# EUROPEAN PATENT SPECIFICATION

(11) **EP 2 140 017 B1**
(45) Date of publication and mention of the grant of the patent: **19.12.2012**
(21) Application number: 08780577.6
(22) Date of filing: 29.04.2008
(51) Int. Cl.: C12P 7/16

(54) **Process for producing isobutanol**
Verfahren zur Herstellung von Isobutanol
Procédé de production d'isobutanol

(30) Priority: 02.05.2007 US 915467 P
(43) Date of publication of application: 06.01.2010
(60) Divisional application: 12173233.3
(73) Proprietor: Butamax (TM) Advanced Biofuels LLC, Wilmington, DE 19803 (US)
(72) Inventor: BRAMUCCI, MICHAEL, G., Boothwyn, PA 19061 (US); FLINT, DENNIS, Newark, DE 19711 (US); MILLER, EDWARD, S., Jr., Knoxville, TN 37934 (US); NAGARAJAN, VASANTHA, Wilmington, DE 19807 (US); SEDKOVA, NATALIA, Cherry Hill, NJ 08003 (US); SINGH, MANJARI, West Chester, PA 19382 (US); VAN DYK, TINA, K., Wilmington, DE 19803 (US)
(74) Representative: Owen, Deborah Jane
(86) International application number: PCT/US2008/061861
(87) International publication number: WO 2008/137406

(56) References cited:
- WO-A-2007/050671
- US-A- 2 198 104
- US-B1- 6 514 733
- JONES D T ET AL: "Acetone-butanol fermentation revisited" MICROBIOLOGICAL REVIEWS, AMERICAN SOCIETY FOR MICROBIOLOGY, WASHINGTON, DC, US, vol. 50, no. 4, 1 December 1986 (1986-12-01), pages 484-524, XP002425366 ISSN: 0146-0749 cited in the application
- JANSEN MICHAEL ET AL: "Growth of the salt-tolerant yeast Zygosaccharomyces rouxii in microtiter plates: Effects of NaCl, pH and temperature on growth and fusel alcohol production from branched-chain amino acids." FEMS YEAST RESEARCH, vol. 3, no. 3, May 2003 (2003-05), pages 313-318, XP002491156 ISSN: 1567-1356
- DICKINSON J RICHARD ET AL: "An investigation of the metabolism of valine to isobutyl alcohol in Saccharomyces cerevisiae" JOURNAL OF BIOLOGICAL CHEMISTRY, AMERICAN SOCIETY OF BIOLOCHEMICAL BIOLOGISTS, BIRMINGHAM,; US, vol. 273, no. 40, 2 October 1998 (1998-10-02), pages 25751-25756, XP002425679 ISSN: 0021-9258
- PENG H-L ET AL: "CLONING SEQUENCING AND HETEROLOGOUS EXPRESSION OF A KLEBSIELLA-PNEUMONIAE GENE ENCODING AN FAD-INDEPENDENT ACETOLACTATE SYNTHASE" GENE, ELSEVIER, AMSTERDAM, NL, vol. 117, no. 1, 1 January 1992 (1992-01-01), pages 125-130, XP002425680 ISSN: 0378-1119
- DE LA PLAZA M ET AL: "Biochemical and molecular characterization of alpha-ketoisovalerate decarboxylase, an enzyme involved in the formation of aldehydes from amino acids by Lactococcus lactis" FEMS MICROBIOLOGY LETTERS, AMSTERDAM, NL, vol. 238, no. 2, 15 September 2004 (2004-09-15), pages 367-374, XP004557199 ISSN: 0378-1097
- BAER S H ET AL: "EFFECT OF BUTANOL CHALLENGE AND TEMPERATURE ON LIPID COMPOSITION AND MEMBRANE FLUIDITY OF BUTANOL-TOLERANT CLOSTRIDIUM-ACETOBUTYLICUM" APPLIED AND ENVIRONMENTAL MICROBIOLOGY,, vol. 53, no. 12, 1 January 1987 (1987-01-01), pages 2854-2861, XP002490712
- ATSUMI SHOTA ET AL: "Non-fermentative pathways for synthesis of branched-chain higher alcohols as biofuels" NATURE (LONDON), vol. 451, no. 7174, January 2008 (2008-01), page 86, XP002491157 ISSN: 0028-0836

### SEQUENCE LISTING

<110> E.I. du Pont de Nemours and Co.
<120> Method for the Pr oduct i on of Isobutanol
<130> CL3892
<140> PCTUS0861861
   <141> 2008-04-29
<160> 267
<170> Patent In version 3. 4
<210> 1
   <211> 1680
   <212> DNA
   <213> K. pneumoniae
<400> 1
   atggacaaac agtatccggt acgccagtgg gcgcacggcg ccgatctcgt cgtcagtcag     60
   ctggaagctc agggagtacg ccaggtgttc ggcatccccg gcgccaaaat cgacaaggtc    120
   tttgattcac tgctggattc ctccattcgc attattccgg tacgccacga agccaacgcc    180
   gcatttatgg ccgccgccgt cggacgcatt accggcaaag cgggcgtggc gctggtcacc    240
   tccggtccgg gctgttccaa cctgatcacc ggcatggcca ccgcgaacag cgaaggcgac    300
   ccggtggtgg ccctgggcgg cgcggtaaaa cgcgccgata aagcgaagca ggtccaccag    360
   agt at ggat a cggt ggcgat gt t cagcccg gt caccaaat acgccat cga ggt gacggcg 420
   ccggatgcgc tggcggaagt ggtctccaac gccttccgcg ccgccgagca gggccggccg    480
   ggcagcgcgt tcgttagcct gccgcaggat gtggtcgatg gcccggtcag cggcaaagtg    540
   ctgccggcca gcggggcccc gcagatgggc gccgcgccgg atgatgccat cgaccaggtg    600
   gcgaagctta tcgcccaggc gaagaacccg atcttcctgc tcggcctgat ggccagccag    660
   ccggaaaaca gcaaggcgct gcgccgtttg ctggagacca gccatattcc agtcaccagc    720
   acctatcagg ccgccggagc ggtgaatcag gataacttct ctcgcttcgc cggccgggtt    780
   gggctgttta acaaccaggc cggggaccgt ctgctgcagc tcgccgacct ggtgatctgc    840
   atcggctaca gcccggtgga atacgaaccg gcgatgtgga acagcggcaa cgcgacgctg    900
   gtgcacatcg acgtgctgcc cgcctatgaa gagcgcaact acaccccgga tgtcgagctg    960
   gt gggcgat a t cgccggcac t ct caacaag ct ggcgcaaa at at cgat ca t cggct ggt g 1020
   ctctccccgc aggcggcgga gatcctccgc gaccgccagc accagcgcga gctgctggac   1080
   cgccgcggcg cgcagctcaa ccagtttgcc ctgcatcccc tgcgcatcgt tcgcgccatg   1140
   caggat at cg t caacagcga cgt cacgt t g accgt ggaca t gggcagct t ccat at ct gg 1200
   attgcccgct acctgtacac gttccgcgcc cgtcaggtga tgatctccaa cggccagcag   1260
   accat gggcg t cgccct gcc ct gggct at c ggcgcct ggc t ggt caat cc t gagcgcaaa 1320
   gtggtctccg tctccggcga cggcggcttc ctgcagtcga gcatggagct ggagaccgcc   1380
   gtccgcctga aagccaacgt gctgcatctt atctgggtcg ataacggcta caacatggtc   1440
   gctatccagg aagagaaaaa atatcagcgc ctgtccggcg tcgagtttgg gccgatggat   1500
   tttaaagcct atgccgaatc cttcggcgcg aaagggtttg ccgtggaaag cgccgaggcg   1560
   ct ggagccga ccct gcgcgc ggcgat ggac gt cgacggcc cggcggt agt ggccat cccg 1620
   gt ggat t at c gcgat aaccc gct gct gat g ggccagct gc at ct gagt ca gat t ct gt aa 1680
<210> 2
   <211> 559
   <212> PRT
   <213> K. pneumoniae
<400> 2
   Met Asp Lys Gln Tyr Pro Val Arg Gln Trp Ala His Gly Ala Asp Leu
   1 5 10 15
   Val Val Ser Gln Leu Glu Ala Gln Gly Val Arg Gln Val Phe Gly Ile
   20 25 30
   Pro Gly Ala Lys Ile Asp Lys Val Phe Asp Ser Leu Leu Asp Ser Ser
   35 40 45
   Ile Arg Ile Ile Pro Val Arg His Glu Ala Asn Ala Ala Phe Met Ala
   50 55 60
   Ala Ala Val Gly Arg Ile Thr Gly Lys Ala Gly Val Ala Leu Val Thr
   65 70 75 80
   Ser Gly Pro Gly Cys Ser Asn Leu Ile Thr Gly Met Ala Thr Ala Asn
   85 90 95
   Ser Glu Gly Asp Pro Val Val Ala Leu Gly Gly Ala Val Lys Arg Ala
   100 105 110
   Asp Lys Ala Lys Gln Val His Gln Ser Met Asp Thr Val Ala Met Phe
   115 120 125
   Ser Pro Val Thr Lys Tyr Ala Ile Glu Val Thr Ala Pro Asp Ala Leu
   130 135 140
   Ala Glu Val Val Ser Asn Ala Phe Arg Ala Ala Glu Gln Gly Arg Pro
   145 150 155 160
   Gly Ser Ala Phe Val Ser Leu Pro Gln Asp Val Val Asp Gly Pro Val
   165 170 175
   Ser Gly Lys Val Leu Pro Ala Ser Gly Ala Pro Gln Met Gly Ala Ala
   180 185 190
   Pro Asp Asp Ala Ile Asp Gln Val Ala Lys Leu Ile Ala Gln Ala Lys
   195 200 205
   Asn Pro Ile Phe Leu Leu Gly Leu Met Ala Ser Gln Pro Glu Asn Ser
   210 215 220
   Lys Ala Leu Arg Arg Leu Leu Glu Thr Ser His Ile Pro Val Thr Ser
   225 230 235 240
   Thr Tyr Gln Ala Ala Gly Ala Val Asn Gln Asp Asn Phe Ser Arg Phe
   245 250 255
   Ala Gly Arg Val Gly Leu Phe Asn Asn Gln Ala Gly Asp Arg Leu Leu
   260 265 270
   Gln Leu Ala Asp Leu Val Ile Cys Ile Gly Tyr Ser Pro Val Glu Tyr
   275 280 285
   Glu Pro Ala Met Trp Asn Ser Gly Asn Ala Thr Leu Val His Ile Asp
   290 295 300
   Val Leu Pro Ala Tyr Glu Glu Arg Asn Tyr Thr Pro Asp Val Glu Leu
   305 310 315 320
   Val Gly Asp Ile Ala Gly Thr Leu Asn Lys Leu Ala Gln Asn Ile Asp
   325 330 335
   His Arg Leu Val Leu Ser Pro Gln Ala Ala Glu Ile Leu Arg Asp Arg
   340 345 350
   Gln His Gln Arg Glu Leu Leu Asp Arg Arg Gly Ala Gln Leu Asn Gln
   355 360 365
   Phe Ala Leu His Pro Leu Arg Ile Val Arg Ala Met Gln Asp Ile Val
   370 375 380
   Asn Ser Asp Val Thr Leu Thr Val Asp Met Gly Ser Phe His Ile Trp
   385 390 395 400
   Ile Ala Arg Tyr Leu Tyr Thr Phe Arg Ala Arg Gln Val Met Ile Ser
   405 410 415
   Asn Gly Gln Gln Thr Met Gly Val Ala Leu Pro Trp Ala Ile Gly Ala
   420 425 430
   Trp Leu Val Asn Pro Glu Arg Lys Val Val Ser Val Ser Gly Asp Gly
   435 440 445
   Gly Phe Leu Gln Ser Ser Met Glu Leu Glu Thr Ala Val Arg Leu Lys
   450 455 460
   Ala Asn Val Leu His Leu Ile Trp Val Asp Asn Gly Tyr Asn Met Val
   465 470 475 480
   Ala Ile Gln Glu Glu Lys Lys Tyr Gln Arg Leu Ser Gly Val Glu Phe
   485 490 495
   Gly Pro Met Asp Phe Lys Ala Tyr Ala Glu Ser Phe Gly Ala Lys Gly
   500 505 510
   Phe Ala Val Glu Ser Ala Glu Ala Leu Glu Pro Thr Leu Arg Ala Ala
   515 520 525
   Met Asp Val Asp Gly Pro Ala Val Val Ala Ile Pro Val Asp Tyr Arg
   530 535 540
   Asp Asn Pro Leu Leu Met Gly Gln Leu His Leu Ser Gln Ile Leu
   545 550 555
<210> 3
   <211> 1476
   <212> DNA
   <213> E. coli
<400> 3
   atggctaact acttcaatac actgaatctg cgccagcagc tggcacagct gggcaaatgt 60
   cgctttatgg gccgcgatga attcgccgat ggcgcgagct accttcaggg taaaaaagta 120
   gtcatcgtcg gctgtggcgc acagggtctg aaccagggcc tgaacatgcg tgattctggt 180
   ctcgatatct cctacgctct gcgtaaagaa gcgattgccg agaagcgcgc gtcctggcgt 240
   aaagcgaccg aaaatggttt taaagtgggt acttacgaag aactgatccc acaggcggat 300
   ctggtgatta acctgacgcc ggacaagcag cactctgatg tagtgcgcac cgtacagcca 360
   ctgatgaaag acggcgcggc gctgggctac tcgcacggtt tcaacatcgt cgaagtgggc 420
   gagcagatcc gtaaagatat caccgtagtg atggttgcgc cgaaatgccc aggcaccgaa 480
   gtgcgtgaag agtacaaacg tgggttcggc gtaccgacgc tgattgccgt tcacccggaa 540
   aacgatccga aaggcgaagg catggcgatt gccaaagcct gggcggctgc aaccggtggt 600
   caccgtgcgg gtgtgctgga atcgtccttc gttgcggaag tgaaatctga cctgatgggc 660
   gagcaaacca tcctgtgcgg tatgttgcag gctggctctc tgctgtgctt cgacaagctg 720
   gtggaagaag gtaccgatcc agcatacgca gaaaaactga ttcagttcgg ttgggaaacc 780
   atcaccgaag cactgaaaca gggcggcatc accctgatga tggaccgtct ctctaacccg 840
   gcgaaactgc gtgcttatgc gctttctgaa cagctgaaag agatcatggc acccctgttc 900
   cagaaacata tggacgacat catctccggc gaattctctt ccggtatgat ggcggactgg 960
   gccaacgatg ataagaaact gctgacctgg cgtgaagaga ccggcaaaac cgcgtttgaa 1020
   accgcgccgc agtatgaagg caaaatcggc gagcaggagt acttcgataa aggcgtactg 1080
   atgattgcga tggtgaaagc gggcgttgaa ctggcgttcg aaaccatggt cgattccggc 1140
   atcattgaag agtctgcata ttatgaatca ctgcacgagc tgccgctgat tgccaacacc 1200
   atcgcccgta agcgtctgta cgaaatgaac gtggttatct ctgataccgc tgagtacggt 1260
   aactatctgt tctcttacgc ttgtgtgccg ttgctgaaac cgtttatggc agagctgcaa 1320
   ccgggcgacc tgggtaaagc tattccggaa ggcgcggtag ataacgggca actgcgtgat 1380
   gtgaacgaag cgattcgcag ccatgcgatt gagcaggtag gtaagaaact gcgcggctat 1440
   atgacagata tgaaacgtat tgctgttgcg ggttaa 1476
<210> 4
   <211> 491
   <212> PRT
   <213> E. coli
<400> 4
   Met Ala Asn Tyr Phe Asn Thr Leu Asn Leu Arg Gln Gln Leu Ala Gln
   1 5 10 15
   Leu Gly Lys Cys Arg Phe Met Gly Arg Asp Glu Phe Ala Asp Gly Ala
   20 25 30
   Ser Tyr Leu Gln Gly Lys Lys Val Val Ile Val Gly Cys Gly Ala Gln
   35 40 45
   Gly Leu Asn Gln Gly Leu Asn Met Arg Asp Ser Gly Leu Asp Ile Ser
   50 55 60
   Tyr Ala Leu Arg Lys Glu Ala Ile Ala Glu Lys Arg Ala Ser Trp Arg
   65 70 75 80
   Lys Ala Thr Glu Asn Gly Phe Lys Val Gly Thr Tyr Glu Glu Leu Ile
   85 90 95
   Pro Gln Ala Asp Leu Val Ile Asn Leu Thr Pro Asp Lys Gln His Ser
   100 105 110
   Asp Val Val Arg Thr Val Gln Pro Leu Met Lys Asp Gly Ala Ala Leu
   115 120 125
   Gly Tyr Ser His Gly Phe Asn Ile Val Glu Val Gly Glu Gln Ile Arg
   130 135 140
   Lys Asp Ile Thr Val Val Met Val Ala Pro Lys Cys Pro Gly Thr Glu
   145 150 155 160
   Val Arg Glu Glu Tyr Lys Arg Gly Phe Gly Val Pro Thr Leu Ile Ala
   165 170 175
   Val His Pro Glu Asn Asp Pro Lys Gly Glu Gly Met Ala Ile Ala Lys
   180 185 190
   Ala Trp Ala Ala Ala Thr Gly Gly His Arg Ala Gly Val Leu Glu Ser
   195 200 205
   Ser Phe Val Ala Glu Val Lys Ser Asp Leu Met Gly Glu Gln Thr Ile
   210 215 220
   Leu Cys Gly Met Leu Gln Ala Gly Ser Leu Leu Cys Phe Asp Lys Leu
   225 230 235 240
   Val Glu Glu Gly Thr Asp Pro Ala Tyr Ala Glu Lys Leu Ile Gln Phe
   245 250 255
   Gly Trp Glu Thr Ile Thr Glu Ala Leu Lys Gln Gly Gly Ile Thr Leu
   260 265 270
   Met Met Asp Arg Leu Ser Asn Pro Ala Lys Leu Arg Ala Tyr Ala Leu
   275 280 285
   Ser Glu Gln Leu Lys Glu Ile Met Ala Pro Leu Phe Gln Lys His Met
   290 295 300
   Asp Asp Ile Ile Ser Gly Glu Phe Ser Ser Gly Met Met Ala Asp Trp
   305 310 315 320
   Ala Asn Asp Asp Lys Lys Leu Leu Thr Trp Arg Glu Glu Thr Gly Lys
   325 330 335
   Thr Ala Phe Glu Thr Ala Pro Gln Tyr Glu Gly Lys Ile Gly Glu Gln
   340 345 350
   Glu Tyr Phe Asp Lys Gly Val Leu Met Ile Ala Met Val Lys Ala Gly
   355 360 365
   Val Glu Leu Ala Phe Glu Thr Met Val Asp Ser Gly Ile Ile Glu Glu
   370 375 380
   Ser Ala Tyr Tyr Glu Ser Leu His Glu Leu Pro Leu Ile Ala Asn Thr
   385 390 395 400
   Ile Ala Arg Lys Arg Leu Tyr Glu Met Asn Val Val Ile Ser Asp Thr
   405 410 415
   Ala Glu Tyr Gly Asn Tyr Leu Phe Ser Tyr Ala Cys Val Pro Leu Leu
   420 425 430
   Lys Pro Phe Met Ala Glu Leu Gln Pro Gly Asp Leu Gly Lys Ala Ile
   435 440 445
   Pro Glu Gly Ala Val Asp Asn Gly Gln Leu Arg Asp Val Asn Glu Ala
   450 455 460
   Ile Arg Ser His Ala Ile Glu Gln Val Gly Lys Lys Leu Arg Gly Tyr
   465 470 475 480
   Met Thr Asp Met Lys Arg Ile Ala Val Ala Gly
   485 490
<210> 5
   <211> 1851
   <212> DNA
   <213> E. coli
<400> 5
   atgcctaagt accgttccgc caccaccact catggtcgta atatggcggg tgctcgtgcg 60
   ctgtggcgcg ccaccggaat gaccgacgcc gatttcggta agccgattat cgcggttgtg 120
   aactcgttca cccaatttgt accgggtcac gtccatctgc gcgatctcgg taaactggtc 180
   gccgaacaaa ttgaagcggc tggcggcgtt gccaaagagt tcaacaccat tgcggtggat 240
   gatgggattg ccatgggcca cggggggatg ctttattcac tgccatctcg cgaactgatc 300
   gctgattccg ttgagtatat ggtcaacgcc cactgcgccg acgccatggt ctgcatctct 360
   aactgcgaca aaatcacccc ggggatgctg atggcttccc tgcgcctgaa tattccggtg 420
   atctttgttt ccggcggccc gatggaggcc gggaaaacca aactttccga tcagatcatc 480
   aagctcgatc tggttgatgc gatgatccag ggcgcagacc cgaaagtatc tgactcccag 540
   agcgatcagg ttgaacgttc cgcgtgtccg acctgcggtt cctgctccgg gatgtttacc 600
   gctaactcaa tgaactgcct gaccgaagcg ctgggcctgt cgcagccggg caacggctcg 660
   ctgctggcaa cccacgccga ccgtaagcag ctgttcctta atgctggtaa acgcattgtt 720
   gaattgacca aacgttatta cgagcaaaac gacgaaagtg cactgccgcg taatatcgcc 780
   agtaaggcgg cgtttgaaaa cgccatgacg ctggatatcg cgatgggtgg atcgactaac 840
   accgtacttc acctgctggc ggcggcgcag gaagcggaaa tcgacttcac catgagtgat 900
   atcgataagc tttcccgcaa ggttccacag ctgtgtaaag ttgcgccgag cacccagaaa 960
   taccatatgg aagatgttca ccgtgctggt ggtgttatcg gtattctcgg cgaactggat 1020
   cgcgcggggt tactgaaccg tgatgtgaaa aacgtacttg gcctgacgtt gccgcaaacg 1080
   ctggaacaat acgacgttat gctgacccag gatgacgcgg taaaaaatat gttccgcgca 1140
   ggtcctgcag gcattcgtac cacacaggca ttctcgcaag attgccgttg ggatacgctg 1200
   gacgacgatc gcgccaatgg ctgtatccgc tcgctggaac acgcctacag caaagacggc 1260
   ggcctggcgg tgctctacgg taactttgcg gaaaacggct gcatcgtgaa aacggcaggc 1320
   gtcgatgaca gcatcctcaa attcaccggc ccggcgaaag tgtacgaaag ccaggacgat 1380
   gcggtagaag cgattctcgg cggtaaagtt gtcgccggag atgtggtagt aattcgctat 1440
   gaaggcccga aaggcggtcc ggggatgcag gaaatgctct acccaaccag cttcctgaaa 1500
   tcaatgggtc tcggcaaagc ctgtgcgctg atcaccgacg gtcgtttctc tggtggcacc 1560
   tctggtcttt ccatcggcca cgtctcaccg gaagcggcaa gcggcggcag cattggcctg 1620
   attgaagatg gtgacctgat cgctatcgac atcccgaacc gtggcattca gttacaggta 1680
   agcgatgccg aactggcggc gcgtcgtgaa gcgcaggacg ctcgaggtga caaagcctgg 1740
   acgccgaaaa atcgtgaacg tcaggtctcc tttgccctgc gtgcttatgc cagcctggca 1800
   accagcgccg acaaaggcgc ggtgcgcgat aaatcgaaac tgggggttaa a 1851
<210> 6
   <211> 616
   <212> PRT
   <213> E. coli
<400> 6
   Met Pro Lys Tyr Arg Ser Ala Thr Thr Thr His Gly Arg Asn Met Ala
   1 5 10 15
   Gly Ala Arg Ala Leu Trp Arg Ala Thr Gly Met Thr Asp Ala Asp Phe
   20 25 30
   Gly Lys Pro Ile Ile Ala Val Val Asn Ser Phe Thr Gln Phe Val Pro
   35 40 45
   Gly His Val His Leu Arg Asp Leu Gly Lys Leu Val Ala Glu Gln Ile
   50 55 60
   Glu Ala Ala Gly Gly Val Ala Lys Glu Phe Asn Thr Ile Ala Val Asp
   65 70 75 80
   Asp Gly Ile Ala Met Gly His Gly Gly Met Leu Tyr Ser Leu Pro Ser
   85 90 95
   Arg Glu Leu Ile Ala Asp Ser Val Glu Tyr Met Val Asn Ala His Cys
   100 105 110
   Ala Asp Ala Met Val Cys Ile Ser Asn Cys Asp Lys Ile Thr Pro Gly
   115 120 125
   Met Leu Met Ala Ser Leu Arg Leu Asn Ile Pro Val Ile Phe Val Ser
   130 135 140
   Gly Gly Pro Met Glu Ala Gly Lys Thr Lys Leu Ser Asp Gln Ile Ile
   145 150 155 160
   Lys Leu Asp Leu Val Asp Ala Met Ile Gln Gly Ala Asp Pro Lys Val
   165 170 175
   Ser Asp Ser Gln Ser Asp Gln Val Glu Arg Ser Ala Cys Pro Thr Cys
   180 185 190
   Gly Ser Cys Ser Gly Met Phe Thr Ala Asn Ser Met Asn Cys Leu Thr
   195 200 205
   Glu Ala Leu Gly Leu Ser Gln Pro Gly Asn Gly Ser Leu Leu Ala Thr
   210 215 220
   His Ala Asp Arg Lys Gln Leu Phe Leu Asn Ala Gly Lys Arg Ile Val
   225 230 235 240
   Glu Leu Thr Lys Arg Tyr Tyr Glu Gln Asn Asp Glu Ser Ala Leu Pro
   245 250 255
   Arg Asn Ile Ala Ser Lys Ala Ala Phe Glu Asn Ala Met Thr Leu Asp
   260 265 270
   Ile Ala Met Gly Gly Ser Thr Asn Thr Val Leu His Leu Leu Ala Ala
   275 280 285
   Ala Gln Glu Ala Glu Ile Asp Phe Thr Met Ser Asp Ile Asp Lys Leu
   290 295 300
   Ser Arg Lys Val Pro Gln Leu Cys Lys Val Ala Pro Ser Thr Gln Lys
   305 310 315 320
   Tyr His Met Glu Asp Val His Arg Ala Gly Gly Val Ile Gly Ile Leu
   325 330 335
   Gly Glu Leu Asp Arg Ala Gly Leu Leu Asn Arg Asp Val Lys Asn Val
   340 345 350
   Leu Gly Leu Thr Leu Pro Gln Thr Leu Glu Gln Tyr Asp Val Met Leu
   355 360 365
   Thr Gln Asp Asp Ala Val Lys Asn Met Phe Arg Ala Gly Pro Ala Gly
   370 375 380
   Ile Arg Thr Thr Gln Ala Phe Ser Gln Asp Cys Arg Trp Asp Thr Leu
   385 390 395 400
   Asp Asp Asp Arg Ala Asn Gly Cys Ile Arg Ser Leu Glu His Ala Tyr
   405 410 415
   Ser Lys Asp Gly Gly Leu Ala Val Leu Tyr Gly Asn Phe Ala Glu Asn
   420 425 430
   Gly Cys Ile Val Lys Thr Ala Gly Val Asp Asp Ser Ile Leu Lys Phe
   435 440 445
   Thr Gly Pro Ala Lys Val Tyr Glu Ser Gln Asp Asp Ala Val Glu Ala
   450 455 460
   Ile Leu Gly Gly Lys Val Val Ala Gly Asp Val Val Val Ile Arg Tyr
   465 470 475 480
   Glu Gly Pro Lys Gly Gly Pro Gly Met Gln Glu Met Leu Tyr Pro Thr
   485 490 495
   Ser Phe Leu Lys Ser Met Gly Leu Gly Lys Ala Cys Ala Leu Ile Thr
   500 505 510
   Asp Gly Arg Phe Ser Gly Gly Thr Ser Gly Leu Ser Ile Gly His Val
   515 520 525
   Ser Pro Glu Ala Ala Ser Gly Gly Ser Ile Gly Leu Ile Glu Asp Gly
   530 535 540
   Asp Leu Ile Ala Ile Asp Ile Pro Asn Arg Gly Ile Gln Leu Gln Val
   545 550 555 560
   Ser Asp Ala Glu Leu Ala Ala Arg Arg Glu Ala Gln Asp Ala Arg Gly
   565 570 575
   Asp Lys Ala Trp Thr Pro Lys Asn Arg Glu Arg Gln Val Ser Phe Ala
   580 585 590
   Leu Arg Ala Tyr Ala Ser Leu Ala Thr Ser Ala Asp Lys Gly Ala Val
   595 600 605
   Arg Asp Lys Ser Lys Leu Gly Gly
   610 615
<210> 7
   <211> 1662
   <212> DNA
   <213> Lactococcus I actis
<400> 7
   tctagacata tgtatactgt gggggattac ctgctggatc gcctgcacga actggggatt     60
   gaagaaattt tcggtgtgcc aggcgattat aacctgcagt tcctggacca gattatctcg    120
   cacaaagata tgaagtgggt cggtaacgcc aacgaactga acgcgagcta tatggcagat    180
   ggttatgccc gtaccaaaaa agctgctgcg tttctgacga cctttggcgt tggcgaactg    240
   agcgccgtca acggactggc aggaagctac gccgagaacc tgccagttgt cgaaattgtt    300
   gggtcgccta cttctaaggt tcagaatgaa ggcaaatttg tgcaccatac tctggctgat     360
   ggggatttta aacattttat gaaaatgcat gaaccggtta ctgcggcccg cacgctgctg    420
   acagcagaga atgctacggt tgagatcgac cgcgtcctgt ctgcgctgct gaaagagcgc    480
   aagccggtat atatcaatct gcctgtcgat gttgccgcag cgaaagccga aaagccgtcg    540
   ctgccactga aaaaagaaaa cagcacctcc aatacatcgg accaggaaat tctgaataaa    600
   atccaggaat cactgaagaa tgcgaagaaa ccgatcgtca tcaccggaca tgagatcatc    660
   tcttttggcc tggaaaaaac ggtcacgcag ttcatttcta agaccaaact gcctatcacc    720
   accctgaact tcggcaaatc tagcgtcgat gaagcgctgc cgagttttct gggtatctat    780
   aatggtaccc tgtccgaacc gaacctgaaa gaattcgtcg aaagcgcgga ctttatcctg    840
   atgctgggcg tgaaactgac ggatagctcc acaggcgcat ttacccacca tctgaacgag    900
   aataaaatga tttccctgaa tatcgacgaa ggcaaaatct ttaacgagcg catccagaac 960
   ttcgattttg aatctctgat tagttcgctg ctggatctgt ccgaaattga gtataaaggt 1020
   aaatatattg ataaaaaaca ggaggatttt gtgccgtcta atgcgctgct gagtcaggat 1080
   cgtctgtggc aagccgtaga aaacctgaca cagtctaatg aaacgattgt tgcggaacag 1140
   ggaacttcat ttttcggcgc ctcatccatt tttctgaaat ccaaaagcca tttcattggc 1200
   caaccgctgt gggggagtat tggttatacc tttccggcgg cgctgggttc acagattgca 1260
   gataaggaat cacgccatct gctgtttatt ggtgacggca gcctgcagct gactgtccag 1320
   gaactggggc tggcgatccg tgaaaaaatc aatccgattt gctttatcat caataacgac 1380
   ggctacaccg tcgaacgcga aattcatgga ccgaatcaaa gttacaatga catcccgatg 1440
   tggaactata gcaaactgcc ggaatccttt ggcgcgacag aggatcgcgt ggtgagtaaa 1500
   attgtgcgta cggaaaacga atttgtgtcg gttatgaaag aagcgcaggc tgacccgaat 1560
   cgcatgtatt ggattgaact gatcctggca aaagaaggcg caccgaaagt tctgaaaaag 1620
   atggggaaac tgtttgcgga gcaaaataaa agctaaggat cc 1662
<210> 8
   <211> 548
   <212> PRT
   <213> Lactococcus I actis
<400> 8
   Met Tyr Thr Val Gly Asp Tyr Leu Leu Asp Arg Leu His Glu Leu Gly
   1 5 10 15
   Ile Glu Glu Ile Phe Gly Val Pro Gly Asp Tyr Asn Leu Gln Phe Leu
   20 25 30
   Asp Gln Ile Ile Ser His Lys Asp Met Lys Trp Val Gly Asn Ala Asn
   35 40 45
   Glu Leu Asn Ala Ser Tyr Met Ala Asp Gly Tyr Ala Arg Thr Lys Lys
   50 55 60
   Ala Ala Ala Phe Leu Thr Thr Phe Gly Val Gly Glu Leu Ser Ala Val
   65 70 75 80
   Asn Gly Leu Ala Gly Ser Tyr Ala Glu Asn Leu Pro Val Val Glu Ile
   85 90 95
   Val Gly Ser Pro Thr Ser Lys Val Gln Asn Glu Gly Lys Phe Val His
   100 105 110
   His Thr Leu Ala Asp Gly Asp Phe Lys His Phe Met Lys Met His Glu
   115 120 125
   Pro Val Thr Ala Ala Arg Thr Leu Leu Thr Ala Glu Asn Ala Thr Val
   130 135 140
   Glu Ile Asp Arg Val Leu Ser Ala Leu Leu Lys Glu Arg Lys Pro Val
   145 150 155 160
   Tyr Ile Asn Leu Pro Val Asp Val Ala Ala Ala Lys Ala Glu Lys Pro
   165 170 175
   Ser Leu Pro Leu Lys Lys Glu Asn Ser Thr Ser Asn Thr Ser Asp Gln
   180 185 190
   Glu Ile Leu Asn Lys Ile Gln Glu Ser Leu Lys Asn Ala Lys Lys Pro
   195 200 205
   Ile Val Ile Thr Gly His Glu Ile Ile Ser Phe Gly Leu Glu Lys Thr
   210 215 220
   Val Thr Gln Phe Ile Ser Lys Thr Lys Leu Pro Ile Thr Thr Leu Asn
   225 230 235 240
   Phe Gly Lys Ser Ser Val Asp Glu Ala Leu Pro Ser Phe Leu Gly Ile
   245 250 255
   Tyr Asn Gly Thr Leu Ser Glu Pro Asn Leu Lys Glu Phe Val Glu Ser
   260 265 270
   Ala Asp Phe Ile Leu Met Leu Gly Val Lys Leu Thr Asp Ser Ser Thr
   275 280 285
   Gly Ala Phe Thr His His Leu Asn Glu Asn Lys Met Ile Ser Leu Asn
   290 295 300
   Ile Asp Glu Gly Lys Ile Phe Asn Glu Arg Ile Gln Asn Phe Asp Phe
   305 310 315 320
   Glu Ser Leu Ile Ser Ser Leu Leu Asp Leu Ser Glu Ile Glu Tyr Lys
   325 330 335
   Gly Lys Tyr Ile Asp Lys Lys Gln Glu Asp Phe Val Pro Ser Asn Ala
   340 345 350
   Leu Leu Ser Gln Asp Arg Leu Trp Gln Ala Val Glu Asn Leu Thr Gln
   355 360 365
   Ser Asn Glu Thr Ile Val Ala Glu Gln Gly Thr Ser Phe Phe Gly Ala
   370 375 380
   Ser Ser Ile Phe Leu Lys Ser Lys Ser His Phe Ile Gly Gln Pro Leu
   385 390 395 400
   Trp Gly Ser Ile Gly Tyr Thr Phe Pro Ala Ala Leu Gly Ser Gln Ile
   405 410 415
   Ala Asp Lys Glu Ser Arg His Leu Leu Phe Ile Gly Asp Gly Ser Leu
   420 425 430
   Gln Leu Thr Val Gln Glu Leu Gly Leu Ala Ile Arg Glu Lys Ile Asn
   435 440 445
   Pro Ile Cys Phe Ile Ile Asn Asn Asp Gly Tyr Thr Val Glu Arg Glu
   450 455 460
   Ile His Gly Pro Asn Gln Ser Tyr Asn Asp Ile Pro Met Trp Asn Tyr
   465 470 475 480
   Ser Lys Leu Pro Glu Ser Phe Gly Ala Thr Glu Asp Arg Val Val Ser
   485 490 495
   Lys Ile Val Arg Thr Glu Asn Glu Phe Val Ser Val Met Lys Glu Ala
   500 505 510
   Gln Ala Asp Pro Asn Arg Met Tyr Trp Ile Glu Leu Ile Leu Ala Lys
   515 520 525
   Glu Gly Ala Pro Lys Val Leu Lys Lys Met Gly Lys Leu Phe Ala Glu
   530 535 540
   Gln Asn Lys Ser
   545
<210> 9
   <211> 1164
   <212> DNA
   <213> E. coli
<400> 9
   atgaacaact ttaatctgca caccccaacc cgcattctgt ttggtaaagg cgcaatcgct 60
   ggtttacgcg aacaaattcc tcacgatgct cgcgtattga ttacctacgg cggcggcagc 120
   gtgaaaaaaa ccggcgttct cgatcaagtt ctggatgccc tgaaaggcat ggacgtgctg 180
   gaatttggcg gtattgagcc aaacccggct tatgaaacgc tgatgaacgc cgtgaaactg 240
   gttcgcgaac agaaagtgac tttcctgctg gcggttggcg gcggttctgt actggacggc 300
   accaaattta tcgccgcagc ggctaactat ccggaaaata tcgatccgtg gcacattctg 360
   caaacgggcg gtaaagagat taaaagcgcc atcccgatgg gctgtgtgct gacgctgcca 420
   gcaaccggtt cagaatccaa cgcaggcgcg gtgatctccc gtaaaaccac aggcgacaag 480
   caggcgttcc attctgccca tgttcagccg gtatttgccg tgctcgatcc ggtttatacc 540
   tacaccctgc cgccgcgtca ggtggctaac ggcgtagtgg acgcctttgt acacaccgtg 600
   gaacagtatg ttaccaaacc ggttgatgcc aaaattcagg accgtttcgc agaaggcatt 660
   ttgctgacgc taatcgaaga tggtccgaaa gccctgaaag agccagaaaa ctacgatgtg 720
   cgcgccaacg tcatgtgggc ggcgactcag gcgctgaacg gtttgattgg cgctggcgta 780
   ccgcaggact gggcaacgca tatgctgggc cacgaactga ctgcgatgca cggtctggat 840
   cacgcgcaaa cactggctat cgtcctgcct gcactgtgga atgaaaaacg cgataccaag 900
   cgcgctaagc tgctgcaata tgctgaacgc gtctggaaca tcactgaagg ttccgatgat 960
   gagcgtattg acgccgcgat tgccgcaacc cgcaatttct ttgagcaatt aggcgtgccg 1020
   acccacctct ccgactacgg tctggacggc agctccatcc cggctttgct gaaaaaactg 1080
   gaagagcacg gcatgaccca actgggcgaa aatcatgaca ttacgttgga tgtcagccgc 1140
   cgtatatacg aagccgcccg ctaa 1164
<210> 10
   <211> 387
   <212> PRT
   <213> E. coli
<400> 10
   Met Asn Asn Phe Asn Leu His Thr Pro Thr Arg Ile Leu Phe Gly Lys
   1 5 10 15
   Gly Ala Ile Ala Gly Leu Arg Glu Gln Ile Pro His Asp Ala Arg Val
   20 25 30
   Leu Ile Thr Tyr Gly Gly Gly Ser Val Lys Lys Thr Gly Val Leu Asp
   35 40 45
   Gln Val Leu Asp Ala Leu Lys Gly Met Asp Val Leu Glu Phe Gly Gly
   50 55 60
   Ile Glu Pro Asn Pro Ala Tyr Glu Thr Leu Met Asn Ala Val Lys Leu
   65 70 75 80
   Val Arg Glu Gln Lys Val Thr Phe Leu Leu Ala Val Gly Gly Gly Ser
   85 90 95
   Val Leu Asp Gly Thr Lys Phe Ile Ala Ala Ala Ala Asn Tyr Pro Glu
   100 105 110
   Asn Ile Asp Pro Trp His Ile Leu Gln Thr Gly Gly Lys Glu Ile Lys
   115 120 125
   Ser Ala Ile Pro Met Gly Cys Val Leu Thr Leu Pro Ala Thr Gly Ser
   130 135 140
   Glu Ser Asn Ala Gly Ala Val Ile Ser Arg Lys Thr Thr Gly Asp Lys
   145 150 155 160
   Gln Ala Phe His Ser Ala His Val Gln Pro Val Phe Ala Val Leu Asp
   165 170 175
   Pro Val Tyr Thr Tyr Thr Leu Pro Pro Arg Gln Val Ala Asn Gly Val
   180 185 190
   Val Asp Ala Phe Val His Thr Val Glu Gln Tyr Val Thr Lys Pro Val
   195 200 205
   Asp Ala Lys Ile Gln Asp Arg Phe Ala Glu Gly Ile Leu Leu Thr Leu
   210 215 220
   Ile Glu Asp Gly Pro Lys Ala Leu Lys Glu Pro Glu Asn Tyr Asp Val
   225 230 235 240
   Arg Ala Asn Val Met Trp Ala Ala Thr Gln Ala Leu Asn Gly Leu Ile
   245 250 255
   Gly Ala Gly Val Pro Gln Asp Trp Ala Thr His Met Leu Gly His Glu
   260 265 270
   Leu Thr Ala Met His Gly Leu Asp His Ala Gln Thr Leu Ala Ile Val
   275 280 285
   Leu Pro Ala Leu Trp Asn Glu Lys Arg Asp Thr Lys Arg Ala Lys Leu
   290 295 300
   Leu Gln Tyr Ala Glu Arg Val Trp Asn Ile Thr Glu Gly Ser Asp Asp
   305 310 315 320
   Glu Arg Ile Asp Ala Ala Ile Ala Ala Thr Arg Asn Phe Phe Glu Gln
   325 330 335
   Leu Gly Val Pro Thr His Leu Ser Asp Tyr Gly Leu Asp Gly Ser Ser
   340 345 350
   Ile Pro Ala Leu Leu Lys Lys Leu Glu Glu His Gly Met Thr Gln Leu
   355 360 365
   Gly Glu Asn His Asp Ile Thr Leu Asp Val Ser Arg Arg Ile Tyr Glu
   370 375 380
   Ala Ala Arg
   385
<210> 11
   <211> 29
   <212> DNA
   <213> Artificial Sequence
<220>
   <223> Primer
<400> 11
   caccat ggac aaacagt at cggtacgcc 29
<210> 12
   <211> 25
   <212> DNA
   <213> Artificial Sequence
<220>
   <223> Primer
<400> 12
   cgaagggcga tagctttacc aat cc 25
<210> 13
   <211> 29
   <212> DNA
   <213> Artificial Sequence
<220>
   <223> Primer
<400> 13
   caccatggct aactacttca atacactga 29
<210> 14
   <211> 28
   <212> DNA
   <213> Artificial Sequence
<220>
   <223> Primer
<400> 14
   ccaggagaag gccttgagtg ttttctcc 28
<210> 15
   <211> 29
   <212> DNA
   <213> Artificial Sequence
<220>
   <223> Primer
<400> 15
   caccatgcct aagtaccgtt ccgccacca 29
<210> 16
   <211> 26
   <212> DNA
   <213> Artificial Sequence
<220>
   <223> Primer
<400> 16
   cgcagcact g ctcttaaat attcggc 26
<210> 17
   <211> 29
   <212> DNA
   <213> Artificial Sequence
<220>
   <223> Primer
<400> 17
   caccatgaac aactttaatc tgcacaccc 29
<210> 18
   <211> 29
   <212> DNA
   <213> Artificial Sequence
<220>
   <223> Primer
<400> 18
   caccat gaac aactttaatc tgcacaccc 29
<210> 19
   <211> 45
   <212> DNA
   <213> Artificial Sequence
<220>
   <223> Primer
<400> 19
   gcat gcct t a agaaaggagg ggggtcacat ggacaaacag tatcc 45
<210> 20
   <211> 39
   <212> DNA
   <213> Artificial Sequence
<220>
   <223> Pr imer
<400> 20
   atgcatttaa ttaattacag aatctgactc agatgcagc 39
<210> 21
   <211> 45
   <212> DNA
   <213> Artificial Sequence
<220>
   <223> Primer
<400> 21
   gtcgacgcta gcaaaggagg gaatcaccat ggctaactac t t caa 45
<210> 22
   <211> 31
   <212> DNA
   <213> Artificial Sequence
<220>
   <223> Primer
<400> 22
   tctagattaa cccgcaacag caatacgttt c 31
<210> 23
   <211> 39
   <212> DNA
   <213> Artificial Sequence
<220>
   <223> Primer
<400> 23
   tctagaaaag gaggaat aaa gt at gcct aa gtaccgttc c 39
<210> 24
   <211> 31
   <212> DNA
   <213> Artificial Sequence
<220>
   <223> Primer
<400> 24
   ggat cct t at t aacccccca gtttcgattt a 31
<210> 25
   <211> 39
   <212> DNA
   <213> Artificial Sequence
<220>
   <223> Primer
<400> 25
   ggat ccaaag gaggct agac at at gt at ac tgtggggga 39
<210> 26
   <211> 31
   <212> DNA
   <213> Artificial Sequence
<220>
   <223> Primer
<400> 26
   gagct ct t ag cttttatttt gctccgcaaa c 31
<210> 27
   <211> 39
   <212> DNA
   <213> Artificial Sequence
<220>
   <223> Primer
<400> 27
   gagctcaaag gaggagcaag t aat gaacaa ctttaatct 39
<210> 28
   <211> 43
   <212> DNA
   <213> Artificial Sequence
<220>
   <223> Primer
<400> 28
   gaat t cact gtcctaggtt agcgggcggc ttcgtatata cgg 43
<210> 29
   <211> 25
   <212> DNA
   <213> Artificial Sequence
<220>
   <223> Primer
<400> 29
   caacatt agc gattttcttt tctct 25
<210> 30
   <211> 45
   <212> DNA
   <213> Artificial Sequence
<220>
   <223> Primer
<400> 30
   catgaagctt actagtgggc ttaagttttg aaaataatga aaact 45
<210> 31
   <211> 61
   <212> DNA
   <213> Artificial Sequence
<220>
   <223> Primer N110.2
<400> 31
   gagct cact a gt caat t gt a agt aagt aaa aggaggt ggg t cacat ggac aaacagt at c 60
   c 61
<210> 32
   <211> 50
   <212> DNA
   <213> Artificial Sequence
<220>
   <223> Primer N111.2
<400> 32
   ggatccgat gacttaagcc tcagcttaca gaatctgact cagatgcagc 50
<210> 33
   <211> 44
   <212> DNA
   <213> Artificial Sequence
<220>
   <223> Primer N112.2
<400> 33
   gagctcctta agaaggaggt aatcaccatg gctaactact t caa 44
<210> 34
   <211> 51
   <212> DNA
   <213> Artificial Sequence
<220>
   <223> Primer N113.2
<400> 34
   ggat ccgat c gagctagcgc ggccgct t aa cccgcaacag caatacgttc 51
<210> 35
   <211> 44
   <212> DNA
   <213> Artificial Sequence
<220>
   <223> Primer N114.2
<400> 35
   gagctcgcta gcaaggaggt ataaagtatg cctaagtacc gt t c 44
<210> 36
   <211> 52
   <212> DNA
   <213> Artificial Sequence
<220>
   <223> Primer N115.2
<400> 36
   ggatccgatc gattaattaa cctaaggtta ttaacccccc agtttcgatt ta 52
<210> 37
   <211> 46
   <212> DNA
   <213> Artificial Sequence
<220>
   <223> Primer N116. 2
<400> 37
   gagct ct t aa t t aaaaggag gt t agacat tgtatactgt ggggga 46
<210> 38
   <211> 49
   <212> DNA
   <213> Artificial Sequence
<220>
   <223> Primer 117.2
<400> 38
   ggatccagat ct cct aggac atgtttagct tttattttgc t ccgcaaac 49
<210> 39
   <211> 3883
   <212> DNA
   <213> Escherichia coli
<400> 39
   ct at at t gct gaaggt acag gcgt t t ccat aact at t t gc t cgcgt t t t t t act caagaa 60
   gaaaat gcca aat agcaaca t caggcagac aat acccgaa at t gcgaaga aaact gt ct g 120
   gtagcctgcg tggtcaaaga gtatcccagt cggcgttgaa agcagcacaa tcccaagcga     180
   act ggcaat t t gaaaaccaa t cagaaagat cgt cgacgac aggcgct t at caaagt t t gc 240
   cacgct gt at t t gaagacgg at at gacaca aagt ggaacc t caat ggcat gt aacaact t 300
   cactaatgaa ataatccagg ggttaacgaa cagcgcgcag gaaaggatac gcaacgccat     360
   aatcacaact ccgataagta atgcattttt tggccctacc cgattcacaa agaaaggaat     420
   aat cgccat g cacagcgct t cgagt accac ct ggaat gag t t gagat aac cat acaggcg 480
   cgttcctaca tcgtgtgatt cgaataaacc tgaataaaag acaggaaaaa gttgttgatc     540
   aaaaatgtta tagaaagacc acgtccccac aataaatatg acgaaaaccc agaagtttcg      600
   at cct t gaaa act gcgat aa aat cct ct t t t t t t acccct cccgcat ct g ccgct acgca 660
   ct ggt gat cc t t at ct t t aa aacgcat gt t gat cat cat a aat acagcgc caaat agcga 720
   gaccaaccag aagttgatat ggggactgat actaaaaaat atgccggcaa agaacgcgcc      780
   aatagcatag ccaaaagatc cccaggcgcg cgctgttcca tattcgaaat gaaaatttcg      840
   cgccattttt tcggtgaagc tatcaagcaa accgcatccc gccagatacc ccaagccaaa      900
   aaat agcgcc cccagaat t a gacct acaga aaaat t gct t t gcagt aacg gt t cat aaac 960
   gt aaat cat a aacggt ccgg t caagaccag gat gaaact c at acaccaga t gagcggt t t 1020
   ct t cagaccg agt t t at cct gaacgat gcc gt agaacat c at aaat agaa t gct ggt aaa 1080
   ct ggt t gacc gaat aaagt g t acct aat t c cgt ccct gt c aaccct agat gt cct t t cag 1140
   ccaaatagcg tataacgacc accacagcga ccaggaaata aaaaagagaa atgagtaact     1200
   ggat gcaaaa cgat agt acg cat t t ct gaa t ggaat at t c agt gccat aa t t acct gcct 1260
   gt cgt t aaaa aat t cacgt c ct at t t agag at aagagcga ct t cgccgt t t act t ct cac 1320
   t at t ccagt t ct t gt cgaca t ggcagcgct gt cat t gccc ct t t cgccgt t act gcaagc 1380
   gct ccgcaac gt t gagcgag at cgat aat t cgt cgcat t t ct ct ct cat c t gt agat aat 1440
   cccgtagagg acagacctgt gagtaacccg gcaacgaacg catctcccgc ccccgtgcta     1500
   tcgacacaat tcacagacat tccagcaaaa tggtgaactt gtcctcgata acagaccacc     1560
   accccttctg cacctttagt caccaacagc atggcgatct catactcttt tgccagggcg     1620
   cat at at cct gat cgt t ct g t gt t t t t cca ct gat aagt c gccat t ct t c t t ccgagagc 1680
   ttgacgacat ccgccagttg tagcgcctgc cgcaaacaca agcggagcaa atgctcgtct     1740
   t gccat agat ct t cacgaat at t aggat cg aagct gacaa aacct ccggc at gccggat c 1800
   gccgtcatcg cagtaaatgc gctggtacgc gaaggctcgg cagacaacgc aattgaacag     1860
   agat gt aacc at t cgccat g t cgccagcag ggcaagt ct g t cgt ct ct aa aaaaagat cg 1920
   gcact ggggc ggaccat aaa cgt aaat gaa cgt t cccct t gat cgt t cag at cgacaagc 1980
   accgt ggat g t ccggt gcca t t cat ct t gc t t cagat acg t gat at cgac t ccct cagt t 2040
   agcagcgttc tttgcattaa cgcaccaaaa ggatcatccc ccacccgacc tataaaccca     2100
   cttgttccgc ctaatctggc gattcccacc gcaacgttag ctggcgcgcc gccaggacaa     2160
   ggcagt aggc gcccgt ct ga t t ct ggcaag agat ct acga ccgcat cccc t aaaacccat 2220
   act t t ggct g acat t t t t t t ccct t aaat t cat ct gagt t acgcat agt g at aaacct ct 2280
   t t t t cgcaaa at cgt cat gg at t t act aaa acat gcat at t cgat cacaa aacgt cat ag 2340
   t t aacgt t aa cat t t gt gat at t cat cgca t t t at gaaag t aagggact t t at t t t t at a 2400
   aaagt t aacg t t aacaat t c accaaat t t g ct t aaccagg at gat t aaaa t gacgcaat c 2460
   tcgattgcat gcggcgcaaa acgccctagc aaaacttcat gagcaccggg gtaacacttt     2520
   ct at ccccat t t t cacct cg cgcct cct gc cgggt ggat g aacgat ccaa acggcct gat 2580
   ct ggt t t aac gat cgt t at c acgcgt t t t a t caacat cat ccgat gagcg aacact gggg 2640
   gccaatgcac tggggacatg ccaccagcga cgatatgatc cactggcagc atgagcctat 2700
   tgcgctagcg ccaggagacg ataatgacaa agacgggtgt ttttcaggta gtgctgtcga 2760
   t gacaat ggt gt cct ct cac t t at ct acac cggacacgt c t ggct cgat g gt gcaggt aa 2820
   t gacgat gca at t cgcgaag t acaat gt ct ggct accagt cgggat ggt a t t cat t t cga 2880
   gaaacagggt gtgatcctca ctccaccaga aggaatcatg cacttccgcg atcctaaagt 2940
   gtggcgtgaa gccgacacat ggtggatggt agtcggggcg aaagatccag gcaacacggg 3000
   gcagat cct g ct t t at cgcg gcagt t cgt t gcgt gaat gg acct t cgat c gcgt act ggc 3060
   ccacgct gat gcgggt gaaa gct at at gt g ggaat gt ccg gact t t t t ca gcct t ggcga 3120
   tcagcattat ctgatgtttt ccccgcaggg aatgaatgcc gagggataca gttaccgaaa 3180
   t cgct t t caa agt ggcgt aa t acccggaat gt ggt cgcca ggacgact t t t t gcacaat c 3240
   cgggcatttt actgaacttg ataacgggca tgacttttat gcaccacaaa gctttttagc 3300
   gaaggat ggt cggcgt at t g t t at cggct g gat ggat at g t gggaat cgc caat gccct c 3360
   aaaacgt gaa ggat gggcag gct gcat gac gct ggcgcgc gagct at cag agagcaat gg 3420
   caaacttcta caacgcccgg tacacgaagc tgagtcgtta cgccagcagc atcaatctgt 3480
   ctctccccgc acaatcagca ataaatatgt tttgcaggaa aacgcgcaag cagttgagat 3540
   t cagt t gcag t gggcgct ga agaacagt ga t gccgaacat t acggat t ac agct cggcac 3600
   t ggaat gcgg ct gt at at t g at aaccaat c t gagcgact t gt t t t gt ggc ggt at t accc 3660
   acacgagaat ttagacggct accgtagtat tcccctcccg cagcgtgaca cgctcgccct 3720
   aaggatattt atcgatacat catccgtgga agtatttatt aacgacgggg aagcggtgat 3780
   gagtagtcga atctatccgc agccagaaga acgggaactg tcgctttatg cctcccacgg 3840
   agt ggct gt g ct gcaacat g gagcact ct g gct act gggt t aa 3883
<210> 40
   <211> 19
   <212> DNA
   <213> Artificial Sequence
<220>
   <223> Primer N130SeqF1
<400> 40
   t gt t ccaacc t gat caccg 19
<210> 41
   <211> 18
   <212> DNA
   <213> Artificial Sequnce
<220>
   <223> Primer N130SeqF2
<400> 41
   ggaaaacagc aaggcgct 18
<210> 42
   <211> 18
   <212> DNA
   <213> Artificial Sequence
<220>
   <223> Primer N130SeqF3
<400> 42
   cagct gaacc agtttgcc 18
<210> 43
   <211> 19
   <212> DNA
   <213> Artificial Sequence
<220>
   <223> Primer N130SeqF4
<400> 43
   aaaataccag cgcctgtcc 19
<210> 44
   <211> 18
   <212> DNA
   <213> Artificial Sequence
<220>
   <223> Primer N130SeqR1
<400> 44
   tgaatggcca ccatgttg 18
<210> 45
   <211> 18
   <212> DNA
   <213> Artificial Sequence
<220>
   <223> Primer N130SeqR2
<400> 45
   gaggatctcc gccgcctg 18
<210> 46
   <211> 18
   <212> DNA
   <213> Artificial Sequence
<220>
   <223> Primer N130SeqR3
<400> 46
   aggccgagca ggaagatc 18
<210> 47
   <211> 19
   <212> DNA
   <213> Artificial Sequence
<220>
   <223> Primer N130SeqR4
<400> 47
   tgatcaggtt ggaacagcc 19

<210> 48
   <211> 19
   <212> DNA
   <213> Artificial Sequence
<220>
   <223> Primer N131SeqF1
<400> 48
   aagaact gat cccacaggc 19
<210> 49
   <211> 19
   <212> DNA
   <213> Artificial Sequence
<220>
   <223> Primer N131SeqF2
<400> 49
   atcctgtgcg gt at gt t gc 19
<210> 50
   <211> 18
   <212> DNA
   <213> Artificial Sequence
<220>
   <223> Primer N131Seqf3
<400> 50
   attgcgatgg t gaaagcg 18
<210> 51
   <211> 19
   <212> DNA
   <213> Artificial Sequence
<220>
   <223> Primer N131 SeqR1
<400> 51
   atggtgttgg caatcagcg 19
<210> 52
   <211> 18
   <212> DNA
   <213> Artificial Sequence
<220>
   <223> Primer N131SeqR2
<400> 52
   gt gct t cggt gatggttt 18
<210> 53
   <211> 19
   <212> DNA
   <213> Artificial Sequence
<220>
   <223> Primer N131SeqR3
<400> 53
   ttgaaaccgt gcgagtagc 19
<210> 54
   <211> 19
   <212> DNA
   <213> Artificial Sequence
<220>
   <223> Primer N132SeqF1
<400> 54
   t at t cact gc catctcgcg 19
<210> 55
   <211> 18
   <212> DNA
   <213> Artificial Sequence
<220>
   <223> Primer N132SeqF2
<400> 55
   ccgtaagcag ctgttcct 18
<210> 56
   <211> 20
   <212> DNA
   <213> Artificial Sequence
<220>
   <223> Primer N132SeqF3
<400> 56
   gctggaacaa tacgacgtta a 20
<210> 57
   <211> 20
   <212> DNA
   <213> Artificial Sequence
<220>
   <223> Primer N132SeqF4
<400> 57
   tgctctaccc aaccagcttc 20
<210> 58
   <211> 20
   <212> DNA
   <213> Artificial Sequence
<220>
   <223> Primer N132SeqR1
<400> 58
   atggaaagac cagaggtgcc 20
<210> 59
   <211> 18
   <212> DNA
   <213> Artificial Sequence
<220>
   <223> Primer N132SeqR2
<400> 59
   tgcctgtgt gtacgaat 18
<210> 60
   <211> 19
   <212> DNA
   <213> Artificial Sequence
<220>
   <223> Primer N132SeqR3
<400> 60
   tattacgcgg cagtgcact 19
<210> 61
   <211> 22
   <212> DNA
   <213> Artificial Sequence
<220>
   <223> Primer N132SeqR4
<400> 61
   ggtgattttg tcgcagttag ag 22
<210> 62
   <211> 18
   <212> DNA
   <213> Artificial Sequence
<220>
   <223> Primer N133SeqF1
<400> 62
   t cgaaat t gt t gggt cgc 18
<210> 63
   <211> 22
   <212> DNA
   <213> Artificial Sequence
<220>
   <223> Primer N133SeqF2
<400> 63
   ggtcacgcag ttcatttcta ag 22
<210> 64
   <211> 18
   <212> DNA
   <213> Artificial Sequence
<220>
   <223> Primer N133SeqF3
<400> 64
   tgtggcaagc cgtagaaa 18
<210> 65
   <211> 19
   <212> DNA
   <213> Artificial Sequence
<220>
   <223> Primer N133SeqF4
<400> 65
   aggatcgcgt ggtgagtaa 19
<210> 66
   <211> 20
   <212> DNA
   <213> Artificial Sequence
<220>
   <223> Primer N133SeqR1
<400> 66
   gtagccgtcg t t at t gat ga 20
<210> 67
   <211> 22
   <212> DNA
   <213> Artificial Sequence
<220>
   <223> Primer N133SeqR2
<400> 67
   gcagcgaact aatcagagat tc 22
<210> 68
   <211> 19
   <212> DNA
   <213> Artificial Sequence
<220>
   <223> Primer N133SeqR3
<400> 68
   tggtccgatg tattggagg 19
<210> 69
   <211> 19
   <212> DNA
   <213> Art i f i ci al Sequence
<220>
   <223> Primer N133SeqR4
<400> 69
   tctgccatat agctcgcgt 19
<210> 70
   <211> 42
   <212> DNA
   <213> Artificial Sequence
<220>
   <223> Promoter 1.6Gl Variant
<400> 70
   gcccttgaca atgccacatc ctgagcaaat aattcaacca ct 42
<210> 71
   <211> 42
   <212> DNA
   <213> Artificial Sequence
<220>
   <223> Promoter 1.5Gl
<400> 71
   gcccttgact atgccacatc ctgagcaaat aattcaacca ct 42
<210> 72
   <211> 18
   <212> DNA
   <213> Artificial Sequence
<220>
   <223> Primer Scr1
<400> 72
   cctttctttg tgaatcgg 18
<210> 73
   <211> 18
   <212> DNA
   <213> Artificial Sequence
<220>
   <223> Primer Scr2
<400> 73
   agaaacaggg t gt gat cc 18
<210> 74
   <211> 20
   <212> DNA
   <213> Artificial Sequence
<220>
   <223> Primer Scr3
<400> 74
   agtgatcatc acctgttgcc 20
<210> 75
   <211> 20
   <212> DNA
   <213> Artificial Sequence
<220>
   <223> Primer Scr4
<400> 75
   agcacggcga gagtcgacgg 20
<210> 76
   <211> 672
   <212> DNA
   <213> Saccharomyces cer evi si ae
<400> 76
   agt t cgagt t t at cat t at c aat act gcca t t t caaagaa t acgt aaat a at t aat agt a 60
   gt gat t t t cc t aact t t at t t agt caaaaa at t agcct t t t aat t ct gct gt aacccgt a 120
   cat gcccaaa at agggggcg ggt t acacag aat at at aac at cgt aggt g t ct gggt gaa 180
   cagt t t at t c ct ggcat cca ct aaat at aa t ggagcccgc t t t t t aagct ggcat ccaga 240
   aaaaaaaaga atcccagcac caaaatattg ttttcttcac caaccatcag ttcataggtc     300
   cattctctta gcgcaactac agagaacagg ggcacaaaca ggcaaaaaac gggcacaacc     360
   tcaatggagt gatgcaacct gcctggagta aatgatgaca caaggcaatt gacccacgca     420
   t gt at ct at c t cat t t t ct t acacct t ct a t t acct t ct g ct ct ct ct ga t t t ggaaaaa 480
   gct gaaaaaa aaggt t gaaa ccagt t ccct gaaat t at t c ccct act t ga ct aat aagt a 540
   t at aaagacg gt aggt at t g at t gt aat t c t gt aaat ct a t t t ct t aaac t t ct t aaat t 600
   ct act t t t at agt t agt ct t t t t t t t agt t t t aaaacacc aagaact t ag t t t cgaat aa 660
   acacacat aa ac 672
<210> 77
   <211> 270
   <212> DNA
   <213> Saccharomyces cerevisiae
<400> 77
   gacctcgagt catgtaatta gttatgtcac gcttacattc acgccctccc cccacatccg 60
   ctctaaccga aaaggaagga gttagacaac ctgaagtcta ggtccctatt tattttttta 120
   tagttatgtt agtattaaga acgttattta tatttcaaat ttttcttttt tttctgtaca 180
   gacgcgtgta cgcatgtaac attatactga aaaccttgct tgagaaggtt ttgggacgct 240
   cgaaggcttt aatttgcggc cggtacccaa 270
<210> 78
   <211> 1716
   <212> DNA
   <213> Bacillus subtilis
<400> 78
   atgttgacaa aagcaacaaa agaacaaaaa tcccttgtga aaaacagagg ggcggagctt 60
   gttgttgatt gcttagtgga gcaaggtgtc acacatgtat ttggcattcc aggtgcaaaa 120
   attgatgcgg tatttgacgc tttacaagat aaaggacctg aaattatcgt tgcccggcac 180
   gaacaaaacg cagcattcat ggcccaagca gtcggccgtt taactggaaa accgggagtc 240
   gtgttagtca catcaggacc gggtgcctct aacttggcaa caggcctgct gacagcgaac 300
   actgaaggag accctgtcgt tgcgcttgct ggaaacgtga tccgtgcaga tcgtttaaaa 360
   cggacacatc aatctttgga taatgcggcg ctattccagc cgattacaaa atacagtgta 420
   gaagttcaag atgtaaaaaa tataccggaa gctgttacaa atgcatttag gatagcgtca 480
   gcagggcagg ctggggccgc ttttgtgagc tttccgcaag atgttgtgaa tgaagtcaca 540
   aatacgaaaa acgtgcgtgc tgttgcagcg ccaaaactcg gtcctgcagc agatgatgca 600
   atcagtgcgg ccatagcaaa aatccaaaca gcaaaacttc ctgtcgtttt ggtcggcatg 660
   aaaggcggaa gaccggaagc aattaaagcg gttcgcaagc ttttgaaaaa ggttcagctt 720
   ccatttgttg aaacatatca agctgccggt accctttcta gagatttaga ggatcaatat 780
   tttggccgta tcggtttgtt ccgcaaccag cctggcgatt tactgctaga gcaggcagat 840
   gttgttctga cgatcggcta tgacccgatt gaatatgatc cgaaattctg gaatatcaat 900
   ggagaccgga caattatcca tttagacgag attatcgctg acattgatca tgcttaccag 960
   cctgatcttg aattgatcgg tgacattccg tccacgatca atcatatcga acacgatgct 1020
   gtgaaagtgg aatttgcaga gcgtgagcag aaaatccttt ctgatttaaa acaatatatg 1080
   catgaaggtg agcaggtgcc tgcagattgg aaatcagaca gagcgcaccc tcttgaaatc 1140
   gttaaagagt tgcgtaatgc agtcgatgat catgttacag taacttgcga tatcggttcg 1200
   cacgccattt ggatgtcacg ttatttccgc agctacgagc cgttaacatt aatgatcagt 1260
   aacggtatgc aaacactcgg cgttgcgctt ccttgggcaa tcggcgcttc attggtgaaa 1320
   ccgggagaaa aagtggtttc tgtctctggt gacggcggtt tcttattctc agcaatggaa 1380
   ttagagacag cagttcgact aaaagcacca attgtacaca ttgtatggaa cgacagcaca 1440
   tatgacatgg ttgcattcca gcaattgaaa aaatataacc gtacatctgc ggtcgatttc 1500
   ggaaatatcg atatcgtgaa atatgcggaa agcttcggag caactggctt gcgcgtagaa 1560
   tcaccagacc agctggcaga tgttctgcgt caaggcatga acgctgaagg tcctgtcatc 1620
   atcgatgtcc cggttgacta cagtgataac attaatttag caagtgacaa gcttccgaaa 1680
   gaattcgggg aactcatgaa aacgaaagct ctctag 1716
<210> 79
   <211> 643
   <212> DNA
   <213> Saccharomyces cerevisiae
<400> 79
   gaaatgaata acaatactga cagtactaaa taattgccta cttggcttca catacgttgc 60
   atacgtcgat atagataata atgataatga cagcaggatt atcgtaatac gtaatagttg 120
   aaaatctcaa aaatgtgtgg gtcattacgt aaataatgat aggaatggga ttcttctatt 180
   tttccttttt ccattctagc agccgtcggg aaaacgtggc atcctctctt tcgggctcaa 240
   ttggagtcac gctgccgtga gcatcctctc tttccatatc taacaactga gcacgtaacc 300
   aatggaaaag catgagctta gcgttgctcc aaaaaagtat tggatggtta ataccatttg 360
   tctgttctct tctgactttg actcctcaaa aaaaaaaaat ctacaatcaa cagatcgctt 420
   caattacgcc ctcacaaaaa cttttttcct tcttcttcgc ccacgttaaa ttttatccct 480
   catgttgtct aacggatttc tgcacttgat ttattataaa aagacaaaga cataatactt 540
   ctctatcaat ttcagttatt gttcttcctt gcgttattct tctgttcttc tttttctttt 600
   gtcatatata accataacca agtaatacat attcaaatct aga 643
<210> 80
   <211> 1188
   <212> DNA
   <213> Saccharomyces cerevisiae
<400> 80
   atgttgagaa ctcaagccgc cagattgatc tgcaactccc gtgtcatcac tgctaagaga 60
   acctttgctt tggccacccg tgctgctgct tacagcagac cagctgcccg tttcgttaag 120
   ccaatgatca ctacccgtgg tttgaagcaa atcaacttcg gtggtactgt tgaaaccgtc 180
   tacgaaagag ctgactggcc aagagaaaag ttgttggact acttcaagaa cgacactttt 240
   gctttgatcg gttacggttc ccaaggttac ggtcaaggtt tgaacttgag agacaacggt 300
   ttgaacgtta tcattggtgt ccgtaaagat ggtgcttctt ggaaggctgc catcgaagac 360
   ggttgggttc caggcaagaa cttgttcact gttgaagatg ctatcaagag aggtagttac 420
   gttatgaact tgttgtccga tgccgctcaa tcagaaacct ggcctgctat caagccattg 480
   ttgaccaagg gtaagacttt gtacttctcc cacggtttct ccccagtctt caaggacttg 540
   actcacgttg aaccaccaaa ggacttagat gttatcttgg ttgctccaaa gggttccggt 600
   agaactgtca gatctttgtt caaggaaggt cgtggtatta actcttctta cgccgtctgg 660
   aacgatgtca ccggtaaggc tcacgaaaag gcccaagctt tggccgttgc cattggttcc 720
   ggttacgttt accaaaccac tttcgaaaga gaagtcaact ctgacttgta cggtgaaaga 780
   ggttgtttaa tgggtggtat ccacggtatg ttcttggctc aatacgacgt cttgagagaa 840
   aacggtcact ccccatctga agctttcaac gaaaccgtcg aagaagctac ccaatctcta 900
   tacccattga tcggtaagta cggtatggat tacatgtacg atgcttgttc caccaccgcc 960
   agaagaggtg ctttggactg gtacccaatc ttcaagaatg ctttgaagcc tgttttccaa 1020
   gacttgtacg aatctaccaa gaacggtacc gaaaccaaga gatctttgga attcaactct 1080
   caacctgact acagagaaaa gctagaaaag gaattagaca ccatcagaaa catggaaatc 1140
   tggaaggttg gtaaggaagt cagaaagttg agaccagaaa accaataa 1188
<210> 81
   <211> 760
   <212> DNA
   <213> Saccharomyces cerevisiae
<400> 81
   tcttttccga tttttttcta aaccgtggaa tatttcggat atccttttgt tgtttccggg 60
   tgtacaatat ggacttcctc ttttctggca accaaaccca tacatcggga ttcctataat 120
   accttcgttg gtctccctaa catgtaggtg gcggagggga gatatacaat agaacagata 180
   ccagacaaga cataatgggc taaacaagac tacaccaatt acactgcctc attgatggtg 240
   gtacataacg aactaatact gtagccctag acttgatagc catcatcata tcgaagtttc 300
   actacccttt ttccatttgc catctattga agtaataata ggcgcatgca acttcttttc 360
   tttttttttc ttttctctct cccccgttgt tgtctcacca tatccgcaat gacaaaaaaa 420
   tgatggaaga cactaaagga aaaaattaac gacaaagaca gcaccaacag atgtcgttgt 480
   tccagagctg atgaggggta tctcgaagca cacgaaactt tttccttcct tcattcacgc 540
   acact act ct ct aat gagca acggt at acg gcct t cct t c cagt t act t g aat t t gaaat 600
   aaaaaaaagt ttgctgtctt gctatcaagt ataaatagac ctgcaattat taatcttttg 660
   t t t cct cgt c at t gt t ct cg t t ccct t t ct t cct t gt t t c t t t t t ct gca caat at t t ca 720
   agct at acca agcat acaat caact at ct c at at acaat g 760
<210> 82
   <211> 316
   <212> DNA
   <213> Saccharomyces cer evi si ae
<400> 82
   gagt aagcga at t t ct t at g at t t at gat t t t t at t at t a aat aagt t at aaaaaaaat a 60
   agt gt at aca aat t t t aaag t gact ct t ag gt t t t aaaac gaaaat t ct t at t ct t gagt 120
   aact ct t t cc t gt aggt cag gt t gct t t ct caggt at agc at gaggt cgc t ct t at t gac 180
   cacacct ct a ccggcat gcc gagcaaat gc ct gcaaat cg ct ccccat t t cacccaat t g 240
   t agat at gct aact ccagca at gagt t gat gaat ct cggt gt gt at t t t a t gt cct caga 300
   ggacaacacc t gt ggt 316
<210> 83
   <211> 1758
   <212> DNA
   <213> Saccharomyces cerevisiae
<400> 83
   atgggcttgt taacgaaagt tgctacatct agacaattct ctacaacgag atgcgttgca 60
   aagaagctca acaagtactc gtatatcatc actgaaccta agggccaagg tgcgtcccag 120
   gccatgcttt atgccaccgg tttcaagaag gaagatttca agaagcctca agtcggggtt 180
   ggttcctgtt ggtggtccgg taacccatgt aacatgcatc tattggactt gaataacaga 240
   tgttctcaat ccattgaaaa agcgggtttg aaagctatgc agttcaacac catcggtgtt 300
   tcagacggta tctctatggg tactaaaggt atgagatact cgttacaaag tagagaaatc 360
   attgcagact cctttgaaac catcatgatg gcacaacact acgatgctaa catcgccatc 420
   ccatcatgtg acaaaaacat gcccggtgtc atgatggcca tgggtagaca taacagacct 480
   tccatcatgg tatatggtgg tactatcttg cccggtcatc caacatgtgg ttcttcgaag 540
   atctctaaaa acatcgatat cgtctctgcg ttccaatcct acggtgaata tatttccaag 600
   caattcactg aagaagaaag agaagatgtt gtggaacatg catgcccagg tcctggttct 660
   tgtggtggta tgtatactgc caacacaatg gcttctgccg ctgaagtgct aggtttgacc 720
   attccaaact cctcttcctt cccagccgtt tccaaggaga agttagctga gtgtgacaac 780
   attggtgaat acatcaagaa gacaatggaa ttgggtattt tacctcgtga tatcctcaca 840
   aaagaggctt ttgaaaacgc cattacttat gtcgttgcaa ccggtgggtc cactaatgct 900
   gttttgcatt tggtggctgt tgctcactct gcgggtgtca agttgtcacc agatgatttc 960
   caaagaatca gtgatactac accattgatc ggtgacttca aaccttctgg taaatacgtc 1020
   atggccgatt tgattaacgt tggtggtacc caatctgtga ttaagtatct atatgaaaac 1080
   aacatgttgc acggtaacac aatgactgtt accggtgaca ctttggcaga acgtgcaaag 1140
   aaagcaccaa gcctacctga aggacaagag attattaagc cactctccca cccaatcaag 1200
   gccaacggtc acttgcaaat tctgtacggt tcattggcac caggtggagc tgtgggtaaa 1260
   attaccggta aggaaggtac ttacttcaag ggtagagcac gtgtgttcga agaggaaggt 1320
   gcctttattg aagccttgga aagaggtgaa atcaagaagg gtgaaaaaac cgttgttgtt 1380
   atcagatatg aaggtccaag aggtgcacca ggtatgcctg aaatgctaaa gccttcctct 1440
   gctctgatgg gttacggttt gggtaaagat gttgcattgt tgactgatgg tagattctct 1500
   ggtggttctc acgggttctt aatcggccac attgttcccg aagccgctga aggtggtcct 1560
   atcgggttgg tcagagacgg cgatgagatt atcattgatg ctgataataa caagattgac 1620
   ctattagtct ctgataagga aatggctcaa cgtaaacaaa gttgggttgc acctccacct 1680
   cgttacacaa gaggtactct atccaagtat gctaagttgg tttccaacgc ttccaacggt 1740
   tgtgttttag atgcttga 1758
<210> 84
   <211> 753
   <212> DNA
   <213> Saccharomyces cerevisiae
<400> 84
   gcatgcttgc atttagtcgt gcaatgtatg actttaagat ttgtgagcag gaagaaaagg 60
   gagaatcttc taacgataaa cccttgaaaa actgggtaga ctacgctatg ttgagttgct 120
   acgcaggctg cacaattaca cgagaatgct cccgcctagg atttaaggct aagggacgtg 180
   caatgcagac gacagatcta aatgaccgtg tcggtgaagt gttcgccaaa cttttcggtt 240
   aacacatgca gtgatgcacg cgcgatggtg ctaagttaca tatatatata tatagccata 300
   gtgatgtcta agtaaccttt atggtatatt tcttaatgtg gaaagatact agcgcgcgca 360
   cccacacaca agcttcgtct tttcttgaag aaaagaggaa gctcgctaaa tgggattcca 420
   ctttccgttc cctgccagct gatggaaaaa ggttagtgga acgatgaaga ataaaaagag 480
   agatccactg aggtgaaatt tcagctgaca gcgagtttca tgatcgtgat gaacaatggt 540
   aacgagttgt ggctgttgcc agggagggtg gttctcaact tttaatgtat ggccaaatcg 600
   ctacttgggt ttgttatata acaaagaaga aataatgaac tgattctctt cctccttctt 660
   gtcctttctt aattctgttg taattacctt cctttgtaat tttttttgta attattcttc 720
   ttaataatcc aaacaaacac acatattaca ata 753
<210> 85
   <211> 20
   <212> DNA
   <213> Artificial Sequence
<220>
   <223> Primer N98SeqF1
<400> 85
   cgtgttagtc acatcaggac 20
<210> 86
   <211> 24
   <212> DNA
   <213> Artificial Sequence
<220>
   <223> Primer N98SeqF2
<400> 86
   ggccatagca aaaat ccaaa cagc 24
<210> 87
   <211> 24
   <212> DNA
   <213> Artificial Sequence
<220>
   <223> Primer N98SeqF3
<400> 87
   ccacgat caatcatatcgaa cacg 24
<210> 88
   <211> 20
   <212> DNA
   <213> Art i f i ci al Sequence
<220>
   <223> Primer N98SeqF4
<400> 88
   ggtttctgtc tctggtgacg 20
<210> 89
   <211> 22
   <212> DNA
   <213> Artificial Sequence
<220>
   <223> Primer N99SeqR1
<400> 89
   gtctggtgat tctacgcgca ag 22
<210> 90
   <211> 22
   <212> DNA
   <213> Artificial Sequence
<220>
   <223> Primer N99SeqR2
<400> 90
   catcgactgc at t acgcaac t c 22
<210> 91
   <211> 22
   <212> DNA
   <213> Artificial Sequence
<220>
   <223> Primer N99SeqR3
<400> 91
   cgatcgtcag aacaacatct gc 22
<210> 92
   <211> 20
   <212> DNA
   <213> Artificial Sequence
<220>
   <223> Primer N99SeqR4
<400> 92
   ccttcagtgt tcgctgtcag 20
<210> 93
   <211> 36
   <212> DNA
   <213> Artificial Sequence
<220>
   <223> Primer N136
<400> 93
   ccgcggatag atctgaaatg g aat aacaat ctgaca 36
<210> 94
   <211> 65
   <212> DNA
   <213> Artificial Sequence
<220>
   <223> Primer N137
<400> 94
   t accaccgaa gt t gat t t gc t t caacat cc t cagct ct ag at t t gaat at gt at t act t g 60
   gt t at 65
<210> 95
   <211> 28
   <212> DNA
   <213> Artificial Sequence
<220>
   <223> Primer N138
<400> 95
   atgttgaagc aaatcaact cggtggta a 28
<210> 96
   <211> 22
   <212> DNA
   <213> Artificial Sequence
<220>
   <223> Primer U139
<400> 96
   ttattggttt tctggtctca ac 22
<210> 97
   <211> 57
   <212> DNA
   <213> Artificial Sequence
<220>
   <223> Primer N140
<400> 97
   aagt tgagac cagaaaacca at aatt aatt aat cat gt aa t agttatgt cacgct t 57
<210> 98
   <211> 30
   <212> DNA
   <213> Artificial Sequence
<220>
   <223> Primer N141
<400> 98
   gcggccgccc gcaaattaaa gccttcgagc 30
<210> 99
   <211> 28
   <212> DNA
   <213> Artificial Sequence
<220>
   <223> Primer N142
<400> 99
   ggatccgcat gcttgcattt agtcgtgc 28
<210> 100
   <211> 56
   <212> DNA
   <213> Artificial Sequence
<220>
   <223> N143
<400> 100
   caggtaatcc cccacagt at acatcctcag ctattgtaat atgtgtgttt gtttgg 56
<210> 101
   <211> 22
   <212> DNA
   <213> Artificial Sequence
<220>
   <223> Primer N144
<400> 101
   atgtatactg tgggggatta cc 22
<210> 102
   <211> 22
   <212> DNA
   <213> Artificial Sequence
<220>
   <223> Primer N145
<400> 102
   ttagctttta ttttgctccg ca 22
<210> 103
   <211> 57
   <212> DNA
   <213> Artificial Sequence
<220>
   <223> Primer N146
<400> 103
   tttgcggagc aaaat aaaag ctaattaatt aagagtaagc gaatttctta tgattta 57
<210> 104
   <211> 28
   <212> DNA
   <213> Artificial Sequence
<220>
   <223> Primer N147
<400> 104
   act agt acca caggt gt t gt cct ct gag 28
<210> 105
   <211> 22
   <212> DNA
   <213> Artificial Sequence
<220>
   <223> Primer N151
<400> 105
   ctagagagct ttcgttttcatg 22
<210> 106
   <211> 57
   <212> DNA
   <213> Artificial Sequence
<220>
   <223> Primer N152
<400> 106
   ctcatgaaaa cgaaagct ct ctagttaatt aatcatgtaa ttagttatgt cacgctt 57
<210> 107
   <211> 25
   <212> DNA
   <213> Artificial Sequence
<220>
   <223> Primer N155
<400> 107
   atggcaaaga agct caacaa gt act 25
<210> 108
   <211> 22
   <212> DNA
   <213> Artificial Sequence
<220>
   <223> Primer N156
<400> 108
   tcaagcatct aaaacacaac cg 22
<210> 109
   <211> 57
   <212> DNA
   <213> Artificial Sequence
<220>
   <223> Primer N157
<400> 109
   aacggtt gtgttttagat gc t tgattaat aagagt aagc gaattt ctt a t gatt t a 57
<210> 110
   <211> 28
   <212> DNA
   <213> Artificial Sequence
<220>
   <223> Primer N158
<400> 110
   ggatcctttt ctggcaacca aacccat a 28
<210> 111
   <211> 56
   <212> DNA
   <213> Artificial Sequence
<220>
   <223> Primer N159
<400> 111
   cgagtacttg ttgagcttct ttgccatcct cagcgagat gttgattgta tgcttg 56
<210> 112
   <211> 19
   <212> DNA
   <213> Artificial Sequence
<220>
   <223> Primer N160SeqF1
<400> 112
   gaaaacgt gg catcctctc 19
<210> 113
   <211> 19
   <212> DNA
   <213> Artificial Sequence
<220>
   <223> Primer N160SeqF2
<400> 113
   gctgactggc caagagaaa 19
<210> 114
   <211> 20
   <212> DNA
   <213> Artificial Sequence
<220>
   <223> Primer N160SeqF3
<400> 114
   tgtacttctc ccacggtttc 20
<210> 115
   <211> 22
   <212> DNA
   <213> Artificial Sequence
<220>
   <223> Primer N160SeqF4
<400> 115
   agct acccaa tctctatacc ca 22
<210> 116
   <211> 22
   <212> DNA
   <213> Artificial Sequence
<220>
   <223> Primer N160SeqF5
<400> 116
   cctgaagtct aggtccctat t t 22
<210> 117
   <211> 19
   <212> DNA
   <213> Artificial Sequence
<220>
   <223> N160SeqR1
<400> 117
   gcgtgaatgt aagcgtgac 19
<210> 118
   <211> 20
   <212> DNA
   <213> Artificial Sequence
<220>
   <223> Primer N160SeqR2
<400> 118
   cgtcgtattg agccaagaac 20
<210> 119
   <211> 20
   <212> DNA
   <213> Artificial Sequence
<220>
   <223> Primer N160SeqR3
<400> 119
   gcat cggaca acaagt t cat 20
<210> 120
   <211> 22
   <212> DNA
   <213> Artificial Sequence
<220>
   <223> Primer N160SeqR4
<400> 120
   t cgt t ct t ga agt agt ccaa ca 22
<210> 121
   <211> 19
   <212> DNA
   <213> Artificial Sequence
<220>
   <223> Primer N160SeqR5
<400> 121
   tgagcccgaa agagaggat 19
<210> 122
   <211> 19
   <212> DNA
   <213> Artificial Sequence
<220>
   <223> Primer N161SeqF1
<400> 122
   acggtatacg gccttcct t 19
<210> 123
   <211> 20
   <212> DNA
   <213> Artificial Sequence
<220>
   <223> Primer N161SeqF2
<400> 123
   gggtttgaaa gctatgcagt 20
<210> 124
   <211> 22
   <212> DNA
   <213> Artificial Sequence
<220>
   <223> Primer N161SeqF3
<400> 124
   ggtggtatgt atactgccaa ca 22
<210> 125
   <211> 22
   <212> DNA
   <213> Artificial Sequence
<220>
   <223> Pr i rrer N161SeqF4
<400> 125
   ggt ggt accc aatctgtgat t a 22
<210> 126
   <211> 20
   <212> DNA
   <213> Artificial Sequence
<220>
   <223> Primer N161SeqF5
<400> 126
   cggtttgggt aaagatgttg 20
<210> 127
   <211> 22
   <212> DNA
   <213> Artificial Sequence
<220>
   <223> Primer N161SeqF6
<400> 127
   aaacgaaaat tcttattctt ga 22
<210> 128
   <211> 22
   <212> DNA
   <213> Artificial Sequence
<220>
   <223> Primer N161SeqR1
<400> 128
   tcgttttaaa acctaagagt ca 22
<210> 129
   <211> 19
   <212> DNA
   <213> Artificial Sequence
<220>
   <223> Primer N161 SeqR2
<400> 129
   ccaaaccgta acccatcag 19
<210> 130
   <211> 19
   <212> DNA
   <213> Artificial Sequence
<220>
   <223> Primer N161SeqR3
<400> 130
   cacagattgg gtaccacca 19
<210> 131
   <211> 20
   <212> DNA
   <213> Artificial Sequence
<220>
   <223> Primer N161Seqr4
<400> 131
   accacaagaa ccaggacctg 20
<210> 132
   <211> 19
   <212> DNA
   <213> Artificial Sequence
<220>
   <223> Primer N161SeqR5
<400> 132
   catagctttc aaacccgct 19
<210> 133
   <211> 22
   <212> DNA
   <213> Artificial Sequence
<220>
   <223> Primer N161SeqR6
<400> 133
   cgtataccgt tgct cattag ag 22
<210> 134
   <211> 23
   <212> DNA
   <213> Artificial Sequence
<220>
   <223> Primer N162
<400> 134
   at gt t gacaa aagcaacaaa aga 23
<210> 135
   <211> 38
   <212> DNA
   <213> Artificial Sequence
<220>
   <223> Primer N189
<400> 135
   at ccgcggat agatctagtt cgagtttatc attatcaa 38
<210> 136
   <211> 53
   <212> DNA
   <213> Artificial Sequence
<220>
   <223> Primer N190.1
<400> 136
   ttcttttgtt gcttttgtca acatcctcag cgtttatgtg tgtttattcg aaa 53
<210> 137
   <211> 38
   <212> DNA
   <213> Artificial Sequence
<220>
   <223> Primer N176
<400> 137
   atccgcggat agatctatta gaagccgccg agcgggcg 38
<210> 138
   <211> 31
   <212> DNA
   <213> Artificial Sequence
<220>
   <223> Pr i rrer N177
<400> 138
   at cct cagct tttctccttg acgttaaagt a 31
<210> 139
   <211> 477
   <212> PRT
   <213> Escherichia coli
<400> 139
   Met Thr Gln Ser Arg Leu His Ala Ala Gln Asn Ala Leu Ala Lys Leu
   1               5                   10                  15
   His Glu His Arg Gly Asn Thr Phe Tyr Pro His Phe His Leu Ala Pro
               20                  25                  30
   Pro Ala Gly Trp Met Asn Asp Pro Asn Gly Leu Ile Trp Phe Asn Asp
           35                  40                  45
   Arg Tyr His Ala Phe Tyr Gln His His Pro Met Ser Glu His Trp Gly
       50                  55                  60
   Pro Met His Trp Gly His Ala Thr Ser Asp Asp Met Ile His Trp Gln
   65                  70                  75                  80
   His Glu Pro Ile Ala Leu Ala Pro Gly Asp Asp Asn Asp Lys Asp Gly
                   85                  90                  95
   Cys Phe Ser Gly Ser Ala Val Asp Asp Asn Gly Val Leu Ser Leu Ile
               100                 105                 110
   Tyr Thr Gly His Val Trp Leu Asp Gly Ala Gly Asn Asp Asp Ala Ile
           115                 120                 125
   Arg Glu Val Gln Cys Leu Ala Thr Ser Arg Asp Gly Ile His Phe Glu
       130                 135                 140
   Lys Gln Gly Val Ile Leu Thr Pro Pro Glu Gly Ile Met His Phe Arg
   145                 150                 155                 160
   Asp Pro Lys Val Trp Arg Glu Ala Asp Thr Trp Trp Met Val Val Gly
                   165                 170                 175
   Ala Lys Asp Pro Gly Asn Thr Gly Gln Ile Leu Leu Tyr Arg Gly Ser
               180                 185                 190
   Ser Leu Arg Glu Trp Thr Phe Asp Arg Val Leu Ala His Ala Asp Ala
           195                 200                 205
   Gly Glu Ser Tyr Met Trp Glu Cys Pro Asp Phe Phe Ser Leu Gly Asp
       210                 215                 220
   Gln His Tyr Leu Met Phe Ser Pro Gln Gly Met Asn Ala Glu Gly Tyr
   225                 230                 235                 240
   Ser Tyr Arg Asn Arg Phe Gln Ser Gly Val Ile Pro Gly Met Trp Ser
                   245                 250                 255
   Pro Gly Arg Leu Phe Ala Gln Ser Gly His Phe Thr Glu Leu Asp Asn
               260                 265                 270
   Gly His Asp Phe Tyr Ala Pro Gln Ser Phe Leu Ala Lys Asp Gly Arg
           275                 280                 285
   Arg Ile Val Ile Gly Trp Met Asp Met Trp Glu Ser Pro Met Pro Ser
       290                 295                 300
   Lys Arg Glu Gly Trp Ala Gly Cys Met Thr Leu Ala Arg Glu Leu Ser
   305                 310                 315                 320
   Glu Ser Asn Gly Lys Leu Leu Gln Arg Pro Val His Glu Ala Glu Ser
                   325                 330                 335
   Leu Arg Gln Gln His Gln Ser Val Ser Pro Arg Thr Ile Ser Asn Lys
               340                 345                 350
   Tyr Val Leu Gln Glu Asn Ala Gln Ala Val Glu Ile Gln Leu Gln Trp
           355                 360                 365
   Ala Leu Lys Asn Ser Asp Ala Glu His Tyr Gly Leu Gln Leu Gly Thr
       370                 375                 380
   Gly Met Arg Leu Tyr Ile Asp Asn Gln Ser Glu Arg Leu Val Leu Trp
   385                 390                 395                 400
   Arg Tyr Tyr Pro His Glu Asn Leu Asp Gly Tyr Arg Ser Ile Pro Leu
                   405                 410                 415
   Pro Gln Arg Asp Thr Leu Ala Leu Arg Ile Phe Ile Asp Thr Ser Ser
               420                 425                 430
   Val Glu Val Phe Ile Asn Asp Gly Glu Ala Val Met Ser Ser Arg Ile
           435                 440                 445
   Tyr Pro Gln Pro Glu Glu Arg Glu Leu Ser Leu Tyr Ala Ser His Gly
   450 455 460
   Val Ala Val Leu Gln His Gly Ala Leu Trp Leu Leu Gly
   465 470 475

<210> 140
   <211> 304
   <212> PRT
   <213> Escherichia coli
<400> 140
   Met Ser Ala Lys Val Trp Val Leu Gly Asp Ala Val Val Asp Leu Leu
   1 5 10 15
   Pro Glu Ser Asp Gly Arg Leu Leu Pro Cys Pro Gly Gly Ala Pro Ala
   20 25 30
   Asn Val Ala Val Gly Ile Ala Arg Leu Gly Gly Thr Ser Gly Phe Ile
   35 40 45
   Gly Arg Val Gly Asp Asp Pro Phe Gly Ala Leu Met Gln Arg Thr Leu
   50 55 60
   Leu Thr Glu Gly Val Asp Ile Thr Tyr Leu Lys Gln Asp Glu Trp His
   65 70 75 80
   Arg Thr Ser Thr Val Leu Val Asp Leu Asn Asp Gln Gly Glu Arg Ser
   85 90 95
   Phe Thr Phe Met Val Arg Pro Ser Ala Asp Leu Phe Leu Glu Thr Thr
   100 105 110
   Asp Leu Pro Cys Trp Arg His Gly Glu Trp Leu His Leu Cys Ser Ile
   115 120 125
   Ala Leu Ser Ala Glu Pro Ser Arg Thr Ser Ala Phe Thr Ala Met Thr
   130 135 140
   Ala Ile Arg His Ala Gly Gly Phe Val Ser Phe Asp Pro Asn Ile Arg
   145 150 155 160
   Glu Asp Leu Trp Gln Asp Glu His Leu Leu Arg Leu Cys Leu Arg Gln
   165 170 175
   Ala Leu Gln Leu Ala Asp Val Val Lys Leu Ser Glu Glu Glu Trp Arg
   180 185 190
   Leu Ile Ser Gly Lys Thr Gln Asn Asp Gln Asp Ile Cys Ala Leu Ala
   195 200 205
   Lys Glu Tyr Glu Ile Ala Met Leu Leu Val Thr Lys Gly Ala Glu Gly
   210 215 220
   Val Val Val Cys Tyr Arg Gly Gln Val His His Phe Ala Gly Met Ser
   225 230 235 240
   Val Asn Cys Val Asp Ser Thr Gly Ala Gly Asp Ala Phe Val Ala Gly
   245 250 255
   Leu Leu Thr Gly Leu Ser Ser Thr Gly Leu Ser Thr Asp Glu Arg Glu
   260 265 270
   Met Arg Arg Ile Ile Asp Leu Ala Gln Arg Cys Gly Ala Leu Ala Val
   275 280 285
   Thr Ala Lys Gly Ala Met Thr Ala Leu Pro Cys Arg Gln Glu Leu Glu
   290 295 300
<210> 141
   <211> 415
   <212> PRT
   <213> Escherichia coli
<400> 141
   Met Ala Leu Asn Ile Pro Phe Arg Asn Ala Tyr Tyr Arg Phe Ala Ser
   1 5 10 15
   Ser Tyr Ser Phe Leu Phe Phe Ile Ser Trp Ser Leu Trp Trp Ser Leu
   20 25 30
   Tyr Ala Ile Trp Leu Lys Gly His Leu Gly Leu Thr Gly Thr Glu Leu
   35 40 45
   Gly Thr Leu Tyr Ser Val Asn Gln Phe Thr Ser Ile Leu Phe Met Met
   50 55 60
   Phe Tyr Gly Ile Val Gln Asp Lys Leu Gly Leu Lys Lys Pro Leu Ile
   65 70 75 80
   Trp Cys Met Ser Phe Ile Leu Val Leu Thr Gly Pro Phe Met Ile Tyr
   85 90 95
   Val Tyr Glu Pro Leu Leu Gln Ser Asn Phe Ser Val Gly Leu Ile Leu
   100 105 110
   Gly Ala Leu Phe Phe Gly Leu Gly Tyr Leu Ala Gly Cys Gly Leu Leu
   115 120 125
   Asp Ser Phe Thr Glu Lys Met Ala Arg Asn Phe His Phe Glu Tyr Gly
   130 135 140
   Thr Ala Arg Ala Trp Gly Ser Phe Gly Tyr Ala Ile Gly Ala Phe Phe
   145 150 155 160
   Ala Gly Ile Phe Phe Ser Ile Ser Pro His Ile Asn Phe Trp Leu Val
   165 170 175
   Ser Leu Phe Gly Ala Val Phe Met Met Ile Asn Met Arg Phe Lys Asp
   180 185 190
   Lys Asp His Gln Cys Val Ala Ala Asp Ala Gly Gly Val Lys Lys Glu
   195 200 205
   Asp Phe Ile Ala Val Phe Lys Asp Arg Asn Phe Trp Val Phe Val Ile
   210 215 220
   Phe Ile Val Gly Thr Trp Ser Phe Tyr Asn Ile Phe Asp Gln Gln Leu
   225 230 235 240
   Phe Pro Val Phe Tyr Ser Gly Leu Phe Glu Ser His Asp Val Gly Thr
   245 250 255
   Arg Leu Tyr Gly Tyr Leu Asn Ser Phe Gln Val Val Leu Glu Ala Leu
   260 265 270
   Cys Met Ala Ile Ile Pro Phe Phe Val Asn Arg Val Gly Pro Lys Asn
   275 280 285
   Ala Leu Leu Ile Gly Val Val Ile Met Ala Leu Arg Ile Leu Ser Cys
   290 295 300
   Ala Leu Phe Val Asn Pro Trp Ile Ile Ser Leu Val Lys Leu Leu His
   305 310 315 320
   Ala Ile Glu Val Pro Leu Cys Val Ile Ser Val Phe Lys Tyr Ser Val
   325 330 335
   Ala Asn Phe Asp Lys Arg Leu Ser Ser Thr Ile Phe Leu Ile Gly Phe
   340 345 350
   Gln Ile Ala Ser Ser Leu Gly Ile Val Leu Leu Ser Thr Pro Thr Gly
   355 360 365
   Ile Leu Phe Asp His Ala Gly Tyr Gln Thr Val Phe Phe Ala Ile Ser
   370 375 380
   Gly Ile Val Cys Leu Met Leu Leu Phe Gly Ile Phe Phe Leu Ser Lys
   385 390 395 400
   Lys Arg Glu Gln Ile Val Met Glu Thr Pro Val Pro Ser Ala Ile
   405 410 415
<210> 142
   <211> 6341
   <212> DNA
   <213> Artificial Sequence
<220>
   <223> Plasmid pFP988DssPspac
<400> 142
   gatccaagtt taaactgtac actagatatt tcttctccgc ttaaatcatc aaagaaatct 60
   ttatcacttg taaccagtcc gtccacatgt cgaattgcat ctgaccgaat tttacgtttc 120
   cctgaataat tctcatcaat cgtttcatca attttatctt tatactttat attttgtgcg 180
   ttaatcaaat cataattttt atatgtttcc tcatgattta tgtctttatt attatagttt 240
   ttattctctc tttgattatg tctttgtatc ccgtttgtat tacttgatcc tttaactctg 300
   gcaaccctca aaattgaatg agacatgcta cacctccgga taataaatat atataaacgt 360
   atatagattt cataaagtct aacacactag acttatttac ttcgtaatta agtcgttaaa 420
   ccgtgtgctc tacgaccaaa actataaaac ctttaagaac tttctttttt tacaagaaaa 480
   aagaaattag ataaatctct catatctttt attcaataat cgcatccgat tgcagtataa 540
   atttaacgat cactcatcat gttcatattt atcagagctc gtgctataat tatactaatt 600
   ttataaggag gaaaaaatat gggcattttt agtatttttg taatcagcac agttcattat 660
   caaccaaaca aaaaataagt ggttataatg aatcgttaat aagcaaaatt catataacca 720
   aattaaagag ggttataatg aacgagaaaa atataaaaca cagtcaaaac tttattactt 780
   caaaacataa tatagataaa ataatgacaa atataagatt aaatgaacat gataatatct 840
   ttgaaatcgg ctcaggaaaa ggccatttta cccttgaatt agtaaagagg tgtaatttcg 900
   taactgccat tgaaatagac cataaattat gcaaaactac agaaaataaa cttgttgatc 960
   acgataattt ccaagtttta aacaaggata tattgcagtt taaatttcct aaaaaccaat 1020
   cctataaaat atatggtaat ataccttata acataagtac ggatataata cgcaaaattg 1080
   tttttgatag tatagctaat gagatttatt taatcgtgga atacgggttt gctaaaagat 1140
   tattaaatac aaaacgctca ttggcattac ttttaatggc agaagttgat atttctatat 1200
   taagtatggt tccaagagaa tattttcatc ctaaacctaa agtgaatagc tcacttatca 1260
   gattaagtag aaaaaaatca agaatatcac acaaagataa acaaaagtat aattatttcg 1320
   ttatgaaatg ggttaacaaa gaatacaaga aaatatttac aaaaaatcaa tttaacaatt 1380
   ccttaaaaca tgcaggaatt gacgatttaa acaatattag ctttgaacaa ttcttatctc 1440
   ttttcaatag ctataaatta tttaataagt aagttaaggg atgcagttca tcgatgaagg 1500
   caactacagc tcaggcgaca accatacgct gagagatcct cactacgtag aagataaagg 1560
   ccacaaatac ttagtatttg aagcaaacac tggaactgaa gatggctacc aaggcgaaga 1620
   atctttattt aacaaagcat actatggcaa aagcacatca ttcttccgtc aagaaagtca 1680
   aaaacttctg caaagcgata aaaaacgcac ggctgagtta gcaaacggcg ctctcggtat 1740
   gattgagcta aacgatgatt acacactgaa aaaagtgatg aaaccgctga ttgcatctaa 1800
   cacagtaaca gatgaaattg aacgcgcgaa cgtctttaaa atgaacggca aatggtacct 1860
   gttcactgac tcccgcggat caaaaatgac gattgacggc attacgtcta acgatattta 1920
   catgcttggt tatgtttcta attctttaac tggcccatac aagccgctga acaaaactgg 1980
   ccttgtgtta aaaatggatc ttgatcctaa cgatgtaacc tttacttact cacacttcgc 2040
   tgtacctcaa gcgaaaggaa acaatgtcgt gattacaagc tatatgacaa acagaggatt 2100
   ctacgcagac aaacaatcaa cgtttgcgcc aagcttgcat gcgagagtag ggaactgcca 2160
   ggcatcaaat aaaacgaaag gctcagtcga aagactgggc ctttcgtttt atctgttgtt 2220
   tgtcggtgaa cgctctcctg agtaggacaa atccgccggg agcggatttg aacgttgcga 2280
   agcaacggcc cggagggtgg cgggcaggac gcccgccata aactgccagg catcaaatta 2340
   agcagaaggc catcctgacg gatggccttt ttgcgtttct acaaactctt tttgtttatt 2400
   tttctaaata cattcaaata tgtatccgct catgctccgg atctgcatcg caggatgctg 2460
   ctggctaccc tgtggaacac ctacatctgt attaacgaag cgctggcatt gaccctgagt 2520
   gatttttctc tggtcccgcc gcatccatac cgccagttgt ttaccctcac aacgttccag 2580
   taaccgggca tgttcatcat cagtaacccg tatcgtgagc atcctctctc gtttcatcgg 2640
   tatcattacc cccatgaaca gaaattcccc cttacacgga ggcatcaagt gaccaaacag 2700
   gaaaaaaccg cccttaacat ggcccgcttt atcagaagcc agacattaac gcttctggag 2760
   aaactcaacg agctggacgc ggatgaacag gcagacatct gtgaatcgct tcacgaccac 2820
   gctgatgagc tttaccgcag ctgcctcgcg cgtttcggtg atgacggtga aaacctctga 2880
   cacatgcagc tcccggagac ggtcacagct tgtctgtaag cggatgccgg gagcagacaa 2940
   gcccgtcagg gcgcgtcagc gggtgttggc gggtgtcggg gcgcagccat gacccagtca 3000
   cgtagcgata gcggagtgta tactggctta actatgcggc atcagagcag attgtactga 3060
   gagtgcacca tatgcggtgt gaaataccgc acagatgcgt aaggagaaaa taccgcatca 3120
   ggcgctcttc cgcttcctcg ctcactgact cgctgcgctc ggtcgttcgg ctgcggcgag 3180
   cggtatcagc tcactcaaag gcggtaatac ggttatccac agaatcaggg gataacgcag 3240
   gaaagaacat gtgagcaaaa ggccagcaaa aggccaggaa ccgtaaaaag gccgcgttgc 3300
   tggcgttttt ccataggctc cgcccccctg acgagcatca caaaaatcga cgctcaagtc 3360
   agaggtggcg aaacccgaca ggactataaa gataccaggc gtttccccct ggaagctccc 3420
   tcgtgcgctc tcctgttccg accctgccgc ttaccggata cctgtccgcc tttctccctt 3480
   cgggaagcgt ggcgctttct caatgctcac gctgtaggta tctcagttcg gtgtaggtcg 3540
   ttcgctccaa gctgggctgt gtgcacgaac cccccgttca gcccgaccgc tgcgccttat 3600
   ccggtaacta tcgtcttgag tccaacccgg taagacacga cttatcgcca ctggcagcag 3660
   ccactggtaa caggattagc agagcgaggt atgtaggcgg tgctacagag ttcttgaagt 3720
   ggtggcctaa ctacggctac actagaagga cagtatttgg tatctgcgct ctgctgaagc 3780
   cagttacctt cggaaaaaga gttggtagct cttgatccgg caaacaaacc accgctggta 3840
   gcggtggttt ttttgtttgc aagcagcaga ttacgcgcag aaaaaaagga tctcaagaag 3900
   atcctttgat cttttctacg gggtctgacg ctcagtggaa cgaaaactca cgttaaggga 3960
   ttttggtcat gagattatca aaaggatctt cacctagatc cttttaaatt taaaaatgaa 4020
   gttttaaatc aatctaaagt atatatgagt aaacttggtc tgacagttac caatgcttaa 4080
   tcagtgaggc acctatctca gcgatctgtc tatttcgttc atccatagtt gcctgactcc 4140
   ccgtcgtgta gataactacg atacgggagg gcttaccatc tggccccagt gctgcaatga 4200
   taccgcgaga cccacgctca ccggctccag atttatcagc aataaaccag ccagccggaa 4260
   gggccgagcg cagaagtggt cctgcaactt tatccgcctc catccagtct attaattgtt 4320
   gccgggaagc tagagtaagt agttcgccag ttaatagttt gcgcaacgtt gttgccattg 4380
   ctgcaggcat cgtggtgtca cgctcgtcgt ttggtatggc ttcattcagc tccggttccc 4440
   aacgatcaag gcgagttaca tgatccccca tgttgtgcaa aaaagcggtt agctccttcg 4500
   gtcctccgat cgttgtcaga agtaagttgg ccgcagtgtt atcactcatg gttatggcag 4560
   cactgcataa ttctcttact gtcatgccat ccgtaagatg cttttctgtg actggtgagt 4620
   actcaaccaa gtcattctga gaatagtgta tgcggcgacc gagttgctct tgcccggcgt 4680
   caatacggga taataccgcg ccacatagca gaactttaaa agtgctcatc attggaaaac 4740
   gttcttcggg gcgaaaactc tcaaggatct taccgctgtt gagatccagt tcgatgtaac 4800
   ccactcgtgc acccaactga tcttcagcat cttttacttt caccagcgtt tctgggtgag 4860
   caaaaacagg aaggcaaaat gccgcaaaaa agggaataag ggcgacacgg aaatgttgaa 4920
   tactcatact cttccttttt caatattatt gaagcattta tcagggttat tgtctcatga 4980
   gcggatacat atttgaatgt atttagaaaa ataaacaaat aggggttccg cgcacatttc 5040
   cccgaaaagt gccacctgac gtctaagaaa ccattattat catgacatta acctataaaa 5100
   ataggcgtat cacgaggccc tttcgtctcg cgcgtttcgg tgatgacggt gaaaacctct 5160
   gacacatgca gctcccggag acggtcacag cttgtctgta agcggatgcc gggagcagac 5220
   aagcccgtca gggcgcgtca gcgggtgttc atgtgcgtaa ctaacttgcc atcttcaaac 5280
   aggagggctg gaagaagcag accgctaaca cagtacataa aaaaggagac atgaacgatg 5340
   aacatcaaaa agtttgcaaa acaagcaaca gtattaacct ttactaccgc actgctggca 5400
   ggaggcgcaa ctcaagcgtt tgcgaaagaa acgaaccaaa agccatataa ggaaacatac 5460
   ggcatttccc atattacacg ccatgatatg ctgcaaatcc ctgaacagca aaaaaatgaa 5520
   aaatatcaag ttcctgaatt cgattcgtcc acaattaaaa atatctcttc tgcaaaaggc 5580
   ctggacgttt gggacagctg gccattacaa aacgctgacg gcactgtcgc aaactatcac 5640
   ggctaccaca tcgtctttgc attagccgga gatcctaaaa atgcggatga cacatcgatt 5700
   tacatgttct atcaaaaagt cggcgaaact tctattgaca gctggaaaaa cgctggccgc 5760
   gtctttaaag acagcgacaa attcgatgca aatgattcta tcctaaaaga ccaaacacaa 5820
   gaatggtcag gttcagccac atttacatct gacggaaaaa tccgtttatt ctacactgat 5880
   ttctccggta aacattacgg caaacaaaca ctgacaactg cacaagttaa cgtatcagca 5940
   tcagacagct ctttgaacat caacggtgta gaggattata aatcaatctt tgacggtgac 6000
   ggaaaaacgt atcaaaatgt acagaattcg agctctcgag taattctaca cagcccagtc 6060
   cagactattc ggcactgaaa ttatgggtga agtggtcaag acctcactag gcaccttaaa 6120
   aatagcgcac cctgaagaag atttatttga ggtagccctt gcctacctag cttccaagaa 6180
   agatatccta acagcacaag agcggaaaga tgttttgttc tacatccaga acaacctctg 6240
   ctaaaattcc tgaaaaattt tgcaaaaagt tgttgacttt atctacaagg tgtggcataa 6300
   tgtgtggaat tgtgagcgct cacaattaag cttgaattcc c 6341
<210> 143
   <211> 6221
   <212> DNA
   <213> Artificial Sequence
<220>
   <223> Plasmid pFP988DssPgr oE
<400> 143
   tcgagagcta ttgtaacata atcggtacgg gggtgaaaaa gctaacggaa aagggagcgg 60
   aaaagaatga tgtaagcgtg aaaaattttt tatcttatca cttgaaattg gaagggagat 120
   tctttattat aagaattgtg gaattgtgag cggataacaa ttcccaatta aaggaggaaa 180
   ctagtggatc caagtttaaa ctgtacacta gatatttctt ctccgcttaa atcatcaaag 240
   aaatctttat cacttgtaac cagtccgtcc acatgtcgaa ttgcatctga ccgaatttta 300
   cgtttccctg aataattctc atcaatcgtt tcatcaattt tatctttata ctttatattt 360
   tgtgcgttaa tcaaatcata atttttatat gtttcctcat gatttatgtc tttattatta 420
   tagtttttat tctctctttg attatgtctt tgtatcccgt ttgtattact tgatccttta 480
   actctggcaa ccctcaaaat tgaatgagac atgctacacc tccggataat aaatatatat 540
   aaacgtatat agatttcata aagtctaaca cactagactt atttacttcg taattaagtc 600
   gttaaaccgt gtgctctacg accaaaacta taaaaccttt aagaactttc tttttttaca 660
   agaaaaaaga aattagataa atctctcata tcttttattc aataatcgca tccgattgca 720
   gtataaattt aacgatcact catcatgttc atatttatca gagctcgtgc tataattata 780
   ctaattttat aaggaggaaa aaatatgggc atttttagta tttttgtaat cagcacagtt 840
   cattatcaac caaacaaaaa ataagtggtt ataatgaatc gttaataagc aaaattcata 900
   taaccaaatt aaagagggtt ataatgaacg agaaaaatat aaaacacagt caaaacttta 960
   ttacttcaaa acataatata gataaaataa tgacaaatat aagattaaat gaacatgata 1020
   atatctttga aatcggctca ggaaaaggcc attttaccct tgaattagta aagaggtgta 1080
   atttcgtaac tgccattgaa atagaccata aattatgcaa aactacagaa aataaacttg 1140
   ttgatcacga taatttccaa gttttaaaca aggatatatt gcagtttaaa tttcctaaaa 1200
   accaatccta taaaatatat ggtaatatac cttataacat aagtacggat ataatacgca 1260
   aaattgtttt tgatagtata gctaatgaga tttatttaat cgtggaatac gggtttgcta 1320
   aaagattatt aaatacaaaa cgctcattgg cattactttt aatggcagaa gttgatattt 1380
   ctatattaag tatggttcca agagaatatt ttcatcctaa acctaaagtg aatagctcac 1440
   ttatcagatt aagtagaaaa aaatcaagaa tatcacacaa agataaacaa aagtataatt 1500
   atttcgttat gaaatgggtt aacaaagaat acaagaaaat atttacaaaa aatcaattta 1560
   acaattcctt aaaacatgca ggaattgacg atttaaacaa tattagcttt gaacaattct 1620
   tatctctttt caatagctat aaattattta ataagtaagt taagggatgc agttcatcga 1680
   tgaaggcaac tacagctcag gcgacaacca tacgctgaga gatcctcact acgtagaaga 1740
   taaaggccac aaatacttag tatttgaagc aaacactgga actgaagatg gctaccaagg 1800
   cgaagaatct ttatttaaca aagcatacta tggcaaaagc acatcattct tccgtcaaga 1860
   aagtcaaaaa cttctgcaaa gcgataaaaa acgcacggct gagttagcaa acggcgctct 1920
   cggtatgatt gagctaaacg atgattacac actgaaaaaa gtgatgaaac cgctgattgc 1980
   atctaacaca gtaacagatg aaattgaacg cgcgaacgtc tttaaaatga acggcaaatg 2040
   gtacctgttc actgactccc gcggatcaaa aatgacgatt gacggcatta cgtctaacga 2100
   tatttacatg cttggttatg tttctaattc tttaactggc ccatacaagc cgctgaacaa 2160
   aactggcctt gtgttaaaaa tggatcttga tcctaacgat gtaaccttta cttactcaca 2220
   cttcgctgta cctcaagcga aaggaaacaa tgtcgtgatt acaagctata tgacaaacag 2280
   aggattctac gcagacaaac aatcaacgtt tgcgccaagc ttgcatgcga gagtagggaa 2340
   ctgccaggca tcaaataaaa cgaaaggctc agtcgaaaga ctgggccttt cgttttatct 2400
   gttgtttgtc ggtgaacgct ctcctgagta ggacaaatcc gccgggagcg gatttgaacg 2460
   ttgcgaagca acggcccgga gggtggcggg caggacgccc gccataaact gccaggcatc 2520
   aaattaagca gaaggccatc ctgacggatg gcctttttgc gtttctacaa actctttttg 2580
   tttatttttc taaatacatt caaatatgta tccgctcatg ctccggatct gcatcgcagg 2640
   atgctgctgg ctaccctgtg gaacacctac atctgtatta acgaagcgct ggcattgacc 2700
   ctgagtgatt tttctctggt cccgccgcat ccataccgcc agttgtttac cctcacaacg 2760
   ttccagtaac cgggcatgtt catcatcagt aacccgtatc gtgagcatcc tctctcgttt 2820
   catcggtatc attaccccca tgaacagaaa ttccccctta cacggaggca tcaagtgacc 2880
   aaacaggaaa aaaccgccct taacatggcc cgctttatca gaagccagac attaacgctt 2940
   ctggagaaac tcaacgagct ggacgcggat gaacaggcag acatctgtga atcgcttcac 3000
   gaccacgctg atgagcttta ccgcagctgc ctcgcgcgtt tcggtgatga cggtgaaaac 3060
   ctctgacaca tgcagctccc ggagacggtc acagcttgtc tgtaagcgga tgccgggagc 3120
   agacaagccc gtcagggcgc gtcagcgggt gttggcgggt gtcggggcgc agccatgacc 3180
   cagtcacgta gcgatagcgg agtgtatact ggcttaacta tgcggcatca gagcagattg 3240
   tactgagagt gcaccatatg cggtgtgaaa taccgcacag atgcgtaagg agaaaatacc 3300
   gcatcaggcg ctcttccgct tcctcgctca ctgactcgct gcgctcggtc gttcggctgc 3360
   ggcgagcggt atcagctcac tcaaaggcgg taatacggtt atccacagaa tcaggggata 3420
   acgcaggaaa gaacatgtga gcaaaaggcc agcaaaaggc caggaaccgt aaaaaggccg 3480
   cgttgctggc gtttttccat aggctccgcc cccctgacga gcatcacaaa aatcgacgct 3540
   caagtcagag gtggcgaaac ccgacaggac tataaagata ccaggcgttt ccccctggaa 3600
   gctccctcgt gcgctctcct gttccgaccc tgccgcttac cggatacctg tccgcctttc 3660
   tcccttcggg aagcgtggcg ctttctcaat gctcacgctg taggtatctc agttcggtgt 3720
   aggtcgttcg ctccaagctg ggctgtgtgc acgaaccccc cgttcagccc gaccgctgcg 3780
   ccttatccgg taactatcgt cttgagtcca acccggtaag acacgactta tcgccactgg 3840
   cagcagccac tggtaacagg attagcagag cgaggtatgt aggcggtgct acagagttct 3900
   tgaagtggtg gcctaactac ggctacacta gaaggacagt atttggtatc tgcgctctgc 3960
   tgaagccagt taccttcgga aaaagagttg gtagctcttg atccggcaaa caaaccaccg 4020
   ctggtagcgg tggttttttt gtttgcaagc agcagattac gcgcagaaaa aaaggatctc 4080
   aagaagatcc tttgatcttt tctacggggt ctgacgctca gtggaacgaa aactcacgtt 4140
   aagggatttt ggtcatgaga ttatcaaaaa ggatcttcac ctagatcctt ttaaattaaa 4200
   aatgaagttt taaatcaatc taaagtatat atgagtaaac ttggtctgac agttaccaat 4260
   gcttaatcag tgaggcacct atctcagcga tctgtctatt tcgttcatcc atagttgcct 4320
   gactccccgt cgtgtagata actacgatac gggagggctt accatctggc cccagtgctg 4380
   caatgatacc gcgagaccca cgctcaccgg ctccagattt atcagcaata aaccagccag 4440
   ccggaagggc cgagcgcaga agtggtcctg caactttatc cgcctccatc cagtctatta 4500
   attgttgccg ggaagctaga gtaagtagtt cgccagttaa tagtttgcgc aacgttgttg 4560
   ccattgctgc aggcatcgtg gtgtcacgct cgtcgtttgg tatggcttca ttcagctccg 4620
   gttcccaacg atcaaggcga gttacatgat cccccatgtt gtgcaaaaaa gcggttagct 4680
   ccttcggtcc tccgatcgtt gtcagaagta agttggccgc agtgttatca ctcatggtta 4740
   tggcagcact gcataattct cttactgtca tgccatccgt aagatgcttt tctgtgactg 4800
   gtgagtactc aaccaagtca ttctgagaat agtgtatgcg gcgaccgagt tgctcttgcc 4860
   cggcgtcaat acgggataat accgcgccac atagcagaac tttaaaagtg ctcatcattg 4920
   gaaaacgttc ttcggggcga aaactctcaa ggatcttacc gctgttgaga tccagttcga 4980
   tgtaacccac tcgtgcaccc aactgatctt cagcatcttt tactttcacc agcgtttctg 5040
   ggtgagcaaa aacaggaagg caaaatgccg caaaaaaggg aataagggcg acacggaaat 5100
   gttgaatact catactcttc ctttttcaat attattgaag catttatcag ggttattgtc 5160
   tcatgagcgg atacatattt gaatgtattt agaaaaataa acaaataggg gttccgcgca 5220
   catttccccg aaaagtgcca cctgacgtct aagaaaccat tattatcatg acattaacct 5280
   ataaaaatag gcgtatcacg aggccctttc gtctcgcgcg tttcggtgat gacggtgaaa 5340
   acctctgaca catgcagctc ccggagacgg tcacagcttg tctgtaagcg gatgccggga 5400
   gcagacaagc ccgtcagggc gcgtcagcgg gtgttcatgt gcgtaactaa cttgccatct 5460
   tcaaacagga gggctggaag aagcagaccg ctaacacagt acataaaaaa ggagacatga 5520
   acgatgaaca tcaaaaagtt tgcaaaacaa gcaacagtat taacctttac taccgcactg 5580
   ctggcaggag gcgcaactca agcgtttgcg aaagaaacga accaaaagcc atataaggaa 5640
   acatacggca tttcccatat tacacgccat gatatgctgc aaatccctga acagcaaaaa 5700
   aat gaaaaat at caagt t cc t gaat t cgat t cgt ccacaa t t aaaaat at ct ct t ct gca 5760
   aaaggcct gg acgt t t ggga cagct ggcca t t acaaaacg ct gacggcac t gt cgcaaac 5820
   t at cacggct accacat cgt ct t t gcat t a gccggagat c ct aaaaat gc ggat gacaca 5880
   tcgatttaca tgttctatca aaaagtcggc gaaacttcta ttgacagctg gaaaaacgct 5940
   ggccgcgtct ttaaagacag cgacaaattc gatgcaaatg attctatcct aaaagaccaa 6000
   acacaagaat ggtcaggttc agccacattt acatctgacg gaaaaatccg tttattctac 6060
   act gat t t ct ccggt aaaca t t acggcaaa caaacact ga caact gcaca agt t aacgt a 6120
   t cagcat cag acagct ct t t gaacat caac ggt gt agagg at t at aaat c aat ct t t gac 6180
   ggt gacggaa aaacgt at ca aaat gt acag aat t cgagct c 6221
<210> 144
   <211> 40
   <212> DNA
   <213> Artificial Sequence
<220>
   <223> Primer T- budB (BamHI)
<400> 144
   agatagatgg atccggaggt gggtcacatg gacaaacagt 40
<210> 145
   <211> 29
   <212> DNA
   <213> Artificial Sequence
<220>
   <223> Primer B- ki vD (BamHI)
<400> 145
   ctctagagga tccagactcc t aggacat g 29
<210> 146
   <211> 6039
   <212> DNA
   <213> Artificial Sequence
<220>
   <223> Vect or fragment pFP988Dss
<400> 146
   gatccaagtt taaactgtac actagatatt tcttctccgc ttaaatcatc aaagaaatct 60
   ttatcacttg taaccagtcc gtccacatgt cgaattgcat ctgaccgaat tttacgtttc 120
   cctgaataat tctcatcaat cgtttcatca attttatctt tatactttat attttgtgcg 180
   ttaatcaaat cataattttt atatgtttcc tcatgattta tgtctttatt attatagttt 240
   ttattctctc tttgattatg tctttgtatc ccgtttgtat tacttgatcc tttaactctg 300
   gcaaccctca aaattgaatg agacatgcta cacctccgga taataaatat atataaacgt 360
   atatagattt cataaagtct aacacactag acttatttac ttcgtaatta agtcgttaaa 420
   ccgtgtgctc tacgaccaaa actataaaac ctttaagaac tttctttttt tacaagaaaa 480
   aagaaattag ataaatctct catatctttt attcaataat cgcatccgat tgcagtataa 540
   atttaacgat cactcatcat gttcatattt atcagagctc gtgctataat tatactaatt 600
   ttataaggag gaaaaaatat gggcattttt agtatttttg taatcagcac agttcattat 660
   caaccaaaca aaaaataagt ggttataatg aatcgttaat aagcaaaatt catataacca 720
   aattaaagag ggttataatg aacgagaaaa atataaaaca cagtcaaaac tttattactt 780
   caaaacataa tatagataaa ataatgacaa atataagatt aaatgaacat gataatatct 840
   ttgaaatcgg ctcaggaaaa ggccatttta cccttgaatt agtaaagagg tgtaatttcg 900
   taactgccat tgaaatagac cataaattat gcaaaactac agaaaataaa cttgttgatc 960
   acgataattt ccaagtttta aacaaggata tattgcagtt taaatttcct aaaaaccaat 1020
   cctataaaat atatggtaat ataccttata acataagtac ggatataata cgcaaaattg 1080
   tttttgatag tatagctaat gagatttatt taatcgtgga atacgggttt gctaaaagat 1140
   tattaaatac aaaacgctca ttggcattac ttttaatggc agaagttgat atttctatat 1200
   taagtatggt tccaagagaa tattttcatc ctaaacctaa agtgaatagc tcacttatca 1260
   gattaagtag aaaaaaatca agaatatcac acaaagataa acaaaagtat aattatttcg 1320
   ttatgaaatg ggttaacaaa gaatacaaga aaatatttac aaaaaatcaa tttaacaatt 1380
   ccttaaaaca tgcaggaatt gacgatttaa acaatattag ctttgaacaa ttcttatctc 1440
   ttttcaatag ctataaatta tttaataagt aagttaaggg atgcagttca tcgatgaagg 1500
   caactacagc tcaggcgaca accatacgct gagagatcct cactacgtag aagataaagg 1560
   ccacaaatac ttagtatttg aagcaaacac tggaactgaa gatggctacc aaggcgaaga 1620
   atctttattt aacaaagcat actatggcaa aagcacatca ttcttccgtc aagaaagtca 1680
   aaaacttctg caaagcgata aaaaacgcac ggctgagtta gcaaacggcg ctctcggtat 1740
   gattgagcta aacgatgatt acacactgaa aaaagtgatg aaaccgctga ttgcatctaa 1800
   cacagtaaca gatgaaattg aacgcgcgaa cgtctttaaa atgaacggca aatggtacct 1860
   gttcactgac tcccgcggat caaaaatgac gattgacggc attacgtcta acgatattta 1920
   catgcttggt tatgtttcta attctttaac tggcccatac aagccgctga acaaaactgg 1980
   ccttgtgtta aaaatggatc ttgatcctaa cgatgtaacc tttacttact cacacttcgc 2040
   tgtacctcaa gcgaaaggaa acaatgtcgt gattacaagc tatatgacaa acagaggatt 2100
   ctacgcagac aaacaatcaa cgtttgcgcc aagcttgcat gcgagagtag ggaactgcca 2160
   ggcatcaaat aaaacgaaag gctcagtcga aagactgggc ctttcgtttt atctgttgtt 2220
   tgtcggtgaa cgctctcctg agtaggacaa atccgccggg agcggatttg aacgttgcga 2280
   agcaacggcc cggagggtgg cgggcaggac gcccgccata aactgccagg catcaaatta 2340
   agcagaaggc catcctgacg gatggccttt ttgcgtttct acaaactctt tttgtttatt 2400
   tttctaaata cattcaaata tgtatccgct catgctccgg atctgcatcg caggatgctg 2460
   ctggctaccc tgtggaacac ctacatctgt attaacgaag cgctggcatt gaccctgagt 2520
   gat t t t t ct c t ggt cccgcc gcat ccat ac cgccagt t gt t t accct cac aacgt t ccag 2580
   t aaccgggca t gt t cat cat cagt aacccg t at cgt gagc at cct ct ct c gt t t cat cgg 2640
   tatcattacc cccatgaaca gaaattcccc cttacacgga ggcatcaagt gaccaaacag 2700
   gaaaaaaccg cccttaacat ggcccgcttt atcagaagcc agacattaac gcttctggag 2760
   aaact caacg agct ggacgc ggat gaacag gcagacat ct gt gaat cgct t cacgaccac 2820
   gct gat gagc t t t accgcag ct gcct cgcg cgt t t cggt g at gacggt ga aaacct ct ga 2880
   cacatgcagc tcccggagac ggtcacagct tgtctgtaag cggatgccgg gagcagacaa 2940
   gcccgtcagg gcgcgtcagc gggtgttggc gggtgtcggg gcgcagccat gacccagtca 3000
   cgt agcgat a gcggagt gt a t act ggct t a act at gcggc at cagagcag at t gt act ga 3060
   gagtgcacca tatgcggtgt gaaataccgc acagatgcgt aaggagaaaa taccgcatca 3120
   ggcgct ct t c cgct t cct cg ct cact gact cgct gcgct c ggt cgt t cgg ct gcggcgag 3180
   cggt at cagc t cact caaag gcggt aat ac ggt t at ccac agaat caggg gat aacgcag 3240
   gaaagaacat gtgagcaaaa ggccagcaaa aggccaggaa ccgtaaaaag gccgcgttgc 3300
   t ggcgt t t t t ccat aggct c cgcccccct g acgagcat ca caaaaat cga cgct caagt c 3360
   agaggtggcg aaacccgaca ggactataaa gataccaggc gtttccccct ggaagctccc 3420
   t cgt gcgct c t cct gt t ccg accct gccgc t t accggat a cct gt ccgcc t t t ct ccct t 3480
   cgggaagcgt ggcgctttct caatgctcac gctgtaggta tctcagttcg gtgtaggtcg 3540
   ttcgctccaa gctgggctgt gtgcacgaac cccccgttca gcccgaccgc tgcgccttat 3600
   ccggt aact a t cgt ct t gag t ccaacccgg t aagacacga ct t at cgcca ct ggcagcag 3660
   ccactggtaa caggattagc agagcgaggt atgtaggcgg tgctacagag ttcttgaagt 3720
   ggt ggcct aa ct acggct ac act agaagga cagt at t t gg t at ct gcgct ct gct gaagc 3780
   cagttacctt cggaaaaaga gttggtagct cttgatccgg caaacaaacc accgctggta 3840
   gcggtggttt ttttgtttgc aagcagcaga ttacgcgcag aaaaaaagga tctcaagaag 3900
   at cct t t gat ct t t t ct acg gggt ct gacg ct cagt ggaa cgaaaact ca cgt t aaggga 3960
   t t t t ggt cat gagat t at ca aaaaggat ct t cacct agat cct t t t aaat t aaaaat gaa 4020
   gt t t t aaat c aat ct aaagt at at at gagt aaact t ggt c t gacagt t ac caat gct t aa 4080
   t cagt gaggc acct at ct ca gcgat ct gt c t at t t cgt t c at ccat agt t gcct gact cc 4140
   ccgt cgt gt a gat aact acg at acgggagg gct t accat c t ggccccagt gct gcaat ga 4200
   taccgcgaga cccacgctca ccggctccag atttatcagc aataaaccag ccagccggaa 4260
   gggccgagcg cagaagtggt cctgcaactt tatccgcctc catccagtct attaattgtt 4320
   gccgggaagc tagagtaagt agttcgccag ttaatagttt gcgcaacgtt gttgccattg 4380
   ct gcaggcat cgt ggt gt ca cgct cgt cgt t t ggt at ggc t t cat t cagc t ccggt t ccc 4440
   aacgat caag gcgagt t aca t gat ccccca t gt t gt gcaa aaaagcggt t agct cct t cg 4500
   gt cct ccgat cgt t gt caga agt aagt t gg ccgcagt gt t at cact cat g gt t at ggcag 4560
   cact gcat aa t t ct ct t act gt cat gccat ccgt aagat g ct t t t ct gt g act ggt gagt 4620
   actcaaccaa gtcattctga gaatagtgta tgcggcgacc gagttgctct tgcccggcgt 4680
   caatacggga taataccgcg ccacatagca gaactttaaa agtgctcatc attggaaaac 4740
   gttcttcggg gcgaaaactc tcaaggatct taccgctgtt gagatccagt tcgatgtaac 4800
   ccactcgtgc acccaactga tcttcagcat cttttacttt caccagcgtt tctgggtgag 4860
   caaaaacagg aaggcaaaat gccgcaaaaa agggaataag ggcgacacgg aaatgttgaa 4920
   tactcatact cttccttttt caatattatt gaagcattta tcagggttat tgtctcatga 4980
   gcggatacat atttgaatgt atttagaaaa ataaacaaat aggggttccg cgcacatttc 5040
   cccgaaaagt gccacctgac gtctaagaaa ccattattat catgacatta acctataaaa 5100
   ataggcgtat cacgaggccc tttcgtctcg cgcgtttcgg tgatgacggt gaaaacctct 5160
   gacacatgca gctcccggag acggtcacag cttgtctgta agcggatgcc gggagcagac 5220
   aagcccgtca gggcgcgtca gcgggtgttc atgtgcgtaa ctaacttgcc atcttcaaac 5280
   aggagggctg gaagaagcag accgctaaca cagtacataa aaaaggagac atgaacgatg 5340
   aacatcaaaa agtttgcaaa acaagcaaca gtattaacct ttactaccgc actgctggca 5400
   ggaggcgcaa ctcaagcgtt tgcgaaagaa acgaaccaaa agccatataa ggaaacatac 5460
   ggcatttccc atattacacg ccatgatatg ctgcaaatcc ctgaacagca aaaaaatgaa 5520
   aaatatcaag ttcctgaatt cgattcgtcc acaattaaaa atatctcttc tgcaaaaggc 5580
   ctggacgttt gggacagctg gccattacaa aacgctgacg gcactgtcgc aaactatcac 5640
   ggctaccaca tcgtctttgc attagccgga gatcctaaaa atgcggatga cacatcgatt 5700
   tacatgttct atcaaaaagt cggcgaaact tctattgaca gctggaaaaa cgctggccgc 5760
   gtctttaaag acagcgacaa attcgatgca aatgattcta tcctaaaaga ccaaacacaa 5820
   gaatggtcag gttcagccac atttacatct gacggaaaaa tccgtttatt ctacactgat 5880
   ttctccggta aacattacgg caaacaaaca ctgacaactg cacaagttaa cgtatcagca 5940
   tcagacagct ctttgaacat caacggtgta gaggattata aatcaatctt tgacggtgac 6000
   ggaaaaacgt atcaaaatgt acagaattcg agctctcga 6039
<210> 147
   <211> 44
   <212> DNA
   <213> Artificial Sequence
<220>
   <223> Primer T-groE(XhoI)
<400> 147
   agatagatct cgagagctat tgtaacataa tcggtacggg ggt g 44
<210> 148
   <211> 51
   <212> DNA
   <213> Artificial Sequence
<220>
   <223> Primer B-groEL (SpeI BamH1)
<400> 148
   at t at gt cag gat ccact ag tttcctcctt taattgggaa ttgttatccg c 51
<210> 149
   <211> 30
   <212> DNA
   <213> Artificial Sequence
<220>
   <223> Primer T-groEL
<400> 149
   agctattgta acataatcgg tacgggggtg 30
<210> 150
   <211> 45
   <212> DNA
   <213> Artificial Sequence
<220>
   <223> Primer T- ilvCB.s. (BamHI)
<400> 150
   acattgatgg at cccataac aagggagaga t t gaaat ggt aaaag 45
<210> 151
   <211> 47
   <212> DNA
   <213> Artificial Sequence
<220>
   <223> Primer B-ilvCB. s. (SpeI BamHI)
<400> 151
   tagacaacgg at ccact agt ttaattttgc gcaacggaga ccaccgc 47
<210> 152
   <211> 47
   <212> DNA
   <213> Artificial Sequence
<220>
   <223> Primer T-BD64 (DraIII
<400> 152
   ttaccgtgga ctcaccgagt gggtaactag cctcgccgga aagagcg 47
<210> 153
   <211> 48
   <212> DNA
   <213> Artificial Sequence
<220>
   <223> Primer B-BD64 (DraIII)
<400> 153
   t cacagt t aa gacacctggt gccgttaatg cgccatgaca gccat gat 48
<210> 154
   <211> 49
   <212> DNA
   <213> Artificial Sequence
<220>
   <223> Primer T-Iaclq (DraIII )
<400> 154
   acagatagat caccaggtgc aagctaattc cggtggaaac gaggtcatc c 49
<210> 155
   <211> 48
   <212> DNA
   <213> Artificial Sequence
<220>
   <223> Primer B-I aclq (DraIII)
<400> 155
   acagt acgat acacggggt tcactgcccg ctttccagtc gggaaacc 48
<210> 156
   <211> 49
   <212> DNA
   <213> Artificial Sequence
<220>
   <223> Primer T-groE (DraIII)
<400> 156
   t cggat t acg caccccgt ga gct at t gt aa cataatcggt acgggggt g 49
<210> 157
   <211> 48
   <212> DNA
   <213> Artificial Sequence
<220>
   <223> Primer B- B.s. ilvC (DraIII)
<400> 157
   ctgctgatct cacaccgt gt gttaattttg cgcaacggag accaccgc 48
<210> 158
   <211> 1221
   <212> DNA
   <213> Clostridium acetobutylicum
<400> 158
   cacacggt gt  aaat aat aat  ct aaacagga  ggggt t aaaa  t ggt t gat t t  cgaat at t ca    60
   at accaact a  gaat t t t t t t  cggt aaagat  aagat aaat g  t act t ggaag  agagct t aaa   120
   aaat at ggt t  ct aaagt gct  t at agt t t at  ggt ggaggaa  gt at aaagag  aaat ggaat a   180
   t at gat aaag  ct gt aagt at  act t gaaaaa  aacagt at t a  aat t t t at ga  act t gcagga   240
   gtagagccaa atccaagagt aactacagtt gaaaaaggag ttaaaatatg tagagaaaat     300
   ggagt t gaag  t agt act agc  t at aggt gga  ggaagt gcaa  t agat t gcgc  aaaggt t at a   360
   gcagcagcat gtgaatatga tggaaatcca tgggatattg tgttagatgg ctcaaaaata     420
   aaaagggt gc  t t cct at agc  t agt at at t a  accat t gct g  caacaggat c  agaaat ggat   480
   acgtgggcag taataaataa tatggataca aacgaaaaac taattgcggc acatccagat     540
   at ggct cct a  agt t t t ct at  at t agat cca  acgt at acgt  at accgt acc  t accaat caa   600
   acagcagcag gaacagctga tattatgagt catatatttg aggtgtattt tagtaataca     660
   aaaacagcat atttgcagga tagaatggca gaagcgttat taagaacttg tattaaatat     720
   ggaggaat ag  ct ct t gagaa  gccggat gat  t at gaggcaa  gagccaat ct  aat gt gggct   780
   t caagt ct t g  cgat aaat gg  act t t t aaca  t at ggt aaag  acact aat t g  gagt gt acac   840
   t t aat ggaac  at gaat t aag  t gct t at t ac  gacat aacac  acggcgt agg  gct t gcaat t   900
   t t aacacct a  at t ggat gga  gt at at t t t a  aat aat gat a  cagt gt acaa  gt t t gt t gaa   960
   t at ggt gt aa  at gt t t gggg  aat agacaaa  gaaaaaaat c  act at gacat  agcacat caa  1020
   gcaatacaaa aaacaagaga ttactttgta aatgtactag gtttaccatc tagactgaga    1080
   gat gt t ggaa  t t gaagaaga  aaaat t ggac  at aat ggcaa  aggaat cagt  aaagct t aca  1140
   ggaggaacca taggaaacct aagaccagta aacgcctccg aagtcctaca aatattcaaa    1200
   aaatctgtgt aacaccgagt g                                                1221
<210> 159
   <211> 54
   <212> DNA
   <213> Artificial Sequence
<220>
   <223> Primer T- bdhB (DraIII)
<400> 159
   tcgatagcat acacacggt gttaacaaag gaggggt t aa aat ggt t gat t t cg 54
<210> 160
   <211> 91
   <212> DNA
   <213> Artificial Sequence
<220>
   <223> Primer B- bdhB (r nBT1 DraIII)
<400> 160
   atctacgcac t cggt gat aa aacgaaaggc ccagtctttc gactgagcct ttcgttttat 60
cttacacaga ttttttgaat atttgtagga c 91
<210> 161
   <211> 29
   <212> DNA
   <213> Artifcial Sequence
<220>
   <223> Primer LDH EcoRV F
<400> 161
   gacgtcatga ccacccgccg atccctttt 29
<210> 162
   <211> 30
   <212> DNA
   <213> Artificial Sequence
<220>
   <223> Primer LDH AatIIR
<400> 162
   gatatccaac accagcgacc gacgtattac 30
<210> 163
   <211> 47
   <212> DNA
   <213> Artificial Sequence
<220>
   <223> Pr i rrer Cm F
<400> 163
   atttaaatct cgagtagagg at cccaacaa acgaaaat t gataaag 47
<210> 164
   <211> 29
   <212> DNA
   <213> Artificial Sequence
<220>
   <223> Primer Cm R
<400> 164
   acgcgttatt at aaaagcca gt catt agg 29
<210> 165
   <211> 58
   <212> DNA
   <213> Artificial Sequence
<220>
   <223> Primer P11 F-Stul
<400> 165
   cctagcgcta tagttgttga cagaatggac at act at gat at at t gt t gc tatagcga 58
<210> 166
   <211> 62
   <212> DNA
   <213> Artificial Sequence
<220>
   <223> Primer P11R- SpeI
<400> 166
   ct agt cgct a t agcaacaat at at cat agt at gt ccat t c t gt caacaac t at agcgct a 60
   gg 62
<210> 167
   <211> 38
   <212> DNA
   <213> Artificial Sequence
<220>
   <223> Primer PidhL F- HindIII
<400> 167
   aagct t gt cg acaaaccaac at t at gacgt gt ct gggc 38
<210> 168
   <211> 28
   <212> DNA
   <213> Artificial Sequence
<220>
   <223> Primer PldhL R- BamHI
<400> 168
   ggat cct cat cctctcgtag tgaaaatt 28
<210> 169
   <211> 36
   <212> DNA
   <213> Artificial Sequence
<220>
   <223> Primer F-bdhB-AvrII
<400> 169
   t t cct aggaa ggaggtggtt aaaatggttg atttcg 36
<210> 170
   <211> 29
   <212> DNA
   <213> Artificial Sequence
<220>
   <223> Primer R- bdhB-BamHI
<400> 170
   ttggatcctt acacagatt tttgaatat 29
<210> 171
   <211> 39
   <212> DNA
   <213> Artificial Sequence
<220>
   <223> Primer F-ilvC(B.s.) Af III
<400> 171
   aact t aagaa ggaggtgatt gaaatggtaa aagtatatt 39
<210> 172
   <211> 32
   <212> DNA
   <213> Artificial Sequence
<220>
   <223> Primer R-ilvC(B.s.)-Notl
<400> 172
   aagcggccgc ttaattttgc gcaacggaga cc 32
<210> 173
   <211> 30
   <212> DNA
   <213> Artificial Sequence
<220>
   <223> Primer F- Pni sA( Hi ndIII)
<400> 173
   ttaagcttga catacttgaa tgacctagtc c 30
<210> 174
   <211> 39
   <212> DNA
   <213> Artificial Sequence
<220>
   <223> Primer R- Pni sA(Spel BamHI)
<400> 174
   ttggatccaa actagtataa tttattttgt agttccttc 39
<210> 175
   <211> 38
   <212> DNA
   <213> Artificial Sequence
<220>
   <223> Primer N191
<400> 175
   atccgcggat agat ct ccca t t accgacat t t gggcgc 38
<210> 176
   <211> 31
   <212> DNA
   <213> Artificial Sequence
<220>
   <223> Primer N192
<400> 176
   at cct cagcg af gat t gat t gat t gat t gt a 31
<210> 177
   <211> 6509
   <212> DNA
   <213> Artificial Sequence
<220>
   <223> Vect or pFP988
<400> 177
   t cgaggcccc gcacat acga aaagact ggc t gaaaacat t gagcct t t ga t gact gat ga 60
   tttggctgaa gaagtggatc gattgtttga gaaaagaaga agaccataaa aataccttgt    120
   ct gt cat cag acagggt at t t t t t at gct g t ccagact gt ccgct gt gt a aaaaat agga 180
   at aaaggggg gt t gt t at t a t t t t act gat at gt aaaat a t aat t t gt at aaggaat t gt 240
   gagcggataa caattcctac gaaaatgaga gggagaggaa acatgattca aaaacgaaag    300
   cggacagt t t cgt t cagact t gt gct t at g t gcacgct gt t at t t gt cag t t t gccgat t 360
   acaaaaacat cagccggatc ccaccatcac catcaccatt aagaattcct agaaactcca    420
   agct at ct t t aaaaaat ct a gt aaat gcac gagcaacat c t t t t gt t gct cagt gcat t t 480
   t t t at t t t gt acact agat a t t t ct t ct cc gct t aaat ca t caaagaaat ct t t at cact 540
   t gt aaccagt ccgt ccacat gt cgaat t gc at ct gaccga at t t t acgt t t ccct gaat a 600
   at t ct cat ca at cgt t t cat caat t t t at c t t t at act t t at at t t t gt g cgt t aat caa 660
   at cat aat t t t t at at gt t t cct cat gat t t at gt ct t t a t t at t at agt t t t t at t ct c 720
   tctttgatta tgtctttgta tcccgtttgt attacttgat cctttaactc tggcaaccct 780
   caaaattgaa tgagacatgc tacacctccg gataataaat atatataaac gtatatagat 840
   ttcataaagt ctaacacact agacttattt acttcgtaat taagtcgtta aaccgtgtgc 900
   tctacgacca aaactataaa acctttaaga actttctttt tttacaagaa aaaagaaatt 960
   agataaatct ctcatatctt ttattcaata atcgcatccg attgcagtat aaatttaacg 1020
   atcactcatc atgttcatat ttatcagagc tcgtgctata attatactaa ttttataagg 1080
   aggaaaaaat atgggcattt ttagtatttt tgtaatcagc acagttcatt atcaaccaaa 1140
   caaaaaataa gtggttataa tgaatcgtta ataagcaaaa ttcatataac caaattaaag 1200
   agggttataa tgaacgagaa aaatataaaa cacagtcaaa actttattac ttcaaaacat 1260
   aatatagata aaataatgac aaatataaga ttaaatgaac atgataatat ctttgaaatc 1320
   ggctcaggaa aaggccattt tacccttgaa ttagtaaaga ggtgtaattt cgtaactgcc 1380
   attgaaatag accataaatt atgcaaaact acagaaaata aacttgttga tcacgataat 1440
   ttccaagttt taaacaagga tatattgcag tttaaatttc ctaaaaacca atcctataaa 1500
   atatatggta atatacctta taacataagt acggatataa tacgcaaaat tgtttttgat 1560
   agtatagcta atgagattta tttaatcgtg gaatacgggt ttgctaaaag attattaaat 1620
   acaaaacgct cattggcatt acttttaatg gcagaagttg atatttctat attaagtatg 1680
   gttccaagag aatattttca tcctaaacct aaagtgaata gctcacttat cagattaagt 1740
   agaaaaaaat caagaatatc acacaaagat aaacaaaagt ataattattt cgttatgaaa 1800
   tgggttaaca aagaatacaa gaaaatattt acaaaaaatc aatttaacaa ttccttaaaa 1860
   catgcaggaa ttgacgattt aaacaatatt agctttgaac aattcttatc tcttttcaat 1920
   agctataaat tatttaataa gtaagttaag ggatgcagtt catcgatgaa ggcaactaca 1980
   gctcaggcga caaccatacg ctgagagatc ctcactacgt agaagataaa ggccacaaat 2040
   acttagtatt tgaagcaaac actggaactg aagatggcta ccaaggcgaa gaatctttat 2100
   ttaacaaagc atactatggc aaaagcacat cattcttccg tcaagaaagt caaaaacttc 2160
   tgcaaagcga taaaaaacgc acggctgagt tagcaaacgg cgctctcggt atgattgagc 2220
   taaacgatga ttacacactg aaaaaagtga tgaaaccgct gattgcatct aacacagtaa 2280
   cagatgaaat tgaacgcgcg aacgtcttta aaatgaacgg caaatggtac ctgttcactg 2340
   actcccgcgg atcaaaaatg acgattgacg gcattacgtc taacgatatt tacatgcttg 2400
   gttatgtttc taattcttta actggcccat acaagccgct gaacaaaact ggccttgtgt 2460
   taaaaatgga tcttgatcct aacgatgtaa cctttactta ctcacacttc gctgtacctc 2520
   aagcgaaagg aaacaatgtc gtgattacaa gctatatgac aaacagagga ttctacgcag 2580
   acaaacaatc aacgtttgcg ccaagcttgc atgcgagagt agggaactgc caggcatcaa 2640
   ataaaacgaa aggctcagtc gaaagactgg gcctttcgtt ttatctgttg tttgtcggtg 2700
   aacgctctcc tgagtaggac aaatccgccg ggagcggatt tgaacgttgc gaagcaacgg 2760
   cccggagggt ggcgggcagg acgcccgcca taaactgcca ggcatcaaat taagcagaag 2820
   gccatcctga cggatggcct ttttgcgttt ctacaaactc tttttgttta tttttctaaa 2880
   tacattcaaa tatgtatccg ctcatgctcc ggatctgcat cgcaggatgc tgctggctac 2940
   cctgtggaac acctacatct gtattaacga agcgctggca ttgaccctga gtgatttttc 3000
   tctggtcccg ccgcatccat accgccagtt gtttaccctc acaacgttcc agtaaccggg 3060
   catgttcatc atcagtaacc cgtatcgtga gcatcctctc tcgtttcatc ggtatcatta 3120
   cccccatgaa cagaaattcc cccttacacg gaggcatcaa gtgaccaaac aggaaaaaac 3180
   cgcccttaac atggcccgct ttatcagaag ccagacatta acgcttctgg agaaactcaa 3240
   cgagctggac gcggatgaac aggcagacat ctgtgaatcg cttcacgacc acgctgatga 3300
   gctttaccgc agctgcctcg cgcgtttcgg tgatgacggt gaaaacctct gacacatgca 3360
   gctcccggag acggtcacag cttgtctgta agcggatgcc gggagcagac aagcccgtca 3420
   gggcgcgtca gcgggtgttg gcgggtgtcg gggcgcagcc atgacccagt cacgtagcga 3480
   tagcggagtg tatactggct taactatgcg gcatcagagc agattgtact gagagtgcac 3540
   catatgcggt gtgaaatacc gcacagatgc gtaaggagaa aataccgcat caggcgctct 3600
   tccgcttcct cgctcactga ctcgctgcgc tcggtcgttc ggctgcggcg agcggtatca 3660
   gctcactcaa aggcggtaat acggttatcc acagaatcag gggataacgc aggaaagaac 3720
   atgtgagcaa aaggccagca aaaggccagg aaccgtaaaa aggccgcgtt gctggcgttt 3780
   ttccataggc tccgcccccc tgacgagcat cacaaaaatc gacgctcaag tcagaggtgg 3840
   cgaaacccga caggactata aagataccag gcgtttcccc ctggaagctc cctcgtgcgc 3900
   tctcctgttc cgaccctgcc gcttaccgga tacctgtccg cctttctccc ttcgggaagc 3960
   gtggcgcttt ctcaatgctc acgctgtagg tatctcagtt cggtgtaggt cgttcgctcc 4020
   aagctgggct gtgtgcacga accccccgtt cagcccgacc gctgcgcctt atccggtaac 4080
   tatcgtcttg agtccaaccc ggtaagacac gacttatcgc cactggcagc agccactggt 4140
   aacaggatta gcagagcgag gtatgtaggc ggtgctacag agttcttgaa gtggtggcct 4200
   aactacggct acactagaag gacagtattt ggtatctgcg ctctgctgaa gccagttacc 4260
   ttcggaaaaa gagttggtag ctcttgatcc ggcaaacaaa ccaccgctgg tagcggtggt 4320
   ttttttgttt gcaagcagca gattacgcgc agaaaaaaag gatctcaaga agatcctttg 4380
   atcttttcta cggggtctga cgctcagtgg aacgaaaact cacgttaagg gattttggtc 4440
   atgagattat caaaaaggat cttcacctag atccttttaa attaaaaatg aagttttaaa 4500
   tcaatctaaa gtatatatga gtaaacttgg tctgacagtt accaatgctt aatcagtgag 4560
   gcacctatct cagcgatctg tctatttcgt tcatccatag ttgcctgact ccccgtcgtg 4620
   tagataacta cgatacggga gggcttacca tctggcccca gtgctgcaat gataccgcga 4680
   gacccacgct caccggctcc agatttatca gcaataaacc agccagccgg aagggccgag 4740
   cgcagaagtg gtcctgcaac tttatccgcc tccatccagt ctattaattg ttgccgggaa 4800
   gctagagtaa gtagttcgcc agttaatagt ttgcgcaacg ttgttgccat tgctgcaggc 4860
   atcgtggtgt cacgctcgtc gtttggtatg gcttcattca gctccggttc ccaacgatca 4920
   aggcgagtta catgatcccc catgttgtgc aaaaaagcgg ttagctcctt cggtcctccg 4980
   atcgttgtca gaagtaagtt ggccgcagtg ttatcactca tggttatggc agcactgcat 5040
   aattctctta ctgtcatgcc atccgtaaga tgcttttctg tgactggtga gtactcaacc 5100
   aagtcattct gagaatagtg tatgcggcga ccgagttgct cttgcccggc gtcaatacgg 5160
   gataataccg cgccacatag cagaacttta aaagtgctca tcattggaaa acgttcttcg 5220
   gggcgaaaac tctcaaggat cttaccgctg ttgagatcca gttcgatgta acccactcgt 5280
   gcacccaact gatcttcagc atcttttact ttcaccagcg tttctgggtg agcaaaaaca 5340
   ggaaggcaaa atgccgcaaa aaagggaata agggcgacac ggaaatgttg aatactcata 5400
   ctcttccttt ttcaatatta ttgaagcatt tatcagggtt attgtctcat gagcggatac 5460
   atatttgaat gtatttagaa aaataaacaa ataggggttc cgcgcacatt tccccgaaaa 5520
   gtgccacctg acgtctaaga aaccattatt atcatgacat taacctataa aaataggcgt 5580
   atcacgaggc cctttcgtct cgcgcgtttc ggtgatgacg gtgaaaacct ctgacacatg 5640
   cagctcccgg agacggtcac agcttgtctg taagcggatg ccgggagcag acaagcccgt 5700
   cagggcgcgt cagcgggtgt tcatgtgcgt aactaacttg ccatcttcaa acaggagggc 5760
   tggaagaagc agaccgctaa cacagtacat aaaaaaggag acatgaacga tgaacatcaa 5820
   aaagtttgca aaacaagcaa cagtattaac ctttactacc gcactgctgg caggaggcgc 5880
   aactcaagcg tttgcgaaag aaacgaacca aaagccatat aaggaaacat acggcatttc 5940
   ccatattaca cgccatgata tgctgcaaat ccctgaacag caaaaaaatg aaaaatatca 6000
   agttcctgaa ttcgattcgt ccacaattaa aaatatctct tctgcaaaag gcctggacgt 6060
   ttgggacagc tggccattac aaaacgctga cggcactgtc gcaaactatc acggctacca 6120
   catcgtcttt gcattagccg gagatcctaa aaatgcggat gacacatcga tttacatgtt 6180
   ctatcaaaaa gtcggcgaaa cttctattga cagctggaaa aacgctggcc gcgtctttaa 6240
   agacagcgac aaattcgatg caaatgattc tatcctaaaa gaccaaacac aagaatggtc 6300
   aggttcagcc acatttacat ctgacggaaa aatccgttta ttctacactg atttctccgg 6360
   taaacattac ggcaaacaaa cactgacaac tgcacaagtt aacgtatcag catcagacag 6420
   ctctttgaac atcaacggtg tagaggatta taaatcaatc tttgacggtg acggaaaaac 6480
   gtatcaaaat gtacagcatg ccacgcgtc 6509

<210> 178
   <211> 571
   <212> PRT
   <213> Bacillus subtilis
<400> 178
   Met Leu Thr Lys Ala Thr Lys Glu Gln Lys Ser Leu Val Lys Asn Arg
   1 5 10 15
   Gly Ala Glu Leu Val Val Asp Cys Leu Val Glu Gln Gly Val Thr His
   20 25 30
   Val Phe Gly Ile Pro Gly Ala Lys Ile Asp Ala Val Phe Asp Ala Leu
   35 40 45
   Gln Asp Lys Gly Pro Glu Ile Ile Val Ala Arg His Glu Gln Asn Ala
   50 55 60
   Ala Phe Met Ala Gln Ala Val Gly Arg Leu Thr Gly Lys Pro Gly Val
   65 70 75 80
   Val Leu Val Thr Ser Gly Pro Gly Ala Ser Asn Leu Ala Thr Gly Leu
   85 90 95
   Leu Thr Ala Asn Thr Glu Gly Asp Pro Val Val Ala Leu Ala Gly Asn
   100 105 110
   Val Ile Arg Ala Asp Arg Leu Lys Arg Thr His Gln Ser Leu Asp Asn
   115 120 125
   Ala Ala Leu Phe Gln Pro Ile Thr Lys Tyr Ser Val Glu Val Gln Asp
   130 135 140
   Val Lys Asn Ile Pro Glu Ala Val Thr Asn Ala Phe Arg Ile Ala Ser
   145 150 155 160
   Ala Gly Gln Ala Gly Ala Ala Phe Val Ser Phe Pro Gln Asp Val Val
   165 170 175
   Asn Glu Val Thr Asn Thr Lys Asn Val Arg Ala Val Ala Ala Pro Lys
   180 185 190
   Leu Gly Pro Ala Ala Asp Asp Ala Ile Ser Ala Ala Ile Ala Lys Ile
   195 200 205
   Gln Thr Ala Lys Leu Pro Val Val Leu Val Gly Met Lys Gly Gly Arg
   210 215 220
   Pro Glu Ala Ile Lys Ala Val Arg Lys Leu Leu Lys Lys Val Gln Leu
   225 230 235 240
   Pro Phe Val Glu Thr Tyr Gln Ala Ala Gly Thr Leu Ser Arg Asp Leu
   245 250 255
   Glu Asp Gln Tyr Phe Gly Arg Ile Gly Leu Phe Arg Asn Gln Pro Gly
   260 265 270
   Asp Leu Leu Leu Glu Gln Ala Asp Val Val Leu Thr Ile Gly Tyr Asp
   275 280 285
   Pro Ile Glu Tyr Asp Pro Lys Phe Trp Asn Ile Asn Gly Asp Arg Thr
   290 295 300
   Ile Ile His Leu Asp Glu Ile Ile Ala Asp Ile Asp His Ala Tyr Gln
   305 310 315 320
   Pro Asp Leu Glu Leu Ile Gly Asp Ile Pro Ser Thr Ile Asn His Ile
   325 330 335
   Glu His Asp Ala Val Lys Val Glu Phe Ala Glu Arg Glu Gln Lys Ile
   340 345 350
   Leu Ser Asp Leu Lys Gln Tyr Met His Glu Gly Glu Gln Val Pro Ala
   355 360 365
   Asp Trp Lys Ser Asp Arg Ala His Pro Leu Glu Ile Val Lys Glu Leu
   370 375 380
   Arg Asn Ala Val Asp Asp His Val Thr Val Thr Cys Asp Ile Gly Ser
   385 390 395 400
   His Ala Ile Trp Met Ser Arg Tyr Phe Arg Ser Tyr Glu Pro Leu Thr
   405 410 415
   Leu Met Ile Ser Asn Gly Met Gln Thr Leu Gly Val Ala Leu Pro Trp
   420 425 430
   Ala Ile Gly Ala Ser Leu Val Lys Pro Gly Glu Lys Val Val Ser Val
   435 440 445
   Ser Gly Asp Gly Gly Phe Leu Phe Ser Ala Met Glu Leu Glu Thr Ala
   450 455 460
   Val Arg Leu Lys Ala Pro Ile Val His Ile Val Trp Asn Asp Ser Thr
   465 470 475 480
   Tyr Asp Met Val Ala Phe Gln Gln Leu Lys Lys Tyr Asn Arg Thr Ser
   485 490 495
   Ala Val Asp Phe Gly Asn Ile Asp Ile Val Lys Tyr Ala Glu Ser Phe
   500 505 510
   Gly Ala Thr Gly Leu Arg Val Glu Ser Pro Asp Gln Leu Ala Asp Val
   515 520 525
   Leu Arg Gln Gly Met Asn Ala Glu Gly Pro Val Ile Ile Asp Val Pro
   530 535 540
   Val Asp Tyr Ser Asp Asn Ile Asn Leu Ala Ser Asp Lys Leu Pro Lys
   545 550 555 560
   Glu Phe Gly Glu Leu Met Lys Thr Lys Ala Leu
   565 570
<210> 179
   <211> 1665
   <212> DNA
   <213> Lactococcus lactis
<400> 179
   atgtctgaga aacaatttgg ggcgaacttg gttgtcgata gtttgattaa ccataaagtg      60
   aagtatgtat ttgggattcc aggagcaaaa attgaccggg tttttgattt attagaaaat     120
   gaagaaggcc ctcaaatggt cgtgactcgt catgagcaag gagctgcttt catggctcaa     180
   gctgtcggtc gtttaactgg cgaacctggt gtagtagttg ttacgagtgg gcctggtgta     240
   tcaaaccttg cgactccgct tttgaccgcg acatcagaag gtgatgctat tttggctatc     300
   ggtggacaag ttaaacgaag tgaccgtctt aaacgtgcgc accaatcaat ggataatgct     360
   ggaatgatgc aatcagcaac aaaatattca gcagaagttc ttgaccctaa tacactttct     420
   gaatcaattg ccaacgctta tcgtattgca aaatcaggac atccaggtgc aactttctta     480
   tcaatccccc aagatgtaac ggatgccgaa gtatcaatca aagccattca accactttca     540
   gaccctaaaa tggggaatgc ctctattgat gacattaatt atttagcaca agcaattaaa     600
   aatgctgtat tgccagtaat tttggttgga gctggtgctt cagatgctaa agtcgcttca     660
   tccttgcgta atctattgac tcatgttaat attcctgtcg ttgaaacatt ccaaggtgca     720
   ggggttattt cacatgattt agaacatact ttttatggac gtatcggtct tttccgcaat     780
   caaccaggcg atatgcttct gaaacgttct gaccttgtta ttgctgttgg ttatgaccca     840
   attgaatatg aagctcgtaa ctggaatgca gaaattgata gtcgaattat cgttattgat     900
   aatgccattg ctgaaattga tacttactac caaccagagc gtgaattaat tggtgatatc     960
   gcagcaacat tggataatct tttaccagct gttcgtggct acaaaattcc aaaaggaaca    1020
   aaagattatc tcgatggcct tcatgaagtt gctgagcaac acgaatttga tactgaaaat    1080
   actgaagaag gtagaatgca ccctcttgat ttggtcagca ctttccaaga aatcgtcaag    1140
   gatgatgaaa cagtaaccgt tgacgtaggt tcactctaca tttggatggc acgtcatttc    1200
   aaatcatacg aaccacgtca tctcctcttc tcaaacggaa tgcaaacact cggagttgca    1260
   cttccttggg caattacagc cgcattgttg cgcccaggta aaaaagttta ttcacactct    1320
   ggtgatggag gcttcctttt cacagggcaa gaattggaaa cagctgtacg tttgaatctt    1380
   ccaatcgttc aaattatctg gaatgacggc cattatgata tggttaaatt ccaagaagaa    1440
   atgaaatatg gtcgttcagc agccgttgat tttggctatg ttgattacgt aaaatatgct    1500
   gaagcaatga gagcaaaagg ttaccgtgca cacagcaaag aagaacttgc tgaaattctc    1560
   aaatcaatcc cagatactac tggaccggtg gtaattgacg ttcctttgga ctattctgat    1620
   aacattaaat tagcagaaaa attattgcct gaagagtttt attga                    1665
<210> 180
   <211> > 554
   <212> PRT
   <213> Lact ococcus lactis
<400> 180
<210> 181
   <211> 395
   <212> PRT
   <213> Saccharomyces cerevisiae
<400> 181
   Met Leu Arg Thr Gln Ala Ala Arg Leu Ile Cys Asn Ser Arg Val Ile
   1               5                   10                  15
   Thr Ala Lys Arg Thr Phe Ala Leu Ala Thr Arg Ala Ala Ala Tyr Ser
               20                  25                  30
   Arg Pro Ala Ala Arg Phe Val Lys Pro Met Ile Thr Thr Arg Gly Leu
           35                  40                  45
   Lys Gln Ile Asn Phe Gly Gly Thr Val Glu Thr Val Tyr Glu Arg Ala
       50                  55                  60
   Asp Trp Pro Arg Glu Lys Leu Leu Asp Tyr Phe Lys Asn Asp Thr Phe
   65                  70                  75                  80
   Ala Leu Ile Gly Tyr Gly Ser Gln Gly Tyr Gly Gln Gly Leu Asn Leu
                   85                  90                  95
   Arg Asp Asn Gly Leu Asn Val Ile Ile Gly Val Arg Lys Asp Gly Ala
               100                 105                 110
   Ser Trp Lys Ala Ala Ile Glu Asp Gly Trp Val Pro Gly Lys Asn Leu
           115                 120                 125
   Phe Thr Val Glu Asp Ala Ile Lys Arg Gly Ser Tyr Val Met Asn Leu
       130                 135                 140
   Leu Ser Asp Ala Ala Gln Ser Glu Thr Trp Pro Ala Ile Lys Pro Leu
   145                 150                 155                 160
   Leu Thr Lys Gly Lys Thr Leu Tyr Phe Ser His Gly Phe Ser Pro Val
                   165                 170                 175
   Phe Lys Asp Leu Thr His Val Glu Pro Pro Lys Asp Leu Asp Val Ile
               180                 185                 190
   Leu Val Ala Pro Lys Gly Ser Gly Arg Thr Val Arg Ser Leu Phe Lys
           195                 200                 205
   Glu Gly Arg Gly Ile Asn Ser Ser Tyr Ala Val Trp Asn Asp Val Thr
       210                 215                 220
   Gly Lys Ala His Glu Lys Ala Gln Ala Leu Ala Val Ala Ile Gly Ser
   225 230 235 240
   Gly Tyr Val Tyr Gln Thr Thr Phe Glu Arg Glu Val Asn Ser Asp Leu
   245 250 255
   Tyr Gly Glu Arg Gly Cys Leu Met Gly Gly Ile His Gly Met Phe Leu
   260 265 270
   Ala Gln Tyr Asp Val Leu Arg Glu Asn Gly His Ser Pro Ser Glu Ala
   275 280 285
   Phe Asn Glu Thr Val Glu Glu Ala Thr Gln Ser Leu Tyr Pro Leu Ile
   290 295 300
   Gly Lys Tyr Gly Met Asp Tyr Met Tyr Asp Ala Cys Ser Thr Thr Ala
   305 310 315 320
   Arg Arg Gly Ala Leu Asp Trp Tyr Pro Ile Phe Lys Asn Ala Leu Lys
   325 330 335
   Pro Val Phe Gln Asp Leu Tyr Glu Ser Thr Lys Asn Gly Thr Glu Thr
   340 345 350
   Lys Arg Ser Leu Glu Phe Asn Ser Gln Pro Asp Tyr Arg Glu Lys Leu
   355 360 365
   Glu Lys Glu Leu Asp Thr Ile Arg Asn Met Glu Ile Trp Lys Val Gly
   370 375 380
   Lys Glu Val Arg Lys Leu Arg Pro Glu Asn Gln
   385 390 395
<210> 182
   <211> 993
   <212> DNA
   <213> Methanococcus maripaludis
<400> 182
   atgaaggtat tctatgactc agattttaaa ttagatgctt taaaagaaaa aacaattgca 60
   gtaatcggtt atggaagtca aggtagggca cagtccttaa acatgaaaga cagcggatta 120
   aacgttgttg ttggtttaag aaaaaacggt gcttcatgga acaacgctaa agcagacggt 180
   cacaatgtaa tgaccattga agaagctgct gaaaaagcgg acatcatcca catcttaata 240
   cctgatgaat tacaggcaga agtttatgaa agccagataa aaccatacct aaaagaagga 300
   aaaacactaa gcttttcaca tggttttaac atccactatg gattcattgt tccaccaaaa 360
   ggagttaacg tggttttagt tgctccaaaa tcacctggaa aaatggttag aagaacatac 420
   gaagaaggtt tcggtgttcc aggtttaatc tgtattgaaa ttgatgcaac aaacaacgca 480
   tttgatattg tttcagcaat ggcaaaagga atcggtttat caagagctgg agttatccag 540
   acaactttca aagaagaaac agaaactgac cttttcggtg aacaagctgt tttatgcggt 600
   ggagttaccg aattaatcaa ggcaggattt gaaacactcg ttgaagcagg atacgcacca    660
   gaaatggcat actttgaaac ctgccacgaa ttgaaattaa tcgttgactt aatctaccaa    720
   aaaggattca aaaacatgtg gaacgatgta agtaacactg cagaatacgg cggacttaca    780
   agaagaagca gaatcgttac agctgattca aaagctgcaa tgaaagaaat cttaagagaa    840
   atccaagatg gaagattcac aaaagaattc cttctcgaaa aacaggtaag ctatgctcat    900
   ttaaaatcaa tgagaagact cgaaggagac ttacaaatcg aagaagtcgg cgcaaaatta    960
   agaaaaatgt gcggtcttga aaaagaagaa taa                                 993
<210> 183
   <211> 330
   <212> PRT
   <213> Methanococcus maripaludis
<400> 183
   Met Lys Val Phe Tyr Asp Ser Asp Phe Lys Leu Asp Ala Leu Lys Glu
   1               5                   10                  15
   Lys Thr Ile Ala Val Ile Gly Tyr Gly Ser Gln Gly Arg Ala Gln Ser
               20                  25                  30
   Leu Asn Met Lys Asp Ser Gly Leu Asn Val Val Val Gly Leu Arg Lys
           35                  40                  45
   Asn Gly Ala Ser Trp Asn Asn Ala Lys Ala Asp Gly His Asn Val Met
       50                  55                  60
   Thr Ile Glu Glu Ala Ala Glu Lys Ala Asp Ile Ile His Ile Leu Ile
   65                  70                  75                  80
   Pro Asp Glu Leu Gln Ala Glu Val Tyr Glu Ser Gln Ile Lys Pro Tyr
                   85                  90                  95
   Leu Lys Glu Gly Lys Thr Leu Ser Phe Ser His Gly Phe Asn Ile His
               100                 105                 110
   Tyr Gly Phe Ile Val Pro Pro Lys Gly Val Asn Val Val Leu Val Ala
           115                 120                 125
   Pro Lys Ser Pro Gly Lys Met Val Arg Arg Thr Tyr Glu Glu Gly Phe
       130                 135                 140
   Gly Val Pro Gly Leu Ile Cys Ile Glu Ile Asp Ala Thr Asn Asn Ala
   145                 150                 155                 160
   Phe Asp Ile Val Ser Ala Met Ala Lys Gly Ile Gly Leu Ser Arg Ala
                   165                 170                 175
   Gly Val Ile Gln Thr Thr Phe Lys Glu Glu Thr Glu Thr Asp Leu Phe
               180                 185                 190
   Gly Glu Gln Ala Val Leu Cys Gly Gly Val Thr Glu Leu Ile Lys Ala
   195 200 205
   Gly Phe Glu Thr Leu Val Glu Ala Gly Tyr Ala Pro Glu Met Ala Tyr
   210 215 220
   Phe Glu Thr Cys His Glu Leu Lys Leu Ile Val Asp Leu Ile Tyr Gln
   225 230 235 240
   Lys Gly Phe Lys Asn Met Trp Asn Asp Val Ser Asn Thr Ala Glu Tyr
   245 250 255
   Gly Gly Leu Thr Arg Arg Ser Arg Ile Val Thr Ala Asp Ser Lys Ala
   260 265 270
   Ala Met Lys Glu Ile Leu Arg Glu Ile Gln Asp Gly Arg Phe Thr Lys
   275 280 285
   Glu Phe Leu Leu Glu Lys Gln Val Ser Tyr Ala His Leu Lys Ser Met
   290 295 300
   Arg Arg Leu Glu Gly Asp Leu Gln Ile Glu Glu Val Gly Ala Lys Leu
   305 310 315 320
   Arg Lys Met Cys Gly Leu Glu Lys Glu Glu
   325 330
<210> 184
   <211> 1476
   <212> DIVA
   <213> Bacillus subtilis
<400> 184
   atggctaact acttcaatac actgaatctg cgccagcagc tggcacagct gggcaaatgt 60
   cgctttatgg gccgcgatga attcgccgat ggcgcgagct accttcaggg taaaaaagta 120
   gtcatcgtcg gctgtggcgc acagggtctg aaccagggcc tgaacatgcg tgattctggt 180
   ctcgatatct cctacgctct gcgtaaagaa gcgattgccg agaagcgcgc gtcctggcgt 240
   aaagcgaccg aaaatggttt taaagtgggt acttacgaag aactgatccc acaggcggat 300
   ctggtgatta acctgacgcc ggacaagcag cactctgatg tagtgcgcac cgtacagcca 360
   ctgatgaaag acggcgcggc gctgggctac tcgcacggtt tcaacatcgt cgaagtgggc 420
   gagcagatcc gtaaagatat caccgtagtg atggttgcgc cgaaatgccc aggcaccgaa 480
   gtgcgtgaag agtacaaacg tgggttcggc gtaccgacgc tgattgccgt tcacccggaa 540
   aacgatccga aaggcgaagg catggcgatt gccaaagcct gggcggctgc aaccggtggt 600
   caccgtgcgg gtgtgctgga atcgtccttc gttgcggaag tgaaatctga cctgatgggc 660
   gagcaaacca tcctgtgcgg tatgttgcag gctggctctc tgctgtgctt cgacaagctg 720
   gtggaagaag gtaccgatcc agcatacgca gaaaaactga ttcagttcgg ttgggaaacc 780
   atcaccgaag cactgaaaca gggcggcatc accctgatga tggaccgtct ctctaacccg 840
   gcgaaactgc gtgcttatgc gctttctgaa cagctgaaag agatcatggc acccctgttc 900
   cagaaacata tggacgacat catctccggc gaattctctt ccggtatgat ggcggactgg 960
   gccaacgatg ataagaaact gctgacctgg cgtgaagaga ccggcaaaac cgcgtttgaa 1020
   accgcgccgc agtatgaagg caaaatcggc gagcaggagt acttcgataa aggcgtactg 1080
   atgattgcga tggtgaaagc gggcgttgaa ctggcgttcg aaaccatggt cgattccggc 1140
   atcattgaag agtctgcata ttatgaatca ctgcacgagc tgccgctgat tgccaacacc 1200
   atcgcccgta agcgtctgta cgaaatgaac gtggttatct ctgataccgc tgagtacggt 1260
   aactatctgt tctcttacgc ttgtgtgccg ttgctgaaac cgtttatggc agagctgcaa 1320
   ccgggcgacc tgggtaaagc tattccggaa ggcgcggtag ataacgggca actgcgtgat 1380
   gtgaacgaag cgattcgcag ccatgcgatt gagcaggtag gtaagaaact gcgcggctat 1440
   atgacagata tgaaacgtat tgctgttgcg ggttaa 1476
<210> 185
   <211> 342
   <212> PRT
   <213> Bacillus subtilis
<400> 185
   Met Val Lys Val Tyr Tyr Asn Gly Asp Ile Lys Glu Asn Val Leu Ala
   1               5                   10                  15
   Gly Lys Thr Val Ala Val Ile Gly Tyr Gly Ser Gln Gly His Ala His
               20                  25                  30
   Ala Leu Asn Leu Lys Glu Ser Gly Val Asp Val Ile Val Gly Val Arg
           35                  40                  45
   Gln Gly Lys Ser Phe Thr Gln Ala Gln Glu Asp Gly His Lys Val Phe
       50                  55                  60
   Ser Val Lys Glu Ala Ala Ala Gln Ala Glu Ile Ile Met Val Leu Leu
   65                  70                  75                  80
   Pro Asp Glu Gln Gln Gln Lys Val Tyr Glu Ala Glu Ile Lys Asp Glu
                   85                  90                  95
   Leu Thr Ala Gly Lys Ser Leu Val Phe Ala His Gly Phe Asn Val His
               100                 105                 110
   Phe His Gln Ile Val Pro Pro Ala Asp Val Asp Val Phe Leu Val Ala
           115                 120                 125
   Pro Lys Gly Pro Gly His Leu Val Arg Arg Thr Tyr Glu Gln Gly Ala
       130                 135                 140
   Gly Val Pro Ala Leu Phe Ala Ile Tyr Gln Asp Val Thr Gly Glu Ala
   145 150 155 160
   Arg Asp Lys Ala Leu Ala Tyr Ala Lys Gly Ile Gly Gly Ala Arg Ala
   165 170 175
   Gly Val Leu Glu Thr Thr Phe Lys Glu Glu Thr Glu Thr Asp Leu Phe
   180 185 190
   Gly Glu Gln Ala Val Leu Cys Gly Gly Leu Ser Ala Leu Val Lys Ala
   195 200 205
   Gly Phe Glu Thr Leu Thr Glu Ala Gly Tyr Gln Pro Glu Leu Ala Tyr
   210 215 220
   Phe Glu Cys Leu His Glu Leu Lys Leu Ile Val Asp Leu Met Tyr Glu
   225 230 235 240
   Glu Gly Leu Ala Gly Met Arg Tyr Ser Ile Ser Asp Thr Ala Gln Trp
   245 250 255
   Gly Asp Phe Val Ser Gly Pro Arg Val Val Asp Ala Lys Val Lys Glu
   260 265 270
   Ser Met Lys Glu Val Leu Lys Asp Ile Gln Asn Gly Thr Phe Ala Lys
   275 280 285
   Glu Trp Ile Val Glu Asn Gln Val Asn Arg Pro Arg Phe Asn Ala Ile
   290 295 300
   Asn Ala Ser Glu Asn Glu His Gln Ile Glu Val Val Gly Arg Lys Leu
   305 310 315 320
   Arg Glu Met Met Pro Phe Val Lys Gln Gly Lys Lys Lys Glu Ala Val
   325 330 335
   Val Ser Val Ala Gln Asn
   340
<210> 186
   <211> 585
   <212> PRT
   <213> Saccharomyces cer evi si ae
<400> 186
   Met Gly Leu Leu Thr Lys Val Ala Thr Ser Arg Gln Phe Ser Thr Thr
   1 5 10 15
   Arg Cys Val Ala Lys Lys Leu Asn Lys Tyr Ser Tyr Ile Ile Thr Glu
   20 25 30
   Pro Lys Gly Gln Gly Ala Ser Gln Ala Met Leu Tyr Ala Thr Gly Phe
   35 40 45
   Lys Lys Glu Asp Phe Lys Lys Pro Gln Val Gly Val Gly Ser Cys Trp
   50 55 60
   Trp Ser Gly Asn Pro Cys Asn Met His Leu Leu Asp Leu Asn Asn Arg
   65 70 75 80
   Cys Ser Gln Ser Ile Glu Lys Ala Gly Leu Lys Ala Met Gln Phe Asn
   85 90 95
   Thr Ile Gly Val Ser Asp Gly Ile Ser Met Gly Thr Lys Gly Met Arg
   100 105 110
   Tyr Ser Leu Gln Ser Arg Glu Ile Ile Ala Asp Ser Phe Glu Thr Ile
   115 120 125
   Met Met Ala Gln His Tyr Asp Ala Asn Ile Ala Ile Pro Ser Cys Asp
   130 135 140
   Lys Asn Met Pro Gly Val Met Met Ala Met Gly Arg His Asn Arg Pro
   145 150 155 160
   Ser Ile Met Val Tyr Gly Gly Thr Ile Leu Pro Gly His Pro Thr Cys
   165 170 175
   Gly Ser Ser Lys Ile Ser Lys Asn Ile Asp Ile Val Ser Ala Phe Gln
   180 185 190
   Ser Tyr Gly Glu Tyr Ile Ser Lys Gln Phe Thr Glu Glu Glu Arg Glu
   195 200 205
   Asp Val Val Glu His Ala Cys Pro Gly Pro Gly Ser Cys Gly Gly Met
   210 215 220
   Tyr Thr Ala Asn Thr Met Ala Ser Ala Ala Glu Val Leu Gly Leu Thr
   225 230 235 240
   Ile Pro Asn Ser Ser Ser Phe Pro Ala Val Ser Lys Glu Lys Leu Ala
   245 250 255
   Glu Cys Asp Asn Ile Gly Glu Tyr Ile Lys Lys Thr Met Glu Leu Gly
   260 265 270
   Ile Leu Pro Arg Asp Ile Leu Thr Lys Glu Ala Phe Glu Asn Ala Ile
   275 280 285
   Thr Tyr Val Val Ala Thr Gly Gly Ser Thr Asn Ala Val Leu His Leu
   290 295 300
   Val Ala Val Ala His Ser Ala Gly Val Lys Leu Ser Pro Asp Asp Phe
   305 310 315 320
   Gln Arg Ile Ser Asp Thr Thr Pro Leu Ile Gly Asp Phe Lys Pro Ser
   325 330 335
   Gly Lys Tyr Val Met Ala Asp Leu Ile Asn Val Gly Gly Thr Gln Ser
   340 345 350
   Val Ile Lys Tyr Leu Tyr Glu Asn Asn Met Leu His Gly Asn Thr Met
   355 360 365
   Thr Val Thr Gly Asp Thr Leu Ala Glu Arg Ala Lys Lys Ala Pro Ser
   370 375 380
   Leu Pro Glu Gly Gln Glu Ile Ile Lys Pro Leu Ser His Pro Ile Lys
   385 390 395 400
   Ala Asn Gly His Leu Gln Ile Leu Tyr Gly Ser Leu Ala Pro Gly Gly
   405 410 415
   Ala Val Gly Lys Ile Thr Gly Lys Glu Gly Thr Tyr Phe Lys Gly Arg
   420 425 430
   Ala Arg Val Phe Glu Glu Glu Gly Ala Phe Ile Glu Ala Leu Glu Arg
   435 440 445
   Gly Glu Ile Lys Lys Gly Glu Lys Thr Val Val Val Ile Arg Tyr Glu
   450 455 460
   Gly Pro Arg Gly Ala Pro Gly Met Pro Glu Met Leu Lys Pro Ser Ser
   465 470 475 480
   Ala Leu Met Gly Tyr Gly Leu Gly Lys Asp Val Ala Leu Leu Thr Asp
   485 490 495
   Gly Arg Phe Ser Gly Gly Ser His Gly Phe Leu Ile Gly His Ile Val
   500 505 510
   Pro Glu Ala Ala Glu Gly Gly Pro Ile Gly Leu Val Arg Asp Gly Asp
   515 520 525
   Glu Ile Ile Ile Asp Ala Asp Asn Asn Lys Ile Asp Leu Leu Val Ser
   530 535 540
   Asp Lys Glu Met Ala Gln Arg Lys Gln Ser Trp Val Ala Pro Pro Pro
   545 550 555 560
   Arg Tyr Thr Arg Gly Thr Leu Ser Lys Tyr Ala Lys Leu Val Ser Asn
   565 570 575
   Ala Ser Asn Gly Cys Val Leu Asp Ala
   580 585
<210> 187
   <211> 1653
   <212> DNA
   <213> Methanococcus maripal udi s
<400> 187
   atgataagtg ataacgtcaa aaagggagtt ataagaactc caaaccgagc tcttttaaag      60
   gct t gcggat at acagacga agacat ggaa aaaccat t t a t t ggaat t gt aaacagct t t 120
   acagaagttg ttcccggcca cattcactta agaacattat cagaagcggc taaacatggt     180
   gt t t at gcaa acggt ggaac accat t t gaa t t t aat acca t t ggaat t t g cgacggt at t 240
   gcaat gggcc acgaaggt at gaaat act ct t t acct t caa gagaaat t at t gcagacgct 300
   gt t gaat caa t ggcaagagc acat ggat t t gat ggt ct t g t t t t aat t cc t acgt gt gat 360
   aaaat cgt t c ct ggaat gat aat gggt gct t t aagact aa acat t ccat t t at t gt agt t 420
   act ggaggac caat gct t cc cggagaat t c caaggt aaaa aat acgaact t at cagcct t 480
   t t t gaaggt g t cggagaat a ccaagt t gga aaaat t act g aagaagagt t aaagt gcat t 540
   gaagact gt g cat gt t cagg t gct ggaagt t gt gcagggc t t t acact gc aaacagt at g 600
   gcct gcct t a cagaagct t t gggact ct ct ct t ccaat gt gt gcaacaac gcat gcagt t 660
   gat gcccaaa aagt t aggct t gct aaaaaa agt ggct caa aaat t gt t ga t at ggt aaaa 720
   gaagacctaa aaccaacaga catattaaca aaagaagctt ttgaaaatgc tattttagtt     780
   gaccttgcac ttggtggatc aacaaacaca acattacaca ttcctgcaat tgcaaatgaa     840
   at t gaaaat a aat t cat aac t ct cgat gac t t t gacaggt t aagcgat ga agt t ccacac 900
   at t gcat caa t caaaccagg t ggagaacac t acat gat t g at t t acacaa t gct ggaggt 960
   at t cct gcgg t at t gaacgt t t t aaaagaa aaaat t agag at acaaaaac agt t gat gga 1020
   agaagcat t t t ggaaat cgc agaat ct gt t aaat acat aa at t acgacgt t at aagaaaa 1080
   gt ggaagct c cggt t cacga aact gct ggt t t aagggt t t t aaagggaaa t ct t gct cca 1140
   aacggt t gcg t t gt aaaaat cggt gcagt a cat ccgaaaa t gt acaaaca cgat ggacct 1200
   gcaaaagt t t acaat t ccga agat gaagca at t t ct gcga t act t ggcgg aaaaat t gt a 1260
   gaaggggacg ttatagtaat cagatacgaa ggaccatcag gaggccctgg aatgagagaa    1320
   at gct ct ccc caact t cagc aat ct gt gga at gggt ct t g at gacagcgt t gcat t gat t 1380
   act gat ggaa gat t cagt gg t ggaagt agg ggcccat gt a t cggacacgt t t ct ccagaa 1440
   gctgcagctg gcggagtaat tgctgcaatt gaaaacgggg atatcatcaa aatcgacatg    1500
   at t gaaaaag aaat aaat gt t gat t t agat gaat cagt ca t t aaagaaag act ct caaaa 1560
   ct gggagaat t t gagcct aa aat caaaaaa ggct at t t at caagat act c aaaact t gt c 1620
   tcatctgctg acgaaggggc agttttaaaa taa                                 1653
<210> 188
   <211> 550
   <212> PRT
   <213> Methanococcus maripaludis
<400> 188
   Met Ile Ser Asp Asn Val Lys Lys Gly Val Ile Arg Thr Pro Asn Arg
   1 5 10 15
   Ala Leu Leu Lys Ala Cys Gly Tyr Thr Asp Glu Asp Met Glu Lys Pro
   20 25 30
   Phe Ile Gly Ile Val Asn Ser Phe Thr Glu Val Val Pro Gly His Ile
   35 40 45
   His Leu Arg Thr Leu Ser Glu Ala Ala Lys His Gly Val Tyr Ala Asn
   50 55 60
   Gly Gly Thr Pro Phe Glu Phe Asn Thr Ile Gly Ile Cys Asp Gly Ile
   65 70 75 80
   Ala Met Gly His Glu Gly Met Lys Tyr Ser Leu Pro Ser Arg Glu Ile
   85 90 95
   Ile Ala Asp Ala Val Glu Ser Met Ala Arg Ala His Gly Phe Asp Gly
   100 105 110
   Leu Val Leu Ile Pro Thr Cys Asp Lys Ile Val Pro Gly Met Ile Met
   115 120 125
   Gly Ala Leu Arg Leu Asn Ile Pro Phe Ile Val Val Thr Gly Gly Pro
   130 135 140
   Met Leu Pro Gly Glu Phe Gln Gly Lys Lys Tyr Glu Leu Ile Ser Leu
   145 150 155 160
   Phe Glu Gly Val Gly Glu Tyr Gln Val Gly Lys Ile Thr Glu Glu Glu
   165 170 175
   Leu Lys Cys Ile Glu Asp Cys Ala Cys Ser Gly Ala Gly Ser Cys Ala
   180 185 190
   Gly Leu Tyr Thr Ala Asn Ser Met Ala Cys Leu Thr Glu Ala Leu Gly
   195 200 205
   Leu Ser Leu Pro Met Cys Ala Thr Thr His Ala Val Asp Ala Gln Lys
   210 215 220
   Val Arg Leu Ala Lys Lys Ser Gly Ser Lys Ile Val Asp Met Val Lys
   225 230 235 240
   Glu Asp Leu Lys Pro Thr Asp Ile Leu Thr Lys Glu Ala Phe Glu Asn
   245 250 255
   Ala Ile Leu Val Asp Leu Ala Leu Gly Gly Ser Thr Asn Thr Thr Leu
   260 265 270
   His Ile Pro Ala Ile Ala Asn Glu Ile Glu Asn Lys Phe Ile Thr Leu
               275                 280                 285
   Asp Asp Phe Asp Arg Leu Ser Asp Glu Val Pro His Ile Ala Ser Ile
       290                 295                 300
   Lys Pro Gly Gly Glu His Tyr Met Ile Asp Leu His Asn Ala Gly Gly
   305                 310                 315                 320
   Ile Pro Ala Val Leu Asn Val Leu Lys Glu Lys Ile Arg Asp Thr Lys
                   325                 330                 335
   Thr Val Asp Gly Arg Ser Ile Leu Glu Ile Ala Glu Ser Val Lys Tyr
               340                 345                 350
   Ile Asn Tyr Asp Val Ile Arg Lys Val Glu Ala Pro Val His Glu Thr
           355                 360                 365
   Ala Gly Leu Arg Val Leu Lys Gly Asn Leu Ala Pro Asn Gly Cys Val
       370                 375                 380
   Val Lys Ile Gly Ala Val His Pro Lys Met Tyr Lys His Asp Gly Pro
   385                 390                 395                 400
   Ala Lys Val Tyr Asn Ser Glu Asp Glu Ala Ile Ser Ala Ile Leu Gly
                   405                 410                 415
   Gly Lys Ile Val Glu Gly Asp Val Ile Val Ile Arg Tyr Glu Gly Pro
               420                 425                 430
   Ser Gly Gly Pro Gly Met Arg Glu Met Leu Ser Pro Thr Ser Ala Ile
           435                 440                 445
   Cys Gly Met Gly Leu Asp Asp Ser Val Ala Leu Ile Thr Asp Gly Arg
       450                 455                 460
   Phe Ser Gly Gly Ser Arg Gly Pro Cys Ile Gly His Val Ser Pro Glu
   465                 470                 475                 480
   Ala Ala Ala Gly Gly Val Ile Ala Ala Ile Glu Asn Gly Asp Ile Ile
                   485                 490                 495
   Lys Ile Asp Met Ile Glu Lys Glu Ile Asn Val Asp Leu Asp Glu Ser
               500                 505                 510
   Val Ile Lys Glu Arg Leu Ser Lys Leu Gly Glu Phe Glu Pro Lys Ile
           515                 520                 525
   Lys Lys Gly Tyr Leu Ser Arg Tyr Ser Lys Leu Val Ser Ser Ala Asp
       530                 535                 540
   Glu Gly Ala Val Leu Lys
   545                 550
<210> 189
   <211> 1677
   <212> DNA
   <213> Bacillus subtilis
<400> 189
   atggcagaat tacgcagtaa tatgatcaca caaggaatcg atagagctcc gcaccgcagt      60
   ttgcttcgtg cagcaggggt aaaagaagag gatttcggca agccgtttat tgcggtgtgt     120
   aattcataca ttgatatcgt tcccggtcat gttcacttgc aggagtttgg gaaaatcgta     180
   aaagaagcaa tcagagaagc agggggcgtt ccgtttgaat ttaataccat tggggtagat     240
   gatggcatcg caatggggca tatcggtatg agatattcgc tgccaagccg tgaaattatc     300
   gcagactctg tggaaacggt tgtatccgca cactggtttg acggaatggt ctgtattccg     360
   aactgcgaca aaatcacacc gggaatgctt atggcggcaa tgcgcatcaa cattccgacg     420
   atttttgtca gcggcggacc gatggcggca ggaagaacaa gttacgggcg aaaaatctcc     480
   ctttcctcag tattcgaagg ggtaggcgcc taccaagcag ggaaaatcaa cgaaaacgag     540
   cttcaagaac tagagcagtt cggatgccca acgtgcgggt cttgctcagg catgtttacg     600
   gcgaactcaa tgaactgtct gtcagaagca cttggtcttg ctttgccggg taatggaacc     660
   attctggcaa catctccgga acgcaaagag tttgtgagaa aatcggctgc gcaattaatg     720
   gaaacgattc gcaaagatat caaaccgcgt gatattgtta cagtaaaagc gattgataac     780
   gcgtttgcac tcgatatggc gctcggaggt tctacaaata ccgttcttca tacccttgcc     840
   cttgcaaacg aagccggcgt tgaatactct ttagaacgca ttaacgaagt cgctgagcgc     900
   gtgccgcact tggctaagct ggcgcctgca tcggatgtgt ttattgaaga tcttcacgaa     960
   gcgggcggcg tttcagcggc tctgaatgag ctttcgaaga aagaaggagc gcttcattta    1020
   gatgcgctga ctgttacagg aaaaactctt ggagaaacca ttgccggaca tgaagtaaag    1080
   gattatgacg tcattcaccc gctggatcaa ccattcactg aaaagggagg ccttgctgtt    1140
   ttattcggta atctagctcc ggacggcgct atcattaaaa caggcggcgt acagaatggg    1200
   attacaagac acgaagggcc ggctgtcgta ttcgattctc aggacgaggc gcttgacggc    1260
   attatcaacc gaaaagtaaa agaaggcgac gttgtcatca tcagatacga agggccaaaa    1320
   ggcggacctg gcatgccgga aatgctggcg ccaacatccc aaatcgttgg aatgggactc    1380
   gggccaaaag tggcattgat tacggacgga cgtttttccg gagcctcccg tggcctctca    1440
   atcggccacg tatcacctga ggccgctgag ggcgggccgc ttgcctttgt tgaaaacgga    1500
   gaccatatta tcgttgatat tgaaaaacgc atcttggatg tacaagtgcc agaagaagag    1560
   tgggaaaaac gaaaagcgaa ctggaaaggt tttgaaccga aagtgaaaac cggctacctg    1620
   gcacgttatt ctaaacttgt gacaagtgcc aacaccggcg gtattatgaa aatctag       1677
<210> 190
   <211> 558
   <212> PRT
   <213> Bacillus subtilis
<400> 190
   Met Ala Glu Leu Arg Ser Asn Met Ile Thr Gln Gly Ile Asp Arg Ala
   1 5 10 15
   Pro His Arg Ser Leu Leu Arg Ala Ala Gly Val Lys Glu Glu Asp Phe
   20 25 30
   Gly Lys Pro Phe Ile Ala Val Cys Asn Ser Tyr Ile Asp Ile Val Pro
   35 40 45
   Gly His Val His Leu Gln Glu Phe Gly Lys Ile Val Lys Glu Ala Ile
   50 55 60
   Arg Glu Ala Gly Gly Val Pro Phe Glu Phe Asn Thr Ile Gly Val Asp
   65 70 75 80
   Asp Gly Ile Ala Met Gly His Ile Gly Met Arg Tyr Ser Leu Pro Ser
   85 90 95
   Arg Glu Ile Ile Ala Asp Ser Val Glu Thr Val Val Ser Ala His Trp
   100 105 110
   Phe Asp Gly Met Val Cys Ile Pro Asn Cys Asp Lys Ile Thr Pro Gly
   115 120 125
   Met Leu Met Ala Ala Met Arg Ile Asn Ile Pro Thr Ile Phe Val Ser
   130 135 140
   Gly Gly Pro Met Ala Ala Gly Arg Thr Ser Tyr Gly Arg Lys Ile Ser
   145 150 155 160
   Leu Ser Ser Val Phe Glu Gly Val Gly Ala Tyr Gln Ala Gly Lys Ile
   165 170 175
   Asn Glu Asn Glu Leu Gln Glu Leu Glu Gln Phe Gly Cys Pro Thr Cys
   180 185 190
   Gly Ser Cys Ser Gly Met Phe Thr Ala Asn Ser Met Asn Cys Leu Ser
   195 200 205
   Glu Ala Leu Gly Leu Ala Leu Pro Gly Asn Gly Thr Ile Leu Ala Thr
   210 215 220
   Ser Pro Glu Arg Lys Glu Phe Val Arg Lys Ser Ala Ala Gln Leu Met
   225 230 235 240
   Glu Thr Ile Arg Lys Asp Ile Lys Pro Arg Asp Ile Val Thr Val Lys
   245 250 255
   Ala Ile Asp Asn Ala Phe Ala Leu Asp Met Ala Leu Gly Gly Ser Thr
   260 265 270
   Asn Thr Val Leu His Thr Leu Ala Leu Ala Asn Glu Ala Gly Val Glu
   275 280 285
   Tyr Ser Leu Glu Arg Ile Asn Glu Val Ala Glu Arg Val Pro His Leu
   290 295 300
   Ala Lys Leu Ala Pro Ala Ser Asp Val Phe Ile Glu Asp Leu His Glu
   305 310 315 320
   Ala Gly Gly Val Ser Ala Ala Leu Asn Glu Leu Ser Lys Lys Glu Gly
   325 330 335
   Ala Leu His Leu Asp Ala Leu Thr Val Thr Gly Lys Thr Leu Gly Glu
   340 345 350
   Thr Ile Ala Gly His Glu Val Lys Asp Tyr Asp Val Ile His Pro Leu
   355 360 365
   Asp Gln Pro Phe Thr Glu Lys Gly Gly Leu Ala Val Leu Phe Gly Asn
   370 375 380
   Leu Ala Pro Asp Gly Ala Ile Ile Lys Thr Gly Gly Val Gln Asn Gly
   385 390 395 400
   Ile Thr Arg His Glu Gly Pro Ala Val Val Phe Asp Ser Gln Asp Glu
   405 410 415
   Ala Leu Asp Gly Ile Ile Asn Arg Lys Val Lys Glu Gly Asp Val Val
   420 425 430
   Ile Ile Arg Tyr Glu Gly Pro Lys Gly Gly Pro Gly Met Pro Glu Met
   435 440 445
   Leu Ala Pro Thr Ser Gln Ile Val Gly Met Gly Leu Gly Pro Lys Val
   450 455 460
   Ala Leu Ile Thr Asp Gly Arg Phe Ser Gly Ala Ser Arg Gly Leu Ser
   465 470 475 480
   Ile Gly His Val Ser Pro Glu Ala Ala Glu Gly Gly Pro Leu Ala Phe
   485 490 495
   Val Glu Asn Gly Asp His Ile Ile Val Asp Ile Glu Lys Arg Ile Leu
   500 505 510
   Asp Val Gln Val Pro Glu Glu Glu Trp Glu Lys Arg Lys Ala Asn Trp
   515 520 525
   Lys Gly Phe Glu Pro Lys Val Lys Thr Gly Tyr Leu Ala Arg Tyr Ser
   530 535 540
   Lys Leu Val Thr Ser Ala Asn Thr Gly Gly Ile Met Lys Ile
   545 550 555

<210> 191
   <211> 1647
   <212> DNA
   <213> Lact ococcus lactis
<400> 191
   atgtatacag taggagatta cctattagac cgattacacg agttaggaat tgaagaaatt      60
   tttggagtcc ctggagacta taacttacaa tttttagatc aaattatttc ccacaaggat     120
   atgaaatggg tcggaaatgc taatgaatta aatgcttcat atatggctga tggctatgct     180
   cgtactaaaa aagctgccgc atttcttaca acctttggag taggtgaatt gagtgcagtt     240
   aatggattag caggaagtta cgccgaaaat ttaccagtag tagaaatagt gggatcacct     300
   acatcaaaag ttcaaaatga aggaaaattt gttcatcata cgctggctga cggtgatttt     360
   aaacacttta tgaaaatgca cgaacctgtt acagcagctc gaactttact gacagcagaa     420
   aatgcaaccg ttgaaattga ccgagtactt tctgcactat taaaagaaag aaaacctgtc     480
   tatatcaact taccagttga tgttgctgct gcaaaagcag agaaaccctc actccctttg     540
   aaaaaggaaa actcaacttc aaatacaagt gaccaagaaa ttttgaacaa aattcaagaa     600
   agcttgaaaa atgccaaaaa accaatcgtg attacaggac atgaaataat tagttttggc     660
   ttagaaaaaa cagtcactca atttatttca aagacaaaac tacctattac gacattaaac     720
   tttggtaaaa gttcagttga tgaagccctc ccttcatttt taggaatcta taatggtaca     780
   ctctcagagc ctaatcttaa agaattcgtg gaatcagccg acttcatctt gatgcttgga     840
   gttaaactca cagactcttc aacaggagcc ttcactcatc atttaaatga aaataaaatg     900
   atttcactga atatagatga aggaaaaata tttaacgaaa gaatccaaaa ttttgatttt     960
   gaatccctca tctcctctct cttagaccta agcgaaatag aatacaaagg aaaatatatc    1020
   gataaaaagc aagaagactt tgttccatca aatgcgcttt tatcacaaga ccgcctatgg    1080
   caagcagttg aaaacctaac tcaaagcaat gaaacaatcg ttgctgaaca agggacatca    1140
   ttctttggcg cttcatcaat tttcttaaaa tcaaagagtc attttattgg tcaaccctta    1200
   tggggatcaa ttggatatac attcccagca gcattaggaa gccaaattgc agataaagaa    1260
   agcagacacc ttttatttat tggtgatggt tcacttcaac ttacagtgca agaattagga    1320
   ttagcaatca gagaaaaaat taatccaatt tgctttatta tcaataatga tggttataca    1380
   gtcgaaagag aaattcatgg accaaatcaa agctacaatg atattccaat gtggaattac    1440
   tcaaaattac cagaatcgtt tggagcaaca gaagatcgag tagtctcaaa aatcgttaga    1500
   actgaaaatg aatttgtgtc tgtcatgaaa gaagctcaag cagatccaaa tagaatgtac    1560
   tggattgagt taattttggc aaaagaaggt gcaccaaaag tactgaaaaa aatgggcaaa    1620
   ctatttgctg aacaaaataa atcataa                                        1647
<210> 192
   <211> 1644
   <212> DNA
   <213> Lactococcus lactis
<400> 192
   atgtatacag taggagatta cctgttagac cgattacacg agttgggaat tgaagaaatt      60
   tttggagttc ctggtgacta taacttacaa tttttagatc aaattatttc acgcgaagat     120
   atgaaatgga ttggaaatgc taatgaatta aatgcttctt atatggctga tggttatgct     180
   cgtactaaaa aagctgccgc atttctcacc acatttggag tcggcgaatt gagtgcgatc     240
   aatggactgg caggaagtta tgccgaaaat ttaccagtag tagaaattgt tggttcacca     300
   acttcaaaag tacaaaatga cggaaaattt gtccatcata cactagcaga tggtgatttt     360
   aaacacttta tgaagatgca tgaacctgtt acagcagcgc ggactttact gacagcagaa     420
   aatgccacat atgaaattga ccgagtactt tctcaattac taaaagaaag aaaaccagtc     480
   tatattaact taccagtcga tgttgctgca gcaaaagcag agaagcctgc attatcttta     540
   gaaaaagaaa gctctacaac aaatacaact gaacaagtga ttttgagtaa gattgaagaa     600
   agtttgaaaa atgcccaaaa accagtagtg attgcaggac acgaagtaat tagttttggt     660
   ttagaaaaaa cggtaactca gtttgtttca gaaacaaaac taccgattac gacactaaat     720
   tttggtaaaa gtgctgttga tgaatctttg ccctcatttt taggaatata taacgggaaa     780
   ctttcagaaa tcagtcttaa aaattttgtg gagtccgcag actttatcct aatgcttgga     840
   gtgaagctta cggactcctc aacaggtgca ttcacacatc atttagatga aaataaaatg     900
   atttcactaa acatagatga aggaataatt ttcaataaag tggtagaaga ttttgatttt     960
   agagcagtgg tttcttcttt atcagaatta aaaggaatag aatatgaagg acaatatatt    1020
   gataagcaat atgaagaatt tattccatca agtgctccct tatcacaaga ccgtctatgg    1080
   caggcagttg aaagtttgac tcaaagcaat gaaacaatcg ttgctgaaca aggaacctca    1140
   ttttttggag cttcaacaat tttcttaaaa tcaaatagtc gttttattgg acaaccttta    1200
   tggggttcta ttggatatac ttttccagcg gctttaggaa gccaaattgc ggataaagag    1260
   agcagacacc ttttatttat tggtgatggt tcacttcaac ttaccgtaca agaattagga    1320
   ctatcaatca gagaaaaact caatccaatt tgttttatca taaataatga tggttataca    1380
   gttgaaagag aaatccacgg acctactcaa agttataacg acattccaat gtggaattac    1440
   tcgaaattac cagaaacatt tggagcaaca gaagatcgtg tagtatcaaa aattgttaga    1500
   acagagaatg aatttgtgtc tgtcatgaaa gaagcccaag cagatgtcaa tagaatgtat    1560
   tggatagaac tagttttgga aaaagaagat gcgccaaaat tactgaaaaa aatgggtaaa    1620
   ttatttgctg agcaaaataa atag                                           1644
<210> 193
   <211> 547
   <212> PRT
   <213> Lactococcus lactis
<400> 193
   Met Tyr Thr Val Gly Asp Tyr Leu Leu Asp Arg Leu His Glu Leu Gly
   1               5                   10                  15
   Ile Glu Glu Ile Phe Gly Val Pro Gly Asp Tyr Asn Leu Gln Phe Leu
               20                  25                  30
   Asp Gln Ile Ile Ser Arg Glu Asp Met Lys Trp Ile Gly Asn Ala Asn
           35                  40                  45
   Glu Leu Asn Ala Ser Tyr Met Ala Asp Gly Tyr Ala Arg Thr Lys Lys
       50                  55                  60
   Ala Ala Ala Phe Leu Thr Thr Phe Gly Val Gly Glu Leu Ser Ala Ile
   65                  70                  75                  80
   Asn Gly Leu Ala Gly Ser Tyr Ala Glu Asn Leu Pro Val Val Glu Ile
                   85                  90                  95
   Val Gly Ser Pro Thr Ser Lys Val Gln Asn Asp Gly Lys Phe Val His
               100                 105                 110
   His Thr Leu Ala Asp Gly Asp Phe Lys His Phe Met Lys Met His Glu
           115                 120                 125
   Pro Val Thr Ala Ala Arg Thr Leu Leu Thr Ala Glu Asn Ala Thr Tyr
       130                 135                 140
   Glu Ile Asp Arg Val Leu Ser Gln Leu Leu Lys Glu Arg Lys Pro Val
   145                 150                 155                 160
   Tyr Ile Asn Leu Pro Val Asp Val Ala Ala Ala Lys Ala Glu Lys Pro
                   165                 170                 175
   Ala Leu Ser Leu Glu Lys Glu Ser Ser Thr Thr Asn Thr Thr Glu Gln
               180                 185                 190
   Val Ile Leu Ser Lys Ile Glu Glu Ser Leu Lys Asn Ala Gln Lys Pro
           195                 200                 205
   Val Val Ile Ala Gly His Glu Val Ile Ser Phe Gly Leu Glu Lys Thr
       210                 215                 220
   Val Thr Gln Phe Val Ser Glu Thr Lys Leu Pro Ile Thr Thr Leu Asn
   225                 230                 235                 240
   Phe Gly Lys Ser Ala Val Asp Glu Ser Leu Pro Ser Phe Leu Gly Ile
                   245                 250                 255
   Tyr Asn Gly Lys Leu Ser Glu Ile Ser Leu Lys Asn Phe Val Glu Ser
               260                 265                 270
   Ala Asp Phe Ile Leu Met Leu Gly Val Lys Leu Thr Asp Ser Ser Thr
   275 280 285
   Gly Ala Phe Thr His His Leu Asp Glu Asn Lys Met Ile Ser Leu Asn
   290 295 300
   Ile Asp Glu Gly Ile Ile Phe Asn Lys Val Val Glu Asp Phe Asp Phe
   305 310 315 320
   Arg Ala Val Val Ser Ser Leu Ser Glu Leu Lys Gly Ile Glu Tyr Glu
   325 330 335
   Gly Gln Tyr Ile Asp Lys Gln Tyr Glu Glu Phe Ile Pro Ser Ser Ala
   340 345 350
   Pro Leu Ser Gln Asp Arg Leu Trp Gln Ala Val Glu Ser Leu Thr Gln
   355 360 365
   Ser Asn Glu Thr Ile Val Ala Glu Gln Gly Thr Ser Phe Phe Gly Ala
   370 375 380
   Ser Thr Ile Phe Leu Lys Ser Asn Ser Arg Phe Ile Gly Gln Pro Leu
   385 390 395 400
   Trp Gly Ser Ile Gly Tyr Thr Phe Pro Ala Ala Leu Gly Ser Gln Ile
   405 410 415
   Ala Asp Lys Glu Ser Arg His Leu Leu Phe Ile Gly Asp Gly Ser Leu
   420 425 430
   Gln Leu Thr Val Gln Glu Leu Gly Leu Ser Ile Arg Glu Lys Leu Asn
   435 440 445
   Pro Ile Cys Phe Ile Ile Asn Asn Asp Gly Tyr Thr Val Glu Arg Glu
   450 455 460
   Ile His Gly Pro Thr Gln Ser Tyr Asn Asp Ile Pro Met Trp Asn Tyr
   465 470 475 480
   Ser Lys Leu Pro Glu Thr Phe Gly Ala Thr Glu Asp Arg Val Val Ser
   485 490 495
   Lys Ile Val Arg Thr Glu Asn Glu Phe Val Ser Val Met Lys Glu Ala
   500 505 510
   Gln Ala Asp Val Asn Arg Met Tyr Trp Ile Glu Leu Val Leu Glu Lys
   515 520 525
   Glu Asp Ala Pro Lys Leu Leu Lys Lys Met Gly Lys Leu Phe Ala Glu
   530 535 540
   Gln Asn Lys
   545
<210> 194
   <211> 1653
   <212> DNA
   <213> Sal monel I a t yphi rrur i um
<400> 194
   ttatcccccg ttgcgggctt ccagcgcccg ggtcacggta cgcagtaatt ccggcagatc      60
   ggcttttggc aacatcactt caataaatga cagacgttgt gggcgcgcca accgttcgag     120
   gacctctgcc agttggatag cctgcgtcac ccgccagcac tccgcctgtt gcgccgcgtt     180
   t agcgccggt ggt at ct gcg t ccagt t cca gct cgcgat g t cgt t at acc gct gggccgc 240
   gccgtgaatg gcgcgctcta cggtatagcc gtcattgttg agcagcagga tgaccggcgc     300
   ct gcccgt cg cgt aacat cg agcccat ct c ct gaat cgt g agct gcgccg cgccat cgcc 360
   gataatcaga atcacccgcc gatcgggaca ggcggtttgc gcgccaaacg cggcgggcaa     420
   ggaatagccg atagaccccc acagcggctg taacacaact tccgcgccgt caggaagcga     480
   cagcgcggca gcgccaaaag ctgctgtccc ctggtcgaca aggataatat ctccgggttt     540
   gagatactgc tgtaaggttt gccagaagct ttcctgggtc agttctcctt tatcaatccg     600
   cactggctgt ccggcggaac gcgtcggcgg cggcgcaaaa gcgcattcca ggcacagttc     660
   gcgcagcgta gacaccgcct gcgccatcgg gaggttgaac caggtttcgc cgatgcgcga     720
   cgcgt aaggc t gaat ct cca gcgt gcgt t c cgccggt aat t gt t gggt aa at ccggccgt 780
   aagggtatcg acaaaacggg tgccgacgca gataacccta tcggcgtcct ctatggcctg     840
   acgcacttct ttgctgctgg cgccagcgct ataggtgcca acgaagttcg ggtgctgttc     900
   at caaaaagc ccct t cccca t cagt agt gt cgcat gagcg at gggcgt t t ccgccat cca 960
   gcgctgcaac agtggtcgta aaccaaaacg cccggcaaga aagtcggcca atagcgcaat    1020
   gcgccgactg ttcatcaggc actgacgggc gtgataacga aaggccgtct ccacgccgct    1080
   ttgcgcttca tgcacgggca acgccagcgc ctgcgtaggt gggatggccg tttttttcgc    1140
   cacatcggcg ggcaacatga tgtatcctgg cctgcgtgcg gcaagcattt cacccaacac    1200
   gcggt caat c t cgaaacagg cgt t ct gt t c at ct aat at t gcgct ggcag cggat at cgc 1260
   ct gact cat g cgat aaaaat gacgaaaat c gccgt caccg agggt at ggt gcat caat t c 1320
   gccacgct gc t gcgcagcgc t acagggcgc gccgacgat a t gcaagaccg ggacat at t c 1380
   cgcgtaactg cccgcgatac cgttaatagc gctaagttct cccacgccaa aggtggtgag    1440
   tagcgctcca gcgcccgaca tgcgcgcata gccgtccgcg gcataagcgg cgttcagctc    1500
   attggcgcat cccacccaac gcagggtcgg gtggtcaatc acatggtcaa gaaactgcaa    1560
   gt t at aat cg cccggt acgc caaaaagat g gccaat gccg cat cct gcca gt ct gt ccag 1620
   caaatagtcg gccacggtat aggggttttg cat                                 1653
<210> 195
   <211> 550
   <212> PRT
   <213> Sal monella typhi muri um
<400> 195
   Met Gln Asn Pro Tyr Thr Val Ala Asp Tyr Leu Leu Asp Arg Leu Ala
   1 5 10 15
   Gly Cys Gly Ile Gly His Leu Phe Gly Val Pro Gly Asp Tyr Asn Leu
   20 25 30
   Gln Phe Leu Asp His Val Ile Asp His Pro Thr Leu Arg Trp Val Gly
   35 40 45
   Cys Ala Asn Glu Leu Asn Ala Ala Tyr Ala Ala Asp Gly Tyr Ala Arg
   50 55 60
   Met Ser Gly Ala Gly Ala Leu Leu Thr Thr Phe Gly Val Gly Glu Leu
   65 70 75 80
   Ser Ala Ile Asn Gly Ile Ala Gly Ser Tyr Ala Glu Tyr Val Pro Val
   85 90 95
   Leu His Ile Val Gly Ala Pro Cys Ser Ala Ala Gln Gln Arg Gly Glu
   100 105 110
   Leu Met His His Thr Leu Gly Asp Gly Asp Phe Arg His Phe Tyr Arg
   115 120 125
   Met Ser Gln Ala Ile Ser Ala Ala Ser Ala Ile Leu Asp Glu Gln Asn
   130 135 140
   Ala Cys Phe Glu Ile Asp Arg Val Leu Gly Glu Met Leu Ala Ala Arg
   145 150 155 160
   Arg Pro Gly Tyr Ile Met Leu Pro Ala Asp Val Ala Lys Lys Thr Ala
   165 170 175
   Ile Pro Pro Thr Gln Ala Leu Ala Leu Pro Val His Glu Ala Gln Ser
   180 185 190
   Gly Val Glu Thr Ala Phe Arg Tyr His Ala Arg Gln Cys Leu Met Asn
   195 200 205
   Ser Arg Arg Ile Ala Leu Leu Ala Asp Phe Leu Ala Gly Arg Phe Gly
   210 215 220
   Leu Arg Pro Leu Leu Gln Arg Trp Met Ala Glu Thr Pro Ile Ala His
   225 230 235 240
   Ala Thr Leu Leu Met Gly Lys Gly Leu Phe Asp Glu Gln His Pro Asn
   245 250 255
   Phe Val Gly Thr Tyr Ser Ala Gly Ala Ser Ser Lys Glu Val Arg Gln
   260 265 270
   Ala Ile Glu Asp Ala Asp Arg Val Ile Cys Val Gly Thr Arg Phe Val
           275                 280                 285
   Asp Thr Leu Thr Ala Gly Phe Thr Gln Gln Leu Pro Ala Glu Arg Thr
       290                 295                 300
   Leu Glu Ile Gln Pro Tyr Ala Ser Arg Ile Gly Glu Thr Trp Phe Asn
   305                 310                 315                 320
   Leu Pro Met Ala Gln Ala Val Ser Thr Leu Arg Glu Leu Cys Leu Glu
                   325                 330                 335
   Cys Ala Phe Ala Pro Pro Pro Thr Arg Ser Ala Gly Gln Pro Val Arg
               340                 345                 350
   Ile Asp Lys Gly Glu Leu Thr Gln Glu Ser Phe Trp Gln Thr Leu Gln
           355                 360                 365
   Gln Tyr Leu Lys Pro Gly Asp Ile Ile Leu Val Asp Gln Gly Thr Ala
       370                 375                 380
   Ala Phe Gly Ala Ala Ala Leu Ser Leu Pro Asp Gly Ala Glu Val Val
   385                 390                 395                 400
   Leu Gln Pro Leu Trp Gly Ser Ile Gly Tyr Ser Leu Pro Ala Ala Phe
                   405                 410                 415
   Gly Ala Gln Thr Ala Cys Pro Asp Arg Arg Val Ile Leu Ile Ile Gly
               420                 425                 430
   Asp Gly Ala Ala Gln Leu Thr Ile Gln Glu Met Gly Ser Met Leu Arg
           435                 440                 445
   Asp Gly Gln Ala Pro Val Ile Leu Leu Leu Asn Asn Asp Gly Tyr Thr
       450                 455                 460
   Val Glu Arg Ala Ile His Gly Ala Ala Gln Arg Tyr Asn Asp Ile Ala
   465                 470                 475                 480
   Ser Trp Asn Trp Thr Gln Ile Pro Pro Ala Leu Asn Ala Ala Gln Gln
                   485                 490                 495
   Ala Glu Cys Trp Arg Val Thr Gln Ala Ile Gln Leu Ala Glu Val Leu
               500                 505                 510
   Glu Arg Leu Ala Arg Pro Gln Arg Leu Ser Phe Ile Glu Val Met Leu
           515                 520                 525
   Pro Lys Ala Asp Leu Pro Glu Leu Leu Arg Thr Val Thr Arg Ala Leu
       530                 535                 540
   Glu Ala Arg Asn Gly Gly
   545                 550
<210> 196
   <211> 1665
   <212> DNA
   <213> Clostr i di um acet obut yl icum
<400> 196
   ttgaagagtg aatacacaat tggaagatat ttgttagacc gtttatcaga gttgggtatt      60
   cggcatatct ttggtgtacc tggagattac aatctatcct ttttagacta tataatggag     120
   tacaaaggga tagattgggt tggaaattgc aatgaattga atgctgggta tgctgctgat     180
   ggatatgcaa gaataaatgg aattggagcc atacttacaa catttggtgt tggagaatta     240
   agtgccatta acgcaattgc tggggcatac gctgagcaag ttccagttgt taaaattaca     300
   ggtatcccca cagcaaaagt tagggacaat ggattatatg tacaccacac attaggtgac     360
   ggaaggtttg atcacttttt tgaaatgttt agagaagtaa cagttgctga ggcattacta     420
   agcgaagaaa atgcagcaca agaaattgat cgtgttctta tttcatgctg gagacaaaaa     480
   cgtcctgttc ttataaattt accgattgat gtatatgata aaccaattaa caaaccatta     540
   aagccattac tcgattatac tatttcaagt aacaaagagg ctgcatgtga atttgttaca     600
   gaaatagtac ctataataaa tagggcaaaa aagcctgtta ttcttgcaga ttatggagta     660
   tatcgttacc aagttcaaca tgtgcttaaa aacttggccg aaaaaaccgg atttcctgtg     720
   gctacactaa gtatgggaaa aggtgttttc aatgaagcac accctcaatt tattggtgtt     780
   tataatggtg atgtaagttc tccttattta aggcagcgag ttgatgaagc agactgcatt     840
   attagcgttg gtgtaaaatt gacggattca accacagggg gattttctca tggattttct     900
   aaaaggaatg taattcacat tgatcctttt tcaataaagg caaaaggtaa aaaatatgca     960
   cctattacga tgaaagatgc tttaacagaa ttaacaagta aaattgagca tagaaacttt    1020
   gaggatttag atataaagcc ttacaaatca gataatcaaa agtattttgc aaaagagaag    1080
   ccaattacac aaaaacgttt ttttgagcgt attgctcact ttataaaaga aaaagatgta    1140
   ttattagcag aacagggtac atgctttttt ggtgcgtcaa ccatacaact acccaaagat    1200
   gcaactttta ttggtcaacc tttatgggga tctattggat acacacttcc tgctttatta    1260
   ggttcacaat tagctgatca aaaaaggcgt aatattcttt taattgggga tggtgcattt    1320
   caaatgacag cacaagaaat ttcaacaatg cttcgtttac aaatcaaacc tattattttt    1380
   ttaattaata acgatggtta tacaattgaa cgtgctattc atggtagaga acaagtatat    1440
   aacaatattc aaatgtggcg atatcataat gttccaaagg ttttaggtcc taaagaatgc    1500
   agcttaacct ttaaagtaca aagtgaaact gaacttgaaa aggctctttt agtggcagat    1560
   aaggattgtg aacatttgat ttttatagaa gttgttatgg atcgttatga taaacccgag     1620
   cctttagaac gtctttcgaa acgttttgca aatcaaaata attag                    1665
<210> 197
   <211> 554
   <212> PRT
   <213> Cl ost r i di um acet obut yl i cum
<400> 197
   Met Lys Ser Glu Tyr Thr Ile Gly Arg Tyr Leu Leu Asp Arg Leu Ser
   1               5                   10                  15
   Glu Leu Gly Ile Arg His Ile Phe Gly Val Pro Gly Asp Tyr Asn Leu
               20                  25                  30
   Ser Phe Leu Asp Tyr Ile Met Glu Tyr Lys Gly Ile Asp Trp Val Gly
           35                  40                  45
   Asn Cys Asn Glu Leu Asn Ala Gly Tyr Ala Ala Asp Gly Tyr Ala Arg
       50                  55                  60
   Ile Asn Gly Ile Gly Ala Ile Leu Thr Thr Phe Gly Val Gly Glu Leu
   65                  70                  75                  80
   Ser Ala Ile Asn Ala Ile Ala Gly Ala Tyr Ala Glu Gln Val Pro Val
                   85                  90                  95
   Val Lys Ile Thr Gly Ile Pro Thr Ala Lys Val Arg Asp Asn Gly Leu
               100                 105                 110
   Tyr Val His His Thr Leu Gly Asp Gly Arg Phe Asp His Phe Phe Glu
           115                 120                 125
   Met Phe Arg Glu Val Thr Val Ala Glu Ala Leu Leu Ser Glu Glu Asn
       130                 135                 140
   Ala Ala Gln Glu Ile Asp Arg Val Leu Ile Ser Cys Trp Arg Gln Lys
   145                 150                 155                 160
   Arg Pro Val Leu Ile Asn Leu Pro Ile Asp Val Tyr Asp Lys Pro Ile
                   165                 170                 175
   Asn Lys Pro Leu Lys Pro Leu Leu Asp Tyr Thr Ile Ser Ser Asn Lys
               180                 185                 190
   Glu Ala Ala Cys Glu Phe Val Thr Glu Ile Val Pro Ile Ile Asn Arg
           195                 200                 205
   Ala Lys Lys Pro Val Ile Leu Ala Asp Tyr Gly Val Tyr Arg Tyr Gln
       210                 215                 220
   Val Gln His Val Leu Lys Asn Leu Ala Glu Lys Thr Gly Phe Pro Val
   225                 230                 235                 240
   Ala Thr Leu Ser Met Gly Lys Gly Val Phe Asn Glu Ala His Pro Gln
                   245                 250                 255
   Phe Ile Gly Val Tyr Asn Gly Asp Val Ser Ser Pro Tyr Leu Arg Gln
   260 265 270
   Arg Val Asp Glu Ala Asp Cys Ile Ile Ser Val Gly Val Lys Leu Thr
   275 280 285
   Asp Ser Thr Thr Gly Gly Phe Ser His Gly Phe Ser Lys Arg Asn Val
   290 295 300
   Ile His Ile Asp Pro Phe Ser Ile Lys Ala Lys Gly Lys Lys Tyr Ala
   305 310 315 320
   Pro Ile Thr Met Lys Asp Ala Leu Thr Glu Leu Thr Ser Lys Ile Glu
   325 330 335
   His Arg Asn Phe Glu Asp Leu Asp Ile Lys Pro Tyr Lys Ser Asp Asn
   340 345 350
   Gln Lys Tyr Phe Ala Lys Glu Lys Pro Ile Thr Gln Lys Arg Phe Phe
   355 360 365
   Glu Arg Ile Ala His Phe Ile Lys Glu Lys Asp Val Leu Leu Ala Glu
   370 375 380
   Gln Gly Thr Cys Phe Phe Gly Ala Ser Thr Ile Gln Leu Pro Lys Asp
   385 390 395 400
   Ala Thr Phe Ile Gly Gln Pro Leu Trp Gly Ser Ile Gly Tyr Thr Leu
   405 410 415
   Pro Ala Leu Leu Gly Ser Gln Leu Ala Asp Gln Lys Arg Arg Asn Ile
   420 425 430
   Leu Leu Ile Gly Asp Gly Ala Phe Gln Met Thr Ala Gln Glu Ile Ser
   435 440 445
   Thr Met Leu Arg Leu Gln Ile Lys Pro Ile Ile Phe Leu Ile Asn Asn
   450 455 460
   Asp Gly Tyr Thr Ile Glu Arg Ala Ile His Gly Arg Glu Gln Val Tyr
   465 470 475 480
   Asn Asn Ile Gln Met Trp Arg Tyr His Asn Val Pro Lys Val Leu Gly
   485 490 495
   Pro Lys Glu Cys Ser Leu Thr Phe Lys Val Gln Ser Glu Thr Glu Leu
   500 505 510
   Glu Lys Ala Leu Leu Val Ala Asp Lys Asp Cys Glu His Leu Ile Phe
   515 520 525
   Ile Glu Val Val Met Asp Arg Tyr Asp Lys Pro Glu Pro Leu Glu Arg
       530                 535                 540
   Leu Ser Lys Arg Phe Ala Asn Gln Asn Asn
   545                 550
<210> 198
   <211> 939
   <212> DNA
   <213> Saccharomyces cerevisiae
<400> 198
   atgcctgcta cgttaaagaa ttcttctgct acattaaaac taaatactgg tgcctccatt      60
   ccagtgttgg gtttcggcac ttggcgttcc gttgacaata acggttacca ttctgtaatt     120
   gcagctttga aagctggata cagacacatt gatgctgcgg ctatctattt gaatgaagaa     180
   gaagttggca gggctattaa agattccgga gtccctcgtg aggaaatttt tattactact     240
   aagctttggg gtacggaaca acgtgatccg gaagctgctc taaacaagtc tttgaaaaga     300
   ctaggcttgg attatgttga cctatatctg atgcattggc cagtgccttt gaaaaccgac     360
   agagttactg atggtaacgt tctgtgcatt ccaacattag aagatggcac tgttgacatc     420
   gatactaagg aatggaattt tatcaagacg tgggagttga tgcaagagtt gccaaagacg     480
   ggcaaaacta aagccgttgg tgtctctaat tttctattta acaacattaa agaattatta     540
   gaatctccaa ataacaaggt ggtaccagct actaatcaaa ttgaaattca tccattgcta     600
   ccacaagacg aattgattgc cttttgtaag gaaaagggta ttgttgttga agcctactca     660
   ccatttggga gtgctaatgc tcctttacta aaagagcaag caattattga tatggctaaa     720
   aagcacggcg ttgagccagc acagcttatt atcagttgga gtattcaaag aggctacgtt     780
   gttctggcca aatcggttaa tcctgaaaga attgtatcca attttaagat tttcactctg     840
   cctgaggatg atttcaagac tattagtaac ctatccaaag tgcatggtac aaagagagtc     900
   gttgatatga agtggggatc cttcccaatt ttccaatga                            939
<210> 199
   <211> 312
   <212> PRT
   <213> Saccharomyces cerevisiae
<400> 199
   Met Pro Ala Thr Leu Lys Asn Ser Ser Ala Thr Leu Lys Leu Asn Thr
   1               5                   10                  15
   Gly Ala Ser Ile Pro Val Leu Gly Phe Gly Thr Trp Arg Ser Val Asp
               20                  25                  30
   Asn Asn Gly Tyr His Ser Val Ile Ala Ala Leu Lys Ala Gly Tyr Arg
           35                  40                  45
   His Ile Asp Ala Ala Ala Ile Tyr Leu Asn Glu Glu Glu Val Gly Arg
       50                  55                  60
   Ala Ile Lys Asp Ser Gly Val Pro Arg Glu Glu Ile Phe Ile Thr Thr
   65 70 75 80
   Lys Leu Trp Gly Thr Glu Gln Arg Asp Pro Glu Ala Ala Leu Asn Lys
   85 90 95
   Ser Leu Lys Arg Leu Gly Leu Asp Tyr Val Asp Leu Tyr Leu Met His
   100 105 110
   Trp Pro Val Pro Leu Lys Thr Asp Arg Val Thr Asp Gly Asn Val Leu
   115 120 125
   Cys Ile Pro Thr Leu Glu Asp Gly Thr Val Asp Ile Asp Thr Lys Glu
   130 135 140
   Trp Asn Phe Ile Lys Thr Trp Glu Leu Met Gln Glu Leu Pro Lys Thr
   145 150 155 160
   Gly Lys Thr Lys Ala Val Gly Val Ser Asn Phe Ser Ile Asn Asn Ile
   165 170 175
   Lys Glu Leu Leu Glu Ser Pro Asn Asn Lys Val Val Pro Ala Thr Asn
   180 185 190
   Gln Ile Glu Ile His Pro Leu Leu Pro Gln Asp Glu Leu Ile Ala Phe
   195 200 205
   Cys Lys Glu Lys Gly Ile Val Val Glu Ala Tyr Ser Pro Phe Gly Ser
   210 215 220
   Ala Asn Ala Pro Leu Leu Lys Glu Gln Ala Ile Ile Asp Met Ala Lys
   225 230 235 240
   Lys His Gly Val Glu Pro Ala Gln Leu Ile Ile Ser Trp Ser Ile Gln
   245 250 255
   Arg Gly Tyr Val Val Leu Ala Lys Ser Val Asn Pro Glu Arg Ile Val
   260 265 270
   Ser Asn Phe Lys Ile Phe Thr Leu Pro Glu Asp Asp Phe Lys Thr Ile
   275 280 285
   Ser Asn Leu Ser Lys Val His Gly Thr Lys Arg Val Val Asp Met Lys
   290 295 300
   Trp Gly Ser Phe Pro Ile Phe Gln
   305 310
<210> 200
   <211> 1083
   <212> DNA
   <213> Saccharomyces cer evi si ae
<400> 200
   ctagtctgaa aattctttgt cgtagccgac taaggtaaat ctatatctaa cgtcaccctt 60
   ttccatcctt tcgaaggctt catggacgcc ggcttcacca acaggtaatg tttccaccca 120
   aattttgata tctttttcag agactaattt caagagttgg ttcaattctt tgatggaacc 180
   taaagcactg taagaaatgg agacagcctt taagccatat ggctttagcg ataacatttc 240
   gtgttgttct ggtatagaga ttgagacaat tctaccacca accttcatag cctttggcat 300
   aatgttgaag tcaatgtcgg taagggagga agcacagact acaatcaggt cgaaggtgtc 360
   aaagtacttt tcaccccaat caccttcttc taatgtagca atgtagtgat cggcgcccat 420
   cttcattgca tcttctcttt ttctcgaaga acgagaaata acatacgtct ctgcccccat 480
   ggctttggaa atcaatgtac ccatactgcc gataccacca agaccaacta taccaacttt 540
   tttacctgga ccgcaaccgt tacgaaccaa tggagagtac acagtcaaac caccacataa 600
   tagtggagca gccaaatgtg atggaatatt ctctgggata ggcaccacaa aatgttcatg 660
   aactctgacg tagtttgcat agccaccctg cgacacatag ccgtcttcat aaggctgact 720
   gtatgtggta acaaacttgg tgcagtatgg ttcattatca ttcttacaac ggtcacattc 780
   caagcatgaa aagacttgag cacctacacc aacacgttga ccgactttca acccactgtt 840
   tgacttgggc cctagcttga caactttacc aacgatttca tgaccaacga ctagcggcat 900
   cttcatattg ccccaatgac cagctgcaca atgaatatca ctaccgcaga caccacatgc 960
   ttcgatctta atgtcaatgt catgatcgta aaatggtttt gggtcatact ttgtcttctt 1020
   tgggtttttc caatcttcgt gtgattgaat agcgatacct tcaaatttct caggataaga 1080
   cat 1083
<210> 201
   <211> 360
   <212> PRT
   <213> Saccharomyces cerevi si ae
<400> 201
   Met Ser Tyr Pro Glu Lys Phe Glu Gly Ile Ala Ile Gln Ser His Glu
   1 5 10 15
   Asp Trp Lys Asn Pro Lys Lys Thr Lys Tyr Asp Pro Lys Pro Phe Tyr
   20 25 30
   Asp His Asp Ile Asp Ile Lys Ile Glu Ala Cys Gly Val Cys Gly Ser
   35 40 45
   Asp Ile His Cys Ala Ala Gly His Trp Gly Asn Met Lys Met Pro Leu
   50 55 60
   Val Val Gly His Glu Ile Val Gly Lys Val Val Lys Leu Gly Pro Lys
   65 70 75 80
   Ser Asn Ser Gly Leu Lys Val Gly Gln Arg Val Gly Val Gly Ala Gln
   85 90 95
   Val Phe Ser Cys Leu Glu Cys Asp Arg Cys Lys Asn Asp Asn Glu Pro
   100 105 110
   Tyr Cys Thr Lys Phe Val Thr Thr Tyr Ser Gln Pro Tyr Glu Asp Gly
   115 120 125
   Tyr Val Ser Gln Gly Gly Tyr Ala Asn Tyr Val Arg Val His Glu His
   130 135 140
   Phe Val Val Pro Ile Pro Glu Asn Ile Pro Ser His Leu Ala Ala Pro
   145 150 155 160
   Leu Leu Cys Gly Gly Leu Thr Val Tyr Ser Pro Leu Val Arg Asn Gly
   165 170 175
   Cys Gly Pro Gly Lys Lys Val Gly Ile Val Gly Leu Gly Gly Ile Gly
   180 185 190
   Ser Met Gly Thr Leu Ile Ser Lys Ala Met Gly Ala Glu Thr Tyr Val
   195 200 205
   Ile Ser Arg Ser Ser Arg Lys Arg Glu Asp Ala Met Lys Met Gly Ala
   210 215 220
   Asp His Tyr Ile Ala Thr Leu Glu Glu Gly Asp Trp Gly Glu Lys Tyr
   225 230 235 240
   Phe Asp Thr Phe Asp Leu Ile Val Val Cys Ala Ser Ser Leu Thr Asp
   245 250 255
   Ile Asp Phe Asn Ile Met Pro Lys Ala Met Lys Val Gly Gly Arg Ile
   260 265 270
   Val Ser Ile Ser Ile Pro Glu Gln His Glu Met Leu Ser Leu Lys Pro
   275 280 285
   Tyr Gly Leu Lys Ala Val Ser Ile Ser Tyr Ser Ala Leu Gly Ser Ile
   290 295 300
   Lys Glu Leu Asn Gln Leu Leu Lys Leu Val Ser Glu Lys Asp Ile Lys
   305 310 315 320
   Ile Trp Val Glu Thr Leu Pro Val Gly Glu Ala Gly Val His Glu Ala
   325 330 335
   Phe Glu Arg Met Glu Lys Gly Asp Val Arg Tyr Arg Phe Thr Leu Val
   340 345 350
   Gly Tyr Asp Lys Glu Phe Ser Asp
   355 360
<210> 202
   <211> 1170
   <212> DNA
   <213> Cl ost r i di um acet obut yl i cum
<400> 202
   ttaataagat tttttaaata tctcaagaac atcctctgca tttattggtc ttaaacttcc      60
   tattgttcct ccagaatttc taacagcttg ctttgccatt agttctagtt tatcttttcc     120
   tattccaact tctctaagct ttgaaggaat acccaatgaa ttaaagtatt ctctcgtatt     180
   tttaatagcc tctcgtgcta tttcatagtt atctttgttc ttgtctattc cccaaacatt     240
   tattccataa gaaacaaatt tatgaagtgt atcgtcattt agaatatatt ccatccaatt     300
   aggtgttaaa attgcaagtc ctacaccatg tgttatatca taatatgcac ttaactcgtg     360
   ttccatagga tgacaactcc attttctatc cttaccaagt gataatagac catttatagc     420
   taaacttgaa gcccacatca aattagctct agcctcgtaa tcatcagtct tctccattgc     480
   tatttttcca tactttatac atgttcttaa gattgcttct gctataccgt cctgcacata     540
   agcaccttca acaccactaa agtaagattc aaaggtgtga ctcataatgt cagctgttcc     600
   cgctgctgtt tgatttttag gtactgtaaa agtatatgta ggatctaaca ctgaaaattt     660
   aggtctcata tcatcatgtc ctactccaag cttttcatta gtctccatat ttgaaattac     720
   tgcaatttga tccatttcag accctgttgc tgaaagagta agtatacttg caattggaag     780
   aactttagtt attttagatg gatctttaac catgtcccat gtatcgccat cataataaac     840
   tccagctgca attaccttag aacagtctat tgcacttcct ccccctattg ctaatactaa     900
   atccacatta ttttctctac atatttctat gccttttttt actgttgtta tcctaggatt     960
   tggctctact cctgaaagtt catagaaagc tatattgttt tcttttaata tagctgttgc    1020
   tctatcatat ataccgttcc tttttatact tcctccgcca taaactataa gcactcttga    1080
   gccatatttc ttaatttctt ctccaattac gtctattttt ccttttccaa aaaaaacttt    1140
   agttggtatt gaataatcaa aacttagcat                                     1170
<210> 203
   <211> 389
   <212> PRT
   <213> Cl ost r i di um acet obut yl i cum
<400> 203
   Met Leu Ser Phe Asp Tyr Ser Ile Pro Thr Lys Val Phe Phe Gly Lys
   1               5                   10                  15
   Gly Lys Ile Asp Val Ile Gly Glu Glu Ile Lys Lys Tyr Gly Ser Arg
               20                  25                  30
   Val Leu Ile Val Tyr Gly Gly Gly Ser Ile Lys Arg Asn Gly Ile Tyr
           35                  40                  45
   Asp Arg Ala Thr Ala Ile Leu Lys Glu Asn Asn Ile Ala Phe Tyr Glu
   50 55 60
   Leu Ser Gly Val Glu Pro Asn Pro Arg Ile Thr Thr Val Lys Lys Gly
   65 70 75 80
   Ile Glu Ile Cys Arg Glu Asn Asn Val Asp Leu Val Leu Ala Ile Gly
   85 90 95
   Gly Gly Ser Ala Ile Asp Cys Ser Lys Val Ile Ala Ala Gly Val Tyr
   100 105 110
   Tyr Asp Gly Asp Thr Trp Asp Met Val Lys Asp Pro Ser Lys Ile Thr
   115 120 125
   Lys Val Leu Pro Ile Ala Ser Ile Leu Thr Leu Ser Ala Thr Gly Ser
   130 135 140
   Glu Met Asp Gln Ile Ala Val Ile Ser Asn Met Glu Thr Asn Glu Lys
   145 150 155 160
   Leu Gly Val Gly His Asp Asp Met Arg Pro Lys Phe Ser Val Leu Asp
   165 170 175
   Pro Thr Tyr Thr Phe Thr Val Pro Lys Asn Gln Thr Ala Ala Gly Thr
   180 185 190
   Ala Asp Ile Met Ser His Thr Phe Glu Ser Tyr Phe Ser Gly Val Glu
   195 200 205
   Gly Ala Tyr Val Gln Asp Gly Ile Ala Glu Ala Ile Leu Arg Thr Cys
   210 215 220
   Ile Lys Tyr Gly Lys Ile Ala Met Glu Lys Thr Asp Asp Tyr Glu Ala
   225 230 235 240
   Arg Ala Asn Leu Met Trp Ala Ser Ser Leu Ala Ile Asn Gly Leu Leu
   245 250 255
   Ser Leu Gly Lys Asp Arg Lys Trp Ser Cys His Pro Met Glu His Glu
   260 265 270
   Leu Ser Ala Tyr Tyr Asp Ile Thr His Gly Val Gly Leu Ala Ile Leu
   275 280 285
   Thr Pro Asn Trp Met Glu Tyr Ile Leu Asn Asp Asp Thr Leu His Lys
   290 295 300
   Phe Val Ser Tyr Gly Ile Asn Val Trp Gly Ile Asp Lys Asn Lys Asp
   305 310 315 320
   Asn Tyr Glu Ile Ala Arg Glu Ala Ile Lys Asn Thr Arg Glu Tyr Phe
   325 330 335
   Asn Ser Leu Gly Ile Pro Ser Lys Leu Arg Glu Val Gly Ile Gly Lys
   340 345 350
   Asp Lys Leu Glu Leu Met Ala Lys Gln Ala Val Arg Asn Ser Gly Gly
   355 360 365
   Thr Ile Gly Ser Leu Arg Pro Ile Asn Ala Glu Asp Val Leu Glu Ile
   370 375 380
   Phe Lys Lys Ser Tyr
   385
<210> 204
   <211> 390
   <212> PRT
   <213> Cl ost r i di um acet obut yl i cum
<400> 204
   Met Val Asp Phe Glu Tyr Ser Ile Pro Thr Arg Ile Phe Phe Gly Lys
   1 5 10 15
   Asp Lys Ile Asn Val Leu Gly Arg Glu Leu Lys Lys Tyr Gly Ser Lys
   20 25 30
   Val Leu Ile Val Tyr Gly Gly Gly Ser Ile Lys Arg Asn Gly Ile Tyr
   35 40 45
   Asp Lys Ala Val Ser Ile Leu Glu Lys Asn Ser Ile Lys Phe Tyr Glu
   50 55 60
   Leu Ala Gly Val Glu Pro Asn Pro Arg Val Thr Thr Val Glu Lys Gly
   65 70 75 80
   Val Lys Ile Cys Arg Glu Asn Gly Val Glu Val Val Leu Ala Ile Gly
   85 90 95
   Gly Gly Ser Ala Ile Asp Cys Ala Lys Val Ile Ala Ala Ala Cys Glu
   100 105 110
   Tyr Asp Gly Asn Pro Trp Asp Ile Val Leu Asp Gly Ser Lys Ile Lys
   115 120 125
   Arg Val Leu Pro Ile Ala Ser Ile Leu Thr Ile Ala Ala Thr Gly Ser
   130 135 140
   Glu Met Asp Thr Trp Ala Val Ile Asn Asn Met Asp Thr Asn Glu Lys
   145 150 155 160
   Leu Ile Ala Ala His Pro Asp Met Ala Pro Lys Phe Ser Ile Leu Asp
   165 170 175
   Pro Thr Tyr Thr Tyr Thr Val Pro Thr Asn Gln Thr Ala Ala Gly Thr
   180 185 190
   Ala Asp Ile Met Ser His Ile Phe Glu Val Tyr Phe Ser Asn Thr Lys
   195 200 205
   Thr Ala Tyr Leu Gln Asp Arg Met Ala Glu Ala Leu Leu Arg Thr Cys
   210 215 220
   Ile Lys Tyr Gly Gly Ile Ala Leu Glu Lys Pro Asp Asp Tyr Glu Ala
   225 230 235 240
   Arg Ala Asn Leu Met Trp Ala Ser Ser Leu Ala Ile Asn Gly Leu Leu
   245 250 255
   Thr Tyr Gly Lys Asp Thr Asn Trp Ser Val His Leu Met Glu His Glu
   260 265 270
   Leu Ser Ala Tyr Tyr Asp Ile Thr His Gly Val Gly Leu Ala Ile Leu
   275 280 285
   Thr Pro Asn Trp Met Glu Tyr Ile Leu Asn Asn Asp Thr Val Tyr Lys
   290 295 300
   Phe Val Glu Tyr Gly Val Asn Val Trp Gly Ile Asp Lys Glu Lys Asn
   305 310 315 320
   His Tyr Asp Ile Ala His Gln Ala Ile Gln Lys Thr Arg Asp Tyr Phe
   325 330 335
   Val Asn Val Leu Gly Leu Pro Ser Arg Leu Arg Asp Val Gly Ile Glu
   340 345 350
   Glu Glu Lys Leu Asp Ile Met Ala Lys Glu Ser Val Lys Leu Thr Gly
   355 360 365
   Gly Thr Ile Gly Asn Leu Arg Pro Val Asn Ala Ser Glu Val Leu Gln
   370 375 380
   Ile Phe Lys Lys Ser Val
   385 390
<210> 205
   <211> 993
   <212> DNA
   <213> Bacillus subtilis
<400> 205
   atgagtacaa accgacatca agcactaggg ctgactgatc aggaagccgt tgatatgtat 60
   agaaccatgc tgttagcaag aaaaatcgat gaaagaatgt ggctgttaaa ccgttctggc 120
   aaaattccat ttgtaatctc ttgtcaagga caggaagcag cacaggtagg agcggctttc 180
   gcacttgacc gtgaaatgga ttatgtattg ccgtactaca gagacatggg tgtcgtgctc 240
   gcgtttggca tgacagcaaa ggacttaatg atgtccgggt ttgcaaaagc agcagatccg 300
   aactcaggag gccgccagat gccgggacat ttcggacaaa agaaaaaccg cattgtgacg 360
   ggatcatctc cggttacaac gcaagtgccg cacgcagtcg gtattgcgct tgcgggacgt 420
   atggagaaaa aggatatcgc agcctttgtt acattcgggg aagggtcttc aaaccaaggc 480
   gatttccatg aaggggcaaa ctttgccgct gtccataagc tgccggttat tttcatgtgt 540
   gaaaacaaca aatacgcaat ctcagtgcct tacgataagc aagtcgcatg tgagaacatt 600
   tccgaccgtg ccataggcta tgggatgcct ggcgtaactg tgaatggaaa tgatccgctg 660
   gaagtttatc aagcggttaa agaagcacgc gaaagggcac gcagaggaga aggcccgaca 720
   ttaattgaaa cgatttctta ccgccttaca ccacattcca gtgatgacga tgacagcagc 780
   tacagaggcc gtgaagaagt agaggaagcg aaaaaaagtg atcccctgct tacttatcaa 840
   gcttacttaa aggaaacagg cctgctgtcc gatgagatag aacaaaccat gctggatgaa 900
   attatggcaa tcgtaaatga agcgacggat gaagcggaga acgccccata tgcagctcct 960
   gagtcagcgc ttgattatgt ttatgcgaag tag 993

<210> 206
   <211> 330
   <212> PRT
   <213> Bacillus subtilis
<400> 206
   Met Ser Thr Asn Arg His Gln Ala Leu Gly Leu Thr Asp Gln Glu Ala
   1 5 10 15
   Val Asp Met Tyr Arg Thr Met Leu Leu Ala Arg Lys Ile Asp Glu Arg
   20 25 30
   Met Trp Leu Leu Asn Arg Ser Gly Lys Ile Pro Phe Val Ile Ser Cys
   35 40 45
   Gln Gly Gln Glu Ala Ala Gln Val Gly Ala Ala Phe Ala Leu Asp Arg
   50 55 60
   Glu Met Asp Tyr Val Leu Pro Tyr Tyr Arg Asp Met Gly Val Val Leu
   65 70 75 80
   Ala Phe Gly Met Thr Ala Lys Asp Leu Met Met Ser Gly Phe Ala Lys
   85 90 95
   Ala Ala Asp Pro Asn Ser Gly Gly Arg Gln Met Pro Gly His Phe Gly
   100 105 110
   Gln Lys Lys Asn Arg Ile Val Thr Gly Ser Ser Pro Val Thr Thr Gln
   115 120 125
   Val Pro His Ala Val Gly Ile Ala Leu Ala Gly Arg Met Glu Lys Lys
   130 135 140
   Asp Ile Ala Ala Phe Val Thr Phe Gly Glu Gly Ser Ser Asn Gln Gly
   145 150 155 160
   Asp Phe His Glu Gly Ala Asn Phe Ala Ala Val His Lys Leu Pro Val
   165 170 175
   Ile Phe Met Cys Glu Asn Asn Lys Tyr Ala Ile Ser Val Pro Tyr Asp
   180 185 190
   Lys Gln Val Ala Cys Glu Asn Ile Ser Asp Arg Ala Ile Gly Tyr Gly
   195 200 205
   Met Pro Gly Val Thr Val Asn Gly Asn Asp Pro Leu Glu Val Tyr Gln
   210 215 220
   Ala Val Lys Glu Ala Arg Glu Arg Ala Arg Arg Gly Glu Gly Pro Thr
   225 230 235 240
   Leu Ile Glu Thr Ile Ser Tyr Arg Leu Thr Pro His Ser Ser Asp Asp
   245 250 255
   Asp Asp Ser Ser Tyr Arg Gly Arg Glu Glu Val Glu Glu Ala Lys Lys
   260 265 270
   Ser Asp Pro Leu Leu Thr Tyr Gln Ala Tyr Leu Lys Glu Thr Gly Leu
   275 280 285
   Leu Ser Asp Glu Ile Glu Gln Thr Met Leu Asp Glu Ile Met Ala Ile
   290 295 300
   Val Asn Glu Ala Thr Asp Glu Ala Glu Asn Ala Pro Tyr Ala Ala Pro
   305 310 315 320
   Glu Ser Ala Leu Asp Tyr Val Tyr Ala Lys
   325 330
<210> 207
   <211> 984
   <212> DNA
   <213> Bacillus subtilis
<400> 207
   atgtcagtaa tgtcatatat tgatgcaatc aatttggcga tgaaagaaga aatggaacga 60
   gattctcgcg ttttcgtcct tggggaagat gtaggaagaa aaggcggtgt gtttaaagcg 120
   acagcgggac tctatgaaca atttggggaa gagcgcgtta tggatacgcc gcttgctgaa 180
   tctgcaatcg caggagtcgg tatcggagcg gcaatgtacg gaatgagacc gattgctgaa 240
   atgcagtttg ctgatttcat tatgccggca gtcaaccaaa ttatttctga agcggctaaa 300
   atccgctacc gcagcaacaa tgactggagc tgtccgattg tcgtcagagc gccatacggc 360
   ggaggcgtgc acggagccct gtatcattct caatcagtcg aagcaatttt cgccaaccag 420
   cccggactga aaattgtcat gccatcaaca ccatatgacg cgaaagggct cttaaaagcc 480
   gcagttcgtg acgaagaccc cgtgctgttt tttgagcaca agcgggcata ccgtctgata 540
   aagggcgagg ttccggctga tgattatgtc ctgccaatcg gcaaggcgga cgtaaaaagg 600
   gaaggcgacg acatcacagt gatcacatac ggcctgtgtg tccacttcgc cttacaagct 660
   gcagaacgtc tcgaaaaaga tggcatttca gcgcatgtgg tggatttaag aacagtttac 720
   ccgcttgata aagaagccat catcgaagct gcgtccaaaa ctggaaaggt tcttttggtc 780
   acagaagata caaaagaagg cagcatcatg agcgaagtag ccgcaattat atccgagcat 840
   tgtctgttcg acttagacgc gccgatcaaa cggcttgcag gtcctgatat tccggctatg 900
   ccttatgcgc cgacaatgga aaaatacttt atggtcaacc ctgataaagt ggaagcggcg 960
   atgagagaat tagcggagtt ttaa 984
<210> 208
   <211> 327
   <212> PRT
   <213> Bacillus subtilis
<400> 208
   Met Ser Val Met Ser Tyr Ile Asp Ala Ile Asn Leu Ala Met Lys Glu
   1 5 10 15
   Glu Met Glu Arg Asp Ser Arg Val Phe Val Leu Gly Glu Asp Val Gly
   20 25 30
   Arg Lys Gly Gly Val Phe Lys Ala Thr Ala Gly Leu Tyr Glu Gln Phe
   35 40 45
   Gly Glu Glu Arg Val Met Asp Thr Pro Leu Ala Glu Ser Ala Ile Ala
   50 55 60
   Gly Val Gly Ile Gly Ala Ala Met Tyr Gly Met Arg Pro Ile Ala Glu
   65 70 75 80
   Met Gln Phe Ala Asp Phe Ile Met Pro Ala Val Asn Gln Ile Ile Ser
   85 90 95
   Glu Ala Ala Lys Ile Arg Tyr Arg Ser Asn Asn Asp Trp Ser Cys Pro
   100 105 110
   Ile Val Val Arg Ala Pro Tyr Gly Gly Gly Val His Gly Ala Leu Tyr
   115 120 125
   His Ser Gln Ser Val Glu Ala Ile Phe Ala Asn Gln Pro Gly Leu Lys
   130 135 140
   Ile Val Met Pro Ser Thr Pro Tyr Asp Ala Lys Gly Leu Leu Lys Ala
   145 150 155 160
   Ala Val Arg Asp Glu Asp Pro Val Leu Phe Phe Glu His Lys Arg Ala
   165 170 175
   Tyr Arg Leu Ile Lys Gly Glu Val Pro Ala Asp Asp Tyr Val Leu Pro
   180 185 190
   Ile Gly Lys Ala Asp Val Lys Arg Glu Gly Asp Asp Ile Thr Val Ile
   195 200 205
   Thr Tyr Gly Leu Cys Val His Phe Ala Leu Gln Ala Ala Glu Arg Leu
   210 215 220
   Glu Lys Asp Gly Ile Ser Ala His Val Val Asp Leu Arg Thr Val Tyr
   225 230 235 240
   Pro Leu Asp Lys Glu Ala Ile Ile Glu Ala Ala Ser Lys Thr Gly Lys
   245 250 255
   Val Leu Leu Val Thr Glu Asp Thr Lys Glu Gly Ser Ile Met Ser Glu
   260 265 270
   Val Ala Ala Ile Ile Ser Glu His Cys Leu Phe Asp Leu Asp Ala Pro
   275 280 285
   Ile Lys Arg Leu Ala Gly Pro Asp Ile Pro Ala Met Pro Tyr Ala Pro
   290 295 300
   Thr Met Glu Lys Tyr Phe Met Val Asn Pro Asp Lys Val Glu Ala Ala
   305 310 315 320
   Met Arg Glu Leu Ala Glu Phe
   325
<210> 209
   <211> 1275
   <212> DNA
   <213> Bacillus subtilis
<400> 209
   atggcaattg aacaaatgac gatgccgcag cttggagaaa gcgtaacaga ggggacgatc 60
   agcaaatggc ttgtcgcccc cggtgataaa gtgaacaaat acgatccgat cgcggaagtc 120
   atgacagata aggtaaatgc agaggttccg tcttctttta ctggtacgat aacagagctt 180
   gtgggagaag aaggccaaac cctgcaagtc ggagaaatga tttgcaaaat tgaaacagaa 240
   ggcgcgaatc cggctgaaca aaaacaagaa cagccagcag catcagaagc cgctgagaac 300
   cctgttgcaa aaagtgctgg agcagccgat cagcccaata aaaagcgcta ctcgccagct 360
   gttctccgtt tggccggaga gcacggcatt gacctcgatc aagtgacagg aactggtgcc 420
   ggcgggcgca tcacacgaaa agatattcag cgcttaattg aaacaggcgg cgtgcaagaa 480
   cagaatcctg aggagctgaa aacagcagct cctgcaccga agtctgcatc aaaacctgag 540
   ccaaaagaag agacgtcata tcctgcgtct gcagccggtg ataaagaaat ccctgtcaca 600
   ggtgtaagaa aagcaattgc ttccaatatg aagcgaagca aaacagaaat tccgcatgct 660
   tggacgatga tggaagtcga cgtcacaaat atggttgcat atcgcaacag tataaaagat 720
   tcttttaaga agacagaagg ctttaattta acgttcttcg ccttttttgt aaaagcggtc 780
   gctcaggcgt taaaagaatt cccgcaaatg aatagcatgt gggcggggga caaaattatt 840
   cagaaaaagg atatcaatat ttcaattgca gttgccacag aggattcttt atttgttccg 900
   gtgattaaaa acgctgatga aaaaacaatt aaaggcattg cgaaagacat taccggccta 960
   gctaaaaaag taagagacgg aaaactcact gcagatgaca tgcagggagg cacgtttacc 1020
   gtcaacaaca caggttcgtt cgggtctgtt cagtcgatgg gcattatcaa ctaccctcag 1080
   gctgcgattc ttcaagtaga atccatcgtc aaacgcccgg ttgtcatgga caatggcatg 1140
   attgctgtca gagacatggt taatctgtgc ctgtcattag atcacagagt gcttgacggt 1200
   ctcgtgtgcg gacgattcct cggacgagtg aaacaaattt tagaatcgat tgacgagaag 1260
   acatctgttt actaa 1275
<210> 210
   <211> 424
   <212> PRT
   <213> Bacillus subtilis
<400> 210
   Met Ala Ile Glu Gln Met Thr Met Pro Gln Leu Gly Glu Ser Val Thr
   1 5 10 15
   Glu Gly Thr Ile Ser Lys Trp Leu Val Ala Pro Gly Asp Lys Val Asn
   20 25 30
   Lys Tyr Asp Pro Ile Ala Glu Val Met Thr Asp Lys Val Asn Ala Glu
   35 40 45
   Val Pro Ser Ser Phe Thr Gly Thr Ile Thr Glu Leu Val Gly Glu Glu
   50 55 60
   Gly Gln Thr Leu Gln Val Gly Glu Met Ile Cys Lys Ile Glu Thr Glu
   65 70 75 80
   Gly Ala Asn Pro Ala Glu Gln Lys Gln Glu Gln Pro Ala Ala Ser Glu
   85 90 95
   Ala Ala Glu Asn Pro Val Ala Lys Ser Ala Gly Ala Ala Asp Gln Pro
   100 105 110
   Asn Lys Lys Arg Tyr Ser Pro Ala Val Leu Arg Leu Ala Gly Glu His
   115 120 125
   Gly Ile Asp Leu Asp Gln Val Thr Gly Thr Gly Ala Gly Gly Arg Ile
   130 135 140
   Thr Arg Lys Asp Ile Gln Arg Leu Ile Glu Thr Gly Gly Val Gln Glu
   145 150 155 160
   Gln Asn Pro Glu Glu Leu Lys Thr Ala Ala Pro Ala Pro Lys Ser Ala
   165 170 175
   Ser Lys Pro Glu Pro Lys Glu Glu Thr Ser Tyr Pro Ala Ser Ala Ala
   180 185 190
   Gly Asp Lys Glu Ile Pro Val Thr Gly Val Arg Lys Ala Ile Ala Ser
   195 200 205
   Asn Met Lys Arg Ser Lys Thr Glu Ile Pro His Ala Trp Thr Met Met
   210 215 220
   Glu Val Asp Val Thr Asn Met Val Ala Tyr Arg Asn Ser Ile Lys Asp
   225 230 235 240
   Ser Phe Lys Lys Thr Glu Gly Phe Asn Leu Thr Phe Phe Ala Phe Phe
   245 250 255
   Val Lys Ala Val Ala Gln Ala Leu Lys Glu Phe Pro Gln Met Asn Ser
   260 265 270
   Met Trp Ala Gly Asp Lys Ile Ile Gln Lys Lys Asp Ile Asn Ile Ser
   275 280 285
   Ile Ala Val Ala Thr Glu Asp Ser Leu Phe Val Pro Val Ile Lys Asn
   290 295 300
   Ala Asp Glu Lys Thr Ile Lys Gly Ile Ala Lys Asp Ile Thr Gly Leu
   305 310 315 320
   Ala Lys Lys Val Arg Asp Gly Lys Leu Thr Ala Asp Asp Met Gln Gly
   325 330 335
   Gly Thr Phe Thr Val Asn Asn Thr Gly Ser Phe Gly Ser Val Gln Ser
   340 345 350
   Met Gly Ile Ile Asn Tyr Pro Gln Ala Ala Ile Leu Gln Val Glu Ser
   355 360 365
   Ile Val Lys Arg Pro Val Val Met Asp Asn Gly Met Ile Ala Val Arg
   370 375 380
   Asp Met Val Asn Leu Cys Leu Ser Leu Asp His Arg Val Leu Asp Gly
   385 390 395 400
   Leu Val Cys Gly Arg Phe Leu Gly Arg Val Lys Gln Ile Leu Glu Ser
   405 410 415
   Ile Asp Glu Lys Thr Ser Val Tyr
   420
<210> 211
   <211> 1374
   <212> DNA
   <213> Bacillus subtilis
<400> 211
   atggcaactg agtatgacgt agtcattctg ggcggcggta ccggcggtta tgttgcggcc    60
   atcagagccg ctcagctcgg cttaaaaaca gccgttgtgg aaaaggaaaa actcggggga   120
   acatgtctgc ataaaggctg tatcccgagt aaagcgctgc ttagaagcgc agaggtatac   180
   cggacagctc gtgaagccga tcaattcgga gtggaaacgg ctggcgtgtc cctcaacttt   240
   gaaaaagtgc agcagcgtaa gcaagccgtt gttgataagc ttgcagcggg tgtaaatcat   300
   ttaatgaaaa aaggaaaaat tgacgtgtac accggatatg gacgtatcct tggaccgtca   360
   atcttctctc cgctgccggg aacaatttct gttgagcggg gaaatggcga agaaaatgac   420
   atgctgatcc cgaaacaagt gatcattgca acaggatcaa gaccgagaat gcttccgggt   480
   cttgaagtgg acggtaagtc tgtactgact tcagatgagg cgctccaaat ggaggagctg   540
   ccacagtcaa tcatcattgt cggcggaggg gttatcggta tcgaatgggc gtctatgctt   600
   catgattttg gcgttaaggt aacggttatt gaatacgcgg atcgcatatt gccgactgaa   660
   gatctagaga tttcaaaaga aatggaaagt cttcttaaga aaaaaggcat ccagttcata   720
   acaggggcaa aagtgctgcc tgacacaatg acaaaaacat cagacgatat cagcatacaa   780
   gcggaaaaag acggagaaac cgttacctat tctgctgaga aaatgcttgt ttccatcggc   840
   agacaggcaa atatcgaagg catcggccta gagaacaccg atattgttac tgaaaatggc   900
   atgatttcag tcaatgaaag ctgccaaacg aaggaatctc atatttatgc aatcggagac   960
   gtaatcggtg gcctgcagtt agctcacgtt gcttcacatg agggaattat tgctgttgag  1020
   cattttgcag gtctcaatcc gcatccgctt gatccgacgc ttgtgccgaa gtgcatttac  1080
   tcaagccctg aagctgccag tgtcggctta accgaagacg aagcaaaggc gaacgggcat  1140
   aatgtcaaaa tcggcaagtt cccatttatg gcgattggaa aagcgcttgt atacggtgaa  1200
   agcgacggtt ttgtcaaaat cgtggctgac cgagatacag atgatattct cggcgttcat  1260
   atgattggcc cgcatgtcac cgacatgatt tctgaagcgg gtcttgccaa agtgctggac  1320
   gcaacaccgt gggaggtcgg gcaaacgatt tcacccgcat ccaacgcttt ctga        1374
<210> 212
   <211> 457
   <212> PRT
   <213> Bacillus subtilis
<400> 212
   Met Ala Thr Glu Tyr Asp Val Val Ile Leu Gly Gly Gly Thr Gly Gly
   1               5                   10                  15
   Tyr Val Ala Ala Ile Arg Ala Ala Gln Leu Gly Leu Lys Thr Ala Val
               20                  25                  30
   Val Glu Lys Glu Lys Leu Gly Gly Thr Cys Leu His Lys Gly Cys Ile
   35 40 45
   Pro Ser Lys Ala Leu Leu Arg Ser Ala Glu Val Tyr Arg Thr Ala Arg
   50 55 60
   Glu Ala Asp Gln Phe Gly Val Glu Thr Ala Gly Val Ser Leu Asn Phe
   65 70 75 80
   Glu Lys Val Gln Gln Arg Lys Gln Ala Val Val Asp Lys Leu Ala Ala
   85 90 95
   Gly Val Asn His Leu Met Lys Lys Gly Lys Ile Asp Val Tyr Thr Gly
   100 105 110
   Tyr Gly Arg Ile Leu Gly Pro Ser Ile Phe Ser Pro Leu Pro Gly Thr
   115 120 125
   Ile Ser Val Glu Arg Gly Asn Gly Glu Glu Asn Asp Met Leu Ile Pro
   130 135 140
   Lys Gln Val Ile Ile Ala Thr Gly Ser Arg Pro Arg Met Leu Pro Gly
   145 150 155 160
   Leu Glu Val Asp Gly Lys Ser Val Leu Thr Ser Asp Glu Ala Leu Gln
   165 170 175
   Met Glu Glu Leu Pro Gln Ser Ile Ile Ile Val Gly Gly Gly Val Ile
   180 185 190
   Gly Ile Glu Trp Ala Ser Met Leu His Asp Phe Gly Val Lys Val Thr
   195 200 205
   Val Ile Glu Tyr Ala Asp Arg Ile Leu Pro Thr Glu Asp Leu Glu Ile
   210 215 220
   Ser Lys Glu Met Glu Ser Leu Leu Lys Lys Lys Gly Ile Gln Phe Ile
   225 230 235 240
   Thr Gly Ala Lys Val Leu Pro Asp Thr Met Thr Lys Thr Ser Asp Asp
   245 250 255
   Ile Ser Ile Gln Ala Glu Lys Asp Gly Glu Thr Val Thr Tyr Ser Ala
   260 265 270
   Glu Lys Met Leu Val Ser Ile Gly Arg Gln Ala Asn Ile Glu Gly Ile
   275 280 285
   Gly Leu Glu Asn Thr Asp Ile Val Thr Glu Asn Gly Met Ile Ser Val
   290 295 300
   Asn Glu Ser Cys Gln Thr Lys Glu Ser His Ile Tyr Ala Ile Gly Asp
   305 310 315 320
   Val Ile Gly Gly Leu Gln Leu Ala His Val Ala Ser His Glu Gly Ile
   325 330 335
   Ile Ala Val Glu His Phe Ala Gly Leu Asn Pro His Pro Leu Asp Pro
   340 345 350
   Thr Leu Val Pro Lys Cys Ile Tyr Ser Ser Pro Glu Ala Ala Ser Val
   355 360 365
   Gly Leu Thr Glu Asp Glu Ala Lys Ala Asn Gly His Asn Val Lys Ile
   370 375 380
   Gly Lys Phe Pro Phe Met Ala Ile Gly Lys Ala Leu Val Tyr Gly Glu
   385 390 395 400
   Ser Asp Gly Phe Val Lys Ile Val Ala Asp Arg Asp Thr Asp Asp Ile
   405 410 415
   Leu Gly Val His Met Ile Gly Pro His Val Thr Asp Met Ile Ser Glu
   420 425 430
   Ala Gly Leu Ala Lys Val Leu Asp Ala Thr Pro Trp Glu Val Gly Gln
   435 440 445
   Thr Ile Ser Pro Ala Ser Asn Ala Phe
   450 455
<210> 213
   <211> 1233
   <212> DNA
   <213> Pseudomonas putida
<400> 213
   atgaacgagt acgcccccct gcgtttgcat gtgcccgagc ccaccggccg gccaggctgc 60
   cagaccgatt tttcctacct gcgcctgaac gatgcaggtc aagcccgtaa accccctgtc 120
   gatgtcgacg ctgccgacac cgccgacctg tcctacagcc tggtccgcgt gctcgacgag 180
   caaggcgacg cccaaggccc gtgggctgaa gacatcgacc cgcagatcct gcgccaaggc 240
   atgcgcgcca tgctcaagac gcggatcttc gacagccgca tggtggttgc ccagcgccag 300
   aagaagatgt ccttctacat gcagagcctg ggcgaagaag ccatcggcag cggccaggcg 360
   ctggcgctta accgcaccga catgtgcttc cccacctacc gtcagcaaag catcctgatg 420
   gcccgcgacg tgtcgctggt ggagatgatc tgccagttgc tgtccaacga acgcgacccc 480
   ctcaagggcc gccagctgcc gatcatgtac tcggtacgcg aggccggctt cttcaccatc 540
   agcggcaacc tggcgaccca gttcgtgcag gcggtcggct gggccatggc ctcggcgatc 600
   aagggcgata ccaagattgc ctcggcctgg atcggcgacg gcgccactgc cgaatcggac 660
   ttccacaccg ccctcacctt tgcccacgtt taccgcgccc cggtgatcct caacgtggtc 720
   aacaaccagt gggccatctc aaccttccag gccatcgccg gtggcgagtc gaccaccttc 780
   gccggccgtg gcgtgggctg cggcatcgct tcgctgcggg tggacggcaa cgacttcgtc 840
   gccgtttacg ccgcttcgcg ctgggctgcc gaacgtgccc gccgtggttt gggcccgagc 900
   ctgatcgagt gggtcaccta ccgtgccggc ccgcactcga cctcggacga cccgtccaag 960
   taccgccctg ccgatgactg gagccacttc ccgctgggtg acccgatcgc ccgcctgaag 1020
   cagcacctga tcaagatcgg ccactggtcc gaagaagaac accaggccac cacggccgag 1080
   ttcgaagcgg ccgtgattgc tgcgcaaaaa gaagccgagc agtacggcac cctggccaac 1140
   ggtcacatcc cgagcgccgc ctcgatgttc gaggacgtgt acaaggagat gcccgaccac 1200
   ctgcgccgcc aacgccagga actggggttt tga 1233
<210> 214
   <211> 410
   <212> PRT
   <213> Pseudomonas putida
<400> 214
   Met Asn Glu Tyr Ala Pro Leu Arg Leu His Val Pro Glu Pro Thr Gly
   1 5 10 15
   Arg Pro Gly Cys Gln Thr Asp Phe Ser Tyr Leu Arg Leu Asn Asp Ala
   20 25 30
   Gly Gln Ala Arg Lys Pro Pro Val Asp Val Asp Ala Ala Asp Thr Ala
   35 40 45
   Asp Leu Ser Tyr Ser Leu Val Arg Val Leu Asp Glu Gln Gly Asp Ala
   50 55 60
   Gln Gly Pro Trp Ala Glu Asp Ile Asp Pro Gln Ile Leu Arg Gln Gly
   65 70 75 80
   Met Arg Ala Met Leu Lys Thr Arg Ile Phe Asp Ser Arg Met Val Val
   85 90 95
   Ala Gln Arg Gln Lys Lys Met Ser Phe Tyr Met Gln Ser Leu Gly Glu
   100 105 110
   Glu Ala Ile Gly Ser Gly Gln Ala Leu Ala Leu Asn Arg Thr Asp Met
   115 120 125
   Cys Phe Pro Thr Tyr Arg Gln Gln Ser Ile Leu Met Ala Arg Asp Val
   130 135 140
   Ser Leu Val Glu Met Ile Cys Gln Leu Leu Ser Asn Glu Arg Asp Pro
   145 150 155 160
   Leu Lys Gly Arg Gln Leu Pro Ile Met Tyr Ser Val Arg Glu Ala Gly
   165 170 175
   Phe Phe Thr Ile Ser Gly Asn Leu Ala Thr Gln Phe Val Gln Ala Val
   180 185 190
   Gly Trp Ala Met Ala Ser Ala Ile Lys Gly Asp Thr Lys Ile Ala Ser
   195 200 205
   Ala Trp Ile Gly Asp Gly Ala Thr Ala Glu Ser Asp Phe His Thr Ala
   210 215 220
   Leu Thr Phe Ala His Val Tyr Arg Ala Pro Val Ile Leu Asn Val Val
   225 230 235 240
   Asn Asn Gln Trp Ala Ile Ser Thr Phe Gln Ala Ile Ala Gly Gly Glu
   245 250 255
   Ser Thr Thr Phe Ala Gly Arg Gly Val Gly Cys Gly Ile Ala Ser Leu
   260 265 270
   Arg Val Asp Gly Asn Asp Phe Val Ala Val Tyr Ala Ala Ser Arg Trp
   275 280 285
   Ala Ala Glu Arg Ala Arg Arg Gly Leu Gly Pro Ser Leu Ile Glu Trp
   290 295 300
   Val Thr Tyr Arg Ala Gly Pro His Ser Thr Ser Asp Asp Pro Ser Lys
   305 310 315 320
   Tyr Arg Pro Ala Asp Asp Trp Ser His Phe Pro Leu Gly Asp Pro Ile
   325 330 335
   Ala Arg Leu Lys Gln His Leu Ile Lys Ile Gly His Trp Ser Glu Glu
   340 345 350
   Glu His Gln Ala Thr Thr Ala Glu Phe Glu Ala Ala Val Ile Ala Ala
   355 360 365
   Gln Lys Glu Ala Glu Gln Tyr Gly Thr Leu Ala Asn Gly His Ile Pro
   370 375 380
   Ser Ala Ala Ser Met Phe Glu Asp Val Tyr Lys Glu Met Pro Asp His
   385 390 395 400
   Leu Arg Arg Gln Arg Gln Glu Leu Gly Val
   405 410
<210> 215
   <211> 1059
   <212> DNA
   <213> Pseudomonas putida
<400> 215
   atgaacgacc acaacaacag catcaacccg gaaaccgcca tggccaccac taccatgacc     60
   atgatccagg ccctgcgctc ggccatggat gtcatgcttg agcgcgacga caatgtggtg    120
   gtgtacggcc aggacgtcgg ctacttcggc ggcgtgttcc gctgcaccga aggcctgcag    180
   accaagtacg gcaagtcccg cgtgttcgac gcgcccatct ctgaaagcgg catcgtcggc    240
   accgccgtgg gcatgggtgc ctacggcctg cgcccggtgg tggaaatcca gttcgctgac    300
   tacttctacc cggcctccga ccagatcgtt tctgaaatgg cccgcctgcg ctaccgttcg    360
   gccggcgagt tcatcgcccc gctgaccctg cgtatgccct gcggtggcgg tatctatggc    420
   ggccagacac acagccagag cccggaagcg atgttcactc aggtgtgcgg cctgcgcacc    480
   gtaatgccat ccaacccgta cgacgccaaa ggcctgctga ttgcctcgat cgaatgcgac    540
   gacccggtga tcttcctgga gcccaagcgc ctgtacaacg gcccgttcga cggccaccat    600
   gaccgcccgg ttacgccgtg gtcgaaacac ccgcacagcg ccgtgcccga tggctactac    660
   accgtgccac tggacaaggc cgccatcacc cgccccggca atgacgtgag cgtgctcacc    720
   tatggcacca ccgtgtacgt ggcccaggtg gccgccgaag aaagtggcgt ggatgccgaa    780
   gtgatcgacc tgcgcagcct gtggccgcta gacctggaca ccatcgtcga gtcggtgaaa    840
   aagaccggcc gttgcgtggt agtacacgag gccacccgta cttgtggctt tggcgcagaa    900
   ctggtgtcgc tggtgcagga gcactgcttc caccacctgg aggcgccgat cgagcgcgtc    960
   accggttggg acacccccta ccctcacgcg caggaatggg cttacttccc agggccttcg   1020
   cgggtaggtg cggcattgaa aaaggtcatg gaggtctga                          1059
<210> 216
   <211> 352
   <212> PRT
   <213> Pseudomonas putida
<400> 216
   Met Asn Asp His Asn Asn Ser Ile Asn Pro Glu Thr Ala Met Ala Thr
   1               5                   10                  15
   Thr Thr Met Thr Met Ile Gln Ala Leu Arg Ser Ala Met Asp Val Met
               20                  25                  30
   Leu Glu Arg Asp Asp Asn Val Val Val Tyr Gly Gln Asp Val Gly Tyr
           35                  40                  45
   Phe Gly Gly Val Phe Arg Cys Thr Glu Gly Leu Gln Thr Lys Tyr Gly
       50                  55                  60
   Lys Ser Arg Val Phe Asp Ala Pro Ile Ser Glu Ser Gly Ile Val Gly
   65                  70                  75                  80
   Thr Ala Val Gly Met Gly Ala Tyr Gly Leu Arg Pro Val Val Glu Ile
                   85                  90                  95
   Gln Phe Ala Asp Tyr Phe Tyr Pro Ala Ser Asp Gln Ile Val Ser Glu
   100 105 110
   Met Ala Arg Leu Arg Tyr Arg Ser Ala Gly Glu Phe Ile Ala Pro Leu
   115 120 125
   Thr Leu Arg Met Pro Cys Gly Gly Gly Ile Tyr Gly Gly Gln Thr His
   130 135 140
   Ser Gln Ser Pro Glu Ala Met Phe Thr Gln Val Cys Gly Leu Arg Thr
   145 150 155 160
   Val Met Pro Ser Asn Pro Tyr Asp Ala Lys Gly Leu Leu Ile Ala Ser
   165 170 175
   Ile Glu Cys Asp Asp Pro Val Ile Phe Leu Glu Pro Lys Arg Leu Tyr
   180 185 190
   Asn Gly Pro Phe Asp Gly His His Asp Arg Pro Val Thr Pro Trp Ser
   195 200 205
   Lys His Pro His Ser Ala Val Pro Asp Gly Tyr Tyr Thr Val Pro Leu
   210 215 220
   Asp Lys Ala Ala Ile Thr Arg Pro Gly Asn Asp Val Ser Val Leu Thr
   225 230 235 240
   Tyr Gly Thr Thr Val Tyr Val Ala Gln Val Ala Ala Glu Glu Ser Gly
   245 250 255
   Val Asp Ala Glu Val Ile Asp Leu Arg Ser Leu Trp Pro Leu Asp Leu
   260 265 270
   Asp Thr Ile Val Glu Ser Val Lys Lys Thr Gly Arg Cys Val Val Val
   275 280 285
   His Glu Ala Thr Arg Thr Cys Gly Phe Gly Ala Glu Leu Val Ser Leu
   290 295 300
   Val Gln Glu His Cys Phe His His Leu Glu Ala Pro Ile Glu Arg Val
   305 310 315 320
   Thr Gly Trp Asp Thr Pro Tyr Pro His Ala Gln Glu Trp Ala Tyr Phe
   325 330 335
   Pro Gly Pro Ser Arg Val Gly Ala Ala Leu Lys Lys Val Met Glu Val
   340 345 350
<210> 217
   <211> 1272
   <212> DNA
   <213> Pseudomonas put i da
<400> 217
   atgggcacgc acgtcatcaa gatgccggac attggcgaag gcatcgcgca ggtcgaattg 60
   gtggaatggt tcgtcaaggt gggcgacatc atcgccgagg accaagtggt agccgacgtc 120
   atgaccgaca aggccaccgt ggaaatcccg tcgccggtca gcggcaaggt gctggccctg 180
   ggtggccagc caggtgaagt gatggcggtc ggcagtgagc tgatccgcat cgaagtggaa 240
   ggcagcggca accatgtgga tgtgccgcaa gccaagccgg ccgaagtgcc tgcggcaccg 300
   gtagccgcta aacctgaacc acagaaagac gttaaaccgg cggcgtacca ggcgtcagcc 360
   agccacgagg cagcgcccat cgtgccgcgc cagccgggcg acaagccgct ggcctcgccg 420
   gcggtgcgca aacgcgccct cgatgccggc atcgaattgc gttatgtgca cggcagcggc 480
   ccggccgggc gcatcctgca cgaagacctc gacgcgttca tgagcaaacc gcaaagcgct 540
   gccgggcaaa cccccaatgg ctatgccagg cgcaccgaca gcgagcaggt gccggtgatc 600
   ggcctgcgcc gcaagatcgc ccagcgcatg caggacgcca agcgccgggt cgcgcacttc 660
   agctatgtgg aagaaatcga cgtcaccgcc ctggaagccc tgcgccagca gctcaacagc 720
   aagcacggcg acagccgcgg caagctgaca ctgctgccgt tcctggtgcg cgccctggtc 780
   gtggcactgc gtgacttccc gcagataaac gccacctacg atgacgaagc gcagatcatc 840
   acccgccatg gcgcggtgca tgtgggcatc gccacccaag gtgacaacgg cctgatggta 900
   cccgtgctgc gccacgccga agcgggcagc ctgtgggcca atgccggtga gatttcacgc 960
   ctggccaacg ctgcgcgcaa caacaaggcc agccgcgaag agctgtccgg ttcgaccatt 1020
   accctgacca gcctcggcgc cctgggcggc atcgtcagca cgccggtggt caacaccccg 1080
   gaagtggcga tcgtcggtgt caaccgcatg gttgagcggc ccgtggtgat cgacggccag 1140
   atcgtcgtgc gcaagatgat gaacctgtcc agctcgttcg accaccgcgt ggtcgatggc 1200
   atggacgccg ccctgttcat ccaggccgtg cgtggcctgc tcgaacaacc cgcctgcctg 1260
   ttcgtggagt ga 1272
<210> 218
   <211> 423
   <212> PRT
   <213> Pseudomonas put i da
<400> 218
   Met Gly Thr His Val Ile Lys Met Pro Asp Ile Gly Glu Gly Ile Ala
   1 5 10 15
   Gln Val Glu Leu Val Glu Trp Phe Val Lys Val Gly Asp Ile Ile Ala
   20 25 30
   Glu Asp Gln Val Val Ala Asp Val Met Thr Asp Lys Ala Thr Val Glu
   35 40 45
   Ile Pro Ser Pro Val Ser Gly Lys Val Leu Ala Leu Gly Gly Gln Pro
   50 55 60
   Gly Glu Val Met Ala Val Gly Ser Glu Leu Ile Arg Ile Glu Val Glu
   65 70 75 80
   Gly Ser Gly Asn His Val Asp Val Pro Gln Ala Lys Pro Ala Glu Val
   85 90 95
   Pro Ala Ala Pro Val Ala Ala Lys Pro Glu Pro Gln Lys Asp Val Lys
   100 105 110
   Pro Ala Ala Tyr Gln Ala Ser Ala Ser His Glu Ala Ala Pro Ile Val
   115 120 125
   Pro Arg Gln Pro Gly Asp Lys Pro Leu Ala Ser Pro Ala Val Arg Lys
   130 135 140
   Arg Ala Leu Asp Ala Gly Ile Glu Leu Arg Tyr Val His Gly Ser Gly
   145 150 155 160
   Pro Ala Gly Arg Ile Leu His Glu Asp Leu Asp Ala Phe Met Ser Lys
   165 170 175
   Pro Gln Ser Ala Ala Gly Gln Thr Pro Asn Gly Tyr Ala Arg Arg Thr
   180 185 190
   Asp Ser Glu Gln Val Pro Val Ile Gly Leu Arg Arg Lys Ile Ala Gln
   195 200 205
   Arg Met Gln Asp Ala Lys Arg Arg Val Ala His Phe Ser Tyr Val Glu
   210 215 220
   Glu Ile Asp Val Thr Ala Leu Glu Ala Leu Arg Gln Gln Leu Asn Ser
   225 230 235 240
   Lys His Gly Asp Ser Arg Gly Lys Leu Thr Leu Leu Pro Phe Leu Val
   245 250 255
   Arg Ala Leu Val Val Ala Leu Arg Asp Phe Pro Gln Ile Asn Ala Thr
   260 265 270
   Tyr Asp Asp Glu Ala Gln Ile Ile Thr Arg His Gly Ala Val His Val
   275 280 285
   Gly Ile Ala Thr Gln Gly Asp Asn Gly Leu Met Val Pro Val Leu Arg
   290 295 300
   His Ala Glu Ala Gly Ser Leu Trp Ala Asn Ala Gly Glu Ile Ser Arg
   305 310 315 320
   Leu Ala Asn Ala Ala Arg Asn Asn Lys Ala Ser Arg Glu Glu Leu Ser
   325 330 335
   Gly Ser Thr Ile Thr Leu Thr Ser Leu Gly Ala Leu Gly Gly Ile Val
   340 345 350
   Ser Thr Pro Val Val Asn Thr Pro Glu Val Ala Ile Val Gly Val Asn
   355 360 365
   Arg Met Val Glu Arg Pro Val Val Ile Asp Gly Gln Ile Val Val Arg
   370 375 380
   Lys Met Met Asn Leu Ser Ser Ser Phe Asp His Arg Val Val Asp Gly
   385 390 395 400
   Met Asp Ala Ala Leu Phe Ile Gln Ala Val Arg Gly Leu Leu Glu Gln
   405 410 415
   Pro Ala Cys Leu Phe Val Glu
   420
<210> 219
   <211> 1380
   <212> DNA
   <213> Pseudomonas putida
<400> 219
   atgcaacaga ctatccagac aaccctgttg atcatcggcg gcggccctgg cggctatgtg 60
   gcggccatcc gcgccgggca actgggcatc cctaccgtgc tggtggaagg ccaggcgctg 120
   ggcggtacct gcctgaacat cggctgcatt ccgtccaagg cgctgatcca tgtggccgag 180
   cagttccacc aggcctcgcg ctttaccgaa ccctcgccgc tgggcatcag cgtggcttcg 240
   ccacgcctgg acatcggcca gagcgtggcc tggaaagacg gcatcgtcga tcgcctgacc 300
   actggtgtcg ccgccctgct gaaaaagcac ggggtgaagg tggtgcacgg ctgggccaag 360
   gtgcttgatg gcaagcaggt cgaggtggat ggccagcgca tccagtgcga gcacctgttg 420
   ctggccacgg gctccagcag tgtcgaactg ccgatgctgc cgttgggtgg gccggtgatt 480
   tcctcgaccg aggccctggc accgaaagcc ctgccgcaac acctggtggt ggtgggcggt 540
   ggctacatcg gcctggagct gggtatcgcc taccgcaagc tcggcgcgca ggtcagcgtg 600
   gtggaagcgc gcgagcgcat cctgccgact tacgacagcg aactgaccgc cccggtggcc 660
   gagtcgctga aaaagctggg tatcgccctg caccttggcc acagcgtcga aggttacgaa 720
   aatggctgcc tgctggccaa cgatggcaag ggcggacaac tgcgcctgga agccgaccgg 780
   gtgctggtgg ccgtgggccg ccgcccacgc accaagggct tcaacctgga atgcctggac 840
   ctgaagatga atggtgccgc gattgccatc gacgagcgct gccagaccag catgcacaac 900
   gtctgggcca tcggcgacgt ggccggcgaa ccgatgctgg cgcaccgggc catggcccag 960
   ggcgagatgg tggccgagat catcgccggc aaggcacgcc gcttcgaacc cgctgcgata 1020
   gccgccgtgt gcttcaccga cccggaagtg gtcgtggtcg gcaagacgcc ggaacaggcc 1080
   agtcagcaag gcctggactg catcgtcgcg cagttcccgt tcgccgccaa cggccgggcc 1140
   atgagcctgg agtcgaaaag cggtttcgtg cgcgtggtcg cgcggcgtga caaccacctg 1200
   atcctgggct ggcaagcggt tggcgtggcg gtttccgagc tgtccacggc gtttgcccag 1260
   tcgctggaga tgggtgcctg cctggaggat gtggccggta ccatccatgc ccacccgacc 1320
   ctgggtgaag cggtacagga agcggcactg cgtgccctgg gccacgccct gcatatctga 1380
<210> 220
   <211> 459
   <212> PRT
   <213> Pseudomonas put i da
<400> 220
   Met Gln Gln Thr Ile Gln Thr Thr Leu Leu Ile Ile Gly Gly Gly Pro
   1               5                   10                  15
   Gly Gly Tyr Val Ala Ala Ile Arg Ala Gly Gln Leu Gly Ile Pro Thr
               20                  25                  30
   Val Leu Val Glu Gly Gln Ala Leu Gly Gly Thr Cys Leu Asn Ile Gly
           35                  40                  45
   Cys Ile Pro Ser Lys Ala Leu Ile His Val Ala Glu Gln Phe His Gln
       50                  55                  60
   Ala Ser Arg Phe Thr Glu Pro Ser Pro Leu Gly Ile Ser Val Ala Ser
   65                  70                  75                  80
   Pro Arg Leu Asp Ile Gly Gln Ser Val Ala Trp Lys Asp Gly Ile Val
                   85                  90                  95
   Asp Arg Leu Thr Thr Gly Val Ala Ala Leu Leu Lys Lys His Gly Val
               100                 105                 110
   Lys Val Val His Gly Trp Ala Lys Val Leu Asp Gly Lys Gln Val Glu
           115                 120                 125
   Val Asp Gly Gln Arg Ile Gln Cys Glu His Leu Leu Leu Ala Thr Gly
       130                 135                 140
   Ser Ser Ser Val Glu Leu Pro Met Leu Pro Leu Gly Gly Pro Val Ile
   145                 150                 155                 160
   Ser Ser Thr Glu Ala Leu Ala Pro Lys Ala Leu Pro Gln His Leu Val
                   165                 170                 175
   Val Val Gly Gly Gly Tyr Ile Gly Leu Glu Leu Gly Ile Ala Tyr Arg
               180                 185                 190
   Lys Leu Gly Ala Gln Val Ser Val Val Glu Ala Arg Glu Arg Ile Leu
           195                 200                 205
   Pro Thr Tyr Asp Ser Glu Leu Thr Ala Pro Val Ala Glu Ser Leu Lys
       210                 215                 220
   Lys Leu Gly Ile Ala Leu His Leu Gly His Ser Val Glu Gly Tyr Glu
   225 230 235 240
   Asn Gly Cys Leu Leu Ala Asn Asp Gly Lys Gly Gly Gln Leu Arg Leu
   245 250 255
   Glu Ala Asp Arg Val Leu Val Ala Val Gly Arg Arg Pro Arg Thr Lys
   260 265 270
   Gly Phe Asn Leu Glu Cys Leu Asp Leu Lys Met Asn Gly Ala Ala Ile
   275 280 285
   Ala Ile Asp Glu Arg Cys Gln Thr Ser Met His Asn Val Trp Ala Ile
   290 295 300
   Gly Asp Val Ala Gly Glu Pro Met Leu Ala His Arg Ala Met Ala Gln
   305 310 315 320
   Gly Glu Met Val Ala Glu Ile Ile Ala Gly Lys Ala Arg Arg Phe Glu
   325 330 335
   Pro Ala Ala Ile Ala Ala Val Cys Phe Thr Asp Pro Glu Val Val Val
   340 345 350
   Val Gly Lys Thr Pro Glu Gln Ala Ser Gln Gln Gly Leu Asp Cys Ile
   355 360 365
   Val Ala Gln Phe Pro Phe Ala Ala Asn Gly Arg Ala Met Ser Leu Glu
   370 375 380
   Ser Lys Ser Gly Phe Val Arg Val Val Ala Arg Arg Asp Asn His Leu
   385 390 395 400
   Ile Leu Gly Trp Gln Ala Val Gly Val Ala Val Ser Glu Leu Ser Thr
   405 410 415
   Ala Phe Ala Gln Ser Leu Glu Met Gly Ala Cys Leu Glu Asp Val Ala
   420 425 430
   Gly Thr Ile His Ala His Pro Thr Leu Gly Glu Ala Val Gln Glu Ala
   435 440 445
   Ala Leu Arg Ala Leu Gly His Ala Leu His Ile
   450 455
<210> 221
   <211> 1407
   <212> DNA
   <213> Clost r i di um bei j er i ncki i
<400> 221
   atgaataaag acacactaat acctacaact aaagatttaa aattaaaaac aaatgttgaa 60
   aacattaatt taaagaacta caaggataat tcttcatgtt tcggagtatt cgaaaatgtt 120
   gaaaatgcta taaacagcgc tgtacacgcg caaaagatat tatcccttca ttatacaaaa 180
   gaacaaagag aaaaaatcat aactgagata agaaaggccg cattagaaaa taaagaggtt 240
   ttagctacca tgattctgga agaaacacat atgggaaggt atgaagataa aatattaaag 300
   catgaattag tagctaaata tactcctggt acagaagatt taactactac tgcttggtca 360
   ggtgataatg gtcttacagt tgtagaaatg tctccatatg gcgttatagg tgcaataact 420
   ccttctacga atccaactga aactgtaata tgtaatagca tcggcatgat agctgctgga 480
   aatgctgtag tatttaacgg acacccaggc gctaaaaaat gtgttgcttt tgctattgaa 540
   atgataaata aagcaattat ttcatgtggc ggtcctgaga atttagtaac aactataaaa 600
   aatccaacta tggaatccct agatgcaatt attaagcatc ctttaataaa acttctttgc 660
   ggaactggag gtccaggaat ggtaaaaacc ctcttaaatt ctggcaagaa agctataggt 720
   gctggtgctg gaaatccacc agttattgta gatgataccg ctgatataga aaaggctggt 780
   aagagtatca ttgaaggctg ttcttttgat aataatttac cttgtattgc agaaaaagaa 840
   gtatttgttt ttgagaatgt tgcagatgat ttaatatcta acatgctaaa aaataatgct 900
   gtaattataa atgaagatca agtatcaaaa ttaatagatt tagtattaca aaaaaataat 960
   gaaactcaag aatactttat aaacaaaaaa tgggtaggaa aagatgcaaa attattctca 1020
   gatgaaatag atgttgagtc tccttcaaat attaaatgca tagtctgcga agtaaatgca 1080
   aatcatccat ttgtcatgac agaactcatg atgccaatat taccaattgt aagagttaaa 1140
   gatatagatg aagctgttaa atatacaaag atagcagaac aaaatagaaa acatagtgcc 1200
   tatatttatt ctaaaaatat agacaaccta aatagatttg aaagagaaat tgatactact 1260
   atttttgtaa agaatgctaa atcttttgct ggtgttggtt atgaagctga aggatttaca 1320
   actttcacta ttgctggatc tactggtgaa ggcataacct ctgcaagaaa ttttacaaga 1380
   caaagaagat gtgtacttgc cggctaa 1407
<210> 222
   <211> 468
   <212> PRT
   <213> Clost r i dium bei j er i ncki i
<400> 222
   Met Asn Lys Asp Thr Leu Ile Pro Thr Thr Lys Asp Leu Lys Leu Lys
   1 5 10 15
   Thr Asn Val Glu Asn Ile Asn Leu Lys Asn Tyr Lys Asp Asn Ser Ser
   20 25 30
   Cys Phe Gly Val Phe Glu Asn Val Glu Asn Ala Ile Asn Ser Ala Val
   35 40 45
   His Ala Gln Lys Ile Leu Ser Leu His Tyr Thr Lys Glu Gln Arg Glu
   50 55 60
   Lys Ile Ile Thr Glu Ile Arg Lys Ala Ala Leu Glu Asn Lys Glu Val
   65                  70                  75                  80
   Leu Ala Thr Met Ile Leu Glu Glu Thr His Met Gly Arg Tyr Glu Asp
                   85                  90                  95
   Lys Ile Leu Lys His Glu Leu Val Ala Lys Tyr Thr Pro Gly Thr Glu
               100                 105                 110
   Asp Leu Thr Thr Thr Ala Trp Ser Gly Asp Asn Gly Leu Thr Val Val
           115                 120                 125
   Glu Met Ser Pro Tyr Gly Val Ile Gly Ala Ile Thr Pro Ser Thr Asn
       130                 135                 140
   Pro Thr Glu Thr Val Ile Cys Asn Ser Ile Gly Met Ile Ala Ala Gly
   145                 150                 155                 160
   Asn Ala Val Val Phe Asn Gly His Pro Gly Ala Lys Lys Cys Val Ala
                   165                 170                 175
   Phe Ala Ile Glu Met Ile Asn Lys Ala Ile Ile Ser Cys Gly Gly Pro
               180                 185                 190
   Glu Asn Leu Val Thr Thr Ile Lys Asn Pro Thr Met Glu Ser Leu Asp
           195                 200                 205
   Ala Ile Ile Lys His Pro Leu Ile Lys Leu Leu Cys Gly Thr Gly Gly
       210                 215                 220
   Pro Gly Met Val Lys Thr Leu Leu Asn Ser Gly Lys Lys Ala Ile Gly
   225                 230                 235                 240
   Ala Gly Ala Gly Asn Pro Pro Val Ile Val Asp Asp Thr Ala Asp Ile
                   245                 250                 255
   Glu Lys Ala Gly Lys Ser Ile Ile Glu Gly Cys Ser Phe Asp Asn Asn
               260                 265                 270
   Leu Pro Cys Ile Ala Glu Lys Glu Val Phe Val Phe Glu Asn Val Ala
           275                 280                 285
   Asp Asp Leu Ile Ser Asn Met Leu Lys Asn Asn Ala Val Ile Ile Asn
       290                 295                 300
   Glu Asp Gln Val Ser Lys Leu Ile Asp Leu Val Leu Gln Lys Asn Asn
   305                 310                 315                 320
   Glu Thr Gln Glu Tyr Phe Ile Asn Lys Lys Trp Val Gly Lys Asp Ala
                   325                 330                 335
   Lys Leu Phe Ser Asp Glu Ile Asp Val Glu Ser Pro Ser Asn Ile Lys
   340 345 350
   Cys Ile Val Cys Glu Val Asn Ala Asn His Pro Phe Val Met Thr Glu
   355 360 365
   Leu Met Met Pro Ile Leu Pro Ile Val Arg Val Lys Asp Ile Asp Glu
   370 375 380
   Ala Val Lys Tyr Thr Lys Ile Ala Glu Gln Asn Arg Lys His Ser Ala
   385 390 395 400
   Tyr Ile Tyr Ser Lys Asn Ile Asp Asn Leu Asn Arg Phe Glu Arg Glu
   405 410 415
   Ile Asp Thr Thr Ile Phe Val Lys Asn Ala Lys Ser Phe Ala Gly Val
   420 425 430
   Gly Tyr Glu Ala Glu Gly Phe Thr Thr Phe Thr Ile Ala Gly Ser Thr
   435 440 445
   Gly Glu Gly Ile Thr Ser Ala Arg Asn Phe Thr Arg Gln Arg Arg Cys
   450 455 460
   Val Leu Ala Gly
   465

<210> 223
   <211> 2589
   <212> DNA
   <213> Cl ost r i di um acet obut yl i cum
<400> 223
   atgaaagtca caacagtaaa ggaattagat gaaaaactca aggtaattaa agaagctcaa 60
   aaaaaattct cttgttactc gcaagaaatg gttgatgaaa tctttagaaa tgcagcaatg 120
   gcagcaatcg acgcaaggat agagctagca aaagcagctg ttttggaaac cggtatgggc 180
   ttagttgaag acaaggttat aaaaaatcat tttgcaggcg aatacatcta taacaaatat 240
   aaggatgaaa aaacctgcgg tataattgaa cgaaatgaac cctacggaat tacaaaaata 300
   gcagaaccta taggagttgt agctgctata atccctgtaa caaaccccac atcaacaaca 360
   atatttaaat ccttaatatc ccttaaaact agaaatggaa ttttcttttc gcctcaccca 420
   agggcaaaaa aatccacaat actagcagct aaaacaatac ttgatgcagc cgttaagagt 480
   ggtgccccgg aaaatataat aggttggata gatgaacctt caattgaact aactcaatat 540
   ttaatgcaaa aagcagatat aacccttgca actggtggtc cctcactagt taaatctgct 600
   tattcttccg gaaaaccagc aataggtgtt ggtccgggta acaccccagt aataattgat 660
   gaatctgctc atataaaaat ggcagtaagt tcaattatat tatccaaaac ctatgataat 720
   ggtgttatat gtgcttctga acaatctgta atagtcttaa aatccatata taacaaggta 780
   aaagatgagt tccaagaaag aggagcttat ataataaaga aaaacgaatt ggataaagtc 840
   cgt gaagt ga t t t t t aaaga t ggat ccgt a aaccct aaaa t agt cggaca gt cagct t at 900
   act at agcag ct at ggct gg cat aaaagt a cct aaaacca caagaat at t aat aggagaa 960
   gttacctcct taggtgaaga agaacctttt gcccacgaaa aactatctcc tgttttggct    1020
   at gt at gagg ct gacaat t t t gat gat gct t t aaaaaaag cagt aact ct aat aaact t a 1080
   ggaggcct cg gccat acct c aggaat at at gcagat gaaa t aaaagcacg agat aaaat a 1140
   gat agat t t a gt agt gccat gaaaaccgt a agaacct t t g t aaat at ccc aacct cacaa 1200
   ggt gcaagt g gagat ct at a t aat t t t aga at accacct t ct t t cacgct t ggct gcgga 1260
   t t t t ggggag gaaat t ct gt t t ccgagaat gt t ggt ccaa aacat ct t t t gaat at t aaa 1320
   accgtagctg aaaggagaga aaacatgctt tggtttagag ttccacataa agtatatttt    1380
   aagttcggtt gtcttcaatt tgctttaaaa gatttaaaag atctaaagaa aaaaagagcc    1440
   t t t at agt t a ct gat agt ga cccct at aat t t aaact at g t t gat t caat aat aaaaat a 1500
   ct t gagcacc t agat at t ga t t t t aaagt a t t t aat aagg t t ggaagaga agct gat ct t 1560
   aaaaccataa aaaaagcaac tgaagaaatg tcctccttta tgccagacac tataatagct    1620
   t t aggt ggt a cccct gaaat gagct ct gca aagct aat gt gggt act at a t gaacat cca 1680
   gaagt aaaat t t gaagat ct t gcaat aaaa t t t at ggaca t aagaaagag aat at at act 1740
   t t cccaaaac t cggt aaaaa ggct at gt t a gt t gcaat t a caact t ct gc t ggt t ccggt 1800
   t ct gaggt t a ct cct t t t gc t t t agt aact gacaat aaca ct ggaaat aa gt acat gt t a 1860
   gcagat t at g aaat gacacc aaat at ggca at t gt agat g cagaact t at gat gaaaat g 1920
   ccaaagggat taaccgctta ttcaggtata gatgcactag taaatagtat agaagcatac    1980
   acat ccgt at at gct t caga at acacaaac ggact agcac t agaggcaat acgat t aat a 2040
   tttaaatatt tgcctgaggc ttacaaaaac ggaagaacca atgaaaaagc aagagagaaa    2100
   at ggct cacg ct t caact at ggcaggt at g gcat ccgct a at gcat t t ct aggt ct at gt 2160
   cat t ccat gg caat aaaat t aagt t cagaa cacaat at t c ct agt ggcat t gccaat gca 2220
   ttactaatag aagaagtaat aaaatttaac gcagttgata atcctgtaaa acaagcccct    2280
   t gcccacaat at aagt at cc aaacaccat a t t t agat at g ct cgaat t gc agat t at at a 2340
   aagct t ggag gaaat act ga t gaggaaaag gt agat ct ct t aat t aacaa aat acat gaa 2400
   ctaaaaaaag ctttaaatat accaacttca ataaaggatg caggtgtttt ggaggaaaac    2460
   ttctattcct cccttgatag aatatctgaa cttgcactag atgatcaatg cacaggcgct    2520
   aat cct agat t t cct ct t ac aagt gagat a aaagaaat gt at at aaat t g t t t t aaaaaa 2580
   caacct t aa 2589
<210> 224
   <211> 862
   <212> PRT
   <213> Clost r i di um acet obut yl i cum
<400> 224
   Met Lys Val Thr Thr Val Lys Glu Leu Asp Glu Lys Leu Lys Val Ile
   1 5 10 15
   Lys Glu Ala Gln Lys Lys Phe Ser Cys Tyr Ser Gln Glu Met Val Asp
   20 25 30
   Glu Ile Phe Arg Asn Ala Ala Met Ala Ala Ile Asp Ala Arg Ile Glu
   35 40 45
   Leu Ala Lys Ala Ala Val Leu Glu Thr Gly Met Gly Leu Val Glu Asp
   50 55 60
   Lys Val Ile Lys Asn His Phe Ala Gly Glu Tyr Ile Tyr Asn Lys Tyr
   65 70 75 80
   Lys Asp Glu Lys Thr Cys Gly Ile Ile Glu Arg Asn Glu Pro Tyr Gly
   85 90 95
   Ile Thr Lys Ile Ala Glu Pro Ile Gly Val Val Ala Ala Ile Ile Pro
   100 105 110
   Val Thr Asn Pro Thr Ser Thr Thr Ile Phe Lys Ser Leu Ile Ser Leu
   115 120 125
   Lys Thr Arg Asn Gly Ile Phe Phe Ser Pro His Pro Arg Ala Lys Lys
   130 135 140
   Ser Thr Ile Leu Ala Ala Lys Thr Ile Leu Asp Ala Ala Val Lys Ser
   145 150 155 160
   Gly Ala Pro Glu Asn Ile Ile Gly Trp Ile Asp Glu Pro Ser Ile Glu
   165 170 175
   Leu Thr Gln Tyr Leu Met Gln Lys Ala Asp Ile Thr Leu Ala Thr Gly
   180 185 190
   Gly Pro Ser Leu Val Lys Ser Ala Tyr Ser Ser Gly Lys Pro Ala Ile
   195 200 205
   Gly Val Gly Pro Gly Asn Thr Pro Val Ile Ile Asp Glu Ser Ala His
   210 215 220
   Ile Lys Met Ala Val Ser Ser Ile Ile Leu Ser Lys Thr Tyr Asp Asn
   225 230 235 240
   Gly Val Ile Cys Ala Ser Glu Gln Ser Val Ile Val Leu Lys Ser Ile
   245 250 255
   Tyr Asn Lys Val Lys Asp Glu Phe Gln Glu Arg Gly Ala Tyr Ile Ile
   260 265 270
   Lys Lys Asn Glu Leu Asp Lys Val Arg Glu Val Ile Phe Lys Asp Gly
   275 280 285
   Ser Val Asn Pro Lys Ile Val Gly Gln Ser Ala Tyr Thr Ile Ala Ala
   290 295 300
   Met Ala Gly Ile Lys Val Pro Lys Thr Thr Arg Ile Leu Ile Gly Glu
   305 310 315 320
   Val Thr Ser Leu Gly Glu Glu Glu Pro Phe Ala His Glu Lys Leu Ser
   325 330 335
   Pro Val Leu Ala Met Tyr Glu Ala Asp Asn Phe Asp Asp Ala Leu Lys
   340 345 350
   Lys Ala Val Thr Leu Ile Asn Leu Gly Gly Leu Gly His Thr Ser Gly
   355 360 365
   Ile Tyr Ala Asp Glu Ile Lys Ala Arg Asp Lys Ile Asp Arg Phe Ser
   370 375 380
   Ser Ala Met Lys Thr Val Arg Thr Phe Val Asn Ile Pro Thr Ser Gln
   385 390 395 400
   Gly Ala Ser Gly Asp Leu Tyr Asn Phe Arg Ile Pro Pro Ser Phe Thr
   405 410 415
   Leu Gly Cys Gly Phe Trp Gly Gly Asn Ser Val Ser Glu Asn Val Gly
   420 425 430
   Pro Lys His Leu Leu Asn Ile Lys Thr Val Ala Glu Arg Arg Glu Asn
   435 440 445
   Met Leu Trp Phe Arg Val Pro His Lys Val Tyr Phe Lys Phe Gly Cys
   450 455 460
   Leu Gln Phe Ala Leu Lys Asp Leu Lys Asp Leu Lys Lys Lys Arg Ala
   465 470 475 480
   Phe Ile Val Thr Asp Ser Asp Pro Tyr Asn Leu Asn Tyr Val Asp Ser
   485 490 495
   Ile Ile Lys Ile Leu Glu His Leu Asp Ile Asp Phe Lys Val Phe Asn
   500 505 510
   Lys Val Gly Arg Glu Ala Asp Leu Lys Thr Ile Lys Lys Ala Thr Glu
   515 520 525
   Glu Met Ser Ser Phe Met Pro Asp Thr Ile Ile Ala Leu Gly Gly Thr
   530 535 540
   Pro Glu Met Ser Ser Ala Lys Leu Met Trp Val Leu Tyr Glu His Pro
   545 550 555 560
   Glu Val Lys Phe Glu Asp Leu Ala Ile Lys Phe Met Asp Ile Arg Lys
   565 570 575
   Arg Ile Tyr Thr Phe Pro Lys Leu Gly Lys Lys Ala Met Leu Val Ala
   580 585 590
   Ile Thr Thr Ser Ala Gly Ser Gly Ser Glu Val Thr Pro Phe Ala Leu
   595 600 605
   Val Thr Asp Asn Asn Thr Gly Asn Lys Tyr Met Leu Ala Asp Tyr Glu
   610 615 620
   Met Thr Pro Asn Met Ala Ile Val Asp Ala Glu Leu Met Met Lys Met
   625 630 635 640
   Pro Lys Gly Leu Thr Ala Tyr Ser Gly Ile Asp Ala Leu Val Asn Ser
   645 650 655
   Ile Glu Ala Tyr Thr Ser Val Tyr Ala Ser Glu Tyr Thr Asn Gly Leu
   660 665 670
   Ala Leu Glu Ala Ile Arg Leu Ile Phe Lys Tyr Leu Pro Glu Ala Tyr
   675 680 685
   Lys Asn Gly Arg Thr Asn Glu Lys Ala Arg Glu Lys Met Ala His Ala
   690 695 700
   Ser Thr Met Ala Gly Met Ala Ser Ala Asn Ala Phe Leu Gly Leu Cys
   705 710 715 720
   His Ser Met Ala Ile Lys Leu Ser Ser Glu His Asn Ile Pro Ser Gly
   725 730 735
   Ile Ala Asn Ala Leu Leu Ile Glu Glu Val Ile Lys Phe Asn Ala Val
   740 745 750
   Asp Asn Pro Val Lys Gln Ala Pro Cys Pro Gln Tyr Lys Tyr Pro Asn
   755 760 765
   Thr Ile Phe Arg Tyr Ala Arg Ile Ala Asp Tyr Ile Lys Leu Gly Gly
   770 775 780
   Asn Thr Asp Glu Glu Lys Val Asp Leu Leu Ile Asn Lys Ile His Glu
   785 790 795 800
   Leu Lys Lys Ala Leu Asn Ile Pro Thr Ser Ile Lys Asp Ala Gly Val
   805 810 815
   Leu Glu Glu Asn Phe Tyr Ser Ser Leu Asp Arg Ile Ser Glu Leu Ala
   820 825 830
   Leu Asp Asp Gln Cys Thr Gly Ala Asn Pro Arg Phe Pro Leu Thr Ser
   835 840 845
   Glu Ile Lys Glu Met Tyr Ile Asn Cys Phe Lys Lys Gln Pro
   850 855 860
<210> 225
   <211> 2577
   <212> DNA
   <213> Clost r i di um acet obut yl i cum
<400> 225
   atgaaagtta caaatcaaaa agaactaaaa caaaagctaa atgaattgag agaagcgcaa     60
   aagaagtttg caacctatac tcaagagcaa gttgataaaa tttttaaaca atgtgccata    120
   gccgcagcta aagaaagaat aaacttagct aaattagcag tagaagaaac aggaataggt    180
   cttgtagaag ataaaattat aaaaaatcat tttgcagcag aatatatata caataaatat    240
   aaaaatgaaa aaacttgtgg cataatagac catgacgatt ctttaggcat aacaaaggtt    300
   gctgaaccaa ttggaattgt tgcagccata gttcctacta ctaatccaac ttccacagca    360
   attttcaaat cattaatttc tttaaaaaca agaaacgcaa tattcttttc accacatcca    420
   cgtgcaaaaa aatctacaat tgctgcagca aaattaattt tagatgcagc tgttaaagca    480
   ggagcaccta aaaatataat aggctggata gatgagccat caatagaact ttctcaagat    540
   ttgatgagtg aagctgatat aatattagca acaggaggtc cttcaatggt taaagcggcc    600
   tattcatctg gaaaacctgc aattggtgtt ggagcaggaa atacaccagc aataatagat    660
   gagagtgcag atatagatat ggcagtaagc tccataattt tatcaaagac ttatgacaat    720
   ggagtaatat gcgcttctga acaatcaata ttagttatga attcaatata cgaaaaagtt    780
   aaagaggaat ttgtaaaacg aggatcatat atactcaatc aaaatgaaat agctaaaata    840
   aaagaaacta tgtttaaaaa tggagctatt aatgctgaca tagttggaaa atctgcttat    900
   ataattgcta aaatggcagg aattgaagtt cctcaaacta caaagatact tataggcgaa    960
   gtacaatctg ttgaaaaaag cgagctgttc tcacatgaaa aactatcacc agtacttgca   1020
   atgtataaag ttaaggattt tgatgaagct ctaaaaaagg cacaaaggct aatagaatta   1080
   ggtggaagtg gacacacgtc atctttatat atagattcac aaaacaataa ggataaagtt   1140
   aaagaatttg gattagcaat gaaaacttca aggacattta ttaacatgcc ttcttcacag   1200
   ggagcaagcg gagatttata caattttgcg atagcaccat catttactct tggatgcggc   1260
   acttggggag gaaactctgt atcgcaaaat gtagagccta aacatttatt aaatattaaa   1320
   agtgttgctg aaagaaggga aaatatgctt tggtttaaag tgccacaaaa aatatatttt   1380
   aaatatggat gtcttagatt tgcattaaaa gaattaaaag atatgaataa gaaaagagcc   1440
   tttatagtaa cagataaaga tctttttaaa cttggatatg ttaataaaat aacaaaggta   1500
   ctagatgaga tagatattaa atacagtata tttacagata ttaaatctga tccaactatt   1560
   gattcagtaa aaaaaggtgc taaagaaatg cttaactttg aacctgatac tataatctct   1620
   attggtggtg gatcgccaat ggatgcagca aaggttatgc acttgttata tgaatatcca   1680
   gaagcagaaa ttgaaaatct agctataaac tttatggata taagaaagag aatatgcaat 1740
   ttccctaaat taggtacaaa ggcgatttca gtagctattc ctacaactgc tggtaccggt 1800
   tcagaggcaa caccttttgc agttataact aatgatgaaa caggaatgaa atacccttta 1860
   acttcttatg aattgacccc aaacatggca ataatagata ctgaattaat gttaaatatg 1920
   cctagaaaat taacagcagc aactggaata gatgcattag ttcatgctat agaagcatat 1980
   gtttcggtta tggctacgga ttatactgat gaattagcct taagagcaat aaaaatgata 2040
   tttaaatatt tgcctagagc ctataaaaat gggactaacg acattgaagc aagagaaaaa 2100
   atggcacatg cctctaatat tgcggggatg gcatttgcaa atgctttctt aggtgtatgc 2160
   cattcaatgg ctcataaact tggggcaatg catcacgttc cacatggaat tgcttgtgct 2220
   gtattaatag aagaagttat taaatataac gctacagact gtccaacaaa gcaaacagca 2280
   ttccctcaat ataaatctcc taatgctaag agaaaatatg ctgaaattgc agagtatttg 2340
   aatttaaagg gtactagcga taccgaaaag gtaacagcct taatagaagc tatttcaaag 2400
   ttaaagatag atttgagtat tccacaaaat ataagtgccg ctggaataaa taaaaaagat 2460
   ttttataata cgctagataa aatgtcagag cttgcttttg atgaccaatg tacaacagct 2520
   aatcctaggt atccacttat aagtgaactt aaggatatct atataaaatc attttaa 2577
<210> 226
   <211 > 800
   <212> PRT
   <213> Cl ost r i di um acet obut yl i cum
<400> 226
   Met Lys Val Thr Asn Gln Lys Glu Leu Lys Gln Lys Leu Asn Glu Leu
   1 5 10 15
   Arg Glu Ala Gln Lys Lys Phe Ala Thr Tyr Thr Gln Glu Gln Val Asp
   20 25 30
   Lys Ile Phe Lys Gln Cys Ala Ile Ala Ala Ala Lys Glu Arg Ile Asn
   35 40 45
   Leu Ala Lys Leu Ala Val Glu Glu Thr Gly Ile Gly Leu Val Glu Asp
   50 55 60
   Lys Ile Ile Lys Asn His Phe Ala Ala Glu Tyr Ile Tyr Asn Lys Tyr
   65 70 75 80
   Lys Asn Glu Lys Thr Cys Gly Ile Ile Asp His Asp Asp Ser Leu Gly
   85 90 95
   Ile Thr Lys Val Ala Glu Pro Ile Gly Ile Val Ala Ala Ile Val Pro
   100 105 110
   Thr Thr Asn Pro Thr Ser Thr Ala Ile Phe Lys Ser Leu Ile Ser Leu
   115 120 125
   Lys Thr Arg Asn Ala Ile Phe Phe Ser Pro His Pro Arg Ala Lys Lys
   130 135 140
   Ser Thr Ile Ala Ala Ala Lys Leu Ile Leu Asp Ala Ala Val Lys Ala
   145 150 155 160
   Gly Ala Pro Lys Asn Ile Ile Gly Trp Ile Asp Glu Pro Ser Ile Glu
   165 170 175
   Leu Ser Gln Asp Leu Met Ser Glu Ala Asp Ile Ile Leu Ala Thr Gly
   180 185 190
   Gly Pro Ser Met Val Lys Ala Ala Tyr Ser Ser Gly Lys Pro Ala Ile
   195 200 205
   Gly Val Gly Ala Gly Asn Thr Pro Ala Ile Ile Asp Glu Ser Ala Asp
   210 215 220
   Ile Asp Met Ala Val Ser Ser Ile Ile Leu Ser Lys Thr Tyr Asp Asn
   225 230 235 240
   Gly Val Ile Cys Ala Ser Glu Gln Ser Ile Leu Val Met Asn Ser Ile
   245 250 255
   Tyr Glu Lys Val Lys Glu Glu Phe Val Lys Arg Gly Ser Tyr Ile Leu
   260 265 270
   Asn Gln Asn Glu Ile Ala Lys Ile Lys Glu Thr Met Phe Lys Asn Gly
   275 280 285
   Ala Ile Asn Ala Asp Ile Val Gly Lys Ser Ala Tyr Ile Ile Ala Lys
   290 295 300
   Met Ala Gly Ile Glu Val Pro Gln Thr Thr Lys Ile Leu Ile Gly Glu
   305 310 315 320
   Val Gln Ser Val Glu Lys Ser Glu Leu Phe Ser His Glu Lys Leu Ser
   325 330 335
   Pro Val Leu Ala Met Tyr Lys Val Lys Asp Phe Asp Glu Ala Leu Lys
   340 345 350
   Lys Ala Gln Arg Leu Ile Glu Leu Gly Gly Ser Gly His Thr Ser Ser
   355 360 365
   Leu Tyr Ile Asp Ser Gln Asn Asn Lys Asp Lys Val Lys Glu Phe Gly
   370 375 380
   Leu Ala Met Lys Thr Ser Arg Thr Phe Ile Asn Met Pro Ser Ser Gln
   385 390 395 400
   Gly Ala Ser Gly Asp Leu Tyr Asn Phe Ala Ile Ala Pro Ser Phe Thr
   405 410 415
   Leu Gly Cys Gly Thr Trp Gly Gly Asn Ser Val Ser Gln Asn Val Glu
   420 425 430
   Pro Lys His Leu Leu Asn Ile Lys Ser Val Ala Glu Arg Arg Glu Asn
   435 440 445
   Met Leu Trp Phe Lys Val Pro Gln Lys Ile Tyr Phe Lys Tyr Gly Cys
   450 455 460
   Leu Arg Phe Ala Leu Lys Glu Leu Lys Asp Met Asn Lys Lys Arg Ala
   465 470 475 480
   Phe Ile Val Thr Asp Lys Asp Leu Phe Lys Leu Gly Tyr Val Asn Lys
   485 490 495
   Ile Thr Lys Val Leu Asp Glu Ile Asp Ile Lys Tyr Ser Ile Phe Thr
   500 505 510
   Asp Ile Lys Ser Asp Pro Thr Ile Asp Ser Val Lys Lys Gly Ala Lys
   515 520 525
   Glu Met Leu Asn Phe Glu Pro Asp Thr Ile Ile Ser Ile Gly Gly Gly
   530 535 540
   Ser Pro Met Asp Ala Ala Lys Val Met His Leu Leu Tyr Glu Tyr Pro
   545 550 555 560
   Glu Ala Glu Ile Glu Asn Leu Ala Ile Asn Phe Met Asp Ile Arg Lys
   565 570 575
   Arg Ile Cys Asn Phe Pro Lys Leu Gly Thr Lys Ala Ile Ser Val Ala
   580 585 590
   Ile Pro Thr Thr Ala Gly Thr Gly Ser Glu Ala Thr Pro Phe Ala Val
   595 600 605
   Ile Thr Asn Asp Glu Thr Gly Met Lys Tyr Pro Leu Thr Ser Tyr Glu
   610 615 620
   Leu Thr Pro Asn Met Ala Ile Ile Asp Thr Glu Leu Met Leu Asn Met
   625 630 635 640
   Pro Arg Lys Leu Thr Ala Ala Thr Gly Ile Asp Ala Leu Val His Ala
   645 650 655
   Ile Glu Ala Tyr Val Ser Val Met Ala Thr Asp Tyr Thr Asp Glu Leu
   660 665 670
   Ala Leu Arg Ala Ile Lys Met Ile Phe Lys Tyr Leu Pro Arg Ala Tyr
   675 680 685
   Lys Asn Gly Thr Asn Asp Ile Glu Ala Arg Glu Lys Met Ala His Ala
   690 695 700
   Ser Asn Ile Ala Gly Met Ala Phe Ala Asn Ala Phe Leu Gly Val Cys
   705 710 715 720
   His Ser Met Ala His Lys Leu Gly Ala Met His His Val Pro His Gly
   725 730 735
   Ile Ala Cys Ala Val Leu Ile Glu Glu Val Ile Lys Tyr Asn Ala Thr
   740 745 750
   Asp Cys Pro Thr Lys Gln Thr Ala Phe Pro Gln Tyr Lys Ser Pro Asn
   755 760 765
   Ala Lys Arg Lys Tyr Ala Glu Ile Ala Glu Tyr Leu Asn Leu Lys Gly
   770 775 780
   Thr Ser Asp Thr Glu Lys Val Thr Ala Leu Ile Glu Ala Ile Ser Lys
   785 790 795 800
<210> 227
   <211> 924
   <212> DNA
   <213> Pseudomonas put i da
<400> 227
   atgagcaaga aactcaaggc ggccatcata ggccccggca atatcggtac cgatctggtg     60
   atgaagatgc tccgttccga gtggattgag ccggtgtgga tggtcggcat cgaccccaac    120
   tccgacggcc tcaaacgcgc ccgcgatttc ggcatgaaga ccacagccga aggcgtcgac    180
   ggcctgctcc cgcacgtgct ggacgacgac atccgcatcg ccttcgacgc cacctcggcc    240
   tatgtgcatg ccgagaatag ccgcaagctc aacgcgcttg gcgtgctgat ggtcgacctg    300
   accccggcgg ccatcggccc ctactgcgtg ccgccggtca acctcaagca gcatgtcggc    360
   cgcctggaaa tgaacgtcaa catggtcacc tgcggcggcc aggccaccat ccccatggtc    420
   gccgcggtgt cccgcgtgca gccggtggcc tacgccgaga tcgtcgccac cgtctcctcg    480
   cgctcggtcg gcccgggcac gcgcaagaac atcgacgagt tcacccgcac caccgccggc    540
   gccatcgagc aggtcggcgg cgccagggaa ggcaaggcga tcatcgtcat caacccggcc    600
   gagccgccgc tgatgatgcg cgacaccatc cactgcctga ccgacagcga gccggaccag    660
   gctgcgatca ccgcttcggt tcacgcgatg atcgccgagg tgcagaaata cgtgcccggc    720
   taccgcctga agaacggccc ggtgttcgac ggcaaccgcg tgtcgatctt catggaagtc    780
   gaaggcctgg gcgactacct gcccaagtac gccggcaacc tcgacatcat gaccgccgcc    840
   gcgctgcgta ccggcgagat gttcgccgag gaaatcgccg ccggcaccat tcaactgccg    900
   cgtcgcgaca tcgcgctggc ttga                                           924
<210> 228
   <211> 307
   <212> PRT
   <213> Pseudomonas put i da
<400> 228
   Met Ser Lys Lys Leu Lys Ala Ala Ile Ile Gly Pro Gly Asn Ile Gly
   1 5 10 15
   Thr Asp Leu Val Met Lys Met Leu Arg Ser Glu Trp Ile Glu Pro Val
   20 25 30
   Trp Met Val Gly Ile Asp Pro Asn Ser Asp Gly Leu Lys Arg Ala Arg
   35 40 45
   Asp Phe Gly Met Lys Thr Thr Ala Glu Gly Val Asp Gly Leu Leu Pro
   50 55 60
   His Val Leu Asp Asp Asp Ile Arg Ile Ala Phe Asp Ala Thr Ser Ala
   65 70 75 80
   Tyr Val His Ala Glu Asn Ser Arg Lys Leu Asn Ala Leu Gly Val Leu
   85 90 95
   Met Val Asp Leu Thr Pro Ala Ala Ile Gly Pro Tyr Cys Val Pro Pro
   100 105 110
   Val Asn Leu Lys Gln His Val Gly Arg Leu Glu Met Asn Val Asn Met
   115 120 125
   Val Thr Cys Gly Gly Gln Ala Thr Ile Pro Met Val Ala Ala Val Ser
   130 135 140
   Arg Val Gln Pro Val Ala Tyr Ala Glu Ile Val Ala Thr Val Ser Ser
   145 150 155 160
   Arg Ser Val Gly Pro Gly Thr Arg Lys Asn Ile Asp Glu Phe Thr Arg
   165 170 175
   Thr Thr Ala Gly Ala Ile Glu Gln Val Gly Gly Ala Arg Glu Gly Lys
   180 185 190
   Ala Ile Ile Val Ile Asn Pro Ala Glu Pro Pro Leu Met Met Arg Asp
   195 200 205
   Thr Ile His Cys Leu Thr Asp Ser Glu Pro Asp Gln Ala Ala Ile Thr
   210 215 220
   Ala Ser Val His Ala Met Ile Ala Glu Val Gln Lys Tyr Val Pro Gly
   225 230 235 240
   Tyr Arg Leu Lys Asn Gly Pro Val Phe Asp Gly Asn Arg Val Ser Ile
   245 250 255
   Phe Met Glu Val Glu Gly Leu Gly Asp Tyr Leu Pro Lys Tyr Ala Gly
   260 265 270
   Asn Leu Asp Ile Met Thr Ala Ala Ala Leu Arg Thr Gly Glu Met Phe
   275 280 285
   Ala Glu Glu Ile Ala Ala Gly Thr Ile Gln Leu Pro Arg Arg Asp Ile
   290 295 300
   Ala Leu Ala
   305
<210> 229
   <211> 924
   <212> DNA
   <213> Ther mus thermophilus
<400> 229
   atgtccgaaa gggttaaggt agccatcctg ggctccggca acatcgggac ggacctgatg 60
   tacaagctcc tgaagaaccc gggccacatg gagcttgtgg cggtggtggg gatagacccc 120
   aagtccgagg gcctggcccg ggcgcgggcc ttagggttag aggcgagcca cgaagggatc 180
   gcctacatcc tggagaggcc ggagatcaag atcgtctttg acgccaccag cgccaaggcc 240
   cacgtgcgcc acgccaagct cctgagggag gcggggaaga tcgccataga cctcacgccg 300
   gcggcccggg gcccttacgt ggtgcccccg gtgaacctga aggaacacct ggacaaggac 360
   aacgtgaacc tcatcacctg cggggggcag gccaccatcc ccctggtcta cgcggtgcac 420
   cgggtggccc ccgtgctcta cgcggagatg gtctccacgg tggcctcccg ctccgcgggc 480
   cccggcaccc ggcagaacat cgacgagttc accttcacca ccgcccgggg cctggaggcc 540
   atcggggggg ccaagaaggg gaaggccatc atcatcctga acccggcgga accccccatc 600
   ctcatgacca acaccgtgcg ctgcatcccc gaggacgagg gctttgaccg ggaggccgtg 660
   gtggcgagcg tccgggccat ggagcgggag gtccaggcct acgtgcccgg ctaccgcctg 720
   aaggcggacc cggtgtttga gaggcttccc accccctggg gggagcgcac cgtggtctcc 780
   atgctcctgg aggtggaggg ggcgggggac tatttgccca aatacgccgg caacctggac 840
   atcatgacgg cttctgcccg gagggtgggg gaggtcttcg cccagcacct cctggggaag 900
   cccgtggagg aggtggtggc gtga 924
<210> 230
   <211> 307
   <212> PRT
   <213> Thermus thermophilus
<400> 230
   Met Ser Glu Arg Val Lys Val Ala Ile Leu Gly Ser Gly Asn Ile Gly
   1 5 10 15
   Thr Asp Leu Met Tyr Lys Leu Leu Lys Asn Pro Gly His Met Glu Leu
   20 25 30
   Val Ala Val Val Gly Ile Asp Pro Lys Ser Glu Gly Leu Ala Arg Ala
   35 40 45
   Arg Ala Leu Gly Leu Glu Ala Ser His Glu Gly Ile Ala Tyr Ile Leu
   50 55 60
   Glu Arg Pro Glu Ile Lys Ile Val Phe Asp Ala Thr Ser Ala Lys Ala
   65 70 75 80
   His Val Arg His Ala Lys Leu Leu Arg Glu Ala Gly Lys Ile Ala Ile
   85 90 95
   Asp Leu Thr Pro Ala Ala Arg Gly Pro Tyr Val Val Pro Pro Val Asn
   100 105 110
   Leu Lys Glu His Leu Asp Lys Asp Asn Val Asn Leu Ile Thr Cys Gly
   115 120 125
   Gly Gln Ala Thr Ile Pro Leu Val Tyr Ala Val His Arg Val Ala Pro
   130 135 140
   Val Leu Tyr Ala Glu Met Val Ser Thr Val Ala Ser Arg Ser Ala Gly
   145 150 155 160
   Pro Gly Thr Arg Gln Asn Ile Asp Glu Phe Thr Phe Thr Thr Ala Arg
   165 170 175
   Gly Leu Glu Ala Ile Gly Gly Ala Lys Lys Gly Lys Ala Ile Ile Ile
   180 185 190
   Leu Asn Pro Ala Glu Pro Pro Ile Leu Met Thr Asn Thr Val Arg Cys
   195 200 205
   Ile Pro Glu Asp Glu Gly Phe Asp Arg Glu Ala Val Val Ala Ser Val
   210 215 220
   Arg Ala Met Glu Arg Glu Val Gln Ala Tyr Val Pro Gly Tyr Arg Leu
   225 230 235 240
   Lys Ala Asp Pro Val Phe Glu Arg Leu Pro Thr Pro Trp Gly Glu Arg
   245 250 255
   Thr Val Val Ser Met Leu Leu Glu Val Glu Gly Ala Gly Asp Tyr Leu
   260 265 270
   Pro Lys Tyr Ala Gly Asn Leu Asp Ile Met Thr Ala Ser Ala Arg Arg
   275 280 285
   Val Gly Glu Val Phe Ala Gln His Leu Leu Gly Lys Pro Val Glu Glu
   290 295 300
   Val Val Ala
   305
<210> 231
   <211> 1254
   <212> DNA
   <213> Escher i chi a col i
<400> 231
   atgacattct ccctttttgg tgacaaattt acccgccact ccggcattac gctgttgatg 60
   gaagatctga acgacggttt acgcacgcct ggcgcgatta tgctcggcgg cggtaatccg 120
   gcgcagatcc cggaaatgca ggactacttc cagacgctac tgaccgacat gctggaaagt 180
   ggcaaagcga ctgatgcact gtgtaactac gacggtccac aggggaaaac ggagctactc 240
   acactgcttg ccggaatgct gcgcgagaag ttgggttggg atatcgaacc acagaatatt 300
   gcactaacaa acggcagcca gagcgcgttt ttctacttat ttaacctgtt tgccggacgc 360
   cgtgccgatg gtcgggtcaa aaaagtgctg ttcccgcttg caccggaata cattggctat 420
   gctgacgccg gactggaaga agatctgttt gtctctgcgc gtccgaatat tgaactgctg 480
   ccggaaggcc agtttaaata ccacgtcgat tttgagcatc tgcatattgg cgaagaaacc 540
   gggatgattt gcgtctcccg gccgacgaat ccaacaggca atgtgattac tgacgaagag 600
   ttgctgaagc ttgacgcgct ggcgaatcaa cacggcattc cgctggtgat tgataacgct 660
   tatggcgtcc cgttcccggg tatcatcttc agtgaagcgc gcccgctatg gaatccgaat 720
   atcgtgctgt gcatgagtct ttccaagctg ggtctacctg gctcccgctg cggcattatc 780
   atcgccaatg aaaaaatcat caccgccatc accaatatga acggcattat cagcctggca 840
   cctggcggta ttggtccggc gatgatgtgt gaaatgatta agcgtaacga tctgctgcgc 900
   ctgtctgaaa cagtcatcaa accgttttac taccagcgtg ttcaggaaac tatcgccatc 960
   attcgccgct atttaccgga aaatcgctgc ctgattcata aaccggaagg agccattttc 1020
   ctctggctat ggtttaagga tttgcccatt acgaccaagc agctctatca gcgcctgaaa 1080
   gcacgcggcg tgctgatggt gccggggcac aacttcttcc cagggctgga taaaccgtgg 1140
   ccgcatacgc atcaatgtat gcgcatgaac tacgtaccag agccggagaa aattgaggcg 1200
   ggggtgaaga ttctggcgga agagatagaa agagcctggg ctgaaagtca ctaa 1254
<210> 232
   <211> 417
   <212> PRT
   <213> Escherichia coli
<400> 232
   Met Thr Phe Ser Leu Phe Gly Asp Lys Phe Thr Arg His Ser Gly Ile
   1 5 10 15
   Thr Leu Leu Met Glu Asp Leu Asn Asp Gly Leu Arg Thr Pro Gly Ala
   20 25 30
   Ile Met Leu Gly Gly Gly Asn Pro Ala Gln Ile Pro Glu Met Gln Asp
   35 40 45
   Tyr Phe Gln Thr Leu Leu Thr Asp Met Leu Glu Ser Gly Lys Ala Thr
   50 55 60
   Asp Ala Leu Cys Asn Tyr Asp Gly Pro Gln Gly Lys Thr Glu Leu Leu
   65 70 75 80
   Thr Leu Leu Ala Gly Met Leu Arg Glu Lys Leu Gly Trp Asp Ile Glu
   85 90 95
   Pro Gln Asn Ile Ala Leu Thr Asn Gly Ser Gln Ser Ala Phe Phe Tyr
   100 105 110
   Leu Phe Asn Leu Phe Ala Gly Arg Arg Ala Asp Gly Arg Val Lys Lys
   115 120 125
   Val Leu Phe Pro Leu Ala Pro Glu Tyr Ile Gly Tyr Ala Asp Ala Gly
   130 135 140
   Leu Glu Glu Asp Leu Phe Val Ser Ala Arg Pro Asn Ile Glu Leu Leu
   145 150 155 160
   Pro Glu Gly Gln Phe Lys Tyr His Val Asp Phe Glu His Leu His Ile
   165 170 175
   Gly Glu Glu Thr Gly Met Ile Cys Val Ser Arg Pro Thr Asn Pro Thr
   180 185 190
   Gly Asn Val Ile Thr Asp Glu Glu Leu Leu Lys Leu Asp Ala Leu Ala
   195 200 205
   Asn Gln His Gly Ile Pro Leu Val Ile Asp Asn Ala Tyr Gly Val Pro
   210 215 220
   Phe Pro Gly Ile Ile Phe Ser Glu Ala Arg Pro Leu Trp Asn Pro Asn
   225 230 235 240
   Ile Val Leu Cys Met Ser Leu Ser Lys Leu Gly Leu Pro Gly Ser Arg
   245 250 255
   Cys Gly Ile Ile Ile Ala Asn Glu Lys Ile Ile Thr Ala Ile Thr Asn
   260 265 270
   Met Asn Gly Ile Ile Ser Leu Ala Pro Gly Gly Ile Gly Pro Ala Met
   275 280 285
   Met Cys Glu Met Ile Lys Arg Asn Asp Leu Leu Arg Leu Ser Glu Thr
   290 295 300
   Val Ile Lys Pro Phe Tyr Tyr Gln Arg Val Gln Glu Thr Ile Ala Ile
   305 310 315 320
   Ile Arg Arg Tyr Leu Pro Glu Asn Arg Cys Leu Ile His Lys Pro Glu
   325 330 335
   Gly Ala Ile Phe Leu Trp Leu Trp Phe Lys Asp Leu Pro Ile Thr Thr
   340 345 350
   Lys Gln Leu Tyr Gln Arg Leu Lys Ala Arg Gly Val Leu Met Val Pro
   355 360 365
   Gly His Asn Phe Phe Pro Gly Leu Asp Lys Pro Trp Pro His Thr His
   370 375 380
   Gln Cys Met Arg Met Asn Tyr Val Pro Glu Pro Glu Lys Ile Glu Ala
   385 390 395 400
   Gly Val Lys Ile Leu Ala Glu Glu Ile Glu Arg Ala Trp Ala Glu Ser
   405 410 415
   His
<210> 233
   <211> 1278
   <212> DNA
   <213> Bacillus li cheni formis
<400> 233
   ttataagtat tcaacctgtt tctcatatac acccttcgca attttagcta aaacatcgat 60
   tccccttata atatcttcat ccgccgcggt taggctgatt cgtatacact ggtgtgaatg 120
   cgccaggcgc cgggattgac ggtgaaagaa agatgatccg ggaacgataa tgactccatc 180
   cgctttcata tactcataca gcgctgcatc ggtcaccggc aggtcttcaa accacagcca 240
   tccgaaaagc gatccttccc cttgatgcag ataccatttg atgtcttcag gcatcttgca 300
   taaaagcgtt tccttgagca gcatgaattt attgcggtaa tatggcctga cttcattcag 360
   cgacacgtcg gcgaggcgcc cgtcattcaa tactgatgca gccatatact gccccagcct 420
   tgaagaatgg atcgccgcat tcgactgaaa agcttccatt gcctgaatat accgggacgg 480
   cccgatggcg attccgatcc tttcgccagg caggccggct tttgaaaggc tcatacagtg 540
   aatgatctgc tcgttgaaaa tcggttccat gtcgataaag tgaatcgccg gaaaaggcgg 600
   agcatatgcg gaatcaatga acagcggaac attcgcttct cggcatgcgt ctgaaatgaa 660
   tgctacatct tctttaggca agatgtttcc gcaaggattg ttcgggcgcg atagcaagac 720
   agcaccgatg cgcatcctct ctaaaaaccc cttacggtcg agctcatatc gaaacgtatg 780
   atcatccaat ttcgatatga gcggagggat cccctcaatc atctcccgct ccagtgccgc 840
   cccgctgtat cccgaatagt caggcagcat cgggatcaag gcttttttca tcacagatcc 900
   gcttcccatt ccgcaaaacg aattgatcgc cagaaaaaac agctgctggc ttccggctgt 960
   aatcaacacg ttctcttttc gaatgccggc gctataccgc tctgaaaaga agcggacaac 1020
   acttgcaatc agttcatcgg ttccatagct cgatccgtat tggccgatca ccgaagaaaa 1080
   cctgtcatcg tcaaggagat cggcaagagc cgacttccac atggctgaca cgccgggcaa 1140
   aatcatcgga ttgcccgcac ttaaattaat gtatgaccgt tcaccgccgg ccaggacttc 1200
   ctgaatatcg ctcatcacag ccctgacccc tgttttctca atcattttct ctccgatttt 1260
   gcttaatggc ggcttcac 1278
<210> 234
   <211> 425
   <212> PRT
   <213> Bacillus licheni formis
<400> 234
   Met Lys Pro Pro Leu Ser Lys Ile Gly Glu Lys Met Ile Glu Lys Thr
   1 5 10 15
   Gly Val Arg Ala Val Met Ser Asp Ile Gln Glu Val Leu Ala Gly Gly
   20 25 30
   Glu Arg Ser Tyr Ile Asn Leu Ser Ala Gly Asn Pro Met Ile Leu Pro
   35 40 45
   Gly Val Ser Ala Met Trp Lys Ser Ala Leu Ala Asp Leu Leu Asp Asp
   50 55 60
   Asp Arg Phe Ser Ser Val Ile Gly Gln Tyr Gly Ser Ser Tyr Gly Thr
   65 70 75 80
   Asp Glu Leu Ile Ala Ser Val Val Arg Phe Phe Ser Glu Arg Tyr Ser
   85 90 95
   Ala Gly Ile Arg Lys Glu Asn Val Leu Ile Thr Ala Gly Ser Gln Gln
   100 105 110
   Leu Phe Phe Leu Ala Ile Asn Ser Phe Cys Gly Met Gly Ser Gly Ser
   115 120 125
   Val Met Lys Lys Ala Leu Ile Pro Met Leu Pro Asp Tyr Ser Gly Tyr
   130 135 140
   Ser Gly Ala Ala Leu Glu Arg Glu Met Ile Glu Gly Ile Pro Pro Leu
   145 150 155 160
   Ile Ser Lys Leu Asp Asp His Thr Phe Arg Tyr Glu Leu Asp Arg Lys
   165 170 175
   Gly Phe Leu Glu Arg Met Arg Ile Gly Ala Val Leu Leu Ser Arg Pro
   180 185 190
   Asn Asn Pro Cys Gly Asn Ile Leu Pro Lys Glu Asp Val Ala Phe Ile
   195 200 205
   Ser Asp Ala Cys Arg Glu Ala Asn Val Pro Leu Phe Ile Asp Ser Ala
   210 215 220
   Tyr Ala Pro Pro Phe Pro Ala Ile His Phe Ile Asp Met Glu Pro Ile
   225 230 235 240
   Phe Asn Glu Gln Ile Ile His Cys Met Ser Leu Ser Lys Ala Gly Leu
   245 250 255
   Pro Gly Glu Arg Ile Gly Ile Ala Ile Gly Pro Ser Arg Tyr Ile Gln
   260 265 270
   Ala Met Glu Ala Phe Gln Ser Asn Ala Ala Ile His Ser Ser Arg Leu
   275 280 285
   Gly Gln Tyr Met Ala Ala Ser Val Leu Asn Asp Gly Arg Leu Ala Asp
   290 295 300
   Val Ser Leu Asn Glu Val Arg Pro Tyr Tyr Arg Asn Lys Phe Met Leu
   305 310 315 320
   Leu Lys Glu Thr Leu Leu Cys Lys Met Pro Glu Asp Ile Lys Trp Tyr
   325 330 335
   Leu His Gln Gly Glu Gly Ser Leu Phe Gly Trp Leu Trp Phe Glu Asp
   340 345 350
   Leu Pro Val Thr Asp Ala Ala Leu Tyr Glu Tyr Met Lys Ala Asp Gly
   355 360 365
   Val Ile Ile Val Pro Gly Ser Ser Phe Phe His Arg Gln Ser Arg Arg
   370 375 380
   Leu Ala His Ser His Gln Cys Ile Arg Ile Ser Leu Thr Ala Ala Asp
   385 390 395 400
   Glu Asp Ile Ile Arg Gly Ile Asp Val Leu Ala Lys Ile Ala Lys Gly
   405 410 415
   Val Tyr Glu Lys Gln Val Glu Tyr Leu
   420 425
<210> 235
   <211> 930
   <212> DNA
   <213> Escherichiacoli
<400> 235
   atgaccacga agaaagctga ttacatttgg ttcaatgggg agatggttcg ctgggaagac 60
   gcgaaggtgc atgtgatgtc gcacgcgctg cactatggca cttcggtttt tgaaggcatc 120
   cgttgctacg actcgcacaa aggaccggtt gtattccgcc atcgtgagca tatgcagcgt 180
   ctgcatgact ccgccaaaat ctatcgcttc ccggtttcgc agagcattga tgagctgatg 240
   gaagcttgtc gtgacgtgat ccgcaaaaac aatctcacca gcgcctatat ccgtccgctg 300
   atcttcgtcg gtgatgttgg catgggagta aacccgccag cgggatactc aaccgacgtg 360
   attatcgctg ctttcccgtg gggagcgtat ctgggcgcag aagcgctgga gcaggggatc 420
   gatgcgatgg tttcctcctg gaaccgcgca gcaccaaaca ccatcccgac ggcggcaaaa 480
   gccggtggta actacctctc ttccctgctg gtgggtagcg aagcgcgccg ccacggttat 540
   caggaaggta tcgcgctgga tgtgaacggt tatatctctg aaggcgcagg cgaaaacctg 600
   tttgaagtga aagatggtgt gctgttcacc ccaccgttca cctcctccgc gctgccgggt 660
   attacccgtg atgccatcat caaactggcg aaagagctgg gaattgaagt acgtgagcag 720
   gtgctgtcgc gcgaatccct gtacctggcg gatgaagtgt ttatgtccgg tacggcggca 780
   gaaatcacgc cagtgcgcag cgtagacggt attcaggttg gcgaaggccg ttgtggcccg 840
   gttaccaaac gcattcagca agccttcttc ggcctcttca ctggcgaaac cgaagataaa 900
   tggggctggt tagatcaagt taatcaataa 930
<210> 236
   <211> 309
   <212> PRT
   <213> Escherichia coli
<400> 236
   Met Thr Thr Lys Lys Ala Asp Tyr Ile Trp Phe Asn Gly Glu Met Val
   1 5 10 15
   Arg Trp Glu Asp Ala Lys Val His Val Met Ser His Ala Leu His Tyr
   20 25 30
   Gly Thr Ser Val Phe Glu Gly Ile Arg Cys Tyr Asp Ser His Lys Gly
   35 40 45
   Pro Val Val Phe Arg His Arg Glu His Met Gln Arg Leu His Asp Ser
   50 55 60
   Ala Lys Ile Tyr Arg Phe Pro Val Ser Gln Ser Ile Asp Glu Leu Met
   65 70 75 80
   Glu Ala Cys Arg Asp Val Ile Arg Lys Asn Asn Leu Thr Ser Ala Tyr
   85 90 95
   Ile Arg Pro Leu Ile Phe Val Gly Asp Val Gly Met Gly Val Asn Pro
   100 105 110
   Pro Ala Gly Tyr Ser Thr Asp Val Ile Ile Ala Ala Phe Pro Trp Gly
   115 120 125
   Ala Tyr Leu Gly Ala Glu Ala Leu Glu Gln Gly Ile Asp Ala Met Val
   130 135 140
   Ser Ser Trp Asn Arg Ala Ala Pro Asn Thr Ile Pro Thr Ala Ala Lys
   145 150 155 160
   Ala Gly Gly Asn Tyr Leu Ser Ser Leu Leu Val Gly Ser Glu Ala Arg
   165 170 175
   Arg His Gly Tyr Gln Glu Gly Ile Ala Leu Asp Val Asn Gly Tyr Ile
   180 185 190
   Ser Glu Gly Ala Gly Glu Asn Leu Phe Glu Val Lys Asp Gly Val Leu
   195 200 205
   Phe Thr Pro Pro Phe Thr Ser Ser Ala Leu Pro Gly Ile Thr Arg Asp
   210 215 220
   Ala Ile Ile Lys Leu Ala Lys Glu Leu Gly Ile Glu Val Arg Glu Gln
   225 230 235 240
   Val Leu Ser Arg Glu Ser Leu Tyr Leu Ala Asp Glu Val Phe Met Ser
   245 250 255
   Gly Thr Ala Ala Glu Ile Thr Pro Val Arg Ser Val Asp Gly Ile Gln
   260 265 270
   Val Gly Glu Gly Arg Cys Gly Pro Val Thr Lys Arg Ile Gln Gln Ala
   275 280 285
   Phe Phe Gly Leu Phe Thr Gly Glu Thr Glu Asp Lys Trp Gly Trp Leu
   290 295 300
   Asp Gln Val Asn Gln
   305
<210> 237
   <211> 1131
   <212> DNA
   <213> Saccharomyces cerevisiae
<400> 237
   atgaccttgg cacccctaga cgcctccaaa gttaagataa ctaccacaca acatgcatct 60
   aagccaaaac cgaacagtga gttagtgttt ggcaagagct tcacggacca catgttaact 120
   gcggaatgga cagctgaaaa agggtggggt accccagaga ttaaacctta tcaaaatctg 180
   tctttagacc cttccgcggt ggttttccat tatgcttttg agctattcga agggatgaag 240
   gcttacagaa cggtggacaa caaaattaca atgtttcgtc cagatatgaa tatgaagcgc 300
   atgaataagt ctgctcagag aatctgtttg ccaacgttcg acccagaaga gttgattacc 360
   ctaattggga aactgatcca gcaagataag tgcttagttc ctgaaggaaa aggttactct 420
   ttatatatca ggcctacatt aatcggcact acggccggtt taggggtttc cacgcctgat 480
   agagccttgc tatatgtcat ttgctgccct gtgggtcctt attacaaaac tggatttaag 540
   gcggtcagac tggaagccac tgattatgcc acaagagctt ggccaggagg ctgtggtgac 600
   aagaaactag gtgcaaacta cgccccctgc gtcctgccac aattgcaagc tgcttcaagg 660
   ggttaccaac aaaatttatg gctatttggt ccaaataaca acattactga agtcggcacc 720
   atgaatgctt ttttcgtgtt taaagatagt aaaacgggca agaaggaact agttactgct 780
   ccactagacg gtaccatttt ggaaggtgtt actagggatt ccattttaaa tcttgctaaa 840
   gaaagactcg aaccaagtga atggaccatt agtgaacgct acttcactat aggcgaagtt 900
   actgagagat ccaagaacgg tgaactactt gaagcctttg gttctggtac tgctgcgatt 960
   gtttctccca ttaaggaaat cggctggaaa ggcgaacaaa ttaatattcc gttgttgccc 1020
   ggcgaacaaa ccggtccatt ggccaaagaa gttgcacaat ggattaatgg aatccaatat 1080
   ggcgagactg agcatggcaa ttggtcaagg gttgttactg atttgaactg a 1131

<210> 238
   <211> 376
   <212> PRT
   <213> Saccharomyces cerevisiae
<400> 238
   Met Thr Leu Ala Pro Leu Asp Ala Ser Lys Val Lys Ile Thr Thr Thr
   1 5 10 15
   Gln His Ala Ser Lys Pro Lys Pro Asn Ser Glu Leu Val Phe Gly Lys
   20 25 30
   Ser Phe Thr Asp His Met Leu Thr Ala Glu Trp Thr Ala Glu Lys Gly
   35 40 45
   Trp Gly Thr Pro Glu Ile Lys Pro Tyr Gln Asn Leu Ser Leu Asp Pro
   50 55 60
   Ser Ala Val Val Phe His Tyr Ala Phe Glu Leu Phe Glu Gly Met Lys
   65 70 75 80
   Ala Tyr Arg Thr Val Asp Asn Lys Ile Thr Met Phe Arg Pro Asp Met
   85 90 95
   Asn Met Lys Arg Met Asn Lys Ser Ala Gln Arg Ile Cys Leu Pro Thr
   100 105 110
   Phe Asp Pro Glu Glu Leu Ile Thr Leu Ile Gly Lys Leu Ile Gln Gln
   115 120 125
   Asp Lys Cys Leu Val Pro Glu Gly Lys Gly Tyr Ser Leu Tyr Ile Arg
   130 135 140
   Pro Thr Leu Ile Gly Thr Thr Ala Gly Leu Gly Val Ser Thr Pro Asp
   145                 150                 155                 160
   Arg Ala Leu Leu Tyr Val Ile Cys Cys Pro Val Gly Pro Tyr Tyr Lys
                   165                 170                 175
   Thr Gly Phe Lys Ala Val Arg Leu Glu Ala Thr Asp Tyr Ala Thr Arg
               180                 185                 190
   Ala Trp Pro Gly Gly Cys Gly Asp Lys Lys Leu Gly Ala Asn Tyr Ala
           195                 200                 205
   Pro Cys Val Leu Pro Gln Leu Gln Ala Ala Ser Arg Gly Tyr Gln Gln
       210                 215                 220
   Asn Leu Trp Leu Phe Gly Pro Asn Asn Asn Ile Thr Glu Val Gly Thr
   225                 230                 235                 240
   Met Asn Ala Phe Phe Val Phe Lys Asp Ser Lys Thr Gly Lys Lys Glu
                   245                 250                 255
   Leu Val Thr Ala Pro Leu Asp Gly Thr Ile Leu Glu Gly Val Thr Arg
               260                 265                 270
   Asp Ser Ile Leu Asn Leu Ala Lys Glu Arg Leu Glu Pro Ser Glu Trp
           275                 280                 285
   Thr Ile Ser Glu Arg Tyr Phe Thr Ile Gly Glu Val Thr Glu Arg Ser
       290                 295                 300
   Lys Asn Gly Glu Leu Leu Glu Ala Phe Gly Ser Gly Thr Ala Ala Ile
   305                 310                 315                 320
   Val Ser Pro Ile Lys Glu Ile Gly Trp Lys Gly Glu Gln Ile Asn Ile
                   325                 330                 335
   Pro Leu Leu Pro Gly Glu Gln Thr Gly Pro Leu Ala Lys Glu Val Ala
               340                 345                 350
   Gln Trp Ile Asn Gly Ile Gln Tyr Gly Glu Thr Glu His Gly Asn Trp
           355                 360                 365
   Ser Arg Val Val Thr Asp Leu Asn
       370                 375
<210> 239
   <211> 993
   <212> DNA
   <213> Methanobacterium thermoautotrophicum
<400> 239
   tcagatgtag gtgagccatc cgaagctgtc ctctgtctct gccctgatta tcctgaagaa      60
   ctcatcctgc agcagctttg taacgggacc ccttcgcccg gcacctatct ctataccatc     120
   aactgatctg atgggtgtta tctctgcggc tgtacctgtg aagaaggcct catctgcgat 180
   gtagagcatc tccctggtta tgggttcctc atgcacggta acaccctcgg tcctggctat 240
   ctttattacg gagtcccttg ttatccccct cagaagggat gatgaaacag ggggggtgta 300
   aatttcaccc tcactgacga ggaatatgtt ctccccgcta ccctcactta tgtagccatg 360
   gtagtccagc attatggcct catcatagcc gtgtctcaca gcctccatct tggcaagctg 420
   tgagttgagg tagttaccgc cggcctttgc catgttgggc attgtgtttg gtgccatcct 480
   ccgccaggtt gaaacaccag catcgacacc aacctcaagg gcctctgcac ccagataggc 540
   cccccattcc caggcagcca cagcgacgtc cactgggcag ttcaccgggt gaacacccat 600
   ctcaccgtat cccctgaata ccacgggtct tatatagcac tcctcaagtc cgttctccct 660
   gacggtctca actatggcat cacatatctg ctcctgggtg tagggtatgt ccatccggta 720
   tatctttgca gaatcaaaaa ggcgtttaac atgctcccgc aaacggaaga tggctgaccc 780
   cttactgttc ctgtagcacc ttattccctc aaagacagat gatccataat gcacaacatg 840
   tgagagtacg tggacggtgg cttcttccca ttcaaccatt tcaccgttta accatatctt 900
   tccactggct tcgcatgaca tgataataac ctcaggtgat ttactaggat aggttatggt 960
   tggaggccta tataatgctc tccataaccg caa 993
<210> 240
   <211> 330
   <212> PRT
   <213> Methanobacterium thermoautotrophicum
<400> 240
   Met Arg Leu Trp Arg Ala Leu Tyr Arg Pro Pro Thr Ile Thr Tyr Pro
   1 5 10 15
   Ser Lys Ser Pro Glu Val Ile Ile Met Ser Cys Glu Ala Ser Gly Lys
   20 25 30
   Ile Trp Leu Asn Gly Glu Met Val Glu Trp Glu Glu Ala Thr Val His
   35 40 45
   Val Leu Ser His Val Val His Tyr Gly Ser Ser Val Phe Glu Gly Ile
   50 55 60
   Arg Cys Tyr Arg Asn Ser Lys Gly Ser Ala Ile Phe Arg Leu Arg Glu
   65 70 75 80
   His Val Lys Arg Leu Phe Asp Ser Ala Lys Ile Tyr Arg Met Asp Ile
   85 90 95
   Pro Tyr Thr Gln Glu Gln Ile Cys Asp Ala Ile Val Glu Thr Val Arg
   100 105 110
   Glu Asn Gly Leu Glu Glu Cys Tyr Ile Arg Pro Val Val Phe Arg Gly
   115 120 125
   Tyr Gly Glu Met Gly Val His Pro Val Asn Cys Pro Val Asp Val Ala
   130 135 140
   Val Ala Ala Trp Glu Trp Gly Ala Tyr Leu Gly Ala Glu Ala Leu Glu
   145 150 155 160
   Val Gly Val Asp Ala Gly Val Ser Thr Trp Arg Arg Met Ala Pro Asn
   165 170 175
   Thr Met Pro Asn Met Ala Lys Ala Gly Gly Asn Tyr Leu Asn Ser Gln
   180 185 190
   Leu Ala Lys Met Glu Ala Val Arg His Gly Tyr Asp Glu Ala Ile Met
   195 200 205
   Leu Asp Tyr His Gly Tyr Ile Ser Glu Gly Ser Gly Glu Asn Ile Phe
   210 215 220
   Leu Val Ser Glu Gly Glu Ile Tyr Thr Pro Pro Val Ser Ser Ser Leu
   225 230 235 240
   Leu Arg Gly Ile Thr Arg Asp Ser Val Ile Lys Ile Ala Arg Thr Glu
   245 250 255
   Gly Val Thr Val His Glu Glu Pro Ile Thr Arg Glu Met Leu Tyr Ile
   260 265 270
   Ala Asp Glu Ala Phe Phe Thr Gly Thr Ala Ala Glu Ile Thr Pro Ile
   275 280 285
   Arg Ser Val Asp Gly Ile Glu Ile Gly Ala Gly Arg Arg Gly Pro Val
   290 295 300
   Thr Lys Leu Leu Gln Asp Glu Phe Phe Arg Ile Ile Arg Ala Glu Thr
   305 310 315 320
   Glu Asp Ser Phe Gly Trp Leu Thr Tyr Ile
   325 330
<210> 241
   <211> 1095
   <212> DNA
   <213> Streptomyces coelicolor
<400> 241
   tcacggccgg ggacgggcct ccgccatccg ctgctcggcg atccggtcgg ccgccgcggc 60
   cggcggaata ccgtcctcct tcgcacgtgc gaatatggcc agcgtggtgt cgtagatctt 120
   cgaggccttc gccttgcacc ggtcgaagtc gaacccgtgc agctcgtcgg cgacctggat 180
   gacaccgccg gcgttcacca catagtccgg cgcgtagagg atcccgcggt cggcgaggtc 240
   cttctcgacg cccgggtggg cgagctggtt gttggccgcg ccgcacacca ccttggcggt 300
   cagcaccggc acggtgtcgt cgttcagcgc gccgccgagc gcgcagggcg cgtagatgtc 360
   caggttctcc acccggatca gcgcgtcggt gtcggcgacg gcgaccaccg acgggtgccg 420
   ctccgtgatc ccgcgcacca cgtccttgcg cacgtccgtg acgacgacgt gggcgccctc 480
   ggcgagcagg tgctcgacca ggtggtggcc gaccttgccg acgcccgcga tgccgacggt 540
   gcggtcgcgc agcgtcgggt cgccccacag gtgctgggcg gcggcccgca tgccctggta 600
   gacgccgaag gaggtgagca cggaggagtc gcccgcgccg ccgttctccg gggaacgccc 660
   ggtcgtccag cggcactcgc gggccacgac gtccatgtcg gcgacgtagg tgccgacgtc 720
   gcacgcggtg acgtagcggc cgcccagcga ggcgacgaac cggccgtagg cgaggagcag 780
   ctcctcgctc ttgatctgct ccggatcgcc gatgatcacg gccttgccgc caccgtggtc 840
   cagaccggcc atggcgttct tgtacgacat cccgcgggcg aggttcagcg cgtcggcgac 900
   ggcctccgcc tcgctcgcgt acgggtagaa gcgggtaccg ccgagcgccg ggcccagggc 960
   ggtggagtgg agggcgatca cggccttgag gccgctggca cggtcctggc agagcacgac 1020
   ttgctcatgt cccccctgat ccgagtggaa cagggtgtgc agtacatcag caggtgcgcc    1080
   gtttacgtcg gtcac 1095
<210> 242
   <211> 364
   <212> PRT
   <213> Streptomyces coelicolor
<400> 242
   Met Thr Asp Val Asn Gly Ala Pro Ala Asp Val Leu His Thr Leu Phe
   1 5 10 15
   His Ser Asp Gln Gly Gly His Glu Gln Val Val Leu Cys Gln Asp Arg
   20 25 30
   Ala Ser Gly Leu Lys Ala Val Ile Ala Leu His Ser Thr Ala Leu Gly
   35 40 45
   Pro Ala Leu Gly Gly Thr Arg Phe Tyr Pro Tyr Ala Ser Glu Ala Glu
   50 55 60
   Ala Val Ala Asp Ala Leu Asn Leu Ala Arg Gly Met Ser Tyr Lys Asn
   65 70 75 80
   Ala Met Ala Gly Leu Asp His Gly Gly Gly Lys Ala Val Ile Ile Gly
   85 90 95
   Asp Pro Glu Gln Ile Lys Ser Glu Glu Leu Leu Leu Ala Tyr Gly Arg
   100 105 110
   Phe Val Ala Ser Leu Gly Gly Arg Tyr Val Thr Ala Cys Asp Val Gly
   115 120 125
   Thr Tyr Val Ala Asp Met Asp Val Val Ala Arg Glu Cys Arg Trp Thr
   130 135 140
   Thr Gly Arg Ser Pro Glu Asn Gly Gly Ala Gly Asp Ser Ser Val Leu
   145 150 155 160
   Thr Ser Phe Gly Val Tyr Gln Gly Met Arg Ala Ala Ala Gln His Leu
   165 170 175
   Trp Gly Asp Pro Thr Leu Arg Asp Arg Thr Val Gly Ile Ala Gly Val
   180 185 190
   Gly Lys Val Gly His His Leu Val Glu His Leu Leu Ala Glu Gly Ala
   195 200 205
   His Val Val Val Thr Asp Val Arg Lys Asp Val Val Arg Gly Ile Thr
   210 215 220
   Glu Arg His Pro Ser Val Val Ala Val Ala Asp Thr Asp Ala Leu Ile
   225 230 235 240
   Arg Val Glu Asn Leu Asp Ile Tyr Ala Pro Cys Ala Leu Gly Gly Ala
   245 250 255
   Leu Asn Asp Asp Thr Val Pro Val Leu Thr Ala Lys Val Val Cys Gly
   260 265 270
   Ala Ala Asn Asn Gln Leu Ala His Pro Gly Val Glu Lys Asp Leu Ala
   275 280 285
   Asp Arg Gly Ile Leu Tyr Ala Pro Asp Tyr Val Val Asn Ala Gly Gly
   290 295 300
   Val Ile Gln Val Ala Asp Glu Leu His Gly Phe Asp Phe Asp Arg Cys
   305 310 315 320
   Lys Ala Lys Ala Ser Lys Ile Tyr Asp Thr Thr Leu Ala Ile Phe Ala
   325 330 335
   Arg Ala Lys Glu Asp Gly Ile Pro Pro Ala Ala Ala Ala Asp Arg Ile
   340 345 350
   Ala Glu Gln Arg Met Ala Glu Ala Arg Pro Arg Pro
   355 360
<210> 243
   <211> 1095
   <212> DNA
   <213> Bacillus subtilis
<400> 243
   atggaacttt ttaaatatat ggagaaatac gattacgaac aattggtatt ctgccaggat 60
   gaacaatctg gattaaaagc gattatcgcc attcatgata caacgcttgg tccggcgctt 120
   ggcggaacga gaatgtggac atatgaaaat gaagaagcgg caattgaaga tgcgctcaga 180
   ttggcaagag gcatgaccta taagaacgcg gcggcaggct taaaccttgg cggcggaaaa 240
   acagtcatta tcggcgatcc gcgcaaagac aaaaatgagg aaatgttccg cgcgtttggc 300
   cgctatattc aaggactgaa tggcagatac atcacggctg aagatgtggg cacaacggtc 360
   gaggatatgg atatcattca tgatgagaca gactatgtca cagggatttc tcctgctttc 420
   ggctcttctg gaaatccgtc cccagtcaca gcgtacgggg tgtacagagg aatgaaggca 480
   gcagctaaag ctgctttcgg aaccgattct cttgaaggaa aaaccattgc tgtacagggt 540
   gttgggaacg tagcctataa cctttgccgc cacctgcatg aagaaggagc aaacttaatc 600
   gttacggata tcaacaaaca atctgtacag cgtgcagttg aagattttgg cgcccgtgcg 660
   gtagatcctg atgacattta ttcacaagac tgcgatattt atgcgccgtg tgcccttggt 720
   gcgactatta acgacgacac cattaaacag ctgaaggcga aagtgatcgc aggtgcggct 780
   aacaaccaat taaaagagac acgccatggt gatcaaattc acgaaatggg catcgtttat 840
   gcaccggatt acgtgattaa cgcgggcggt gtcatcaacg tggcagatga gctttacggc 900
   tataatgcag aacgtgcatt gaaaaaagtt gaaggcattt acggcaatat cgagcgtgta 960
   cttgagattt ctcagcgtga cggcattcct gcatatttag cggctgaccg cttagcagag 1020
   gaacggattg aacgcatgcg ccgctcaaga agccagtttt tgcaaaacgg ccacagtgta 1080
   ttaagcagac gttaa 1095
<210> 244
   <211> 364
   <212> PRT
   <213> Bacillus subtilis
<400> 244
   Met Glu Leu Phe Lys Tyr Met Glu Lys Tyr Asp Tyr Glu Gln Leu Val
   1 5 10 15
   Phe Cys Gln Asp Glu Gln Ser Gly Leu Lys Ala Ile Ile Ala Ile His
   20 25 30
   Asp Thr Thr Leu Gly Pro Ala Leu Gly Gly Thr Arg Met Trp Thr Tyr
   35 40 45
   Glu Asn Glu Glu Ala Ala Ile Glu Asp Ala Leu Arg Leu Ala Arg Gly
   50 55 60
   Met Thr Tyr Lys Asn Ala Ala Ala Gly Leu Asn Leu Gly Gly Gly Lys
   65 70 75 80
   Thr Val Ile Ile Gly Asp Pro Arg Lys Asp Lys Asn Glu Glu Met Phe
   85 90 95
   Arg Ala Phe Gly Arg Tyr Ile Gln Gly Leu Asn Gly Arg Tyr Ile Thr
   100 105 110
   Ala Glu Asp Val Gly Thr Thr Val Glu Asp Met Asp Ile Ile His Asp
   115 120 125
   Glu Thr Asp Tyr Val Thr Gly Ile Ser Pro Ala Phe Gly Ser Ser Gly
   130 135 140
   Asn Pro Ser Pro Val Thr Ala Tyr Gly Val Tyr Arg Gly Met Lys Ala
   145 150 155 160
   Ala Ala Lys Ala Ala Phe Gly Thr Asp Ser Leu Glu Gly Lys Thr Ile
   165 170 175
   Ala Val Gln Gly Val Gly Asn Val Ala Tyr Asn Leu Cys Arg His Leu
   180 185 190
   His Glu Glu Gly Ala Asn Leu Ile Val Thr Asp Ile Asn Lys Gln Ser
   195 200 205
   Val Gln Arg Ala Val Glu Asp Phe Gly Ala Arg Ala Val Asp Pro Asp
   210 215 220
   Asp Ile Tyr Ser Gln Asp Cys Asp Ile Tyr Ala Pro Cys Ala Leu Gly
   225 230 235 240
   Ala Thr Ile Asn Asp Asp Thr Ile Lys Gln Leu Lys Ala Lys Val Ile
   245 250 255
   Ala Gly Ala Ala Asn Asn Gln Leu Lys Glu Thr Arg His Gly Asp Gln
   260 265 270
   Ile His Glu Met Gly Ile Val Tyr Ala Pro Asp Tyr Val Ile Asn Ala
   275 280 285
   Gly Gly Val Ile Asn Val Ala Asp Glu Leu Tyr Gly Tyr Asn Ala Glu
   290 295 300
   Arg Ala Leu Lys Lys Val Glu Gly Ile Tyr Gly Asn Ile Glu Arg Val
   305 310 315 320
   Leu Glu Ile Ser Gln Arg Asp Gly Ile Pro Ala Tyr Leu Ala Ala Asp
   325 330 335
   Arg Leu Ala Glu Glu Arg Ile Glu Arg Met Arg Arg Ser Arg Ser Gln
   340 345 350
   Phe Leu Gln Asn Gly His Ser Val Leu Ser Arg Arg
   355 360
<210> 245
   <211> 1785
   <212> DNA
   <213> Streptomyces viridifaciens
<400> 245
   gtgtcaactt cctccgcttc ttccgggccg gacctcccct tcgggcccga ggacacgcca      60
   tggcagaagg ccttcagcag gctgcgggcg gtggatggcg tgccgcgcgt caccgcgccg     120
   t ccagt gat c cgcgt gaggt ct acat ggac at cccggaga t cccct t ct c caaggt ccag 180
   atccccccgg acggaatgga cgagcagcag tacgcagagg ccgagagcct cttccgccgc     240
   tacgtagacg cccagacccg caacttcgcg ggataccagg tcaccagcga cctcgactac     300
   cagcacctca gtcactatct caaccggcat ctgaacaacg tcggcgatcc ctatgagtcc     360
   agctcctaca cgctgaactc caaggtcctt gagcgagccg ttctcgacta cttcgcctcc     420
   ct gt ggaacg ccaagt ggcc ccat gacgca agcgat ccgg aaacgt act g gggt t acgt g 480
   ctgaccatgg gctccagcga aggcaacctg tacgggttgt ggaacgcacg ggactatctg     540
   tcgggcaagc tgctgcggcg ccagcaccgg gaggccggcg gcgacaaggc ctcggtcgtc     600
   tacacgcaag cgctgcgaca cgaagggcag agtccgcatg cctacgagcc ggtggcgttc     660
   t t ct cgcagg acacgcact a ct cgct cacg aaggccgt gc gggt t ct ggg cat cgacacc 720
   ttccacagca tcggcagcag tcggtatccg gacgagaacc cgctgggccc cggcactccg     780
   t ggccgaccg aagt gccct c ggt t gacggt gccat cgat g t cgacaaact cgcct cgt t g 840
   gt ccgct t ct t cgccagcaa gggct acccg at act ggt ca gcct caact a cgggt caacg 900
   t t caagggcg cct acgacga cgt cccggcc gt ggcacagg ccgt gcggga cat ct gcacg 960
   gaatacggtc tggatcggcg gcgggtatac cacgaccgca gtaaggacag tgacttcgac    1020
   gagcgcagcg gcttctggat ccacatcgat gccgccctgg gggcgggcta cgctccctac    1080
   ctgcagatgg cccgggatgc cggcatggtc gaggaggcgc cgcccgtttt cgacttccgg    1140
   ctcccggagg tgcactcgct gaccatgagc ggccacaagt ggatgggaac accgtgggca    1200
   t gcggt gt ct acat gacacg gaccgggct g cagat gaccc cgccgaagt c gt ccgagt ac 1260
   atcggggcgg ccgacaccac cttcgcgggc tcccgcaacg gcttctcgtc actgctgctg    1320
   t gggact acc t gt cccggca t t cgt at gac gat ct ggt gc gcct ggccgc cgact gcgac 1380
   cggctggccg gctacgccca cgaccggttg ctgaccttgc aggacaaact cggcatggat    1440
   ctgtgggtcg cccgcagccc gcagtccctc acggtgcgct tccgtcagcc atgtgcagac    1500
   atcgtccgca agtactcgct gtcgtgtgag acggtctacg aagacaacga gcaacggacc    1560
   t acgt acat c t ct acgccgt t ccccacct c act cgggaac t cgt ggat ga gct cgt gcgc 1620
   gatctgcgcc agcccggagc cttcaccaac gctggtgcac tggaggggga ggcctgggcc    1680
   ggggtgatcg atgccctcgg ccgcccggac cccgacggaa cctatgccgg cgccttgagc    1740
   gctccggctt ccggcccccg ctccgaggac ggcggcggga gctga                    1785
<210> 246
   <211> 594
   <212> PRT
   <213> Streptomyces viridifaciens
<400> 246
   Met Ser Thr Ser Ser Ala Ser Ser Gly Pro Asp Leu Pro Phe Gly Pro
   1 5 10 15
   Glu Asp Thr Pro Trp Gln Lys Ala Phe Ser Arg Leu Arg Ala Val Asp
   20 25 30
   Gly Val Pro Arg Val Thr Ala Pro Ser Ser Asp Pro Arg Glu Val Tyr
   35 40 45
   Met Asp Ile Pro Glu Ile Pro Phe Ser Lys Val Gln Ile Pro Pro Asp
   50 55 60
   Gly Met Asp Glu Gln Gln Tyr Ala Glu Ala Glu Ser Leu Phe Arg Arg
   65 70 75 80
   Tyr Val Asp Ala Gln Thr Arg Asn Phe Ala Gly Tyr Gln Val Thr Ser
   85 90 95
   Asp Leu Asp Tyr Gln His Leu Ser His Tyr Leu Asn Arg His Leu Asn
   100 105 110
   Asn Val Gly Asp Pro Tyr Glu Ser Ser Ser Tyr Thr Leu Asn Ser Lys
   115 120 125
   Val Leu Glu Arg Ala Val Leu Asp Tyr Phe Ala Ser Leu Trp Asn Ala
   130 135 140
   Lys Trp Pro His Asp Ala Ser Asp Pro Glu Thr Tyr Trp Gly Tyr Val
   145 150 155 160
   Leu Thr Met Gly Ser Ser Glu Gly Asn Leu Tyr Gly Leu Trp Asn Ala
   165 170 175
   Arg Asp Tyr Leu Ser Gly Lys Leu Leu Arg Arg Gln His Arg Glu Ala
   180 185 190
   Gly Gly Asp Lys Ala Ser Val Val Tyr Thr Gln Ala Leu Arg His Glu
   195 200 205
   Gly Gln Ser Pro His Ala Tyr Glu Pro Val Ala Phe Phe Ser Gln Asp
   210 215 220
   Thr His Tyr Ser Leu Thr Lys Ala Val Arg Val Leu Gly Ile Asp Thr
   225 230 235 240
   Phe His Ser Ile Gly Ser Ser Arg Tyr Pro Asp Glu Asn Pro Leu Gly
   245 250 255
   Pro Gly Thr Pro Trp Pro Thr Glu Val Pro Ser Val Asp Gly Ala Ile
   260 265 270
   Asp Val Asp Lys Leu Ala Ser Leu Val Arg Phe Phe Ala Ser Lys Gly
   275 280 285
   Tyr Pro Ile Leu Val Ser Leu Asn Tyr Gly Ser Thr Phe Lys Gly Ala
   290 295 300
   Tyr Asp Asp Val Pro Ala Val Ala Gln Ala Val Arg Asp Ile Cys Thr
   305 310 315 320
   Glu Tyr Gly Leu Asp Arg Arg Arg Val Tyr His Asp Arg Ser Lys Asp
   325 330 335
   Ser Asp Phe Asp Glu Arg Ser Gly Phe Trp Ile His Ile Asp Ala Ala
   340 345 350
   Leu Gly Ala Gly Tyr Ala Pro Tyr Leu Gln Met Ala Arg Asp Ala Gly
   355 360 365
   Met Val Glu Glu Ala Pro Pro Val Phe Asp Phe Arg Leu Pro Glu Val
   370 375 380
   His Ser Leu Thr Met Ser Gly His Lys Trp Met Gly Thr Pro Trp Ala
   385 390 395 400
   Cys Gly Val Tyr Met Thr Arg Thr Gly Leu Gln Met Thr Pro Pro Lys
   405 410 415
   Ser Ser Glu Tyr Ile Gly Ala Ala Asp Thr Thr Phe Ala Gly Ser Arg
   420 425 430
   Asn Gly Phe Ser Ser Leu Leu Leu Trp Asp Tyr Leu Ser Arg His Ser
   435 440 445
   Tyr Asp Asp Leu Val Arg Leu Ala Ala Asp Cys Asp Arg Leu Ala Gly
   450 455 460
   Tyr Ala His Asp Arg Leu Leu Thr Leu Gln Asp Lys Leu Gly Met Asp
   465 470 475 480
   Leu Trp Val Ala Arg Ser Pro Gln Ser Leu Thr Val Arg Phe Arg Gln
   485 490 495
   Pro Cys Ala Asp Ile Val Arg Lys Tyr Ser Leu Ser Cys Glu Thr Val
   500 505 510
   Tyr Glu Asp Asn Glu Gln Arg Thr Tyr Val His Leu Tyr Ala Val Pro
   515 520 525
   His Leu Thr Arg Glu Leu Val Asp Glu Leu Val Arg Asp Leu Arg Gln
   530 535 540
   Pro Gly Ala Phe Thr Asn Ala Gly Ala Leu Glu Gly Glu Ala Trp Ala
   545 550 555 560
   Gly Val Ile Asp Ala Leu Gly Arg Pro Asp Pro Asp Gly Thr Tyr Ala
   565 570 575
   Gly Ala Leu Ser Ala Pro Ala Ser Gly Pro Arg Ser Glu Asp Gly Gly
   580 585 590
   Gly Ser
<210> 247
   <211> 1323
   <212> DNA
   <213> Alcaligenes denitrificans
<400> 247
   atgagcgctg ccaaactgcc cgacctgtcc cacctctgga tgccctttac cgccaaccgg      60
   cagttcaagg cgaacccccg cctgctggcc tcggccaagg gcatgtacta cacgtctttc     120
   gacggccgcc agatcctgga cggcacggcc ggcctgtggt gcgtgaacgc cggccactgc     180
   cgcgaagaaa tcgtctccgc catcgccagc caggccggcg tcatggacta cgcgccgggg     240
   ttccagctcg gccacccgct ggccttcgag gccgccaccg ccgtggccgg cctgatgccg     300
   cagggcct gg accgcgt gt t ct t caccaat t cgggct ccg aat cggt gga caccgcgct g 360
   aagatcgccc tggcctacca ccgcgcgcgc ggcgaggcgc agcgcacccg cctcatcggg     420
   cgcgagcgcg gctaccacgg cgtgggcttc ggcggcattt ccgtgggcgg catctcgccc     480
   aaccgcaaga ccttctccgg cgcgctgctg ccggccgtgg accacctgcc gcacacccac     540
   agcctggaac acaacgcctt cacgcgcggc cagcccgagt ggggcgcgca cctggccgac     600
   gagt t ggaac gcat cat cgc cct gcacgac gcct ccacca t cgcggccgt gat cgt cgag 660
   cccatggccg gctccaccgg cgtgctcgtc ccgcccaagg gctatctcga aaaactgcgc     720
   gaaatcaccg cccgccacgg cattctgctg atcttcgacg aagtcatcac cgcgtacggc     780
   cgcctgggcg aggccaccgc cgcggcctat ttcggcgtaa cgcccgacct catcaccatg     840
   gccaagggcg tgagcaacgc cgccgttccg gccggcgccg tcgcggtgcg ccgcgaagtg     900
   catgacgcca tcgtcaacgg accgcaaggc ggcatcgagt tcttccacgg ctacacctac     960
   tcggcccacc cgctggccgc cgccgccgtg ctcgccacgc tggacatcta ccgccgcgaa    1020
   gacctgttcg cccgcgcccg caagctgtcg gccgcgttcg aggaagccgc ccacagcctc    1080
   aagggcgcgc cgcacgtcat cgacgtgcgc aacatcggcc tggtggccgg catcgagctg    1140
   tcgccgcgcg aaggcgcccc gggcgcgcgc gccgccgaag ccttccagaa atgcttcgac    1200
   accggcctca tggtgcgcta cacgggcgac atcctcgcgg tgtcgcctcc gctcatcgtc    1260
   gacgaaaacc agatcggcca gatcttcgag ggcatcggca aggtgctcaa ggaagtggct    1320
   t ag 1323
<210> 248
   <211> 440
   <212> PRT
   <213> omega-amino acid: pyruvate transaminase
<400> 248
   Met Ser Ala Ala Lys Leu Pro Asp Leu Ser His Leu Trp Met Pro Phe
   1 5 10 15
   Thr Ala Asn Arg Gln Phe Lys Ala Asn Pro Arg Leu Leu Ala Ser Ala
   20 25 30
   Lys Gly Met Tyr Tyr Thr Ser Phe Asp Gly Arg Gln Ile Leu Asp Gly
   35 40 45
   Thr Ala Gly Leu Trp Cys Val Asn Ala Gly His Cys Arg Glu Glu Ile
   50 55 60
   Val Ser Ala Ile Ala Ser Gln Ala Gly Val Met Asp Tyr Ala Pro Gly
   65 70 75 80
   Phe Gln Leu Gly His Pro Leu Ala Phe Glu Ala Ala Thr Ala Val Ala
   85 90 95
   Gly Leu Met Pro Gln Gly Leu Asp Arg Val Phe Phe Thr Asn Ser Gly
   100 105 110
   Ser Glu Ser Val Asp Thr Ala Leu Lys Ile Ala Leu Ala Tyr His Arg
   115 120 125
   Ala Arg Gly Glu Ala Gln Arg Thr Arg Leu Ile Gly Arg Glu Arg Gly
   130 135 140
   Tyr His Gly Val Gly Phe Gly Gly Ile Ser Val Gly Gly Ile Ser Pro
   145 150 155 160
   Asn Arg Lys Thr Phe Ser Gly Ala Leu Leu Pro Ala Val Asp His Leu
   165 170 175
   Pro His Thr His Ser Leu Glu His Asn Ala Phe Thr Arg Gly Gln Pro
   180 185 190
   Glu Trp Gly Ala His Leu Ala Asp Glu Leu Glu Arg Ile Ile Ala Leu
   195 200 205
   His Asp Ala Ser Thr Ile Ala Ala Val Ile Val Glu Pro Met Ala Gly
   210 215 220
   Ser Thr Gly Val Leu Val Pro Pro Lys Gly Tyr Leu Glu Lys Leu Arg
   225 230 235 240
   Glu Ile Thr Ala Arg His Gly Ile Leu Leu Ile Phe Asp Glu Val Ile
   245 250 255
   Thr Ala Tyr Gly Arg Leu Gly Glu Ala Thr Ala Ala Ala Tyr Phe Gly
   260 265 270
   Val Thr Pro Asp Leu Ile Thr Met Ala Lys Gly Val Ser Asn Ala Ala
   275 280 285
   Val Pro Ala Gly Ala Val Ala Val Arg Arg Glu Val His Asp Ala Ile
   290 295 300
   Val Asn Gly Pro Gln Gly Gly Ile Glu Phe Phe His Gly Tyr Thr Tyr
   305 310 315 320
   Ser Ala His Pro Leu Ala Ala Ala Ala Val Leu Ala Thr Leu Asp Ile
   325 330 335
   Tyr Arg Arg Glu Asp Leu Phe Ala Arg Ala Arg Lys Leu Ser Ala Ala
   340 345 350
   Phe Glu Glu Ala Ala His Ser Leu Lys Gly Ala Pro His Val Ile Asp
   355 360 365
   Val Arg Asn Ile Gly Leu Val Ala Gly Ile Glu Leu Ser Pro Arg Glu
   370 375 380
   Gly Ala Pro Gly Ala Arg Ala Ala Glu Ala Phe Gln Lys Cys Phe Asp
   385 390 395 400
   Thr Gly Leu Met Val Arg Tyr Thr Gly Asp Ile Leu Ala Val Ser Pro
   405 410 415
   Pro Leu Ile Val Asp Glu Asn Gln Ile Gly Gln Ile Phe Glu Gly Ile
   420 425 430
   Gly Lys Val Leu Lys Glu Val Ala
   435 440
<210> 249
   <211> 1332
   <212> DNA
   <213> Ralstoniaeutropha
<400> 249
   atggacgccg cgaagaccgt gattcccgat ctcgatgccc tgtggatgcc ctttaccgcg 60
   aaccgccagt acaaggcggc gccgcgcctg ctggcctcgg ccagcggcat gtactacacc 120
   acccacgacg gacgccagat cctcgacggt tgcgcgggcc tctggtgcgt agcggccggc 180
   cactgccgca aggagattgc cgaggccgtg gcccgccagg ccgccacgct cgactacgcg 240
   ccgccgttcc agatgggcca tccgctgtcg ttcgaagccg ccaccaaggt ggccgcgatc 300
   atgccgcagg gactggaccg catcttcttc acgaattccg gttcggaatc ggtggacacc 360
   gcgctgaaga ttgcgctggc ctaccaccgt gcgcgcggcg agggccagcg cacccgcttc 420
   atcgggcgcg aacgcggtta ccacggcgtg ggctttggcg gcatggctgt cggtggcatc 480
   gggccgaacc gcaaggcgtt ctcggccaac ctgatgccgg gcaccgacca tctgccggcg 540
   acgctgaata tcgccgaagc ggcgttctcc aagggtcagc cgacatgggg cgcgcacctt 600
   gccgacgaac tcgagcgcat cgtcgcgctg catgatccgt ccacgattgc cgccgtcatc 660
   gtggaaccgc tggcgggctc cgccggggtg ctggtgccgc cggtcggcta cctcgacaag 720
   ctgcgcgaga tcacgaccaa gcacggcatc ctgctgatct tcgacgaggt catcacggcc 780
   tttggtcgcc tgggtaccgc caccgcggcg gaacgcttca aggtcacgcc ggacctgatc 840
   accatggcca aggccatcaa caacgccgcc gtgccgatgg gtgccgtggc cgtgcgccgc 900
   gaagtccatg acaccgtggt caactcggcc gcgccgggcg cgatcgaact cgcgcatggc 960
   tacacctact cgggccaccc gctggccgcc gccgctgcca tcgccacgct ggacctgtat 1020
   cagcgcgaga acctgttcgg ccgtgccgcg gagctgtcgc cggtgttcga agcggccgtt 1080
   cacagcgtac gcagcgcgcc gcatgtgaag gacatccgca acctcggcat ggtggccggc 1140
   atcgagctgg agccgcgtcc gggccagccc ggcgcacgcg cctacgaagc cttcctcaaa 1200
   tgccttgagc gtggcgtgct ggtgcgctac accggcgata tcctcgcgtt ctcgccgccg 1260
   ctgatcatca gcgaggcgca gattgccgag ctgttcgata cggtcaagca ggccttgcag 1320
   gaagtgcagt aa 1332
<210> 250
   <211> 443
   <212> PRT
   <213> Ralstoniaeutropha
<400> 250
   Met Asp Ala Ala Lys Thr Val Ile Pro Asp Leu Asp Ala Leu Trp Met
   1               5                   10                  15
   Pro Phe Thr Ala Asn Arg Gln Tyr Lys Ala Ala Pro Arg Leu Leu Ala
               20                  25                  30
   Ser Ala Ser Gly Met Tyr Tyr Thr Thr His Asp Gly Arg Gln Ile Leu
           35                  40                  45
   Asp Gly Cys Ala Gly Leu Trp Cys Val Ala Ala Gly His Cys Arg Lys
       50                  55                  60
   Glu Ile Ala Glu Ala Val Ala Arg Gln Ala Ala Thr Leu Asp Tyr Ala
   65                  70                  75                  80
   Pro Pro Phe Gln Met Gly His Pro Leu Ser Phe Glu Ala Ala Thr Lys
                   85                  90                  95
   Val Ala Ala Ile Met Pro Gln Gly Leu Asp Arg Ile Phe Phe Thr Asn
               100                 105                 110
   Ser Gly Ser Glu Ser Val Asp Thr Ala Leu Lys Ile Ala Leu Ala Tyr
   115 120 125
   His Arg Ala Arg Gly Glu Gly Gln Arg Thr Arg Phe Ile Gly Arg Glu
   130 135 140
   Arg Gly Tyr His Gly Val Gly Phe Gly Gly Met Ala Val Gly Gly Ile
   145 150 155 160
   Gly Pro Asn Arg Lys Ala Phe Ser Ala Asn Leu Met Pro Gly Thr Asp
   165 170 175
   His Leu Pro Ala Thr Leu Asn Ile Ala Glu Ala Ala Phe Ser Lys Gly
   180 185 190
   Gln Pro Thr Trp Gly Ala His Leu Ala Asp Glu Leu Glu Arg Ile Val
   195 200 205
   Ala Leu His Asp Pro Ser Thr Ile Ala Ala Val Ile Val Glu Pro Leu
   210 215 220
   Ala Gly Ser Ala Gly Val Leu Val Pro Pro Val Gly Tyr Leu Asp Lys
   225 230 235 240
   Leu Arg Glu Ile Thr Thr Lys His Gly Ile Leu Leu Ile Phe Asp Glu
   245 250 255
   Val Ile Thr Ala Phe Gly Arg Leu Gly Thr Ala Thr Ala Ala Glu Arg
   260 265 270
   Phe Lys Val Thr Pro Asp Leu Ile Thr Met Ala Lys Ala Ile Asn Asn
   275 280 285
   Ala Ala Val Pro Met Gly Ala Val Ala Val Arg Arg Glu Val His Asp
   290 295 300
   Thr Val Val Asn Ser Ala Ala Pro Gly Ala Ile Glu Leu Ala His Gly
   305 310 315 320
   Tyr Thr Tyr Ser Gly His Pro Leu Ala Ala Ala Ala Ala Ile Ala Thr
   325 330 335
   Leu Asp Leu Tyr Gln Arg Glu Asn Leu Phe Gly Arg Ala Ala Glu Leu
   340 345 350
   Ser Pro Val Phe Glu Ala Ala Val His Ser Val Arg Ser Ala Pro His
   355 360 365
   Val Lys Asp Ile Arg Asn Leu Gly Met Val Ala Gly Ile Glu Leu Glu
   370 375 380
   Pro Arg Pro Gly Gln Pro Gly Ala Arg Ala Tyr Glu Ala Phe Leu Lys
   385 390 395 400
   Cys Leu Glu Arg Gly Val Leu Val Arg Tyr Thr Gly Asp Ile Leu Ala
   405 410 415
   Phe Ser Pro Pro Leu Ile Ile Ser Glu Ala Gln Ile Ala Glu Leu Phe
   420 425 430
   Asp Thr Val Lys Gln Ala Leu Gln Glu Val Gln
   435 440
<210> 251
   <211> 1341
   <212> DNA
   <213> Shewanella oneidensis
<400> 251
   atggccgact cacccaacaa cctcgctcac gaacatcctt cacttgaaca ctattggatg 60
   ccttttaccg ccaatcgcca attcaaagcg agccctcgtt tactcgccca agctgaaggt 120
   atgtattaca cagatatcaa tggcaacaag gtattagact ctacagcggg cttatggtgt 180
   tgtaatgctg gccatggtcg ccgtgagatc agtgaagccg tcagcaaaca aattcggcag 240
   atggattacg ctccctcctt ccaaatgggc catcccatcg cttttgaact ggccgaacgt 300
   ttaaccgaac tcagcccaga aggactcaac aaagtattct ttaccaactc aggctctgag 360
   tcggttgata ccgcgctaaa aatggctctt tgctaccata gagccaatgg ccaagcgtca 420
   cgcacccgct ttattggccg tgaaatgggt taccatggcg taggatttgg tgggatctcg 480
   gtgggtggtt taagcaataa ccgtaaagcc ttcagcggcc agctattgca aggcgtggat 540
   cacctgcccc acaccttaga cattcaacat gccgccttta gtcgtggctt accgagcctc 600
   ggtgctgaaa aagctgaggt attagaacaa ttagtcacac tccatggcgc cgaaaatatt 660
   gccgccgtta ttgttgaacc catgtcaggt tctgcagggg taattttacc acctcaaggc 720
   tacttaaaac gcttacgtga aatcactaaa aaacacggca tcttattgat tttcgatgaa 780
   gtcattaccg catttggccg tgtaggtgca gcattcgcca gccaacgttg gggcgttatt 840
   ccagacataa tcaccacggc taaagccatt aataatggcg ccatccccat gggcgcagtg 900
   tttgtacagg attatatcca cgatacttgc atgcaagggc caaccgaact gattgaattt 960
   ttccacggtt atacctattc gggccaccca gtcgccgcag cagcagcact cgccacgctc 1020
   tccatctacc aaaacgagca actgtttgag cgcagttttg agcttgagcg gtatttcgaa 1080
   gaagccgttc atagcctcaa agggttaccg aatgtgattg atattcgcaa caccggatta 1140
   gtcgcgggtt tccagctagc accgaatagc caaggtgttg gtaaacgcgg atacagcgtg 1200
   ttcgagcatt gtttccatca aggcacactc gtgcgggcaa cgggcgatat tatcgccatg 1260
   tccccaccac tcattgttga gaaacatcag attgaccaaa tggtaaatag ccttagcgat 1320
   gcaattcacg ccgttggatg a 1341
<210> 252
   <211> 446
   <212> PRT
   <213> bet a alanine-pyruvate transaminase
<400> 252
   Met Ala Asp Ser Pro Asn Asn Leu Ala His Glu His Pro Ser Leu Glu
   1 5 10 15
   His Tyr Trp Met Pro Phe Thr Ala Asn Arg Gln Phe Lys Ala Ser Pro
   20 25 30
   Arg Leu Leu Ala Gln Ala Glu Gly Met Tyr Tyr Thr Asp Ile Asn Gly
   35 40 45
   Asn Lys Val Leu Asp Ser Thr Ala Gly Leu Trp Cys Cys Asn Ala Gly
   50 55 60
   His Gly Arg Arg Glu Ile Ser Glu Ala Val Ser Lys Gln Ile Arg Gln
   65 70 75 80
   Met Asp Tyr Ala Pro Ser Phe Gln Met Gly His Pro Ile Ala Phe Glu
   85 90 95
   Leu Ala Glu Arg Leu Thr Glu Leu Ser Pro Glu Gly Leu Asn Lys Val
   100 105 110
   Phe Phe Thr Asn Ser Gly Ser Glu Ser Val Asp Thr Ala Leu Lys Met
   115 120 125
   Ala Leu Cys Tyr His Arg Ala Asn Gly Gln Ala Ser Arg Thr Arg Phe
   130 135 140
   Ile Gly Arg Glu Met Gly Tyr His Gly Val Gly Phe Gly Gly Ile Ser
   145 150 155 160
   Val Gly Gly Leu Ser Asn Asn Arg Lys Ala Phe Ser Gly Gln Leu Leu
   165 170 175
   Gln Gly Val Asp His Leu Pro His Thr Leu Asp Ile Gln His Ala Ala
   180 185 190
   Phe Ser Arg Gly Leu Pro Ser Leu Gly Ala Glu Lys Ala Glu Val Leu
   195 200 205
   Glu Gln Leu Val Thr Leu His Gly Ala Glu Asn Ile Ala Ala Val Ile
   210 215 220
   Val Glu Pro Met Ser Gly Ser Ala Gly Val Ile Leu Pro Pro Gln Gly
   225 230 235 240
   Tyr Leu Lys Arg Leu Arg Glu Ile Thr Lys Lys His Gly Ile Leu Leu
   245 250 255
   Ile Phe Asp Glu Val Ile Thr Ala Phe Gly Arg Val Gly Ala Ala Phe
   260 265 270
   Ala Ser Gln Arg Trp Gly Val Ile Pro Asp Ile Ile Thr Thr Ala Lys
   275 280 285
   Ala Ile Asn Asn Gly Ala Ile Pro Met Gly Ala Val Phe Val Gln Asp
   290 295 300
   Tyr Ile His Asp Thr Cys Met Gln Gly Pro Thr Glu Leu Ile Glu Phe
   305 310 315 320
   Phe His Gly Tyr Thr Tyr Ser Gly His Pro Val Ala Ala Ala Ala Ala
   325 330 335
   Leu Ala Thr Leu Ser Ile Tyr Gln Asn Glu Gln Leu Phe Glu Arg Ser
   340 345 350
   Phe Glu Leu Glu Arg Tyr Phe Glu Glu Ala Val His Ser Leu Lys Gly
   355 360 365
   Leu Pro Asn Val Ile Asp Ile Arg Asn Thr Gly Leu Val Ala Gly Phe
   370 375 380
   Gln Leu Ala Pro Asn Ser Gln Gly Val Gly Lys Arg Gly Tyr Ser Val
   385 390 395 400
   Phe Glu His Cys Phe His Gln Gly Thr Leu Val Arg Ala Thr Gly Asp
   405 410 415
   Ile Ile Ala Met Ser Pro Pro Leu Ile Val Glu Lys His Gln Ile Asp
   420 425 430
   Gln Met Val Asn Ser Leu Ser Asp Ala Ile His Ala Val Gly
   435 440 445
<210> 253
   <211> 1347
   <212> DNA
   <213> Pseudomonas putida
<400> 253
   atgaacatgc ccgaaactgg tcctgccggt atcgccagcc agctcaagct ggacgcccac 60
   tggatgccct acaccgccaa ccgcaacttc cagcgcgacc cacgcctgat cgtggcggcc 120
   gaaggcaact acctggtcga tgaccacggg cgcaagatct tcgacgccct gtccggcctg 180
   tggacctgcg gcgcagggca cactcgcaag gaaatcgctg acgcggtgac ccgtcaactg 240
   agtacgctgg actactcccc agcgttccag ttcggccacc cgctgtcgtt ccagctggcg 300
   gaaaagatcg ccgagctggt tccgggcaat ctgaatcacg tcttctatac caactccggt 360
   tccgagtgcg ccgataccgc actgaagatg gtgcgtgcct actggcgcct gaaaggccag 420
   gcaaccaaga ccaagatcat cggccgtgcc cgtggttacc atggcgtgaa catcgccggt 480
   accagcctgg gtggcgtcaa cggtaaccgc aagatgtttg gccagctgct ggacgtcgac 540
   cacctgcctc acactgtatt gccggtgaac gccttctcga aaggcttgcc ggaagagggc 600
   ggtatcgcgc tggctgacga aatgctcaag ctgatcgagc tgcacgatgc ctccaacatc 660
   gcagcagtca tcgtcgagcc gctggccggt tcggccggtg tgctgccgcc gccaaagggt 720
   tacctgaagc gcctgcgtga aatctgcacc cagcacaaca ttctgctgat cttcgacgaa 780
   gtgatcacag gcttcggccg catgggcgcg atgaccggct cggaagcctt cggcgttacc 840
   ccggacctga tgtgcatcgc caagcaggtg accaacggcg ccatcccgat gggcgcagtg 900
   attgccagca gcgagatcta ccagaccttc atgaaccagc cgaccccgga atacgccgtg 960
   gaattcccac acggctacac ctattcggcg cacccggtag cctgtgccgc cggtctcgcc 1020
   gcgctggacc tgctgcagaa ggaaaacctg gtgcagtccg cggctgaact ggcgccgcat 1080
   ttcgagaagc tgctgcacgg cgtgaagggc accaagaata tcgtcgatat ccgcaactac 1140
   ggcctggccg gcgccatcca gatcgccgcc cgtgacggtg atgccatcgt tcgcccttac 1200
   gaagcggcca tgaagctgtg gaaagcgggc ttctatgtac gctttggtgg cgacaccctg 1260
   cagttcggcc caaccttcaa taccaagccg caggaactgg accgcttgtt cgatgctgtt 1320
   ggcgaaaccc tgaacctgat cgactga 1347
<210> 254
   <211> 448
   <212> PRT
   <213> Pseudomonas putida
<400> 254
   Met Asn Met Pro Glu Thr Gly Pro Ala Gly Ile Ala Ser Gln Leu Lys
   1 5 10 15
   Leu Asp Ala His Trp Met Pro Tyr Thr Ala Asn Arg Asn Phe Gln Arg
   20 25 30
   Asp Pro Arg Leu Ile Val Ala Ala Glu Gly Asn Tyr Leu Val Asp Asp
   35 40 45
   His Gly Arg Lys Ile Phe Asp Ala Leu Ser Gly Leu Trp Thr Cys Gly
   50 55 60
   Ala Gly His Thr Arg Lys Glu Ile Ala Asp Ala Val Thr Arg Gln Leu
   65 70 75 80
   Ser Thr Leu Asp Tyr Ser Pro Ala Phe Gln Phe Gly His Pro Leu Ser
   85 90 95
   Phe Gln Leu Ala Glu Lys Ile Ala Glu Leu Val Pro Gly Asn Leu Asn
   100 105 110
   His Val Phe Tyr Thr Asn Ser Gly Ser Glu Cys Ala Asp Thr Ala Leu
   115 120 125
   Lys Met Val Arg Ala Tyr Trp Arg Leu Lys Gly Gln Ala Thr Lys Thr
   130 135 140
   Lys Ile Ile Gly Arg Ala Arg Gly Tyr His Gly Val Asn Ile Ala Gly
   145 150 155 160
   Thr Ser Leu Gly Gly Val Asn Gly Asn Arg Lys Met Phe Gly Gln Leu
   165 170 175
   Leu Asp Val Asp His Leu Pro His Thr Val Leu Pro Val Asn Ala Phe
   180 185 190
   Ser Lys Gly Leu Pro Glu Glu Gly Gly Ile Ala Leu Ala Asp Glu Met
   195 200 205
   Leu Lys Leu Ile Glu Leu His Asp Ala Ser Asn Ile Ala Ala Val Ile
   210 215 220
   Val Glu Pro Leu Ala Gly Ser Ala Gly Val Leu Pro Pro Pro Lys Gly
   225 230 235 240
   Tyr Leu Lys Arg Leu Arg Glu Ile Cys Thr Gln His Asn Ile Leu Leu
   245 250 255
   Ile Phe Asp Glu Val Ile Thr Gly Phe Gly Arg Met Gly Ala Met Thr
   260 265 270
   Gly Ser Glu Ala Phe Gly Val Thr Pro Asp Leu Met Cys Ile Ala Lys
   275 280 285
   Gln Val Thr Asn Gly Ala Ile Pro Met Gly Ala Val Ile Ala Ser Ser
   290 295 300
   Glu Ile Tyr Gln Thr Phe Met Asn Gln Pro Thr Pro Glu Tyr Ala Val
   305 310 315 320
   Glu Phe Pro His Gly Tyr Thr Tyr Ser Ala His Pro Val Ala Cys Ala
   325 330 335
   Ala Gly Leu Ala Ala Leu Asp Leu Leu Gln Lys Glu Asn Leu Val Gln
   340 345 350
   Ser Ala Ala Glu Leu Ala Pro His Phe Glu Lys Leu Leu His Gly Val
   355 360 365
   Lys Gly Thr Lys Asn Ile Val Asp Ile Arg Asn Tyr Gly Leu Ala Gly
   370 375 380
   Ala Ile Gln Ile Ala Ala Arg Asp Gly Asp Ala Ile Val Arg Pro Tyr
   385 390 395 400
   Glu Ala Ala Met Lys Leu Trp Lys Ala Gly Phe Tyr Val Arg Phe Gly
   405 410 415
   Gly Asp Thr Leu Gln Phe Gly Pro Thr Phe Asn Thr Lys Pro Gln Glu
   420 425 430
   Leu Asp Arg Leu Phe Asp Ala Val Gly Glu Thr Leu Asn Leu Ile Asp
   435 440 445

<210> 255
   <211> 1701
   <212> DNA
   <213> Streptomyces cinnamonensis
<400> 255
   atggacgctg acgcgatcga ggaaggccgc cgacgctggc aggcccgtta cgacaaggcc 60
   cgcaagcgcg acgcggactt caccacgctc tccggggacc ccgtcgaccc cgtctacggc 120
   ccccggcccg gggacacgta cgacgggttc gagcggatcg gctggccggg ggagtacccc 180
   ttcacccgcg ggctctacgc caccgggtac cgcggccgca cctggaccat ccgccagttc 240
   gccggcttcg gcaacgccga gcagacgaac gagcgctaca agatgatcct ggccaacggc 300
   ggcggcggcc tctccgtcgc cttcgacatg ccgaccctca tgggccgcga ctccgacgac 360
   ccgcgctcgc tcggcgaggt cggccactgc ggtgtcgcca tcgactccgc cgccgacatg 420
   gaggtcctct tcaaggacat cccgctcggc gacgtcacga cgtccatgac catcagcggg 480
   cccgccgtgc ccgtcttctg catgtacctc gtcgcggccg agcgccaggg cgtcgacccg 540
   gccgtcctca acggcacgct gcagaccgac atcttcaagg agtacatcgc ccagaaggag 600
   tggctcttcc agcccgagcc gcacctgcgc ctcatcggcg acctgatgga gcactgcgcg 660
   cgcgacatcc ccgcgtacaa gccgctctcg gtctccggct accacatccg cgaggccggg 720
   gcgacggccg cgcaggagct cgcgtacacc ctcgcggacg gcttcgggta cgtggaactg 780
   ggcctctcgc gcggcctgga cgtggacgtc ttcgcgcccg gcctctcctt cttcttcgac 840
   gcgcacgtcg acttcttcga ggagatcgcg aagttccgcg ccgcacgccg catctgggcg 900
   cgctggctcc gggacgagta cggagcgaag accgagaagg cacagtggct gcgcttccac 960
   acgcagaccg cgggggtctc gctcacggcc cagcagccgt acaacaacgt ggtgcggacg 1020
   gcggtggagg ccctcgccgc ggtgctcggc ggcacgaact ccctgcacac caacgctctc 1080
   gacgagaccc ttgccctccc cagcgagcag gccgcggaga tcgcgctgcg cacccagcag 1140
   gtgctgatgg aggagaccgg cgtcgccaac gtcgcggacc cgctgggcgg ctcctggtac 1200
   atcgagcagc tcaccgaccg catcgaggcc gacgccgaga agatcttcga gcagatcagg 1260
   gagcgggggc ggcgggcctg ccccgacggg cagcacccga tcgggccgat cacctccggc 1320
   atcctgcgcg gcatcgagga cggctggttc accggcgaga tcgccgagtc cgccttccag 1380
   taccagcggt ccctggagaa gggcgacaag cgggtcgtcg gcgtcaactg cctcgaaggc 1440
   tccgtcaccg gcgacctgga gatcctgcgc gtcagccacg aggtcgagcg cgagcaggtg 1500
   cgggagcttg cggggcgcaa ggggcggcgt gacgatgcgc gggtgcgggc ctcgctcgac 1560
   gcgatgctcg ccgctgcgcg ggacgggtcg aacatgattg cccccatgct ggaggcggtg 1620
   cgggccgagg cgaccctcgg ggagatctgc ggggtgcttc gcgatgagtg gggggtctac 1680
   gtggagccgc ccgggttctg a 1701
<210> 256
   <211> 566
   <212> PRT
   <213> Streptomyces cinnamonensis
<400> 256
   Met Asp Ala Asp Ala Ile Glu Glu Gly Arg Arg Arg Trp Gln Ala Arg
   1 5 10 15
   Tyr Asp Lys Ala Arg Lys Arg Asp Ala Asp Phe Thr Thr Leu Ser Gly
   20 25 30
   Asp Pro Val Asp Pro Val Tyr Gly Pro Arg Pro Gly Asp Thr Tyr Asp
   35 40 45
   Gly Phe Glu Arg Ile Gly Trp Pro Gly Glu Tyr Pro Phe Thr Arg Gly
   50 55 60
   Leu Tyr Ala Thr Gly Tyr Arg Gly Arg Thr Trp Thr Ile Arg Gln Phe
   65 70 75 80
   Ala Gly Phe Gly Asn Ala Glu Gln Thr Asn Glu Arg Tyr Lys Met Ile
   85 90 95
   Leu Ala Asn Gly Gly Gly Gly Leu Ser Val Ala Phe Asp Met Pro Thr
   100 105 110
   Leu Met Gly Arg Asp Ser Asp Asp Pro Arg Ser Leu Gly Glu Val Gly
   115 120 125
   His Cys Gly Val Ala Ile Asp Ser Ala Ala Asp Met Glu Val Leu Phe
   130 135 140
   Lys Asp Ile Pro Leu Gly Asp Val Thr Thr Ser Met Thr Ile Ser Gly
   145 150 155 160
   Pro Ala Val Pro Val Phe Cys Met Tyr Leu Val Ala Ala Glu Arg Gln
   165 170 175
   Gly Val Asp Pro Ala Val Leu Asn Gly Thr Leu Gln Thr Asp Ile Phe
   180 185 190
   Lys Glu Tyr Ile Ala Gln Lys Glu Trp Leu Phe Gln Pro Glu Pro His
   195 200 205
   Leu Arg Leu Ile Gly Asp Leu Met Glu His Cys Ala Arg Asp Ile Pro
   210 215 220
   Ala Tyr Lys Pro Leu Ser Val Ser Gly Tyr His Ile Arg Glu Ala Gly
   225 230 235 240
   Ala Thr Ala Ala Gln Glu Leu Ala Tyr Thr Leu Ala Asp Gly Phe Gly
   245 250 255
   Tyr Val Glu Leu Gly Leu Ser Arg Gly Leu Asp Val Asp Val Phe Ala
   260 265 270
   Pro Gly Leu Ser Phe Phe Phe Asp Ala His Val Asp Phe Phe Glu Glu
   275 280 285
   Ile Ala Lys Phe Arg Ala Ala Arg Arg Ile Trp Ala Arg Trp Leu Arg
   290 295 300
   Asp Glu Tyr Gly Ala Lys Thr Glu Lys Ala Gln Trp Leu Arg Phe His
   305 310 315 320
   Thr Gln Thr Ala Gly Val Ser Leu Thr Ala Gln Gln Pro Tyr Asn Asn
   325 330 335
   Val Val Arg Thr Ala Val Glu Ala Leu Ala Ala Val Leu Gly Gly Thr
   340 345 350
   Asn Ser Leu His Thr Asn Ala Leu Asp Glu Thr Leu Ala Leu Pro Ser
   355 360 365
   Glu Gln Ala Ala Glu Ile Ala Leu Arg Thr Gln Gln Val Leu Met Glu
   370 375 380
   Glu Thr Gly Val Ala Asn Val Ala Asp Pro Leu Gly Gly Ser Trp Tyr
   385 390 395 400
   Ile Glu Gln Leu Thr Asp Arg Ile Glu Ala Asp Ala Glu Lys Ile Phe
   405 410 415
   Glu Gln Ile Arg Glu Arg Gly Arg Arg Ala Cys Pro Asp Gly Gln His
   420 425 430
   Pro Ile Gly Pro Ile Thr Ser Gly Ile Leu Arg Gly Ile Glu Asp Gly
   435 440 445
   Trp Phe Thr Gly Glu Ile Ala Glu Ser Ala Phe Gln Tyr Gln Arg Ser
   450 455 460
   Leu Glu Lys Gly Asp Lys Arg Val Val Gly Val Asn Cys Leu Glu Gly
   465 470 475 480
   Ser Val Thr Gly Asp Leu Glu Ile Leu Arg Val Ser His Glu Val Glu
   485 490 495
   Arg Glu Gln Val Arg Glu Leu Ala Gly Arg Lys Gly Arg Arg Asp Asp
   500 505 510
   Ala Arg Val Arg Ala Ser Leu Asp Ala Met Leu Ala Ala Ala Arg Asp
   515 520 525
   Gly Ser Asn Met Ile Ala Pro Met Leu Glu Ala Val Arg Ala Glu Ala
   530 535 540
   Thr Leu Gly Glu Ile Cys Gly Val Leu Arg Asp Glu Trp Gly Val Tyr
   545 550 555 560
   Val Glu Pro Pro Gly Phe
   565
<210> 257
   <211> 411
   <212> DNA
   <213> Streptomyces cinnamonensis
<400> 257
   atgggtgtgg cagccgggcc gatccgcgtg gtggtcgcca agccggggct cgacgggcac 60
   gatcgcgggg ccaaggtgat cgcgcgggcg ttgcgtgacg cgggtatgga ggtcatctac 120
   accgggctgc accagacgcc cgagcaggtg gtggacaccg cgatccagga ggacgccgac 180
   gcgatcggcc tctccatcct ctccggagcg cacaacacgc tgttcgcgcg cgtgttggag 240
   ctcttgaagg agcgggacgc ggaggacatc aaggtgtttg gtggcggcat catcccggag 300
   gcggacatcg cgccgctgaa ggagaagggc gtcgcggaga tcttcacgcc cggggccacc 360
   accacgtcga tcgtggagtg ggttcggggg aacgtgcgac aggccgtctg a 411
<210> 258
   <211> 136
   <212> PRT
   <213> Streptomyces cinnamonensis
<400> 258
   Met Gly Val Ala Ala Gly Pro Ile Arg Val Val Val Ala Lys Pro Gly
   1               5                   10                  15
   Leu Asp Gly His Asp Arg Gly Ala Lys Val Ile Ala Arg Ala Leu Arg
               20                  25                  30
   Asp Ala Gly Met Glu Val Ile Tyr Thr Gly Leu His Gln Thr Pro Glu
           35                  40                  45
   Gln Val Val Asp Thr Ala Ile Gln Glu Asp Ala Asp Ala Ile Gly Leu
       50                  55                  60
   Ser Ile Leu Ser Gly Ala His Asn Thr Leu Phe Ala Arg Val Leu Glu
   65                  70                  75                  80
   Leu Leu Lys Glu Arg Asp Ala Glu Asp Ile Lys Val Phe Gly Gly Gly
                   85                  90                  95
   Ile Ile Pro Glu Ala Asp Ile Ala Pro Leu Lys Glu Lys Gly Val Ala
               100                 105                 110
   Glu Ile Phe Thr Pro Gly Ala Thr Thr Thr Ser Ile Val Glu Trp Val
           115                 120                 125
   Arg Gly Asn Val Arg Gln Ala Val
       130                 135
<210> 259
   <211> 1701
   <212> DNA
   <213> Streptomyces coelicolor
<400> 259
   atggacgctc atgccataga ggagggccgc cttcgctggc aggcccggta cgacgcggcg 60
   cgcaagcgcg acgcggactt caccacgctc tccggagacc ccgtggagcc ggtgtacggg 120
   ccccgccccg gggacgagta cgagggcttc gagcggatcg gctggccggg cgagtacccc 180
   ttcacccgcg gcctgtatcc gaccgggtac cgggggcgta cgtggaccat ccggcagttc 240
   gccgggttcg gcaacgccga gcagaccaac gagcgctaca agatgatcct ccgcaacggc 300
   ggcggcgggc tctcggtcgc cttcgacatg ccgaccctga tgggccgcga ctccgacgac 360
   ccgcgctcgc tgggcgaggt cgggcactgc ggggtggcca tcgactcggc cgccgacatg 420
   gaagtgctgt tcaaggacat cccgctcggg gacgtgacga cctccatgac gatcagcggg 480
   cccgccgtgc ccgtgttctg catgtacctc gtcgccgccg agcgccaggg cgtcgacgca 540
   tccgtgctca acggcacgct gcagaccgac atcttcaagg agtacatcgc ccagaaggag 600
   tggctcttcc agcccgagcc ccacctccgg ctcatcggcg acctcatgga gtactgcgcg 660
   gccggcatcc ccgcctacaa gccgctctcc gtctccggct accacatccg cgaggcgggc 720
   gcgacggccg cgcaggagct ggcgtacacg ctcgccgacg gcttcggata cgtggagctg 780
   ggcctcagcc gcgggctcga cgtggacgtc ttcgcgcccg gcctctcctt cttcttcgac 840
   gcgcacctcg acttcttcga ggagatcgcc aagttccgcg cggcccgcag gatctgggcc 900
   cgctggatgc gcgacgtgta cggcgcgcgg accgacaagg cccagtggct gcggttccac 960
   acccagaccg ccggagtctc gctcaccgcg cagcagccgt acaacaacgt cgtacgcacc 1020
   gcggtggagg cgctggcggc cgtgctcggc ggcaccaact ccctgcacac caacgcgctc 1080
   gacgagaccc tcgccctgcc cagcgagcag gccgccgaga tcgccctgcg cacccagcag 1140
   gtgctgatgg aggagaccgg cgtcgccaac gtcgccgacc cgctgggcgg ttcctggttc 1200
   atcgagcagc tgaccgaccg catcgaggcc gacgccgaga agatcttcga gcagatcaag 1260
   gagcgggggc tgcgcgccca ccccgacggg cagcaccccg tcggaccgat cacctccggc 1320
   ctgctgcgcg gcatcgagga cggctggttc accggcgaga tcgccgagtc cgccttccgc 1380
   taccagcagt ccttggagaa ggacgacaag aaggtggtcg gcgtcaacgt ccacaccggc 1440
   tccgtcaccg gcgacctgga gatcctgcgg gtcagccacg aggtcgagcg cgagcaggtg 1500
   cgggtcctgg gcgagcgcaa ggacgcccgg gacgacgccg ccgtgcgcgg cgccctggac 1560
   gccatgctgg ccgcggcccg ctccggcggc aacatgatcg ggccgatgct ggacgcggtg 1620
   cgcgcggagg cgacgctggg cgagatctgc ggtgtgctgc gcgacgagtg gggggtgtac 1680
   acggaaccgg cggggttctg a 1701
<210> 260
   <211> 566
   <212> PRT
   <213> Streptomyces coelicolor
<400> 260
   Met Asp Ala His Ala Ile Glu Glu Gly Arg Leu Arg Trp Gln Ala Arg
   1 5 10 15
   Tyr Asp Ala Ala Arg Lys Arg Asp Ala Asp Phe Thr Thr Leu Ser Gly
   20 25 30
   Asp Pro Val Glu Pro Val Tyr Gly Pro Arg Pro Gly Asp Glu Tyr Glu
   35 40 45
   Gly Phe Glu Arg Ile Gly Trp Pro Gly Glu Tyr Pro Phe Thr Arg Gly
   50 55 60
   Leu Tyr Pro Thr Gly Tyr Arg Gly Arg Thr Trp Thr Ile Arg Gln Phe
   65 70 75 80
   Ala Gly Phe Gly Asn Ala Glu Gln Thr Asn Glu Arg Tyr Lys Met Ile
   85 90 95
   Leu Arg Asn Gly Gly Gly Gly Leu Ser Val Ala Phe Asp Met Pro Thr
   100 105 110
   Leu Met Gly Arg Asp Ser Asp Asp Pro Arg Ser Leu Gly Glu Val Gly
   115 120 125
   His Cys Gly Val Ala Ile Asp Ser Ala Ala Asp Met Glu Val Leu Phe
   130 135 140
   Lys Asp Ile Pro Leu Gly Asp Val Thr Thr Ser Met Thr Ile Ser Gly
   145 150 155 160
   Pro Ala Val Pro Val Phe Cys Met Tyr Leu Val Ala Ala Glu Arg Gln
   165 170 175
   Gly Val Asp Ala Ser Val Leu Asn Gly Thr Leu Gln Thr Asp Ile Phe
   180 185 190
   Lys Glu Tyr Ile Ala Gln Lys Glu Trp Leu Phe Gln Pro Glu Pro His
   195 200 205
   Leu Arg Leu Ile Gly Asp Leu Met Glu Tyr Cys Ala Ala Gly Ile Pro
   210 215 220
   Ala Tyr Lys Pro Leu Ser Val Ser Gly Tyr His Ile Arg Glu Ala Gly
   225 230 235 240
   Ala Thr Ala Ala Gln Glu Leu Ala Tyr Thr Leu Ala Asp Gly Phe Gly
   245 250 255
   Tyr Val Glu Leu Gly Leu Ser Arg Gly Leu Asp Val Asp Val Phe Ala
   260 265 270
   Pro Gly Leu Ser Phe Phe Phe Asp Ala His Leu Asp Phe Phe Glu Glu
   275 280 285
   Ile Ala Lys Phe Arg Ala Ala Arg Arg Ile Trp Ala Arg Trp Met Arg
   290 295 300
   Asp Val Tyr Gly Ala Arg Thr Asp Lys Ala Gln Trp Leu Arg Phe His
   305 310 315 320
   Thr Gln Thr Ala Gly Val Ser Leu Thr Ala Gln Gln Pro Tyr Asn Asn
   325 330 335
   Val Val Arg Thr Ala Val Glu Ala Leu Ala Ala Val Leu Gly Gly Thr
   340 345 350
   Asn Ser Leu His Thr Asn Ala Leu Asp Glu Thr Leu Ala Leu Pro Ser
   355 360 365
   Glu Gln Ala Ala Glu Ile Ala Leu Arg Thr Gln Gln Val Leu Met Glu
   370 375 380
   Glu Thr Gly Val Ala Asn Val Ala Asp Pro Leu Gly Gly Ser Trp Phe
   385 390 395 400
   Ile Glu Gln Leu Thr Asp Arg Ile Glu Ala Asp Ala Glu Lys Ile Phe
   405 410 415
   Glu Gln Ile Lys Glu Arg Gly Leu Arg Ala His Pro Asp Gly Gln His
   420 425 430
   Pro Val Gly Pro Ile Thr Ser Gly Leu Leu Arg Gly Ile Glu Asp Gly
   435 440 445
   Trp Phe Thr Gly Glu Ile Ala Glu Ser Ala Phe Arg Tyr Gln Gln Ser
   450 455 460
   Leu Glu Lys Asp Asp Lys Lys Val Val Gly Val Asn Val His Thr Gly
   465 470 475 480
   Ser Val Thr Gly Asp Leu Glu Ile Leu Arg Val Ser His Glu Val Glu
   485 490 495
   Arg Glu Gln Val Arg Val Leu Gly Glu Arg Lys Asp Ala Arg Asp Asp
   500 505 510
   Ala Ala Val Arg Gly Ala Leu Asp Ala Met Leu Ala Ala Ala Arg Ser
   515 520 525
   Gly Gly Asn Met Ile Gly Pro Met Leu Asp Ala Val Arg Ala Glu Ala
   530 535 540
   Thr Leu Gly Glu Ile Cys Gly Val Leu Arg Asp Glu Trp Gly Val Tyr
   545 550 555 560
   Thr Glu Pro Ala Gly Phe
   565
<210> 261
   <211> 417
   <212> DNA
   <213> Streptomyces coelicolor
<400> 261
   atgggtgtgg cagccggtcc gatccgcgtg gtggtggcca agccggggct cgacggccac 60
   gatcgcgggg ccaaggtgat cgcgagggcc ctgcgtgacg ccggtatgga ggtgatctac 120
   accgggctcc accagacgcc cgagcagatc gtcgacaccg cgatccagga ggacgccgac 180
   gcgatcgggc tgtccatcct ctccggtgcg cacaacacgc tcttcgccgc cgtgatcgag 240
   ctgctccggg agcgggacgc cgcggacatc ctggtcttcg gcggcgggat catccccgag 300
   gcggacatcg ccccgctgaa ggagaagggc gtcgcggaga tcttcacgcc cggcgccacc 360
   acggcgtcca tcgtggactg ggtccgggcg aacgtgcggg agcccgcggg agcatag 417
<210> 262
   <211> 138
   <212> PRT
   <213> Streptomyces coelicolor
<400> 262
   Met Gly Val Ala Ala Gly Pro Ile Arg Val Val Val Ala Lys Pro Gly
   1 5 10 15
   Leu Asp Gly His Asp Arg Gly Ala Lys Val Ile Ala Arg Ala Leu Arg
   20 25 30
   Asp Ala Gly Met Glu Val Ile Tyr Thr Gly Leu His Gln Thr Pro Glu
   35 40 45
   Gln Ile Val Asp Thr Ala Ile Gln Glu Asp Ala Asp Ala Ile Gly Leu
   50 55 60
   Ser Ile Leu Ser Gly Ala His Asn Thr Leu Phe Ala Ala Val Ile Glu
   65 70 75 80
   Leu Leu Arg Glu Arg Asp Ala Ala Asp Ile Leu Val Phe Gly Gly Gly
   85 90 95
   Ile Ile Pro Glu Ala Asp Ile Ala Pro Leu Lys Glu Lys Gly Val Ala
   100 105 110
   Glu Ile Phe Thr Pro Gly Ala Thr Thr Ala Ser Ile Val Asp Trp Val
   115 120 125
   Arg Ala Asn Val Arg Glu Pro Ala Gly Ala
   130 135
<210> 263
   <211> 1701
   <212> DNA
   <213> Streptomyces avermitilis
<400> 263
   tcagaaaccg gcgggctccg tgtagacccc ccactcctcc cggaggacat cgcagatctc      60
   gcccagcgt g gcct ccgcgc ggaccgcgt c cagcat cggg gcgat cat gt t cgacccgt c 120
   gcgcgcggcg gcgagcatcg cgtccagggc cgcggttacg gccgtgtcgt cgcgccccga     180
   cttccgctcg cccagcaccc gcacctgctc gcgctccacc tcgtggctga cgcgcaggat     240
   ctccaggtcg cccgtcacgg acccgtggtg gacgttgacg ccgacgaccc gcttgtcgcc     300
   ct t ct ccagc gcct gct ggt act ggaaggc cgact cggcg at ct ccccgg t gaaccagcc 360
   gtcctcgatg ccgcgcagga tgccggaggt gatgggcccg atcgggtgcc gcccgtccgg     420
   gtgggcccgc agcccgcgct ccctgatctg ttcgaagatc ttctcggcgt cggcctcgat     480
   ccggtcggtc agctgctcca cgtaccagga accgcccagc ggatcggcca cgttggcgac     540
   gcccgtctcc tccatcagca cctgctgggt gcgcagggcg atctcggccg cctgctcgga     600
   cggcagggcg agggtctcgt cgagggcgtt ggtgtgcagc gagttcgtcc cgccgagcac     660
   cgcggcgagg gcctccacgg ccgtccgtac gacgttgttg tacggctgct gcgcggtgag     720
   cgagacgccc gcggtctggg tgtggaagcg cagccactgc gccttctccg acttcgcccc     780
   gtacacgtcc cgcagccagc gcgcccagat gcgccgcgcc gcacggaact tggcgatctc     840
   ctcgaagaag tcgacgtgcg cgtcgaagaa gaaggagagc ccgggcgcga acacgtccac     900
   gt ccaggccg cggct cagcc ccagct ccac gt at ccgaaa ccgt cggcga gggt gt acgc 960
   cagctcctgg gcggccgtgg caccggcctc ccggatgtgg tacccggaga cggacagcgg    1020
   ct t gt acgcg gggat ct t cg aggcgcagt g ct ccat cagg t cgccgat ga gccgcagat g 1080
   gggct cgggc t ggaagagcc act cct t ct g cgcgat gt ac t cct t gaaga t gt cggt ct g 1140
   gagggt gccg t t gaggacgg aggggt cgac gccct gccgc t cggccgcga ccaggt acat 1200
   gcagaagacg ggcacggcgg gcccgctgat cgtcatcgac gtcgtcacgt cacccagcgg    1260
   gatgtccttg aacaggacct ccatgtcggc cgccgagtcg atcgcgaccc cgcagtgccc    1320
   gacctcgccg agcgcgcggc ggtcgtcgga gtcgcgcccc atgagcgtcg gcatgtcgaa    1380
   ggccacggac agcccaccgc cgccgttggc gaggatcttc ttgtagcgct cgttggtctg    1440
   ctcggcgttg ccgaacccgg cgaactgccg gatggtccag gtccggcccc ggtagccggt    1500
   cggatacaga ccgcgcgtga aggggtactc acccggccag ccgatccgct cgaaaccctc    1560
   gtacgcgtcc ccgggccggg gcccgtacgc cggctccacg ggatcgccgg agagcgtggt    1620
   gaaat cggcc t cgcgct t gc gt gaggcgt c gt agcgggcc t gccagcgt c ggcggcct t c 1680
   ct cgat ggcg t cagcgt cca t 1701
<210> 264
   <211> 566
   <212> PRT
   <213> Streptomyces avermitilis
<400> 264
   Met Asp Ala Asp Ala Ile Glu Glu Gly Arg Arg Arg Trp Gln Ala Arg
   1 5 10 15
   Tyr Asp Ala Ser Arg Lys Arg Glu Ala Asp Phe Thr Thr Leu Ser Gly
   20 25 30
   Asp Pro Val Glu Pro Ala Tyr Gly Pro Arg Pro Gly Asp Ala Tyr Glu
   35 40 45
   Gly Phe Glu Arg Ile Gly Trp Pro Gly Glu Tyr Pro Phe Thr Arg Gly
   50 55 60
   Leu Tyr Pro Thr Gly Tyr Arg Gly Arg Thr Trp Thr Ile Arg Gln Phe
   65 70 75 80
   Ala Gly Phe Gly Asn Ala Glu Gln Thr Asn Glu Arg Tyr Lys Lys Ile
   85 90 95
   Leu Ala Asn Gly Gly Gly Gly Leu Ser Val Ala Phe Asp Met Pro Thr
   100 105 110
   Leu Met Gly Arg Asp Ser Asp Asp Arg Arg Ala Leu Gly Glu Val Gly
   115 120 125
   His Cys Gly Val Ala Ile Asp Ser Ala Ala Asp Met Glu Val Leu Phe
   130 135 140
   Lys Asp Ile Pro Leu Gly Asp Val Thr Thr Ser Met Thr Ile Ser Gly
   145 150 155 160
   Pro Ala Val Pro Val Phe Cys Met Tyr Leu Val Ala Ala Glu Arg Gln
   165 170 175
   Gly Val Asp Pro Ser Val Leu Asn Gly Thr Leu Gln Thr Asp Ile Phe
   180 185 190
   Lys Glu Tyr Ile Ala Gln Lys Glu Trp Leu Phe Gln Pro Glu Pro His
   195 200 205
   Leu Arg Leu Ile Gly Asp Leu Met Glu His Cys Ala Ser Lys Ile Pro
   210 215 220
   Ala Tyr Lys Pro Leu Ser Val Ser Gly Tyr His Ile Arg Glu Ala Gly
   225 230 235 240
   Ala Thr Ala Ala Gln Glu Leu Ala Tyr Thr Leu Ala Asp Gly Phe Gly
   245 250 255
   Tyr Val Glu Leu Gly Leu Ser Arg Gly Leu Asp Val Asp Val Phe Ala
   260 265 270
   Pro Gly Leu Ser Phe Phe Phe Asp Ala His Val Asp Phe Phe Glu Glu
   275 280 285
   Ile Ala Lys Phe Arg Ala Ala Arg Arg Ile Trp Ala Arg Trp Leu Arg
   290 295 300
   Asp Val Tyr Gly Ala Lys Ser Glu Lys Ala Gln Trp Leu Arg Phe His
   305 310 315 320
   Thr Gln Thr Ala Gly Val Ser Leu Thr Ala Gln Gln Pro Tyr Asn Asn
   325 330 335
   Val Val Arg Thr Ala Val Glu Ala Leu Ala Ala Val Leu Gly Gly Thr
   340 345 350
   Asn Ser Leu His Thr Asn Ala Leu Asp Glu Thr Leu Ala Leu Pro Ser
   355 360 365
   Glu Gln Ala Ala Glu Ile Ala Leu Arg Thr Gln Gln Val Leu Met Glu
   370 375 380
   Glu Thr Gly Val Ala Asn Val Ala Asp Pro Leu Gly Gly Ser Trp Tyr
   385 390 395 400
   Val Glu Gln Leu Thr Asp Arg Ile Glu Ala Asp Ala Glu Lys Ile Phe
   405 410 415
   Glu Gln Ile Arg Glu Arg Gly Leu Arg Ala His Pro Asp Gly Arg His
   420 425 430
   Pro Ile Gly Pro Ile Thr Ser Gly Ile Leu Arg Gly Ile Glu Asp Gly
   435 440 445
   Trp Phe Thr Gly Glu Ile Ala Glu Ser Ala Phe Gln Tyr Gln Gln Ala
   450 455 460
   Leu Glu Lys Gly Asp Lys Arg Val Val Gly Val Asn Val His His Gly
   465 470 475 480
   Ser Val Thr Gly Asp Leu Glu Ile Leu Arg Val Ser His Glu Val Glu
   485 490 495
   Arg Glu Gln Val Arg Val Leu Gly Glu Arg Lys Ser Gly Arg Asp Asp
   500 505 510
   Thr Ala Val Thr Ala Ala Leu Asp Ala Met Leu Ala Ala Ala Arg Asp
   515 520 525
   Gly Ser Asn Met Ile Ala Pro Met Leu Asp Ala Val Arg Ala Glu Ala
   530 535 540
   Thr Leu Gly Glu Ile Cys Asp Val Leu Arg Glu Glu Trp Gly Val Tyr
   545 550 555 560
   Thr Glu Pro Ala Gly Phe
   565
<210> 265
   <211> 417
   <212> DNA
   <213> Streptomyces avermitilis
<400> 265
   ctacgccccg gcaggctgcc gcacgttcgc ccgcacccac tccacgatcg acgccgtggt 60
   cgcccccgga gtgaagatct ccgcgacacc cttctccttc agcggcgcga tgtccgcctc 120
   ggggatgatg ccgccaccga acaccttgat gtcctcggca tcgcgctcct tgagcagatc 180
   gatgaccgcc gcgaacaacg tgttgtgcgc cccggacagg atcgacagcc cgatcgcgtc 240
   ggcgtcctcc tggatggccg tgcccacgat ctgctccggc gtctggtgca gccccgtgta 300
   aatgacctcc ataccggcat cgcgcagcgc ccgcgcgatc accttggccc cgcgatcgtg 360
   gccatcgagc cccggcttgg ccaccaccac gcggatcgga ccggctgcca cacccat 417
<210> 266
   <211> 138
   <212> PRT
   <213> Streptomyces avermitilis
<400> 266
   Met Gly Val Ala Ala Gly Pro Ile Arg Val Val Val Ala Lys Pro Gly
   1 5 10 15
   Leu Asp Gly His Asp Arg Gly Ala Lys Val Ile Ala Arg Ala Leu Arg
   20 25 30
   Asp Ala Gly Met Glu Val Ile Tyr Thr Gly Leu His Gln Thr Pro Glu
   35 40 45
   Gln Ile Val Gly Thr Ala Ile Gln Glu Asp Ala Asp Ala Ile Gly Leu
   50 55 60
   Ser Ile Leu Ser Gly Ala His Asn Thr Leu Phe Ala Ala Val Ile Asp
   65 70 75 80
   Leu Leu Lys Glu Arg Asp Ala Glu Asp Ile Lys Val Phe Gly Gly Gly
   85 90 95
   Ile Ile Pro Glu Ala Asp Ile Ala Pro Leu Lys Glu Lys Gly Val Ala
   100 105 110
   Glu Ile Phe Thr Pro Gly Ala Thr Thr Ala Ser Ile Val Glu Trp Val
   115 120 125
   Arg Ala Asn Val Arg Gln Pro Ala Gly Ala
   130 135
<210> 267
   <211> 2910
   <212> DNA
   <213> Staphylococcus aureus
<400> 267
   gatcaatttc ttttaagtaa tctaaatccc cattttttaa tttcttttta gcctctttaa 60
   ataatcctga ataaactaat acctgtttac ctttaagtga tttataaaat gcatcaaaga 120
   ctttttgatt tatttattaa ataatcacta tctttaccag aatacttagc catttcatat 180
   aattctttat tattattttg tcttattttt tgaacttgaa cttgtgttat ttctgaaatg 240
   cccgttacat cacgccataa atctaaccat tcttgttggc taatataata tcttttatct 300
   gtgaaatacg atttatttac tgcaattaac acatgaaaat gaggattata atcatctctt 360
   tttttattat atgtaatctc taacttacga acatatccct ttataacact acctactttt 420
   tttctcttta taagttttct aaaagaatta ttataacgtt ttatttcatt ttctaattca 480
   tcactcatta cattaggtgt agtcaaagtt aaaaagataa actccttttt ctcttgctgc 540
   ttaatatatt gcatcatcaa agataaaccc aatgcatctt ttctagcttt tctccaagca 600
   cagacaggac aaaatcgatt tttacaagaa ttagctttat ataatttctg tttttctaaa 660
   gttttatcag ctacaaaaga cagaaatgta ttgcaatctt caactaaatc catttgattc 720
   tctccaatat gacgtttaat aaatttctga aatacttgat ttctttgttt tttctcagta 780
   tacttttcca tgttataaca cataaaaaca acttagtttt cacaaactat gacaataaaa 840
   aaagttgctt tttccccttt ctatgtatgt tttttactag tcatttaaaa cgatacatta 900
   ataggtacga aaaagcaact ttttttgcgc ttaaaaccag tcataccaat aacttaaggg 960
   taactagcct cgccggcaat agttaccctt attatcaaga taagaaagaa aaggattttt 1020
   cgctacgctc aaatccttta aaaaaacaca aaagaccaca ttttttaatg tggtctttat 1080
   tcttcaacta aagcacccat tagttcaaca aacgaaaatt ggataaagtg ggatattttt 1140
   aaaatatata tttatgttac agtaatattg acttttaaaa aaggattgat tctaatgaag    1200
   aaagcagaca agtaagcctc ctaaattcac tttagataaa aatttaggag gcatatcaaa    1260
   tgaactttaa taaaattgat ttagacaatt ggaagagaaa agagatattt aatcattatt    1320
   tgaaccaaca aacgactttt agtataacca cagaaattga tattagtgtt ttataccgaa    1380
   acataaaaca agaaggatat aaattttacc ctgcatttat tttcttagtg acaagggtga    1440
   taaactcaaa tacagctttt agaactggtt acaatagcga cggagagtta ggttattggg    1500
   ataagttaga gccactttat acaatttttg atggtgtatc taaaacattc tctggtattt    1560
   ggactcctgt aaagaatgac ttcaaagagt tttatgattt atacctttct gatgtagaga    1620
   aatataatgg ttcggggaaa ttgtttccca aaacacctat acctgaaaat gctttttctc    1680
   tttctattat tccatggact tcatttactg ggtttaactt aaatatcaat aataatagta    1740
   attaccttct acccattatt acagcaggaa aattcattaa taaaggtaat tcaatatatt    1800
   taccgctatc tttacaggta catcattctg tttgtgatgg ttatcatgca ggattgttta    1860
   tgaactctat tcaggaattg tcagataggc ctaatgactg gcttttataa tatgagataa    1920
   tgccgactgt actttttaca gtcggttttc taatgtcact aacctgcccc gttagttgaa    1980
   gaaggttttt atattacagc tccagatcca tatccttctt tttctgaacc gacttctcct    2040
   ttttcgcttc tttattccaa ttgctttatt gacgttgagc ctcggaaccc ttaacaatcc    2100
   caaaacttgt cgaatggtcg gcttaatagc tcacgctatg ccgacattcg tctgcaagtt    2160
   tagttaaggg ttcttctcaa cgcacaataa attttctcgg cataaatgcg tggtctaatt    2220
   tttattttta ataaccttga tagcaaaaaa tgccattcca atacaaaacc acatacctat    2280
   aatcgataac cacataacag tcataaaacc actccttttt aacaaacttt atcacaagaa    2340
   atatttaaat tttaaatgcc tttattttga attttaaggg gcattttaaa gatttagggg    2400
   taaatcatat agttttatgc ctaaaaacct acagaagctt ttaaaaagca aatatgagcc    2460
   aaataaatat attctaattc tacaaacaaa aatttgagca aattcagtgt cgatttttta    2520
   agacactgcc cagttacatg caaattaaaa ttttcatgat tttttatagt tcctaacagg    2580
   gttaaaattt gtataacgaa agtataatgt ttatataacg ttagtataat aaagcatttt    2640
   aacattatac ttttgataat cgtttatcgt cgtcatcaca ataactttta aaatactcgt    2700
   gcataattca cgctgacctc ccaataacta catggtgtta tcgggaggtc agctgttagc    2760
   acttatattt tgttattgtt cttcctcgat ttcgtctatc attttgtgat taatttctct    2820
   tttttcttgt tctgttaagt cataaagttc actagctaaa tactcttttt gtttccaaat    2880
   ataaaaaatt tgatagatat attacggttg                                     2910

## Description

### FIELD OF THE INVENTION

The invention relates to a method for the production of isobutanol by fermentation using a recombinant microbial host. Specifically, the method employs a decrease in temperature during fermentation that results in more robust tolerance of the production host to the isobutanol product.

### BACKGROUND OF THE INVENTION

Butanol is an important industrial chemical, useful as a fuel additive, as a feedstock chemical in the plastics industry, and as a foodgrade extractant in the food and flavor industry. Each year 10 to12 billion pounds of butanol are produced by petrochemical means and the need for this commodity chemical will likely increase.

Methods for the chemical synthesis of isobutanol are known, such as oxo synthesis, catalytic hydrogenation of carbon monoxide (Ullmann's Encyclopedia of Industrial Chemistry, 6th edition, 2003, Wiley-VCHVerlag GmbH and Co., Weinheim, Germany, Vol. 5, pp. 716-719) and Guerbet condensation of methanol with n-propanol (Carlini et al., J. Mol. Catal. A:Chem. 220:215-220 (2004)). These processes use starting materials derived from petrochemicals and are generally expensive and are not environmentally friendly. The production of isobutanol from plant-derived raw materials would minimize greenhouse gas emissions and would represent an advance in the art.

Isobutanol is produced biologically as a by-product of yeast fermentation. It is a component of "fusel oil" that forms as a result of incomplete metabolism of amino acids by this group of fungi. Isobutanol is specifically produced from catabolism of L-valine. After the amine group of L-valine is harvested as a nitrogen source, the resulting α-keto acid is decarboxylated and reduced to isobutanol by enzymes of the so-called Ehrlich pathway (Dickinson et al., J. Biol. Chem. 273(40):25752-25756 (1998)). Yields of fusel oil and/or its components achieved during beverage fermentation are typically low. For example, the concentration of isobutanol produced in beer fermentation is reported to be less than 16 parts per million (Garcia et al., Process Biochemistry 29:303-309 (1994)). Addition of exogenous L-valine to the fermentation increases the yield of isobutanol, as described by Dickinson *et al*., *supra,* wherein it is reported that a yield of isobutanol of 3 g/L is obtained by providing L-valine at a concentration of 20 g/L in the fermentation. However, the use of valine as a feed-stock would be cost prohibitive for industrial scale isobutanol production. Jansen *et al.* report the use of a high-throughput screening approach in microtiter plates to study the effects of pH, temperature and salt concentration on growth of *Z*. *rouxii* and formation of fusel alcohols from branched-chain amino acids (FEMS Yeast Research, 3, (2003), 313-318). The biosynthesis of isobutanol directly from sugars would be economically viable and would represent an advance in the art.

Recombinant microbial production hosts expressing an isobutanol biosynthetic pathway have been described (Donaldson et al., copending and commonly owned U.S. Patent Application Publication No. US2007 0092957 A1). However, biological production of isobutanol is believed to be limited by butanol toxicity to the host microorganism used in the fermentation.

Some microbial strains that are tolerant to isobutanol are known in the art (Bramucci et al., copending and commonly owned U.S. Patent Application Nos. 11/743220 (US 2007 259411). The document US 2007/0092957 A1 especially discloses a method for producing isobutanol comprising:
a) providing a recombinant microbial production host which produces isobutanol, wherein said recombinant microbial host comprises DNA molecules encoding polypeptides that catalyse each of the following substrate to product conversions:
   i) pyruvate to acetolactate
   ii) acetolactate to 2,3-dihydroxyisovalerate
   iii) 2,3-dihydroxyisovalerate to alpha-ketoisovalerate
   iv) alpha-ketoisovalerate to isobutyraldehyde and
   v) isobutyraldehyde to isobutanol,
   wherein at least one DNA molecule is heterologous to said microbial production host
b) seeding the production host of a) into a fermentation medium comprising a fermentable carbon substrate to create a fermentation culture
c) incubating said production host at the temperature of about 25 °C to about 40 °C, whereby isobutanol is produced.
However, biological methods of producing isobutanol to higher levels are required for cost effective commercial production.

There have been reports describing the effect of temperature on the tolerance of some microbial strains to ethanol. For example, Amartey et al. (Biotechnol. Lett. 13(9):627-632 (1991)) disclose that *Bacillus stearothermophillus* is less tolerant to ethanol at 70 °C than at 60 °C. Herrero et al. (Appl. Environ. Microbiol. 40(3):571-577 (1980)) report that the optimum growth temperature of a wild-type strain of *Clostridium thermocellum* decreases as the concentration of ethanol challenge increases, whereas the optimum growth temperature of an ethanol-tolerant mutant remains constant. Brown et al. (Biotechnol. Lett. 4(4):269-274 (1982)) disclose that the yeast *Saccharomyces uvarum* is more resistant to growth inhibition by ethanol at temperatures 5 °C and 10 °C below its growth optimum of 35 °C. However, fermentation became more resistant to ethanol inhibition with increasing temperature. Additionally, Van Uden (CRC Crit. Rev. Biotechnol. 1(3):263-273 (1984)) report that ethanol and other alkanols depress the maximum and the optimum growth temperature for growth of *Saccharomyces cerevisiae* while thermal death is enhanced. Moreover, Lewis et al. (U.S. Patent Application Publication No. 2004/0234649) describe methods for producing high levels of ethanol during fermentation of plant material comprising decreasing the temperature during saccharifying, fermenting, or simultaneously saccharifying and fermenting.

Much less is known about the effect of temperature on the tolerance of microbial strains to butanols. Harada (Hakko Kyokaishi 20:155-156 (1962)) discloses that the yield of 1-butanol in acetone-butanol-ethanol (ABE) fermentation is increased from 18.4%-18.7% to 19.1%-21.2% by lowering the temperature from 30 °C to 28 °C when the growth of the bacteria reaches a maximum. Jones et al. (Microbiol. Rev. 50(4):484-524 (1986)) review the role of temperature in ABE fermentation. They report that the solvent yields of three different solvent producing strains remains fairly constant at 31 % at 30 °C and 33 °C, but decreases to 23 to 25% at 37 °C. Similar results were reported for *Clostridium acetobutylicum* for which solvent yields decreased from 29% at 25 °C to 24% at 40 °C. In the latter case, the decrease in solvent yield was attributed to a decrease in acetone production while the yield of 1-butanol was unaffected. However, Carnarius (U.S. Patent No. 2,198,104) reports that an increase in the butanol ratio is obtained in the ABE process by decreasing the temperature of the fermentation from 30 °C to 24 °C after 16 hours. However, the effect of temperature on the production of isobutanol by recombinant microbial hosts is not known in the art.

There is a need, therefore, for a cost-effective process for the production of isobutanol by fermentation that provides higher yields than processes known in the art. The present invention addresses this need through the discovery of a method for producing isobutanol by fermentation using a recombinant microbial host, which employs a decrease in temperature during fermentation, resulting in more robust tolerance of the production host to the isobutanol product.

### SUMMARY OF THE INVENTION

The invention provides a method for the production of isobutanol by fermentation using a recombinant microbial host, which employs a decrease in temperature during fermentation that results in more robust tolerance of the production host to the isobutanol product.

Accordingly, the invention provides a method for the production of isobutanol comprising:
a) providing a recombinant microbial production host which produces isobutanol, wherein said recombinant microbial host comprises DNA molecules encoding polypeptides that catalyse each of the following substrate to product conversions:
   i) pyruvate to acetolactate;
   ii) acetolactate to 2,3-dihydroxyisovalerate;
   iii) 2,3-dihydroxyisovalerate to α-ketoisovalerate;
   iv) α-ketoisovalerate to isobutyraldehyde; and
   v) isobutyraldehyde to isobutanol;
   wherein at least one of said DNA molecules is heterologous to said recombinant microbial host;
b) seeding the production host of (a) into a fermentation medium comprising a fermentable carbon substrate to create a fermentation culture;
c) growing the production host in the fermentation culture at a first temperature for a first period of time, wherein the first temperature is from about 25 °C to about 40 °C;
d) lowering the temperature of the fermentation culture to a second temperature; and
e) incubating the production host at the second temperature of step (d) for a second period of time;
whereby isobutanol is produced.

Also disclosed herein is a method for the production of isobutanol comprising:
a) providing a recombinant microbial production host which produces isobutanol;
b) seeding the production host of (a) into a fermentation medium comprising a fermentable carbon substrate to create a fermentation culture;
c) growing the production host in the fermentation culture at a first temperature for a first period of time;
d) lowering the temperature of the fermentation culture to a second temperature; and
e) incubating the production host at the second temperature of step (d) for a second period of time;
whereby isobutanol is produced.

### BRIEF DESCRIPTION OF THE FIGURES AND SEQUENCE DESCRIPTIONS

The invention can be more fully understood from the following detailed description, figure, and the accompanying sequence descriptions, which form a part of this application.

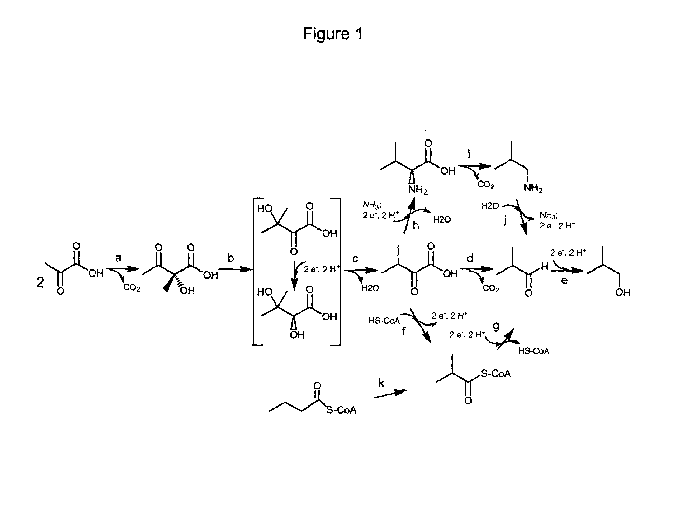

Figure 1 shows four different isobutanol biosynthetic pathways. The steps labeled "a", "b", "c", "d", "e", "f", "g", "h", "i", "j" and "k" represent the substrate to product conversions described below. The following sequences conform with 37 C.F.R. 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

**Table 1**

| Summary of Gene and Protein SEQ ID Numbers | | |
|---|---|---|
| Description | SEQ ID NO: Nucleic acid | SEQ ID NO: Peptide |
| *Klebsiella pneumoniae budB* (acetolactate synthase) | 1 | 2 |
| *Bacillus subtilis* alsS (acetolactate synthase) | 78 | 178 |
| *Lactococcus lactis als* (acetolactate synthase) | 179 | 180 |
| *E. coli ilvC* (acetohydroxy acid reductoisomerase) | 3 | 4 |
| *S. cerevisiae IL V5* (acetohydroxy acid reductoisomerase) | 80 | 181 |
| *M. maripaludis* ilvC (Ketol-acid reductoisomerase) | 182 | 183 |
| *B. subtilis ilvC* (acetohydroxy acid reductoisomerase) | 184 | 185 |
| *E. coli ilvD* (acetohydroxy acid dehydratase) | 5 | 6 |
| *S. cerevisiae ILV3* (Dihydroxyacid dehydratase) | 83 | 186 |
| *M. maripaludis ilvD* (Dihydroxy-acid dehydratase) | 187 | 188 |
| *B. subtilis ilvD* (dihydroxy-acid dehydratase) | 189 | 190 |
| *Lactococcus lactis kivD* (branched-chain α-keto acid decarboxylase), codon optimized | 7 | 8 |
| *Lactococcus lactis kivD* (branched-chain α-keto acid decarboxylase), | 191 | 8 |
| *Lactococcus lactis kdcA* (branched-chain alpha-ketoacid decarboxylase) | 192 | 193 |
| *Salmonella typhimurium* (indolepyruvate decarboxylase) | 194 | 195 |
| *Clostridium acetobutylicum pdc* (Pyruvate decarboxylase) | 196 | 197 |
| *E*. *coli yqhD* (branched-chain alcohol dehydrogenase) | 9 | 10 |
| *S. cerevisiae YPR1* (2-methylbutyraldehyde reductase) | 198 | 199 |
| *S*. *cerevisiae ADH6* (NADPH-dependent cinnamyl alcohol dehydrogenase) | 200 | 201 |
| *Clostridium acetobutylicum bdhA* (NADH-dependent butanol dehydrogenase A) | 202 | 203 |
| *Clostridium acetobutylicum bdhB* Butanol dehydrogenase | 158 | 204 |
| *B. subtilis bkdAA* (branched-chain keto acid dehydrogenase E1 subunit) | 205 | 206 |
| *B. subtilis* bkdAB (branched-chain alpha-keto acid dehydrogenase E1 subunit) | 207 | 208 |
| *B. subtilis bkdB* (branched-chain alpha-keto acid dehydrogenase E2 subunit) | 209 | 210 |
| *B. subtilis IpdV* (branched-chain alpha-keto acid dehydrogenase E3 subunit) | 211 | 212 |
| *P. putida bkdA1* (keto acid dehydrogenase E1-alpha subunit) | 213 | 214 |
| *P. putida bkdA2* (keto acid dehydrogenase E1-beta subunit) | 215 | 216 |
| *P. putida bkdB* (transacylase E2) | 217 | 218 |
| *P. putida 1pdV* (lipoamide dehydrogenase) | 219 | 220 |
| *C*. *beijerinckii ald* (coenzyme A acylating aldehyde dehydrogenase) | 221 | 222 |
| *C. acetobutylicum adhe*1 (aldehyde dehydrogenase) | 223 | 224 |
| *C*. *acetobutylicum adhe* (alcohol-aldehyde dehydrogenase) | 225 | 226 |
| *P. putida nahO* (acetaldehyde dehydrogenase) | 227 | 228 |
| *T. thermophilus* (acetaldehyde dehydrogenase) | 229 | 230 |
| *E. coli avtA* (valine-pyruvate transaminase) | 231 | 232 |
| *B. licheniformis avtA* (valine-pyruvate transaminase) | 233 | 234 |
| *E. coli ilvE* (branched chain amino acid aminotransferase) | 235 | 236 |
| *S. cerevisiae BAT2* (branched chain amino acid aminotransferase) | 237 | 238 |
| *M. thermoautotrophicum* (branched chain amino acid aminotransferase) | 239 | 240 |
| *S. coelicolor* (valine dehydrogenase) | 241 | 242 |
| *B. subtilis bcd* (leucine dehydrogenase) | 243 | 244 |
| *S*. *viridifaciens* (valine decarboxyase) | 245 | 246 |
| *A. denitrificans aptA* (omega-amino acid:pyruvate transaminase) | 247 | 248 |
| *R*. *eutropha* (alanine-pyruvate transaminase) | 249 | 250 |
| *S. oneidensis* (beta alanine-pyruvate transaminase) | 251 | 252 |
| *P. putida* (beta alanine-pyruvate transaminase) | 253 | 254 |
| *S*. *cinnamonensis icm* (isobutyrl-CoA mutase) | 255 | 256 |
| *S*. *cinnamonensis icmB* (isobutyrl-CoA mutase) | 257 | 258 |
| *S. coelicolor* SCO5415 (isobutyrl-CoA mutase) | 259 | 260 |
| *S. coelicolor SCO4800* (isobutyrl-CoA mutase) | 261 | 262 |
| *S. avermitilis icmA* (isobutyrl-CoA mutase) | 263 | 264 |
| *S. avermitilis icmB* (isobutyrl-CoA mutase) | 265 | 266 |

SEQ ID NOs:11-38, 40-69, 72-75, 85-138, 144, 145, 147-157, 159-176 are the nucleotide sequences of oligonucleotide cloning, screening or sequencing primers used in the Examples described herein.

SEQ ID NO:39 is the nucleotide sequence of the *cscBKA* gene cluster described in Example 20.

SEQ ID NO:70 is the nucleotide sequence of the glucose isomerase promoter 1.6GI described in Example 17.

SEQ ID NO:71 is the nucleotide sequence of the 1.5GI promoter described in Example 17.

SEQ ID NO:76 is the nucleotide sequence of the GPD promoter described in Example 21.

SEQ ID NO:77 is the nucleotide sequence of the CYC1 terminator described in Example 21.

SEQ ID NO:79 is the nucleotide sequence of the FBA promoter described in Example 21.

SEQ ID NO:81 is the nucleotide sequence of ADH1 promoter described in Example 21.

SEQW ID NO:82 is the nucleotide sequence of ADH1 terminator described in Example 21.

SEQ ID NO:84 is the nucleotide sequence of GPM promoter described in Example 21.

SEQ ID NO:139 is the amino acid sequence of sucrose hydrolase (CscA).

SEQ ID NO:140 is the amino acid sequence of D-fructokinase (CscK).

SEQ ID NO:141 is the amino acid sequence of sucrose permease (CscB).

SEQ ID NO:142 is the nucleotide sequence of plasmid pFP988DssPspac described in Example 24.

SEQ ID NO:143 is the nucleotide sequence of plasmid pFP988DssPgroE described in Example 24.

SEQ ID NO:146 is the nucleotide sequence of the pFP988Dss vector fragment described in Example 24.

SEQ ID NO:177 is the nucleotide sequence of the pFP988 integration vector described in Example 25.

SEQ ID NO:267 is the nucleotide sequence of plasmid pC194 described in Example 25.

### DETAILED DESCRIPTION OF THE INVENTION

The present invention relates to a method for the production of isobutanol using recombinant microorganisms that employs a decrease in temperature during fermentation, resulting in more robust tolerance of the production host to the isobutanol product and therefore a higher titer of isobutanol. The present invention meets a number of commercial and industrial needs. Butanol is an important industrial commodity chemical with a variety of applications, where its potential as a fuel or fuel additive is particularly significant. Although only a four-carbon alcohol, butanol has an energy content similar to that of gasoline and can be blended with any fossil fuel. Butanol is favored as a fuel or fuel additive as it yields only CO₂ and little or no SOₓ or NOₓ when burned in the standard internal combustion engine. Additionally butanol is less corrosive than ethanol, the most preferred fuel additive to date.

In addition to its utility as a biofuel or fuel additive, butanol has the potential of impacting hydrogen distribution problems in the emerging fuel cell industry. Fuel cells today are plagued by safety concerns associated with hydrogen transport and distribution. Butanol can be easily reformed for its hydrogen content and can be distributed through existing gas stations in the purity required for either fuel cells or vehicles.

Finally the present invention produces isobutanol from plant derived carbon sources, avoiding the negative environmental impact associated with standard petrochemical processes for butanol production.

The following definitions and abbreviations are to be used for the interpretation of the claims and the specification.

As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

The term "invention" or "present invention" as used herein is not intended to refer to any single embodiment of the particular invention but encompasses all possible embodiments as described in the specification and the claims.

As used herein, the term "about" modifying the quantity of an ingredient or reactant of the invention employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities. In one embodiment, the term "about" means within 10% of the reported numerical value, preferably within 5% of the reported numerical value.

The term "isobutanol biosynthetic pathway" refers to an enzyme pathway to produce isobutanol.

The terms "acetolactate synthase" and "acetolactate synthetase" are used intechangeably herein to refer to an enzyme that catalyzes the conversion of pyruvate to acetolactate and CO₂. Preferred acetolactate synthases are known by the EC number 2.2.1.6 9 (Enzyme Nomenclature 1992, Academic Press, San Diego). These enzymes are available from a number of sources, including, but not limited to, *Bacillus subtilis* (GenBank Nos: CAB15618 (SEQ ID NO:178), Z99122 (SEQ ID NO:78), NCBI (National Center for Biotechnology Information) amino acid sequence, NCBI nucleotide sequence, respectively), *Klebsiella pneumoniae* (GenBank Nos: AAA25079 (SEQ ID NO:2), M73842 (SEQ ID NO:1)), and *Lactococcus lactis* (GenBank Nos: AAA25161 (SEQ ID NO:180), L16975 (SEQ ID NO:179)).

The terms "acetohydroxy acid isomeroreductase" and "acetohydroxy acid reductoisomerase" are used interchangeably herein to refer to an enzyme that catalyzes the conversion of acetolactate to 2,3-dihydroxyisovalerate using NADPH (reduced nicotinamide adenine dinucleotide phosphate) as an electron donor. Preferred acetohydroxy acid isomeroreductases are known by the EC number 1.1.1.86 and sequences are available from a vast array of microorganisms, including, but not limited to, *Escherichia coli* (GenBank Nos: NP_418222 (SEQ ID NO:4), NC_000913 (SEQ ID NO:3)), *Saccharomyces cerevisiae* (GenBank Nos: NP_013459 (SEQ ID NO:181), NC_001144 (SEQ ID NO:80)), *Methanococcus maripaludis* (GenBank Nos: CAF30210 (SEQ ID NO:183), BX957220 (SEQ ID NO:182)), and *Bacillus. subtilis* (GenBank Nos: CAB14789 (SEQ ID NO:185), Z99118 (SEQ ID NO:184)).

The term "acetohydroxy acid dehydratase" refers to an enzyme that catalyzes the conversion of 2,3-dihydroxyisovalerate to α-ketoisovalerate. Preferred acetohydroxy acid dehydratases are known by the EC number 4.2.1.9. These enzymes are available from a vast array of microorganisms, including, but not limited to, *E*. *coli* (GenBank Nos: YP_026248 (SEQ ID NO:6), NC_000913 (SEQ ID NO:5)), *S*. *cerevisiae* (GenBank Nos: NP_012550 (SEQ ID NO:186), NC_001142 (SEQ ID NO:83)), *M*. *maripaludis* (GenBank Nos: CAF29874 (SEQ ID NO:188), BX957219 (SEQ ID NO:187)), and *B. subtilis* (GenBank Nos: CAB14105 (SEQ ID NO:190), Z99115 (SEQ ID NO:189)).

The term "branched-chain α-keto acid decarboxylase" refers to an enzyme that catalyzes the conversion of α-ketoisovalerate to isobutyraldehyde and CO₂. Preferred branched-chain α-keto acid decarboxylases are known by the EC number 4.1.1.72 and are available from a number of sources, including, but not limited to, *Lactococcus lactis* (GenBank Nos: AAS49166 (SEQ ID NO:193), AY548760 (SEQ ID NO:192); CAG34226 (SEQ ID NO:8), AJ746364 (SEQ ID NO:191), *Salmonella typhimurium* (GenBank Nos: NP_461346 (SEQ ID NO:195), NC_003197 (SEQ ID NO:194)), and *Clostridium acetobutylicum* (GenBank Nos: NP_149189 (SEQ ID NO:197), NC_001988 (SEQ ID NO:196)).

The term "branched-chain alcohol dehydrogenase" refers to an enzyme that catalyzes the conversion of isobutyraldehyde to isobutanol. Preferred branched-chain alcohol dehydrogenases are known by the EC number 1.1.1.265, but may also be classified under other alcohol dehydrogenases (specifically, EC 1.1.1.1 or 1.1.1.2). These enzymes utilize NADH (reduced nicotinamide adenine dinucleotide) and/or NADPH as electron donor and are available from a number of sources, including, but not limited to, *S*. *cerevisiae* (GenBank Nos: NP_010656 (SEQ ID NO:199), NC_001136 (SEQ ID NO:198); NP_014051 (SEQ ID NO:201) NC_001145 (SEQ ID NO:200)), *E*. *coli* (GenBank Nos: NP_417484 (SEQ ID NO:10), NC_000913 (SEQ ID NO:9)), and *C*. *acetobutylicum* (GenBank Nos: NP_349892 (SEQ ID NO:203), NC_003030 (SEQ ID NO:202); NP_349891 (SEQ ID NO:204), NC_003030 (SEQ ID NO:158)).

The term "branched-chain keto acid dehydrogenase" refers to an enzyme that catalyzes the conversion of α-ketoisovalerate to isobutyryl-CoA (isobutyryl-coenzyme A), using NAD⁺ (nicotinamide adenine dinucleotide) as electron acceptor. Preferred branched-chain keto acid dehydrogenases are known by the EC number 1.2.4.4. These branched-chain keto acid dehydrogenases are comprised of four subunits and sequences from all subunits are available from a vast array of microorganisms, including, but not limited to, *B. subtilis* (GenBank Nos: CAB14336 (SEQ ID NO:206), Z99116 (SEQ ID NO:205); CAB14335 (SEQ ID NO:208), Z99116 (SEQ ID NO:207); CAB14334 (SEQ ID NO:210), Z99116 (SEQ ID NO:209); and CAB14337 (SEQ ID NO:212), Z99116 (SEQ ID NO:211)) and *Pseudomonas putida* (GenBank Nos: AAA65614 (SEQ ID NO:214), M57613 (SEQ ID NO:213); AAA65615 (SEQ ID NO:216), M57613 (SEQ ID NO:215); AAA65617 (SEQ ID NO:218), M57613 (SEQ ID NO:217); and AAA65618 (SEQ ID NO:220), M57613 (SEQ ID NO:219)).

The term "acylating aldehyde dehydrogenase" refers to an enzyme that catalyzes the conversion of isobutyryl-CoA to isobutyraldehyde, using either NADH or NADPH as electron donor. Preferred acylating aldehyde dehydrogenases are known by the EC numbers 1.2.1.10 and 1.2.1.57. These enzymes are available from multiple sources, including, but not limited to, *Clostridium beijerinckii* (GenBank Nos: AAD31841 (SEQ ID NO:222), AF157306 (SEQ ID NO:221)), C. *acetobutylicum* (GenBank Nos: NP_149325 (SEQ ID NO:224), NC_001988 (SEQ ID NO:223); NP_149199 (SEQ ID NO:226), NC_001988 (SEQ ID NO:225)), *P. putida* (GenBank Nos: AAA89106 (SEQ ID NO:228), U13232 (SEQ ID NO:227)), and *Thermus thermophilus* (GenBank Nos: YP_145486 (SEQ ID NO:230), NC_006461 (SEQ ID NO:229)).

The term "transaminase" refers to an enzyme that catalyzes the conversion of α-ketoisovalerate to L-valine, using either alanine or glutamate as amine donor. Preferred transaminases are known by the EC numbers 2.6.1.42 and 2.6.1.66. These enzymes are available from a number of sources. Examples of sources for alanine-dependent enzymes include, but are not limited to, *E*. *coli* (GenBank Nos: YP_026231 (SEQ ID NO:232), NC_000913 (SEQ ID NO:231)) and *Bacillus licheniformis* (GenBank Nos: YP_093743 (SEQ ID NO:234), NC_006322 (SEQ ID NO:233)). Examples of sources for glutamate-dependent enzymes include, but are not limited to, *E*. *coli* (GenBank Nos: YP_026247 (SEQ ID NO:236), NC_000913 (SEQ ID NO:235)), *S*. *cerevisiae* (GenBank Nos: NP_012682 (SEQ ID NO:238), NC_001142 (SEQ ID NO:237)) and *Methanobacterium thermoautotrophicum* (GenBank Nos: NP_276546 (SEQ ID NO:240), NC_000916 (SEQ ID NO:239)).

The term "valine dehydrogenase" refers to an enzyme that catalyzes the conversion of α-ketoisovalerate to L-valine, using NAD(P)H as electron donor and ammonia as amine donor. Preferred valine dehydrogenases are known by the EC numbers 1.4.1.8 and 1.4.1.9 and are available from a number of sources, including, but not limited to, *Streptomyces coelicolor* (GenBank Nos: NP_628270 (SEQ ID NO:242), NC_003888 (SEQ ID NO:241)) and *B. subtilis* (GenBank Nos: CAB14339 (SEQ ID NO:244), Z99116 (SEQ ID NO:243)).

The term "valine decarboxylase" refers to an enzyme that catalyzes the conversion of L-valine to isobutylamine and CO₂. Preferred valine decarboxylases are known by the EC number 4.1.1.14. These enzymes are found in Streptomycetes, such as for example, *Streptomyces viridifaciens* (GenBank Nos: AAN10242 (SEQ ID NO:246), AY116644 (SEQ ID NO:245)).

The term "omega transaminase" refers to an enzyme that catalyzes the conversion of isobutylamine to isobutyraldehyde using a suitable amino acid as amine donor. Preferred omega transaminases are known by the EC number 2.6.1.18 and are available from a number of sources, including, but not limited to, *Alcaligenes denitrificans* (AAP92672 (SEQ ID NO:248), AY330220 (SEQ ID NO:247)), *Ralstonia eutropha* (GenBank Nos: YP_294474 (SEQ ID NO:250), NC_007347 (SEQ ID NO:249)), *Shewanella oneidensis* (GenBank Nos: NP_719046 (SEQ ID NO:252), NC_004347 (SEQ ID NO:251)), and *P. putida* (GenBank Nos: AAN66223 (SEQ ID NO:254), AE016776 (SEQ ID NO:253)).

The term "isobutyryl-CoA mutase" refers to an enzyme that catalyzes the conversion of butyryl-CoA to isobutyryl-CoA. This enzyme uses coenzyme B₁₂ as cofactor. Preferred isobutyryl-CoA mutases are known by the EC number 5.4.99.13. These enzymes are found in a number of Streptomycetes, including, but not limited to, *Streptomyces cinnamonensis* (GenBank Nos: AAC08713 (SEQ ID NO:256), U67612 (SEQ ID NO:255); CAB59633 (SEQ ID NO:258), AJ246005 (SEQ ID NO:257)), *S*. *coelicolor* (GenBank Nos: CAB70645 (SEQ ID NO:260), AL939123 (SEQ ID NO:259); CAB92663 (SEQ ID NO:262), AL939121 (SEQ ID NO:261)), and *Streptomyces avermitilis* (GenBank Nos: NP_824008 (SEQ ID NO:264), NC_003155 (SEQ ID NO:263); NP_824637 (SEQ ID NO:266), NC_003155 (SEQ ID NO:265)).

The term "a facultative anaerobe" refers to a microorganism that can grow in both aerobic and anaerobic environments.

The term "carbon substrate" or "fermentable carbon substrate" refers to a carbon source capable of being metabolized by host organisms disclosed herein and particularly carbon sources selected from the group consisting of monosaccharides, oligosaccharides, polysaccharides, and one-carbon substrates or mixtures thereof.

The term "gene" refers to a nucleic acid fragment that is capable of being expressed as a specific protein, optionally including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign gene" or "heterologous gene" refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

As used herein, an "isolated nucleic acid fragment" or "isolated nucleic acid molecule" or "genetic construct" will be used interchangeably and will mean a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA.

A nucleic acid fragment is "hybridizable" to another nucleic acid fragment, such as a cDNA, genomic DNA, or RNA molecule, when a single-stranded form of the nucleic acid fragment can anneal to the other nucleic acid fragment under the appropriate conditions of temperature and solution ionic strength. Hybridization and washing conditions are well known and exemplified in Sambrook, J., Fritsch, E. F. and Maniatis, T. Molecular Cloning: A Laboratory Manual, 2nd ed., Cold Spring Harbor Laboratory: Cold Spring Harbor, NY (1989), particularly Chapter 11 and Table 11.1 therein. The conditions of temperature and ionic strength determine the "stringency" of the hybridization. Stringency conditions can be adjusted to screen for moderately similar fragments (such as homologous sequences from distantly related organisms), to highly similar fragments (such as genes that duplicate functional enzymes from closely related organisms). Post-hybridization washes determine stringency conditions. One set of preferred conditions uses a series of washes starting with 6X SSC, 0.5% SDS at room temperature for 15 min, then repeated with 2X SSC, 0.5% SDS at 45 °C for 30 min, and then repeated twice with 0.2X SSC, 0.5% SDS at 50 °C for 30 min. A more preferred set of stringent conditions uses higher temperatures in which the washes are identical to those above except for the temperature of the final two 30 min washes in 0.2X SSC, 0.5% SDS was increased to 60 °C. Another preferred set of highly stringent conditions uses two final washes in 0.1X SSC, 0.1 % SDS at 65 °C. An additional set of stringent conditions include hybridization at 0.1X SSC, 0.1% SDS, 65 °C and washes with 2X SSC, 0.1 % SDS followed by 0.1X SSC, 0.1% SDS, for example.

Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher T*m*) of nucleic acid hybridizations decreases in the following order: RNA:RNA, DNA:RNA, DNA:DNA. For hybrids of greater than 100 nucleotides in length, equations for calculating Tm have been derived (see Sambrook et al., *supra,* 9.50-9.51). For hybridizations with shorter nucleic acids, i.e., oligonucleotides, the position of mismatches becomes more important, and the length of the oligonucleotide determines its specificity (see Sambrook et al., *supra,* 11.7-11.8). In one embodiment the length for a hybridizable nucleic acid is at least about 10 nucleotides. Preferably a minimum length for a hybridizable nucleic acid is at least about 15 nucleotides; more preferably at least about 20 nucleotides; and most preferably the length is at least about 30 nucleotides. Furthermore, the skilled artisan will recognize that the temperature and wash solution salt concentration may be adjusted as necessary according to factors such as length of the probe.

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Altschul, S. F., et al., J. Mol. Biol., 215:403-410 (1993)). In general, a sequence of ten or more contiguous amino acids or thirty or more nucleotides is necessary in order to putatively identify a polypeptide or nucleic acid sequence as homologous to a known protein or gene. Moreover, with respect to nucleotide sequences, gene specific oligonucleotide probes comprising 20-30 contiguous nucleotides may be used in sequence-dependent methods of gene identification (e.g., Southern hybridization) and isolation (e.g., *in situ* hybridization of bacterial colonies or bacteriophage plaques). In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence. The instant specification teaches the complete amino acid and nucleotide sequence encoding particular fungal proteins. The skilled artisan, having the benefit of the sequences as reported herein, may now use all or a substantial portion of the disclosed sequences for purposes known to those skilled in this art. Accordingly, the instant specification comprises the complete sequences as reported in the accompanying Sequence Listing, as well as substantial portions of those sequences as defined above.

The term "complementary" is used to describe the relationship between nucleotide bases that are capable of hybridizing to one another. For example, with respect to DNA, adenosine is complementary to thymine and cytosine is complementary to guanine.

The terms "homology" and "homologous" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant discloure such as deletion or insertion of one or more nucleotides that do not substantially alter the functional properties of the resulting nucleic acid fragment relative to the initial, unmodified fragment. It is therefore understood, as those skilled in the art will appreciate, that the disclosure encompasses more than the specific exemplary sequences.

Moreover, the skilled artisan recognizes that homologous nucleic acid sequences encompassed by this disclosure are also defined by their ability to hybridize, under moderately stringent conditions (e.g., 0.5 X SSC, 0.1 % SDS, 60 °C) with the sequences exemplified herein, or to any portion of the nucleotide sequences disclosed herein and which are functionally equivalent to any of the nucleic acid sequences disclosed herein.

"Codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

The term "percent identity", as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in: 1.) Computational Molecular Biology (Lesk, A. M., Ed.) Oxford University: NY (1988); 2.) Biocomputing: Informatics and Genome Projects (Smith, D. W., Ed.) Academic: NY (1993); 3.) Computer Analysis of Sequence Data, Part I (Griffin, A. M., and Griffin, H. G., Eds.) Humania: NJ (1994); 4.) Sequence Analysis in Molecular Biology (von Heinje, G., Ed.) Academic (1987); and 5.) Sequence Analysis Primer (Gribskov, M. and Devereux, J., Eds.) Stockton: NY (1991).

Preferred methods to determine identity are designed to give the best match between the sequences tested. Methods to determine identity and similarity are codified in publicly available computer programs. Sequence alignments and percent identity calculations may be performed using the MegAlign^{™} program of the LASERGENE bioinformatics computing suite (DNASTAR Inc., Madison, WI). Multiple alignment of the sequences is performed using the "Clustal method of alignment" which encompasses several varieties of the algorithm including the "Clustal V method of alignment" corresponding to the alignment method labeled Clustal V (described by Higgins and Sharp, CABIOS. 5:151-153 (1989); Higgins, D.G. et al., Comput. Appl. Biosci., 8:189-191 (1992)) and found in the MegAlign™ program of the LASERGENE bioinformatics computing suite (DNASTAR Inc.). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program. Additionally the "Clustal W method of alignment" is available and corresponds to the alignment method labeled Clustal W (described by Higgins and Sharp, CABIOS. 5:151-153 (1989); Higgins, D.G. et al., Comput. Appl. Biosci. 8:189-191(1992)) and found in the MegAlign™ v6.1 program of the LASERGENE bioinformatics computing suite (DNASTAR Inc.). Default parameters for multiple alignment (GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

It is well understood by one skilled in the art that many levels of sequence identity are useful in identifying polypeptides, from other species, wherein such polypeptides have the same or similar function or activity. Useful examples of percent identities include, but are not limited to: 24%, 30%, 35%, 40%, 45%, 50%, 55%, 60%, 65%, 70%, 75%, 80%, 85%, 90%, or 95%, or any integer percentage from 24% to 100% may be useful in describing the present disclosure such as 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. Suitable nucleic acid fragments not only have the above homologies but typically encode a polypeptide having at least 50 amino acids, preferably at least 100 amino acids, more preferably at least 150 amino acids, still more preferably at least 200 amino acids, and most preferably at least 250 amino acids.

The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1.) the GCG suite of programs (Wisconsin Package Version 9.0, Genetics Computer Group (GCG), Madison, WI); 2.) BLASTP, BLASTN, BLASTX (Altschul et al., J. Mol. Biol., 215:403-410 (1990)); 3.) DNASTAR (DNASTAR, Inc. Madison, WI); 4.) Sequencher (Gene Codes Corporation, Ann Arbor, MI); and 5.) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

As used herein the term "coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, polyadenylation recognition sequences, RNA processing site, effector binding site and stem-loop structure.

The term "promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters which cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The term "operably linked" refers to the association of nucleic acid sequences on a single nucleic acid fragment so that the function of one is affected by the other. For example, a promoter is operably linked with a coding sequence when it is capable of effecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment disclosed herein. Expression may also refer to translation of mRNA into a polypeptide.

As used herein the term "transformation" refers to the transfer of a nucleic acid fragment into a host organism, resulting in genetically stable inheritance. Host organisms containing the transformed nucleic acid fragments are referred to as "transgenic" or "recombinant" or "transformed" organisms.

The terms "plasmid" and "vector" refer to an extra chromosomal element often carrying genes which are not part of the central metabolism of the cell, and usually in the form of circular double-stranded DNA moledules. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell. "Transformation vector" refers to a specific vector containing a foreign gene and having elements in addition to the foreign gene that facilitates transformation of a particular host cell.

As used herein the term "codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

The term "codon-optimized" as it refers to genes or coding regions of nucleic acid molecules for transformation of various hosts, refers to the alteration of codons in the gene or coding regions of the nucleic acid molecules to reflect the typical codon usage of the host organism without altering the polypeptide encoded by the DNA.

Standard recombinant DNA and molecular cloning techniques used herein are well known in the art and are described by Sambrook, J., Fritsch, E. F. and Maniatis, T., Molecular Cloning: A Laboratory Manual, Second Edition, Cold Spring Harbor Laboratory Press, Cold Spring Harbor, NY (1989) (hereinafter "Maniatis"); and by Silhavy, T. J., Bennan, M. L. and Enquist, L. W., Experiments with Gene Fusions, Cold Spring Harbor Laboratory Press, Cold Spring Harbor, NY (1984); and by Ausubel, F. M. et al., Current Protocols in Molecular Biology, published by Greene Publishing Assoc. and Wiley-Interscience (1987).

### Isobutanol Biosynthetic Pathways

Carbohydrate utilizing microorganisms employ the Embden-Meyerhof-Parnas (EMP) pathway, the Entner-Doudoroff pathway and the pentose phosphate cycle as the central, metabolic routes to provide energy and cellular precursors for growth and maintenance. These pathways have in common the intermediate glyceraldehyde-3-phosphate and, ultimately, pyruvate is formed directly or in combination with the EMP pathway. Subsequently, pyruvate is transformed to acetyl-coenzyme A (acetyl-CoA) via a variety of means. Acetyl-CoA serves as a key intermediate, for example, in generating fatty acids, amino acids and secondary metabolites. The combined reactions of sugar conversion to pyruvate produce energy (e.g. adenosine-5'-triphosphate, ATP) and reducing equivalents (e.g. reduced nicotinamide adenine dinucleotide, NADH, and reduced nicotinamide adenine dinucleotide phosphate, NADPH). NADH and NADPH must be recycled to their oxidized forms (NAD⁺ and NADP⁺, respectively). In the presence of inorganic electron acceptors (e.g. O₂, NO₃⁻ and SO₄²⁻), the reducing equivalents may be used to augment the energy pool; alternatively, a reduced carbon by-product may be formed. As described by Donaldson et al., *supra,* isobutanol can be produced from carbohydrate sources by recombinant microorganisms comprising one of four complete isobutanol biosynthetic pathways, as shown in Figure 1.

Three of the pathways comprise conversion of pyruvate to isobutanol via a series of enzymatic steps. The preferred isobutanol pathway (Figure 1, steps a to e), comprises the following substrate to product conversions:
a) pyruvate to acetolactate, as catalyzed for example by acetolactate synthase,
b) acetolactate to 2,3-dihydroxyisovalerate, as catalyzed for example by acetohydroxy acid isomeroreductase,
c) 2,3-dihydroxyisovalerate to α-ketoisovalerate, as catalyzed for example by acetohydroxy acid dehydratase,
d) α-ketoisovalerate to isobutyraldehyde, as catalyzed for example by a branched-chain keto acid decarboxylase, and
e) isobutyraldehyde to isobutanol, as catalyzed for example by, a branched-chain alcohol dehydrogenase.

This pathway combines enzymes known to be involved in well-characterized pathways for valine biosynthesis (pyruvate to α-ketoisovalerate) and valine catabolism (α-ketoisovalerate to isobutanol). Since many valine biosynthetic enzymes also catalyze analogous reactions in the isoleucine biosynthetic pathway, substrate specificity is a major consideration in selecting the gene sources. For this reason, the primary genes of interest for the acetolactate synthase enzyme are those from *Bacillus* (*a*/*sS*) and *Klebsiella (budB).* These particular acetolactate synthases are known to participate in butanediol fermentation in these organisms and show increased affinity for pyruvate over ketobutyrate (Gollop et al., J. Bacteriol. 172(6):3444-3449 (1990); Holtzclaw et al., J. Bacteriol. 121 (3):917-922 (1975)). The second and third pathway steps are catalyzed by acetohydroxy acid reductoisomerase and dehydratase, respectively. These enzymes have been characterized from a number of sources, such as for example, *E*. *coli* (Chunduru et al., Biochemistry 28(2):486-493 (1989); Flint et al., J. Biol. Chem. 268(29):14732-14742 (1993)). The final two steps of the preferred isobutanol pathway are known to occur in yeast, which can use valine as a nitrogen source and, in the process, secrete isobutanol. α-Ketoisovalerate can be converted to isobutyraldehyde by a number of keto acid decarboxylase enzymes, such as for example pyruvate decarboxylase. To prevent misdirection of pyruvate away from isobutanol production, a decarboxylase with decreased affinity for pyruvate is desired. So far, there are two such enzymes known in the art (Smit et al., Appl. Environ. Microbiol. 71(1):303-311 (2005); de la Plaza et al., FEMS Microbiol. Lett. 238(2):367-374 (2004)). Both enzymes are from strains of *Lactococcus lactis* and have a 50-200-fold preference for ketoisovalerate over pyruvate. Finally, a number of aldehyde reductases have been identified in yeast, many with overlapping substrate specificity. Those known to prefer branched-chain substrates over acetaldehyde include, but are not limited to, alcohol dehydrogenase VI (ADH6) and Ypr1p (Larroy et al., Biochem. J. 361 (Pt 1):163-172 (2002); Ford et al., Yeast 19(12):1087-1096 (2002)), both of which use NADPH as electron donor. An NADPH-dependent reductase, YqhD, active with branched-chain substrates has also been recently identified in *E*. *coli* (Sulzenbacher et al., J. Mol. Biol. 342(2):489-502 (2004)).

### Pathway steps.

a) Pyruvate to acetolactate, is catalyzed by acetolactate synthase. The skilled person will appreciate that polypeptides having acetolactate synthase activity isolated from a variety of sources will be useful in the present invention independent of sequence homology. Example of suitable acetolactate synthase enzymes are available from a number of sources, for example, *Bacillus subtilis* (GenBank Nos: CAB15618 (SEQ ID NO:178), Z99122 (SEQ ID NO:78), NCBI (National Center for Biotechnology Information) amino acid sequence, NCBI nucleotide sequence, respectively), *Klebsiella pneumoniae* (GenBank Nos: AAA25079 (SEQ ID NO:2), M73842 (SEQ ID NO:1)), and *Lactococcus lactis* (GenBank Nos: AAA25161 (SEQ ID NO:180), L16975 (SEQ ID NO:179)). Preferred acetolactate synthase enzymes are those that have at least 80% - 85% identity to SEQ ID NO:2, SEQ ID NO:178, and SEQ ID: 180 where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
b) Acetolactate to 2,3-dihydroxyisovalerate, is catalyzed by acetohydroxy acid isomeroreductase. The skilled person will appreciate that polypeptides having acetohydroxy acid isomeroreductase activity isolated from a variety of sources will be useful in the present invention independent of sequence homology. Example of suitable acetohydroxy acid isomeroreductase enzymes are available from a number of sources, for example, *Escherichia coli* (GenBank Nos: NP_418222 (SEQ ID NO:4), NC_000913 (SEQ ID NO:3)), *Saccharomyces cerevisiae* (GenBank Nos: NP_013459 (SEQ ID NO:181), NC_001144 (SEQ ID NO:80)), *Methanococcus maripaludis* (GenBank Nos: CAF30210 (SEQ ID NO:183), BX957220 (SEQ ID NO:182)), and *Bacillus. subtilis* (GenBank Nos: CAB14789 (SEQ ID NO:185), Z99118 (SEQ ID NO:184)). Preferred acetohydroxy acid isomeroreductase enzymes are those that have at least 80% - 85% identity to SEQ ID NO:4, SEQ ID NO:181, SEQ ID NO:183, and SEQ ID NO:185, where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
c) 2,3-dihydroxyisovalerate to α-ketoisovalerate, as catalyzed for example by acetohydroxy acid dehydratase. The skilled person will appreciate that polypeptides having acetohydroxy acid dehydratase activity isolated from a variety of sources will be useful in the present invention independent of sequence homology. Example of suitable acetohydroxy acid dehydratase enzymes are available from a number of sources, for example, *E*. *coli* (GenBank Nos: YP_026248 (SEQ ID NO:6), NC_000913 (SEQ ID NO:5)), *S. cerevisiae* (GenBank Nos: NP_012550 (SEQ ID NO:186), NC_001142 (SEQ ID NO:83)), *M. maripaludis* (GenBank Nos: CAF29874 (SEQ ID NO:188), BX957219 (SEQ ID NO:187)), and *B. subtilis* (GenBank Nos: CAB14105 (SEQ ID NO:190), Z99115 (SEQ ID NO:189)). Preferred acetohydroxy acid dehydratase enzymes are those that have at least 80% - 85% identity to SEQ ID NO:6, SEQ ID NO:186, SEQ ID NO:188, and SEQ ID NO:190, where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
d) α-ketoisovalerate to isobutyraldehyde, as catalyzed for example by a branched-chain keto acid decarboxylase. The skilled person will appreciate that polypeptides having branched-chain keto acid decarboxylase activity isolated from a variety of sources will be useful in the present invention independent of sequence homology. Example of suitable branched-chain keto acid decarboxylase enzymes are available from a number of sources, for example, *Lactococcus lactis* (GenBank Nos: AAS49166 (SEQ ID NO:193), AY548760 (SEQ ID NO:192); CAG34226 (SEQ ID NO:8), AJ746364 (SEQ ID NO:191), *Salmonella typhimurium* (GenBank Nos: NP_461346 (SEQ ID NO:195), NC_003197 (SEQ ID NO:194)), and *Clostridium acetobutylicum* (GenBank Nos: NP_149189 (SEQ ID NO:197), NC_001988 (SEQ ID NO:196)). Preferred branched-chain keto acid decarboxylase enzymes are those that have at least 80% - 85% identity to SEQ ID NO:8, SEQ ID NO:193, SEQ
   ID NO:195, and SEQ ID NO:197, where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
e) Isobutyraldehyde to isobutanol, as catalyzed for example by, a branched-chain alcohol dehydrogenase. The skilled person will appreciate that polypeptides having branched-chain alcohol dehydrogenase activity isolated from a variety of sources will be useful in the present invention independent of sequence homology. Example of suitable branched-chain alcohol dehydrogenase enzymes are available from a number of sources, for example, *S. cerevisiae* (GenBank Nos: NP_010656 (SEQ ID NO:199), NC_001136 (SEQ ID NO:198); NP_014051 (SEQ ID NO:201) NC_001145 (SEQ ID NO:200)), *E*. *coli* (GenBank Nos: NP_417484 (SEQ ID NO:10), NC_000913 (SEQ ID NO:9)), and *C*. *acetobutylicum* (GenBank Nos: NP_349892 (SEQ ID NO:203), NC_003030 (SEQ ID NO:202); NP_349891 (SEQ ID NO:204), NC_003030 (SEQ ID NO:158)). Preferred branched-chain alcohol dehydrogenase enzymes are those that have at least 80% - 85% identity to SEQ ID NO:10, SEQ ID NO:199, SEQ ID NO:201, SEQ ID NO:203, and SEQ ID NO:204.where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.

Additional pathways are discussed below and in commonly owned and copending (US 11/586315 (US 2007 0092957).

Another pathway for converting pyruvate to isobutanol comprises the following substrate to product conversions (Figure 1, steps a,b,c,f,g,e):
a) pyruvate to acetolactate, as catalyzed for example by acetolactate synthase,
b) acetolactate to 2,3-dihydroxyisovalerate, as catalyzed for example by acetohydroxy acid isomeroreductase,
c) 2,3-dihydroxyisovalerate to α-ketoisovalerate, as catalyzed for example by acetohydroxy acid dehydratase,
f) α-ketoisovalerate to isobutyryl-CoA, as catalyzed for example by a branched-chain keto acid dehydrogenase,
g) isobutyryl-CoA to isobutyraldehyde, as catalyzed for example by an acylating aldehyde dehydrogenase, and
e) isobutyraldehyde to isobutanol, as catalyzed for example by, a branched-chain alcohol dehydrogenase.

The first three steps in this pathway (a,b,c) are the same as those described above. The α-ketoisovalerate is converted to isobutyryl-CoA by the action of a branched-chain keto acid dehydrogenase. While yeast can only use valine as a nitrogen source, many other organisms (both eukaryotes and prokaryotes) can use valine as the carbon source as well. These organisms have branched-chain keto acid dehydrogenase (Sokatch et al. J. Bacteriol. 148(2):647-652 (1981)), which generates isobutyryl-CoA. Isobutyryl-CoA may be converted to isobutyraldehyde by an acylating aldehyde dehydrogenase. Dehydrogenases active with the branched-chain substrate have been described, but not cloned, in *Leuconostoc* and *Propionibacterium* (Kazahaya et al., J. Gen. Appl. Microbiol. 18:43-55 (1972); Hosoi et al., J. Ferment. Technol. 57:418-427 (1979)). However, it is also possible that acylating aldehyde dehydrogenases known to function with straight-chain acyl-CoAs (i.e. butyryl-CoA), may also work with isobutyryl-CoA. The isobutyraldehyde is then converted to isobutanol by a branched-chain alcohol dehydrogenase, as described above for the first pathway.

Another pathway for converting pyruvate to isobutanol comprises the following substrate to product conversions (Figure 1, steps a,b,c,h,i,j,e):
a) pyruvate to acetolactate, as catalyzed for example by acetolactate synthase,
b) acetolactate to 2,3-dihydroxyisovalerate, as catalyzed for example by acetohydroxy acid isomeroreductase,
c) 2,3-dihydroxyisovalerate to α-ketoisovalerate, as catalyzed for example by acetohydroxy acid dehydratase,
h) α-ketoisovalerate to valine, as catalyzed for example by valine dehydrogenase or transaminase,
i) valine to isobutylamine, as catalyzed for example by valine decarboxylase,
j) isobutylamine to isobutyraldehyde, as catalyzed for example by omega transaminase, and
e) isobutyraldehyde to isobutanol, as catalyzed for example by, a branched-chain alcohol dehydrogenase.

The first three steps in this pathway (a,b,c) are the same as those described above. This pathway requires the addition of a valine dehydrogenase or a suitable transaminase. Valine (and or leucine) dehydrogenase catalyzes reductive amination and uses ammonia; Kₘ values for ammonia are in the millimolar range (Priestly et al., Biochem J. 261 (3):853-861 (1989); Vancura et al., J. Gen. Microbiol. 134(12):3213-3219 (1988) Zink et al., Arch. Biochem. Biophys. 99:72-77 (1962); Sekimoto et al. J. Biochem (Japan) 116(1):176-182 (1994)). Transaminases typically use either glutamate or alanine as amino donors and have been characterized from a number of organisms (Lee-Peng et al,. J. Bacteriol. 139(2):339-345 (1979); Berg et al., J. Bacteriol. 155(3):1009-1014 (1983)). An alanine-specific enzyme may be desirable, since the generation of pyruvate from this step could be coupled to the consumption of pyruvate later in the pathway when the amine group is removed (see below). The next step is decarboxylation of valine, a reaction that occurs in valanimycin biosynthesis in *Streptomyces* (Garg et al., Mol. Microbiol. 46(2):505-517 (2002)). The resulting isobutylamine may be converted to isobutyraldehyde in a pyridoxal 5'-phosphate-dependent reaction by, for example, an enzyme of the omega-aminotransferase family. Such an enzyme from *Vibrio fluvialis* has demonstrated activity with isobutylamine (Shin et al., Biotechnol. Bioeng. 65(2):206-211 (1999)). Another omega-aminotransferase from *Alcaligenes denitrificans* has been cloned and has some activity with butylamine (Yun et al., Appl. Environ. Microbiol. 70(4):2529-2534 (2004)). In this direction, these enzymes use pyruvate as the amino acceptor, yielding alanine. As mentioned above, adverse affects on the pyruvate pool may be offset by using a pyruvate-producing transaminase earlier in the pathway. The isobutyraldehyde is then converted to isobutanol by a branched-chain alcohol dehydrogenase, as described above for the first pathway.

The fourth isobutanol biosynthetic pathway comprises the substrate to product conversions shown as steps k,g,e in Figure 1. A number of organisms are known to produce butyrate and/or butanol via a butyryl-CoA intermediate (Dürre et al., FEMS Microbiol. Rev. 17(3):251-262 (1995); Abbad-Andaloussi et al., Microbiology 142(5):1149-1158 (1996)). Isobutanol production may be engineered in these organisms by addition of a mutase able to convert butyryl-CoA to isobutyryl-CoA (Figure 1, step k). Genes for both subunits of isobutyryl-CoA mutase, a coenzyme B₁₂-dependent enzyme, have been cloned from a Streptomycete (Ratnatilleke et al., J. Biol. Chem. 274(44):31679-31685 (1999)). The isobutyryl-CoA is converted to isobutyraldehyde (step g in Figure 1), which is converted to isobutanol (step e in Figure 1).

Thus, in providing multiple recombinant pathways from pyruvate to isobutanol, there exist a number of choices to fulfill the individual conversion steps, and the person of skill in the art will be able to utilize publicly available sequences to construct the relevant pathways. A listing of a representative number of genes known in the art and useful in the construction of isobutanol biosynthetic pathways are listed below in Table 2.

**Table 2**

| Sources of Isobuatnol Biosvnthetic Pathway Genes | |
|---|---|
| Gene | GenBank Citation |
| acetolactate synthase | Z99122, *Bacillus subtilis* complete genome (section 19 of 21): from 3608981 to 3809670 |
| | gi\|32468830\|emb\|Z99122.2\|BSUB0019[32468830] |
| | M73842, *Klebsiella pneumoniae* acetolactate synthase (*iluk*) gene, complete cds |
| | gi\|149210\|gb\|M73842.1\|KPNILUK[149210] |
| | L16975, *Lactococcus lactis* alpha-acetolactate synthase (*als*) gene, complete cds |
| | gi\|473900\|gb\|L16975.1\|LACALS[473900] |
| acetohydroxy acid isomeroreductase | NC_000913, *Escherichia coli* K12, complete genome |
| | gi\|49175990\|ref\|NC_000913.2\|[49175990] |
| | NC_001144, Saccharomyces cerevisiae chromosome XII, complete chromosome sequence |
| | gi\|42742286\|ref\|NC_001144.3\|[42742286] |
| | BX957220, *Methanococcus maripaludis* S2 complete genome; segment 2/5 |
| | gi\|44920669\|emb\|BX957220.1\|[44920669] |
| | Z99118, *Bacillus subtilis* complete genome (section 15 of 21): from 2812801 to 3013507 |
| | gi\|32468802\|emb\|Z99118.2\|BSUB0015[32468802] |
| acetohydroxy acid dehydratase | NC_000913, *Escherichia coli* K12, complete genome |
| | gi\|49175990\|ref\|NC_000913.2\|[49175990] |
| | NC_001142, *Saccharomyces cerevisiae* chromosome X, co mplete chromosome sequence |
| | gi\|42742252\|ref\|NC_001142.5\|[42742252] |
| | BX957219, *Methanococcus maripaludis* S2 complete genome; segment 1/5 gi\|45047123\|emb\|BX957219.1\|[45047123] |
| | Z99115, *Bacillus subtilis* complete genome (section 12 of 21): from 2207806 to 2409180 |
| | gi\|32468778\|emb\|Z99115.2\|BSUB0012[32468778] |
| branched-chain α-keto acid decarboxylase | AY548760, *Lactococcus lactis* branched-chain alpha-ketoacid decarboxylase (*kdcA*) gene, complete cds gi\|44921616\|gb\|AY548760.1\|[44921616] |
| | AJ746364, *Lactococcus lactis* subsp. *lactis kivd* gene for alpha-ketoisovalerate decarboxylase, strain IFPL730 |
| | gi\|51870501\|emb\|AJ746364.1\|[51870501] |
| | NC_003197, *Salmonella typhimurium* LT2, complete genome |
| | gi\|16763390\|ref\|NC_003197.1\|[16763390] |
| | NC_001988, *Clostridium* acetobutylicum ATCC 824 plasmid pSOL1, complete sequence |
| | gi\|15004705\|ref\|NC_001988.2\|[15004705] |
| branched-chain alcohol dehydrogenase | NC_001136, *Saccharomyces cerevisiae* chromosome IV, complete chromosome sequence |
| | gi\|50593138\|ref\|NC_001136.6\|[50593138] |
| | NC_001145, *Saccharomyces cerevisiae* chromosome XIII, complete chromosome sequence |
| | gi\|44829554\|ref\|NC_001145.2\|[44829554] |
| | NC_000913, *Escherichia coli K12*, complete genome |
| | gi\|49175990\|ref\|NC_000913.2\|[49175990] |
| | NC_003030, *Clostridium acetobutylicum* ATCC 824, complete genome |
| | gi\|15893298\|ref\|NC_003030.1\|[15893298] |
| branched-chain keto acid dehydrogenase | Z99116, *Bacillus subtilis* complete genome (section 13 of 21): from 2409151 to 2613687 |
| | gi\|32468787\|emb\|Z99116.2\|BSUB0013[32468787] |
| | M57613, *Pseudomonas putida* branched-chain keto acid dehydrogenase operon *(bkdA1, bkdA1* and *bkdA2),* transacylase E2 (*bkdAB*), *bkdR* and lipoamide dehydrogenase (*IpdV*) genes, complete cds |
| | gi\|790512\|gb\|M57613.1\|PSEBKDPPG2[790512] |
| acylating aldehyde | AF157306, *Clostridium beijerinckii* strain NRRL B593 |
| dehydrogenase | hypothetical protein, coenzyme A acylating aldehyde dehydrogenase (*ald*), acetoacetate:butyrate/acetate coenzyme A transferase (*ctfA*), acetoacetate:butyrate/acetate coenzyme A transferase (*ctfB*), and acetoacetate decarboxylase (*adc*) genes, complete cds |
| | gi\|47422980\|gb\|AF 157306.2\|[47422980] |
| | NC_001988, *Clostridium acetobutylicum* ATCC 824 plasmid pSOL1, complete sequence |
| | gi\|15004705\|ref\|NC_001988.2\|[15004705] |
| | U13232, *Pseudomonas putida* NCIB9816 acetaldehyde dehydrogenase (*nahO*) and 4-hydroxy-2-oxovalerate aldolase (*nahM*) genes, complete cds, and 4-oxalocrotonate decarboxylase (*nahK*) and 2-oxopent-4-enoate hydratase (*nahL*) genes, partial cds |
| | gi\|595671\|gb\|U13232.1\|PPU13232[595671] |
| transaminase | NC_000913, *Escherichia coli* K12, complete genome gi\|49175990\|ref\|NC_000913.2\|[49175990] |
| | NC_006322, *Bacillus licheniformis* ATCC 14580, complete genome |
| | gi\|52783855\|ref\|NC_006322.1\|[52783855] |
| | NC_001142, *Saccharomyces cerevisiae* chromosome X, complete chromosome sequence |
| | gi\|42742252\|ref\|NC_001142.5\|[42742252] |
| | NC_000916, *Methanothermobacter thermautotrophicus* str. Delta H, complete genome |
| | gi\|15678031\|ref\|NC_000916.1\|[15678031] |
| valine dehydrogenase | NC_003888, *Streptomyces coelicolor* A3(2), complete genome |
| | gi\|32141095\|ref\|NC_003888.3\|[32141095] |
| | Z99116, *Bacillus subtilis* complete genome (section 13 of 21): from 2409151 to 2613687 |
| | gi\|32468787\|emb\|Z99116.2\|BSUB0013[32468787] |
| valine decarboxylase | AY116644, *Streptomyces viridifaciens* amino acid aminotransferase gene, partial cds; ketol-acid reductoisomerase, acetolactate synthetase small |
| | subunit, acetolactate synthetase large subunit, complete cds; azoxy antibiotic valanimycin gene cluster, complete sequence; and putative transferase, and putative secreted protein genes, complete cds |
| | gi\|27777548\|gb\|AY116644.1\|[27777548] |
| omega transaminase | AY330220, *Achromobacter denitrificans* omega-amino acid:pyruvate transaminase (*aptA*) gene, complete cds |
| | gi\|33086797\|gb\|AY330220.1\|[33086797] |
| | NC_007347, *Ralstonia eutropha* JMP134 chromosome 1, complete sequence |
| | gi\|73539706\|ref\|NC_007347.1\|[73539706] |
| | NC_004347, *Shewanella oneidensis* MR-1, complete genome |
| | gi\|24371600\|ref\|NC_004347.1\|[24371600] |
| | NZ_AAAG02000002, *Rhodospirillum rubrum* Rrub02_2, whole genome shotgun sequence |
| | gi\|48764549\|ref\|NZ_AAAG02000002.1\|[48764549] |
| | AE016776, *Pseudomonas putida* KT2440 section 3 of 21 of the complete genome |
| | gi\|26557019\|gb\|AE016776.1\|[26557019] |
| isobutyryl-CoA mutase | U67612, *Streptomyces cinnamonensis* coenzyme B12-dependent isobutyrylCoA mutase (*icm*) gene, complete cds |
| | gi\|3002491\|gb\|U67612.1\|SCU67612[3002491] |
| | AJ246005, *Streptomyces cinnamonensis icmB* gene for isobutyryl-CoA mutase, small subunit |
| | gi\|6137076\|emb\|AJ246005.1\|SC1246005[6137076] |
| | AL939123, *Streptomyces coelicolor* A3(2) complete genome; segment 20/29 |
| | gi\|24430032\|emb\|AL939123.1\|SC0939123[24430032] |
| | AL9939121, *Streptomyces coelicolor* A3(2) complete genome; segment 18/29 |
| | gi\|24429533\|emb\|AL939121.1\|SC0939121 [24429533] |
| | NC_003155, *Streptomyces avermitilis* MA-4680, complete genome |
| | gi\|57833846\|ref\|NC_003155.3\|[57833846] |

### Microbial Hosts for Isobutanol Production

Microbial hosts for isobutanol production may be selected from bacteria, cyanobacteria, filamentous fungi and yeasts. The microbial host used for isobutanol production is preferably tolerant to isobutanol so that the yield is not limited by isobutanol toxicity. Microbes that are metabolically active at high titer levels of isobutanol are not well known in the art. Although 1-butanol-tolerant mutants have been isolated from solventogenic *Clostridia,* little information is available concerning the butanol tolerance of other potentially useful bacterial strains. Most of the studies on the comparison of alcohol tolerance in bacteria suggest that 1-butanol is more toxic than ethanol (de Cavalho et al., Microsc. Res. Tech. 64:215-22 (2004) and Kabelitz et al., FEMS Microbiol. Lett. 220:223-227 (2003)). Tomas et al. (J. Bacteriol. 186:2006-2018 (2004)) report that the yield of 1-butanol during fermentation in *Clostridium acetobutylicum* may be limited by 1-butanol toxicity. The primary effect of 1-butanol on *Clostridium acetobutylicum* is disruption of membrane functions (Hermann et al., Appl. Environ. Microbiol. 50:1238-1243 (1985)).

The microbial hosts selected for the production of isobutanol are preferably tolerant to isobutanol and should be able to convert carbohydrates to isobutanol. The criteria for selection of suitable microbial hosts include the following: intrinsic tolerance to isobutanol, high rate of glucose utilization, availability of genetic tools for gene manipulation, and the ability to generate stable chromosomal alterations.

Suitable host strains with a tolerance for isobutanol may be identified by screening based on the intrinsic tolerance of the strain. The intrinsic tolerance of microbes to isobutanol may be measured by determining the concentration of isobutanol that is responsible for 50% inhibition of the growth rate (IC50) when grown in a minimal medium. The IC50 values may be determined using methods known in the art. For example, the microbes of interest may be grown in the presence of various amounts of isobutanol and the growth rate monitored by measuring the optical density at 600 nanometers. The doubling time may be calculated from the logarithmic part of the growth curve and used as a measure of the growth rate. The concentration of isobutanol that produces 50% inhibition of growth may be determined from a graph of the percent inhibition of growth versus the isobutanol concentration. Preferably, the host strain should have an IC50 for isobutanol of greater than about 0.5%.

The microbial host for isobutanol production should also utilize glucose at a high rate. Most microbes are capable of utilizing carbohydrates. However, certain environmental microbes cannot utilize carbohydrates to high efficiency, and therefore would not be suitable hosts.

The ability to genetically modify the host is essential for the production of any recombinant microorganism. The mode of gene transfer technology may be by electroporation, conjugation, transduction or natural transformation. A broad range of host conjugative plasmids and drug resistance markers are available. The cloning vectors are tailored to the host organisms based on the nature of antibiotic resistance markers that can function in that host.

The microbial host also has to be manipulated in order to inactivate competing pathways for carbon flow by deleting various genes. This requires the availability of either transposons to direct inactivation or chromosomal integration vectors. Additionally, the production host should be amenable to chemical mutagenesis so that mutations to improve intrinsic isobutanol tolerance may be obtained.

Based on the criteria described above, suitable microbial hosts for the production of isobutanol include, but are not limited to, members of the genera *Clostridium*, *Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* and *Saccharomyces.* Preferred hosts include: *Escherichia coli, Alcaligenes eutrophus, Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis* and *Saccharomyces cerevisiae.*

### Construction of Production Host

Recombinant organisms containing the necessary genes that will encode the enzymatic pathway for the conversion of a fermentable carbon substrate to isobutanol may be constructed using techniques well known in the art. Genes encoding the enzymes of the isobutanol biosynthetic pathway of the disclosure, for example, acetolactate synthase, acetohydroxy acid isomeroreductase, acetohydroxy acid dehydratase, branched-chain α-keto acid decarboxylase, and branched-chain alcohol dehydrogenase, may be isolated from various sources, as described above. Genes encoding the enzymes of the isobutanol pathways disclosed herein may be isolated from various sources, as described above.

Methods of obtaining desired genes from a bacterial genome are common and well known in the art of molecular biology. For example, if the sequence of the gene is known, suitable genomic libraries may be created by restriction endonuclease digestion and may be screened with probes complementary to the desired gene sequence. Once the sequence is isolated, the DNA may be amplified using standard primer-directed amplification methods such as polymerase chain reaction (U.S. 4,683,202) to obtain amounts of DNA suitable for transformation using appropriate vectors. Tools for codon optimization for expression in a heterologous host are readily available. Some tools for codon optimization are available based on the GC content of the host organism. The GC content of some exemplary microbial hosts is given Table 3.

**Table 3**

| GC Content of Microbial Hosts | |
|---|---|
| Strain | % GC |
| *B. licheniformis* | 46 |
| *B. subtilis* | 42 |
| C. acetobutylicum | 37 |
| *E*. *coli* | 50 |
| *P. putida* | 61 |
| *A. eutrophus* | 61 |
| *Paenibacillus macerans* | 51 |
| *Rhodococcus erythropolis* | 62 |
| *Brevibacillus* | 50 |
| *Paenibacillus polymyxa* | 50 |

Once the relevant pathway genes are identified and isolated they may be transformed into suitable expression hosts by means well known in the art. Vectors or cassettes useful for the transformation of a variety of host cells are common and commercially available from companies such as EPICENTRE^{®} (Madison, WI), Invitrogen Corp. (Carlsbad, CA), Stratagene (La Jolla, CA), and New England Biolabs, Inc. (Beverly, MA). Typically the vector or cassette contains sequences directing transcription and translation of the relevant gene, a selectable marker, and sequences allowing autonomous replication or chromosomal integration. Suitable vectors comprise a region 5' of the gene which harbors transcriptional initiation controls and a region 3' of the DNA fragment which controls transcriptional termination. Both control regions may be derived from genes homologous to the transformed host cell, although it is to be understood that such control regions may also be derived from genes that are not native to the specific species chosen as a production host.

Initiation control regions or promoters, which are useful to drive expression of the relevant pathway coding regions in the desired host cell are numerous and familiar to those skilled in the art. Virtually any promoter capable of driving these genetic elements is suitable for the present invention including, but not limited to, *CYC1*, *HIS3*, *GAL1*, *GAL10*, *ADH1, PGK, PHO5*, *GAPDH, ADC1, TRP1, URA3, LEU2, ENO, TPI, CUP1, FBA, GPD, and GPM* (useful for expression in *Saccharomyces*); *AOX1* (useful for expression in *Pichia*); and *lac, ara, tet, trp, IP_{L}, IP_{R}, T7, tac*, *and trc* (useful for expression in *Escherichia coli, Alcaligenes,* and *Pseudomonas*); the *amy, apr, npr* promoters and various phage promoters useful for expression in *Bacillus subtilis, Bacillus licheniformis, and Paenibacillus macerans*; *nisA* (useful for expression Gram-positive bacteria, Eichenbaum et al. Appl. Environ. Microbiol. 64(8):2763-2769 (1998)); and the synthetic P11 promoter (useful for expression in *Lactobacillus plantarum,* Rud et al., Microbiology 152:1011-1019 (2006)).

Termination control regions may also be derived from various genes native to the preferred hosts. Optionally, a termination site may be unnecessary, however, it is most preferred if included.

Certain vectors are capable of replicating in a broad range of host bacteria and can be transferred by conjugation. The complete and annotated sequence of pRK404 and three related vectors-pRK437, pRK442, and pRK442(H) are available. These derivatives have proven to be valuable tools for genetic manipulation in Gram-negative bacteria (Scott et al., Plasmid 50(1):74-79 (2003)). Several plasmid derivatives of broad-host-range Inc P4 plasmid RSF1010 are also available with promoters that can function in a range of Gram-negative bacteria. Plasmid pAYC36 and pAYC37, have active promoters along with multiple cloning sites to allow for the heterologous gene expression in Gram-negative bacteria.

Chromosomal gene replacement tools are also widely available. For example, a thermosensitive variant of the broad-host-range replicon pWV101 has been modified to construct a plasmid pVE6002 which can be used to effect gene replacement in a range of Gram-positive bacteria (Maguin et al., J. Bacteriol. 174(17):5633-5638 (1992)). Additionally, in vitro transposomes are available to create random mutations in a variety of genomes from commercial sources such as EPICENTRE^{®}.

The expression of an isobutanol biosynthetic pathway in various preferred microbial hosts is described in more detail below.

### Expression of an isobutanol biosynthetic pathway in E. coli

Vectors or cassettes useful for the transformation of *E*. *coli* are common and commercially available from the companies listed above. For example, the genes of an isobutanol biosynthetic pathway may be isolated from various sources, cloned into a modified pUC19 vector and transformed into *E*. *coli* NM522, as described in Examples 10 and 11.

### Expression of an isobutanol biosynthetic pathway in Rhodococcus erythropolis

A series of *E*. *coli-Rhodococcus* shuttle vectors are available for expression in *R*. *erythropolis,* including, but not limited to, pRhBR17 and pDA71 (Kostichka et al., Appl. Microbiol. Biotechnol. 62:61-68(2003)). Additionally, a series of promoters are available for heterologous gene expression in *R*. *eythropolis* (see for example Nakashima et al., Appl. Environ. Microbiol. 70:5557-5568 (2004), and Tao et al., Appl. Microbiol. Biotechnol. 2005, DOI 10.1007/s00253-005-0064). Targeted gene disruption of chromosomal genes in R. *erythropolis* may be created using the method described by Tao et al., *supra,* and Brans et al. (Appl. Environ. Microbiol. 66: 2029-2036 (2000)).

The heterologous genes required for the production of isobutanol, as described above, may be cloned initially in pDA71 or pRhBR71 and transformed into *E. coli*. The vectors may then be transformed into *R*. *erythropolis* by electroporation, as described by Kostichka et al., *supra.* The recombinants may be grown in synthetic medium containing glucose and the production of isobutanol can be followed using methods known in the art.

### Expression of an isobutanol biosynthetic pathway in B. Subtilis

Methods for gene expression and creation of mutations in *B. subtilis* are also well known in the art. For example, the genes of an isobutanol biosynthetic pathway may be isolated from various sources, cloned into a modified pUC19 vector and transformed into *Bacillus subtilis* BE1010, as described in Example 12. Additionally, the five genes of an isobutanol biosynthetic pathway can be split into two operons for expression, as described in Example 24. The three genes of the pathway (*bubB*, *ilvD*, and *kivD*) were integrated into the chromosome of *Bacillus subtilis* BE1010 (Payne and Jackson, J. Bacteriol. 173:2278-2282 (1991)). The remaining two genes (*ilvC* and *bdhB*) were cloned into an expression vector and transformed into the *Bacillus* strain carrying the integrated isobutanol genes

### Expression of an isobutanol biosynthetic pathway in B. licheniformis

Most of the plasmids and shuttle vectors that replicate in B. *subtilis* may be used to transform *B. licheniformis* by either protoplast transformation or electroporation. The genes required for the production of isobutanol may be cloned in plasmids pBE20 or pBE60 derivatives (Nagarajan et al., Gene 114:121-126 (1992)). Methods to transform *B*. *licheniformis* are known in the art (for example see Fleming et al. Appl. Environ. Microbiol., 61(11):3775-3780 (1995)). The plasmids constructed for expression in *B. subtilis* may be transformed into *B. licheniformis* to produce a recombinant microbial host that produces isobutanol.

### Expression of an isobutanol biosynthetic pathway in Paenibacillus macerans

Plasmids may be constructed as described above for expression in *B. subtilis* and used to transform *Paenibacillus macerans* by protoplast transformation to produce a recombinant microbial host that produces isobutanol.

### Expression of the isobutanol biosynthetic pathway in Alcaligenes (Ralstonia) eutrophus

Methods for gene expression and creation of mutations in *Alcaligenes eutrophus* are known in the art (see for example Taghavi et al., Appl. Environ. Microbiol., 60(10):3585-3591 (1994)). The genes for an isobutanol biosynthetic pathway may be cloned in any of the broad host range vectors described above, and electroporated to generate recombinants that produce isobutanol. The poly(hydroxybutyrate) pathway in *Alcaligenes* has been described in detail, a variety of genetic techniques to modify the *Alcaligenes eutrophus* genome is known, and those tools can be applied for engineering an isobutanol biosynthetic pathway.

### Expression of an isobutanol biosynthetic pathway in Pseudomonas putida

Methods for gene expression in *Pseudomonas putida* are known in the art (see for example Ben-Bassat et al., U.S. Patent No. 6,586,229 ). The isobutanol pathway genes may be inserted into pPCU18 and this ligated DNA may be electroporated into electrocompetent *Pseudomonas putida* DOT-T1 C5aAR1 cells to generate recombinants that produce isobutanol.

### Expression of an isobutanol biosynthetic pathway in Saccharomyces cerevisiae

Methods for gene expression in *Saccharomyces cerevisiae* are known in the art (see for example Methods in Enzymology, Volume 194, Guide to Yeast Genetics and Molecular and Cell Biology (Part A, 2004, Christine Guthrie and Gerald R. Fink (Eds.), Elsevier Academic Press, San Diego, CA). Expression of genes in yeast typically requires a promoter, followed by the gene of interest, and a transcriptional terminator. A number of yeast promoters can be used in constructing expression cassettes for genes encoding an isobutanol biosynthetic pathway, including, but not limited to constitutive promoters FBA, GPD, ADH1, and GPM, and the inducible promoters GAL1, GAL10, and CUP1. Suitable transcriptional terminators include, but are not limited to FBAt, GPDt, GPMt, ERG10t, GAL1t, CYC1, and ADH1. For example, suitable promoters, transcriptional terminators, and the genes of an isobutanol biosynthetic pathway may be cloned into *E. coli*-yeast shuttle vectors as described in Example 21.

### Expression of an isobutanol biosynthetic pathway in Lactobacillus plantarum

The *Lactobacillus* genus belongs to the *Lactobacillales* family and many plasmids and vectors used in the transformation of *Bacillus subtilis* and *Streptococcus* may be used for *lactobacillus.* Non-limiting examples of suitable vectors include pAMβ1 and derivatives thereof (Renault et al., Gene 183:175-182 (1996); and O'Sullivan et al., Gene 137:227-231 (1993)); pMBB1 and pHW800, a derivative of pMBB1 (Wyckoff et al. Appl. Environ. Microbiol. 62:1481-1486 (1996)); pMG1, a conjugative plasmid (Tanimoto et al., J. Bacteriol. 184:5800-5804 (2002)); pNZ9520 (Kleerebezem et al., Appl. Environ. Microbiol. 63:4581-4584 (1997)); pAM401 (Fujimoto et al., Appl. Environ. Microbiol. 67:1262-1267 (2001)); and pAT392 (Arthur et al., Antimicrob. Agents Chemother. 38:1899-1903 (1994)). Several plasmids from *Lactobacillus plantarum* have also been reported (e.g., van Kranenburg R, Golic N, Bongers R, Leer RJ, de Vos WM, Siezen RJ, Kleerebezem M. Appl. Environ. Microbiol. 2005 Mar; 71 (3): 1223-1230). For example, expression of an isobutanol biosynthetic pathway in *Lactobacillus plantarum* is described in Example 25.

### Expression of an isobutanol biosynthetic pathway in Enterococcus faecium, Enterococcus gallinarium, and Enterococcus faecalis

The *Enterococcus* genus belongs to the *Lactobacillales* family and many plasmids and vectors used in the transformation of *Lactobacillus, Bacillus subtilis,* and *Streptococcus* may be used for *Enterococcus.* Non-limiting examples of suitable vectors include pAMβ1 and derivatives thereof (Renault et al., Gene 183:175-182 (1996); and O'Sullivan et al., Gene 137:227-231 (1993)); pMBB1 and pHW800, a derivative of pMBB1 (Wyckoff et al. Appl. Environ. Microbiol. 62:1481-1486 (1996)); pMG1, a conjugative plasmid (Tanimoto et al., J. Bacteriol. 184:5800-5804 (2002)); pNZ9520 (Kleerebezem et al., Appl. Environ. Microbiol. 63:4581-4584 (1997)); pAM401 (Fujimoto et al., Appl. Environ. Microbiol. 67:1262-1267 (2001)); and pAT392 (Arthur et al., Antimicrob. Agents Chemother. 38:1899-1903 (1994)). Expression vectors for *E*. *faecalis* using the *nisA* gene from *Lactococcus* may also be used (Eichenbaum et al., Appl. Environ. Microbiol. 64:2763-2769 (1998). Additionally, vectors for gene replacement in the *E*. *faecium* chromosome may be used (Nallaapareddy et al., Appl. Environ. Microbiol. 72:334-345 (2006)). For example, expression of an isobutanol biosynthetic pathway in *Enterococcus faecalis* is described in Example 26.

### Fermentation Media

Fermentation media used in the present invention must contain suitable carbon substrates. Suitable substrates may include but are not limited to monosaccharides such as glucose and fructose, oligosaccharides such as lactose or sucrose, polysaccharides such as starch or cellulose or mixtures thereof and unpurified mixtures from renewable feedstocks such as cheese whey permeate, cornsteep liquor, sugar beet molasses, and barley malt. Additionally the carbon substrate may also be one-carbon substrates such as carbon dioxide, or methanol for which metabolic conversion into key biochemical intermediates has been demonstrated. In addition to one and two carbon substrates methylotrophic organisms are also known to utilize a number of other carbon containing compounds such as methylamine, glucosamine and a variety of amino acids for metabolic activity. For example, methylotrophic yeast are known to utilize the carbon from methylamine to form trehalose or glycerol (Bellion et al., Microb. Growth C1 Compd., [Int. Symp.], 7th (1993), 415-32. Editor(s): Murrell, J. Collin; Kelly, Don P. Publisher: Intercept, Andover, UK). Similarly, various species of *Candida* will metabolize alanine or oleic acid (Sulter et al., Arch. Microbiol. 153:485-489 (1990)). Hence it is contemplated that the source of carbon utilized in the present invention may encompass a wide variety of carbon containing substrates and will only be limited by the choice of organism.

Although it is contemplated that all of the above mentioned carbon substrates and mixtures thereof are suitable in the present invention, preferred carbon substrates are glucose, fructose, and sucrose. Sucrose may be derived from renewable sugar sources such as sugar cane, sugar beets, cassava, sweet sorghum, and mixtures thereof. Glucose and dextrose may be derived from renewable grain sources through saccharification of starch based feedstocks including grains such as corn, wheat, rye, barley, oats, and mixtures thereof. In addition, fermentable sugars may be derived from renewable cellulosic or lignocellulosic biomass through processes of pretreatment and saccharification, as described, for example, in co-owned and co-pending U.S. Patent Application Publication No. 2007/0031918A1 . Biomass refers to any cellulosic or lignocellulosic material and includes materials comprising cellulose, and optionally further comprising hemicellulose, lignin, starch, oligosaccharides and/or monosaccharides. Biomass may also comprise additional components, such as protein and/or lipid. Biomass may be derived from a single source, or biomass can comprise a mixture derived from more than one source; for example, biomass may comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn grain, corn cobs, crop residues such as corn husks, corn stover, grasses, wheat, wheat straw, barley, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers, animal manure, and mixtures thereof.

In addition to an appropriate carbon source, fermentation media must contain suitable minerals, salts, cofactors, buffers and other components, known to those skilled in the art, suitable for the growth of the cultures and promotion of the enzymatic pathway necessary for isobutanol production.

### Culture Conditions with Temperature Lowering

In the present method, the recombinant microbial production host which produces isobutanol is seeded into a fermentation medium comprising a fermentable carbon substrate to create a fermentation culture. The production host is grown in the fermentation culture at a first temperature for a first period of time. The first temperature is from about 25 °C to about 40 °C.

Suitable fermentation media used in the present invention include common commercially prepared media such as Luria Bertani (LB) broth, Sabouraud Dextrose (SD) broth or Yeast Medium (YM) broth. Other defined or synthetic growth media may also be used, and the appropriate medium for growth of the particular microorganism will be known by one skilled in the art of microbiology or fermentation science. The use of agents known to modulate catabolite repression directly or indirectly, e.g., cyclic adenosine 2':3'-monophosphate, may also be incorporated into the fermentation medium.

Suitable pH ranges for the fermentation are between pH 5.0 to pH 9.0, where pH 6.0 to pH 8.0 is preferred as the initial condition.

Fermentations may be performed under aerobic or anaerobic conditions, where anaerobic or microaerobic conditions are preferred.

The first period of time to grow the production host at the first temperature may be determined in a variety of ways. For example, during this period of growth a metabolic parameter of the fermentation culture may be monitored. The metabolic parameter that is monitored may be any parameter known in the art, including, but not limited to the optical density, pH, respiratory quotient, fermentable carbon substrate utilization, CO₂ production, and isobutanol production. During this period of growth, additional fermentable carbon substrate may be added, the pH may be adjusted, oxygen may be added for aerobic cells, or other culture parameters may be adjusted to support the metabolic activity of the culture. Though nutrients and culture conditions are supportive of growth, after a period of time the metabolic activity of the fermentation culture decreases as determined by the monitored parameter described above. For example, a decrease in metabolic activity may be indicated by a decrease in one or more of the following parameters: rate of optical density change, rate of pH change, rate of change in respiratory quotient (if the host cells are aerobic), rate of fermentable carbon substrate utilization, rate of isobutanol production, rate of change in CO₂ production, or rate of another metabolic parameter. The decrease in metabolic activity is related to the sensitivity of the host cells to the production of isobutanol and/or the presence of isobutanol in the culture. When decreased metabolic activity is detected, the temperature of the fermentation culture is lowered to reduce the sensitivity of the host cells to isobutanol and thereby allow further production of isobutanol. In one embodiment, the lowering of the temperature coincides with a change in the metabolic parameter that is monitored.

In one embodiment, the change in metabolic activity is a decrease in the rate of isobutanol production. Isobutanol production may be monitored by analyzing the amount of isobutanol present in the fermentation culture medium as a function of time using methods well known in the art, such as using high performance liquid chromatography (HPLC) or gas chromatography (GC), which are described in the Examples herein. GC is preferred due to the short assay time.

Alternatively, the lowering of the temperature of the fermentation culture may occur at a predetermined time. The first period of time may be predetermined by establishing a correlation between a metabolic parameter of the fermentation culture and time in a series of test fermentations runs. A correlation between a metabolic parameter, as described above, and time of culture growth may be established for any isobutanol producing host by one skilled in the art. The specific correlation may vary depending on conditions used including, but not limited to, carbon substrate, fermentation conditions, and the specific recombinant isobutanol producing microbial production host. The correlation is most suitably made between isobutanol production or specific glucose consumption rate and time of culture growth. Once the predetermined time has been established from the correlation, the temperature of the fermentation culture in subsequent fermentation runs is lowered at the predetermined time. For example, if it is determined by monitoring a metabolic parameter in the test fermentation runs that the rate of production of isobutanol decreases after 12 hours, the temperature in subsequent fermentations runs is lowered after 12 hours without the need to monitor isobutanol production in the subsequent runs.

After the first period of time, the temperature of the fermentation culture is lowered to a second temperature. Typically, the second temperature is about 3 °C to about 25 °C lower than the first temperature. Reduction in temperature to enhance tolerance of the host cells to isobutanol is balanced with maintaining the temperature at a level where the cells continue to be metabolically active for isobutanol production. For example, a fermentation culture that has been grown at about 35 °C may be reduced in temperature to about 28 °C; or a culture grown at about 30 °C may be reduced in temperature to about 25 °C. The change in temperature may be done gradually over time or may be made as a step change. The production host is incubated at the second temperature for a second period of time, so that isobutanol production continues. The second period of time may be determined in the same manner as the first period of time described above, e.g., by monitoring a metabolic parameter or by using a predetermined time.

Additionally, the temperature lowering and incubation steps may be repeated one or more times to more finely balance metabolic activity for isobutanol production and isobutanol sensitivity. For example, a culture that has been grown at about 35 °C may be reduced in temperature to about 32 °C, followed by an incubation period. During this period a metabolic parameter of the fermentation culture may be monitored as described above, or a predetermined time may be used. It is particularly suitable to monitor the production of isobutanol during this incubation period. When monitoring indicates a decrease in metabolic activity or at a predetermined time, the temperature may be reduced a second time. For example, the temperature may be reduced from about 32 °C to about 28 °C. The temperature lowering and incubation steps may be repeated a third time where the temperature is reduced, for example, to about 20 °C. The production host is incubated at the lowered temperature so that isobutanol production continues. The steps may be repeated further as necessary to obtain the desired isobutanol titer.

### Industrial Batch and Continuous Fermentations

The present process employs a batch method of fermentation. A classical batch fermentation is a closed system where the composition of the medium is set at the beginning of the fermentation and not subject to artificial alterations during the fermentation. Thus, at the beginning of the fermentation the medium is inoculated with the desired organism or organisms, and fermentation is permitted to occur without adding anything to the system. Typically, however, a "batch" fermentation is batch with respect to the addition of carbon source and attempts are often made at controlling factors such as pH and oxygen concentration. In batch systems the metabolite and biomass compositions of the system change constantly up to the time the fermentation is stopped. Within batch cultures cells moderate through a static lag phase to a high growth log phase and finally to a stationary phase where growth rate is diminished or halted. If untreated, cells in the stationary phase will eventually die. Cells in log phase generally are responsible for the bulk of production of end product or intermediate.

A variation on the standard batch system is the Fed-Batch system. Fed-Batch fermentation processes are also suitable in the present invention and comprise a typical batch system with the exception that the substrate is added in increments as the fermentation progresses. Fed-Batch systems are useful when catabolite repression is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂. Batch and Fed-Batch fermentations are common and well known in the art and examples may be found in Thomas D. Brock in Biotechnology: A Textbook of Industrial Microbiology, Second Edition (1989) Sinauer Associates, Inc., Sunderland, MA., or Deshpande, Mukund V., Appl. Biochem. Biotechnol., 36:227, (1992) .

Although the present invention is performed in batch mode it is contemplated that the method would be adaptable to continuous fermentation methods. Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology and a variety of methods are detailed by Brock, *supra.*

It is contemplated that the present invention may be practiced using either batch, fed-batch or continuous processes and that any known mode of fermentation would be suitable. Additionally, it is contemplated that cells may be immobilized on a substrate as whole cell catalysts and subjected to fermentation conditions for isobutanol production.

### Methods for Isobutanol Isolation from the Fermentation Medium

The bioproduced isobutanol may be isolated from the fermentation medium using methods known in the art. For example, solids may be removed from the fermentation medium by centrifugation, filtration, decantation, or the like. Then, the isobutanol may be isolated from the fermentation medium, which has been treated to remove solids as described above, using methods such as distillation, liquid-liquid extraction, or membrane-based separation. Because isobutanol forms a low boiling point, azeotropic mixture with water, distillation can only be used to separate the mixture up to its azeotropic composition. Distillation may be used in combination with another separation method to obtain separation around the azeotrope. Methods that may be used in combination with distillation to isolate and purify isobutanol include, but are not limited to, decantation, liquid-liquid extraction, adsorption, and membrane-based techniques. Additionally, isobutanol may be isolated using azeotropic distillation using an entrainer (see for example Doherty and Malone, Conceptual Design of Distillation Systems, McGraw Hill, New York, 2001).

The isobutanol-water mixture forms a heterogeneous azeotrope so that distillation may be used in combination with decantation to isolate and purify the isobutanol. In this method, the isobutanol containing fermentation broth is distilled to near the azeotropic composition. Then, the azeotropic mixture is condensed, and the isobutanol is separated from the fermentation medium by decantation. The decanted aqueous phase may be returned to the first distillation column as reflux. The isobutanol-rich decanted organic phase may be further purified by distillation in a second distillation column.

The isobutanol may also be isolated from the fermentation medium using liquid-liquid extraction in combination with distillation. In this method, the isobutanol is extracted from the fermentation broth using liquid-liquid extraction with a suitable solvent. The isobutanol-containing organic phase is then distilled to separate the isobutanol from the solvent.

Distillation in combination with adsorption may also be used to isolate isobutanol from the fermentation medium. In this method, the fermentation broth containing the isobutanol is distilled to near the azeotropic composition and then the remaining water is removed by use of an adsorbent, such as molecular sieves (Aden et al. Lignocellulosic Biomass to Ethanol Process Design and Economics Utilizing Co-Current Dilute Acid Prehydrolysis and Enzymatic Hydrolysis for Corn Stover, Report NREL/TP-510-32438, National Renewable Energy Laboratory, June 2002).

Additionally, distillation in combination with pervaporation may be used to isolate and purify the isobutanol from the fermentation medium. In this method, the fermentation broth containing the isobutanol is distilled to near the azeotropic composition, and then the remaining water is removed by pervaporation through a hydrophilic membrane (Guo et al., J. Membr. Sci. 245, 199-210 (2004)).

### EXAMPLES

The present invention is further defined in the following Examples. It should be understood that these Examples, while indicating preferred embodiments of the invention, are given by way of illustration only.

### General Methods

Standard recombinant DNA and molecular cloning techniques used in the Examples are well known in the art and are described by Sambrook, J., Fritsch, E. F. and Maniatis, T. Molecular Cloning: A Laboratory Manual; Cold Spring Harbor Laboratory Press: Cold Spring Harbor, NY (1989) (Maniatis) and by T. J. Silhavy, M. L. Bennan, and L. W. Enquist, Experiments with Gene Fusions, Cold Spring Harbor Laboratory Press, Cold Spring Harbor, N.Y. (1984) and by Ausubel, F. M. et al., Current Protocols in Molecular Biology, pub. by Greene Publishing Assoc. and Wiley-Interscience (1987).

Materials and methods suitable for the maintenance and growth of bacterial cultures are well known in the art. Techniques suitable for use in the following Examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994)) or by Thomas D. Brock in Biotechnology: A Textbook of Industrial Microbiology, Second Edition, Sinauer Associates, Inc., Sunderland, MA (1989). All reagents, restriction enzymes and materials used for the growth and maintenance of bacterial cells were obtained from Aldrich Chemicals (Milwaukee, WI), BD Diagnostic Systems (Sparks, MD), Life Technologies (Rockville, MD), or Sigma Chemical Company (St. Louis, MO) unless otherwise specified.

Microbial strains were obtained from The American Type Culture Collection (ATCC), Manassas, VA, unless otherwise noted.

The oligonucleotide primers to use in the following Examples are given in Table 4. All the oligonucleotide primers are synthesized by Sigma-Genosys (Woodlands, TX).

**Table 4**

| Oligonucleotide Cloning, Screening, and Sequencing Primers | | | |
|---|---|---|---|
| Name | Sequence | Description | SEQ ID NO: |
| N80 | | budB forward | 11 |
| N81 | | budB reverse | 12 |
| N100 | | ilvC forward | 13 |
| N101 | | ilvC reverse | 14 |
| N102 | | ilvD forward | 15 |
| N103 | | ilvD reverse | 16 |
| N104 | | yqhD forward | 17 |
| N105 | | yqhD reverse | 18 |
| N110 | | budB forward | 19 |
| N111 | | budB reverse | 20 |
| N112 | | ilvC forward | 21 |
| N113 | | ilvC reverse | 22 |
| N114 | | ilvD forward | 23 |
| N115 | | ilvD reverse | 24 |
| N116 | | kivD forward | 25 |
| N117 | | kivD reverse | 26 |
| N118 | | yqhD forward | 27 |
| N119 | | yqhD reverse | 28 |
| BenNF | | Npr forward | 29 |
| BenASR | | Npr reverse | 30 |
| N110.2 | | budB forward | 31 |
| | | | |
| N111.2 | | budB reverse | 32 |
| N112.2 | | ilvC forward | 33 |
| N113.2 | | ilvC reverse | 34 |
| N114.2 | | ilvD forward | 35 |
| N115.2 | | ilvD reverse | 36 |
| N116.2 | | kivD forward | 37 |
| N117.2 | | kivD reverse | 38 |
| N130SeqF1 | TGTTCCAACCTGATCACCG | sequencing primer | 40 |
| N130SeqF2 | GGAAAACAGCAAGGCGCT | sequencing primer | 41 |
| N130SeqF3 | CAGCTGAACCAGTTTGCC | sequencing primer | 42 |
| N130SeqF4 | AAAATACCAGCGCCTGTCC | sequencing primer | 43 |
| N130SeqR1 | TGAATGGCCACCATGTTG | sequencing primer | 44 |
| N130SeqR2 | GAGGATCTCCGCCGCCTG | sequencing primer | 45 |
| N130SeqR3 | AGGCCGAGCAGGAAGATC | sequencing primer | 46 |
| N130SeqR4 | TGATCAGGTTGGAACAGCC | sequencing primer | 47 |
| N131SeqF1 | AAGAACTGATCCCACAGGC | sequencing primer | 48 |
| N131SeqF2 | ATCCTGTGCGGTATGTTGC | sequencing primer | 49 |
| N131SeqF3 | ATTGCGATGGTGAAAGCG | sequencing primer | 50 |
| N131SeqR1 | ATGGTGTTGGCAATCAGCG | sequencing primer | 51 |
| N131SeqR2 | GTGCTTCGGTGATGGTTT | sequencing primer | 52 |
| N131SeqR3 | TTGAAACCGTGCGAGTAGC | sequencing primer | 53 |
| N132SeqF1 | TATTCACTGCCATCTCGCG | sequencing primer | 54 |
| N132SeqF2 | CCGTAAGCAGCTGTTCCT | sequencing primer | 55 |
| N132SeqF3 | GCTGGAACAATACGACGTTA | sequencing primer | 56 |
| N132SeqF4 | TGCTCTACCCAACCAGCTTC | sequencing primer | 57 |
| N132SeqR1 | ATGGAAAGACCAGAGGTGCC | sequencing primer | 58 |
| N132SeqR2 | TGCCTGTGTGGTACGAAT | sequencing primer | 59 |
| N132SeqR3 | TATTACGCGGCAGTGCACT | sequencing primer | 60 |
| N132SeqR4 | GGTGATTTTGTCGCAGTTAGAG | sequencing primer | 61 |
| N133SeqF1 | TCGAAATTGTTGGGTCGC | sequencing primer | 62 |
| N133SeqF2 | GGTCACGCAGTTCATTTCTAAG | sequencing primer | 63 |
| N133SeqF3 | TGTGGCAAGCCGTAGAAA | sequencing primer | 64 |
| N133SeqF4 | AGGATCGCGTGGTGAGTAA | sequencing primer | 65 |
| N133SeqR1 | GTAGCCGTCGTTATTGATGA | sequencing primer | 66 |
| N133SeqR2 | GCAGCGAACTAATCAGAGATTC | sequencing primer | 67 |
| N133SeqR3 | TGGTCCGATGTATTGGAGG | sequencing primer | 68 |
| N133SeqR4 | TCTGCCATATAGCTCGCGT | sequencing primer | 69 |
| Scr1 | CCTTTCTTTGTGAATCGG | sequencing primer | 72 |
| Scr2 | AGAAACAGGGTGTGATCC | sequencing primer | 73 |
| Scr3 | AGTGATCATCACCTGTTGCC | sequencing primer | 74 |
| Scr4 | AGCACGGCGAGAGTCGACGG | sequencing primer | 75 |
| T-budB (BamHI) | | budB forward | 144 |
| B-kivD (BamHI) | | kivD reverse | 145 |
| T-groE(Xhol) | | PgroE forward | 147 |
| B-groEL(Spel, BamH1) | | PgroE reverse | 148 |
| T-groEL | | PgroE forward | 149 |
| T-ilvCB.s. (BamHI) | | ilvC forward | 150 |
| B-ilvCB.s. (SpelBamHI) | | ilvC reverse | 151 |
| T-BD64 (DraIII) | | pBD64 forward | 152 |
| B-BD64 (DraIII) | | pBD64 reverse | 153 |
| | | | |
| T-laclq (DraIII) | | laclq forward | 154 |
| B-laclq (DraIII) | | laclq reverse | 155 |
| T-groE (Dralll) | | PgroE forward | 156 |
| B-B.s.ilvC (DraIII) | | ilvC reverse | 157 |
| T-bdhB (DraIII) | | bdhB forward | 159 |
| B-bdhB (rrnBT1DraIII) | | bdhB reverse | 160 |
| LDH EcoRV F | | IdhL forward | 161 |
| LDH AatIIR | | IdhL reverse | 162 |
| Cm F | | Cm forward | 163 |
| Cm R | | Cm reverse | 164 |
| P11 F-Stul | | P11 promoter forward | 165 |
| P11 R-Spel | | P11 promoter reverse | 166 |
| PldhL F-HindIII | | IdhL forward | 167 |
| PldhL R- | | IdhL reverse | 168 |
| BamHI | | | |
| F-bdhB-AvrII | | bdhB forward | 169 |
| R-bdhB-BamHl | | bdhB reverse | 170 |
| F-ilvC(B.s.)-AfIII | | ilvC forward | 171 |
| R-ilvC(B.s.)-Notl | | ivIC reverse | 172 |
| F-PnisA(Hindlll) | | nisA promoter forward | 173 |
| R-PnisA(Spel BamHI) | | nisA promoter reverse | 174 |

### Methods for Determining Isobutanol Concentration in Culture Media

The concentration of isobutanol in the culture media can be determined by a number of methods known in the art. For example, a specific high performance liquid chromatography (HPLC) method utilized a Shodex SH-1011 column with a Shodex SH-G guard column, both purchased from Waters Corporation (Milford, MA), with refractive index (RI) detection. Chromatographic separation was achieved using 0.01 M H₂SO₄ as the mobile phase with a flow rate of 0.5 mL/min and a column temperature of 50 °C. Isobutanol had a retention time of 46.6 min under the conditions used. Alternatively, gas chromatography (GC) methods are available. For example, a specific GC method utilized an HP-INNOWax column (30 m x 0.53 mm id, 1 µm film thickness, Agilent Technologies, Wilmington, DE), with a flame ionization detector (FID). The carrier gas was helium at a flow rate of 4.5 mL/min, measured at 150 °C with constant head pressure; injector split was 1:25 at 200 °C; oven temperature was 45 °C for 1 min, 45 to 220 °C at 10 °C/min, and 220 °C for 5 min; and FID detection was employed at 240 °C with 26 mL/min helium makeup gas. The retention time of isobutanol was 4.5 min.

The meaning of abbreviations is as follows: "s" means second(s), "min" means minute(s), "h" means hour(s), "psi" means pounds per square inch, "nm" means nanometers, "d" means day(s), "µL" means microliter(s), "mL" means milliliter(s), "L" means liter(s), "mm" means millimeter(s), "nm" means nanometers, "mM" means millimolar, "µM" means micromolar, "M" means molar, "mmol" means millimole(s), "µmol" means micromole(s)", "g" means gram(s), "µg" means microgram(s) and "ng" means nanogram(s), "PCR" means polymerase chain reaction, "OD" means optical density, "OD₆₀₀" means the optical density measured at a wavelength of 600 nm, "kDa" means kilodaltons, "g" means the gravitation constant, "bp" means base pair(s), "kbp" means kilobase pair(s), "% w/v" means weight/volume percent, % v/v" means volume/volume percent, "IPTG" means isopropyl-β-D-thiogalactopyranoiside, "RBS" means ribosome binding site, "nt" means not tested, "HPLC" means high performance liquid chromatography, and "GC" means gas chromatography. The term "molar selectivity" is the number of moles of product produced per mole of sugar substrate consumed and is reported as a percent.

### EXAMPLE 1

### Increased tolerance of Lactobacillus plantarum PN0512 to 1-butanol, isobutanol and 2-butanol at decreased growth temperatures

Tolerance levels of bacterial strain *Lactobacillus plantarum* PN0512 (ATCC # PTA-7727) were determined at 25 °C, 30 °C and 37 °C as follows. The strain was cultured in S30L medium (i.e., 10 mM ammonium sulfate, 5 mM potassium phosphate buffer, pH 7.0, 50 mM MOPS, pH 7.0, 2 mM MgCl₂, 0.7 mM CaCl₂, 50 µM MnCl₂, 1 µM FeCl₃, 1 µM ZnCl₂, 1.72 µM CuCl₂, 2.53 µM CoCl₂, 2.42 µM Na₂MoO₄, 2 µM thiamine hydrochloride, 10 mM glucose, and 0.2% yeast extract). An overnight culture in the absence of any test compound was started in 15 mL of the S30L medium in a 150 mL flask, with incubation at 37 °C in a shaking water bath. The next morning, the overnight culture was diluted into three 500 mL flasks containing 150 mL of fresh medium to an initial OD₆₀₀ of about 0.08. Each flask was incubated in a shaking water bath, one each at 25 °C, 30 °C and 37 °C. Each large culture was allowed to acclimate at the test temperature for at least 0.5 h. After the acclimation period, each large culture was split into flasks in the absence (control) and in the presence of various amounts of 1-butanol, isobutanol or 2-butanol, as listed in Tables 5, 6, and 7, respectively. Growth was followed by measuring OD₆₀₀ for six hours after addition of the compounds. The results are summarized in Tables 5, 6, and 7 below.

**Table 5. Growth of L. plantarum PN0512 in the presence of 1-butanol at different temperatures**

| Concentration 1-butanol (% w/v) | 37 °C | 30 °C | 25 °C |
|---|---|---|---|
| 0.0 | + | + | + |
| 1.0 | + | nt | nt |
| 1.2 | + | nt | nt |
| 1.4 | + | nt | nt |
| 1.5 | + | + | + |
| 1.6 | + | nt | nt |
| 1.8 | + | nt | nt |
| 2.0 | + | + | + |
| 2.1 | + | nt | nt |
| 2.2 | + | nt | nt |
| 2.3 | + | nt | nt |
| 2.4 | - | + | + |
| 2.5 | - | nt | nt |
| 2.7 | - | + | nt |
| 2.9 | - | - | + |
| 3.1 | - | - | + |
| 3.2 | nt | - | - |
| 3.3 | nt | nt | - |
| 3.4 | nt | - | - |

**Table 6. Growth of L. plantarum PN0512 in the presence of isobutanol at different temperatures**

| Concentration isobutanol (% w/v) | 37 °C | 30 °C | 25 °C |
|---|---|---|---|
| 0.0 | + | + | + |
| 0.5 | + | nt | nt |
| 1.0 | + | nt | nt |
| 1.5 | + | + | + |
| 1.6 | + | nt | nt |
| 1.8 | + | nt | nt |
| 2.0 | + | + | + |
| 2.1 | + | nt | nt |
| 2.3 | + | nt | nt |
| 2.4 | + | + | + |
| 2.5 | + | nt | nt |
| 2.7 | + | + | + |
| 2.9 | + | + | + |
| 3.1 | + | + | + |
| 3.3 | nt | - | + |
| 3.4 | - | nt | nt |
| 3.5 | nt | nt | + |
| 3.6 | nt | nt | - |
| 3.8 | - | nt | nt |
| 4.3 | - | nt | nt |

**Table 7. Growth of L. plantarum PN0512 in the presence of 2-butanol at different temperatures**

| Concentration 2-butanol (% w/v) | 37 °C | 30 °C | 25 °C |
|---|---|---|---|
| 0.0 | + | + | + |
| 1.8 | + | nt | nt |
| 2.1 | + | nt | nt |
| 2.5 | + | nt | nt |
| 2.9 | + | + | + |
| 3.1 | + | nt | nt |
| 3.5 | + | nt | nt |
| 3.6 | + | nt | nt |
| 3.8 | + | + | + |
| 4.0 | nt | + | nt |
| 4.3 | + | + | + |
| 4.5 | - | + | nt |
| 4.7 | - | + | + |
| 4.9 | nt | - | + |
| 5.2 | - | nt | + |
| 5.6 | - | nt | - |
| 6.0 | - | nt | nt |
| 6.4 | - | nt | nt |
| 7.3 | - | nt | nt |

| | | | |
|---|---|---|---|
| "+" = growth observed as an increase in OD₆₀₀. "-" = no growth observed, i.e. no change in OD₆₀₀. | | | |

All three butanols showed a similar effect of temperature on growth inhibition of *L*. *plantarum* PN0512. The concentration that resulted in full growth inhibition was greater at 25 °C than at 37 °C. In the case of 1-butanol, growth was observed at 37 °C in 2.3% 1-butanol, but not 2.4%. However, at 30 °C growth was observed in 2.7%, but not 2.9%, and at 25 °C growth was observed even in 3.1% 1-butanol. Thus, the concentration of 1-butanol that completely inhibited growth increased as growth temperature decreased. Likewise, in the case of isobutanol, growth was observed in 3.5% at 25 °C while growth was observed in 3.1% at 30 °C and 37 °C, but not in 3.3% or 3.4%. Similarly, in the case of 2-butanol growth was observed at 37 °C in 4.3%, but not in 4.5%; at 30 °C in 4.7%, but not in 4.9%; and at 25 °C in 5.2 %. Thus the tolerance of *L*. *plantarum* PN0512 to butanols increased with decreased growth temperature.

### EXAMPLE 2

### Increased tolerance of Escherichia coli to 1-butanol at decreased exposure temperature

The effect of growth and exposure temperature on survival of *Escherichia coli* in the presence of 1-butanol was tested using stationary phase cultures in a rich medium and log phase cultures in a defined medium. For the stationary phase studies, *E*. *coli* strain MG1655 (ATCC # 700926) was grown overnight in LB medium (Teknova, Half Moon Bay, CA) with shaking at 250 rpm at 42 °C, 29 °C or 28 °C. Survival of 1-butanol shock was tested at exposure temperatures of 0 °C, 28 °C or 42 °C. The 1-butanol exposure at 28 °C or 42 °C was started immediately after removing the overnight cultures from the growth incubators. The 1-butanol exposure at 0 °C was done after allowing the overnight cultures to cool on ice for about 15 min. A series of solutions of 1-butanol at different concentrations in LB medium was made and 90 µL aliquots were put in microfuge tubes. To these were added 10 µL of the overnight cultures and the tubes were immediately placed in shaking incubators at 42 °C or 28 °C or left on ice for 30 min. To stop the effect of 1-butanol on the cultures, a 10⁻² dilution was done by placing 2 µL of the treated culture into 198 µL of LB medium in wells of a microplate. Then, 5 µL of the undiluted treated cultures were spotted on LB agar plates. Subsequent 10-fold serial dilutions of 10⁻³, 10⁻⁴, 10⁻⁵ and 10⁻⁶ of the exposed cultures were done by serial pipetting of 20 µL, starting with the 10⁻² dilution cultures, into 180 µL of LB medium in the microplate, using a multi-channel pipette. Prior to each transfer, the cultures were mixed by pipetting up and down six times. Each dilution (5 µL) was spotted onto an LB plate using a multi-channel pipette and allowed to soak into the plate. The plates were inverted and incubated overnight at 37 °C. The number of colonies for each dilution was counted and the % growth inhibition was calculated by comparison with a control culture that had not been exposed to 1-butanol. Survival of 0% was recorded when no colonies in the spots of the undiluted or any of the serial dilutions were observed. The results are shown in Table 8.

**Table 8. Survival of stationary phase E. coli in 1-butanol at 42 °C, 28 °C, or 0 °C**

| | Grown at 42 °C | Grown at 29 °C | Grown at 42 °C | Grown at 28 °C | Grown at 42 °C | Grown at 29 °C |
|---|---|---|---|---|---|---|
| 1-Butanol % (w/v) | % survival after 30 min exposure at 42 °C | | % survival after 30 min exposure at 28 °C | | % survival after 30 min exposure at 0 °C | |
| 1.0 | 100 | 100 | 100 | 100 | 100 | 100 |
| 1.5 | 0.1 | 0.1 | 100 | 100 | 100 | 100 |
| 2.0 | 0 | 0.1 | 100 | 100 | 100 | 100 |
| 2.5 | 0 | 0 | 100 | 100 | 100 | 100 |
| 3.0 | 0 | 0 | 100 | 100 | 100 | 100 |
| 3.5 | 0 | 0 | 3 | 10 | 100 | 100 |
| 4.0 | 0 | 0 | 0.0004 | 0.0003 | 100 | 100 |
| 5.0 | nt | nt | nt | nt | 1 | 1 |
| 6.0 | nt | nt | nt | nt | 0 | 0.001 |
| 7.0 | nt | nt | nt | nt | 0 | 0 |

A similar study was done with log-phase cultures of *E. coli* grown in a defined medium. *E*. *coli* strain MG1655 was allowed to grow overnight in MOPS 0.2% glucose medium (Teknova, Half Moon Bay, CA) at 42 °C or 28 °C. The following day, the cultures were diluted into fresh medium and allowed to grow at the same temperature until in the log phase of growth. The OD₆₀₀ was 0.74 for the 28 °C culture and was 0.72 for the 42 °C culture. Both of these log phase cultures were exposed to 1-butanol at 42 °C, 28 °C and 0 °C as follows. A series of solutions of 1-butanol at different concentrations in MOPS 0.2% glucose medium was made and 90 µL aliquots were put in microfuge tubes. To these were added 10 µL of the log phase cultures and the tubes were immediately placed in shaking incubators at 42 °C or 28 °C or left on ice for 30 min. To stop the effect of 1-butanol on the cultures, a 10⁻² dilution was done by placing 2 µL of the treated culture into 198 µL of LB medium in wells of a microplate. Then 5 µL of the undiluted treated cultures were spotted on LB agar plates. Subsequent 10-fold serial dilutions of 10⁻³, 10⁻⁴, 10⁻⁵ and 10⁻⁶ of the exposed cultures were done by serial pipetting of 20 µL, starting with the 10⁻² dilution cultures, into 180 µL of LB medium in the microplate, using a multi-channel pipette. Prior to each transfer, the cultures were mixed by pipetting up and down six times. Each dilution (5 µL) was spotted onto an LB plate using a multi-channel pipette and allowed to soak into the plate. The plates were inverted and incubated overnight at 37 °C. The number of colonies for each dilution was counted and the % growth inhibition was calculated by comparison with a control culture that had not been exposed to 1-butanol. Survival of 0% was recorded when no colonies in the spots of the undiluted or any of the serial dilutions were observed. The results are shown in Table 9.

**Table 9. Survival of log-phase E. coli in 1-butanol at 42 °C, 28 °C, or 0 °C**

| | Grown at 42 °C | Grown at 28 °C | Grown at 42 °C | Grown at 28 °C | Grown at 42 °C | Grown at 29 °C |
|---|---|---|---|---|---|---|
| 1-Butanol % (w/v) | % survival after 30 min exposure at 42 °C | | % survival after 30 min exposure at 28 °C | | % survival after 30 min exposure at 0 °C | |
| 1.0 | 100 | 100 | nt | nt | nt | nt |
| 1.5 | 0 | 0 | 100 | 100 | nt | nt |
| 2.0 | 0 | 0 | 100 | 100 | nt | nt |
| 2.5 | 0 | 0 | 0.1 | 50 | 100 | 100 |
| 3.0 | 0 | 0 | 0 | 0 | 100 | 100 |
| 3.5 | 0 | 0 | 0.01 | 0 | 100 | 100 |
| 4.0 | 0 | 0 | 0.001 | 0 | 100 | 100 |
| 4.5 | nt | nt | 0 | 0 | 100 | 100 |
| 5.0 | nt | nt | nt | nt | 10 | 50 |
| 6.0 | nt | nt | nt | nt | 1 | 1 |

For both the stationary phase and log-phase cultures of *E*. *coli* MG1655, the growth temperature had very little, if any, effect on the survival of a 1-butanol shock. However, the exposure temperature had a major effect on the survival of *E*. *coli* to 1-butanol shock. As can be seen from the data in Tables 8 and 9, the tolerance of *E*. *coli* MG1655 to 1-butanol increased with decreasing exposure temperature.

### EXAMPLE 3

### Increased tolerance of Escherichia coli to 2-butanone at decreased exposure temperature

The effect of exposure temperature on survival of *Escherichia coli* in the presence of 2-butanone (also referred to herein as methyl ethyl ketone or MEK) was tested as follows. *E*. *coli* strain BW25113 (The Coli Genetic Stock Center (CGSC), Yale University; # 7636) was grown overnight in LB medium (Teknova, Half Moon Bay, CA) with shaking at 250 rpm at 37 °C. Survival of MEK shock was tested at exposure temperatures of 28 °C or 37 °C. A series of solutions of MEK at different concentrations in LB medium was made and 90 µL aliquots were put in microfuge tubes. To these were added 10 µL of the overnight culture and the tubes were immediately placed in shaking incubators at 37 °C or 28 °C for 30 min. To stop the effect of MEK on the cultures, a 10⁻² dilution was done by placing 2 µL of the MEK treated culture into 198 µL of LB medium in wells of a microplate. Then 5 µL of the undiluted treated cultures were spotted on LB agar plates. Subsequent 10-fold serial dilutions of 10⁻³, 10⁻⁴, 10⁻⁵ and 10⁻⁶ of the exposed cultures were done by serial pipetting of 20 µL, starting with the 10⁻² dilution cultures, into 180 µL of LB medium in the microplate, using a multi-channel pipette. Prior to each transfer, the cultures were mixed by pipetting up and down six times. Each dilution (5 µL) was spotted onto LB plates using a multi-channel pipette and allowed to soak into the plate. The plates were inverted and incubated overnight at 37 °C. The number of colonies for each dilution was counted and the % growth inhibition was calculated by comparison with a control culture that had not been exposed to MEK. Survival of 0% was recorded when no colonies in the spots of the undiluted or any of the serial dilutions were observed. The results, given as the average of duplicate experiments, are shown in Table 10.

**Table 10. Survival of E. coli in MEK at 37 °C and 28 °C**

| MEK % w/v | % Survival at 37 °C | % Survival at 28 °C |
|---|---|---|
| 0 | 100 | 100 |
| 4 | 100 | 100 |
| 6 | 0 | 100 |
| 8 | 0 | 0.002 |

Reducing the exposure temperature from 37 °C to 28 °C dramatically improved survival of *E. coli* to MEK treatment. At 37 °C there was full survival at 4% w/v and no survival at 6% w/v, while at 28 °C there was full survival at 6% w/v. Thus, the tolerance of *E. coli* to MEK increased with decreasing exposure temperature.

### EXAMPLE 4

### Increased tolerance of E. coli and L. plantarum PN0512 to 1-Butanol at decreased exposure temperature

This Example demonstrates that the toxic effects of 1-butanol and 2-butanol on various microbial cells was reduced at lower temperatures. This was demonstrated by incubating *E. coli* (strain MG1655; ATCC # 700926), and *L. plantarum* (strain PN0512; ATCC # PTA-7727) with either 1-butanol or 2-butanol at different temperatures and then determining the fraction of the cells that survived the treatment at the different temperatures.

Using overnight cultures or cells from plates, 30 mL cultures of the microorganisms to be tested were started in the following culture media:
*E. coli -* Miller's LB medium (Teknova, Half Moon Bay, CA):
*L. plantarum* PN0512 - *Lactobacilli* MRS Broth (BD Diagnostic Systems, Sparks, MD).
The *E. coli* and *L. plantarum* cultures were grown at 37 °C aerobically with shaking until the cultures were in log phase and the OD₆₀₀ was between 0.6 and 0.8. A 50 µL aliquot of each culture was removed for a time zero sample. The remainder of the cultures was divided into six 5 mL portions and placed in six small incubation flasks or tubes. Different amounts of 1-butanol or 2-butanol were added to the six flasks to bring the concentration to predetermined values, as listed in the tables below. The flasks or tubes were incubated at a desired temperature, aerobically without shaking for 1 h. After the incubation with one of the butanols, 2 µL from each of the flasks (and in addition 2 µL of the time zero sample of the culture before exposure to one of the butanols) were pipetted into the "head" wells of a 96 well (8 X 12) microtiter plate, each containing 198 µL of LB medium to give a 10⁻² dilution of the culture. Subsequently, 10⁻³, 10⁻⁴, 10⁻⁵, and 10⁻⁶ serial dilutions of the cultures were prepared as follows. The 10⁻³ dilution was prepared by pipetting 20 µL of the sample from the head well into the 180 µL LB medium in the next well using a multi-channel pipette. This procedure was repeated 3 more times on successive wells to prepare the 10⁻⁴, 10⁻⁵, and 10⁻⁶ dilutions. After each liquid transfer, the solution in the well was mixed by pipetting it up and down 10 times with the multi-channel pipetor. A 5 µL aliquot of each dilution was spotted onto an LB plate using a multi-channel pipette starting with the 10⁻⁶ dilution, then the 10⁻⁵, and so on working from more to less dilute without a change of tips. The spots were allowed to soak into the agar by leaving the lid of the plate slightly open for 15 to 30 min in a sterile transfer hood. The plates were covered, inverted, and incubated overnight at 37 °C. The following day, the number of colonies in the spots were counted from the different dilutions. The number of living cells/mL in each of the original culture solutions from which the 2 µL was withdrawn was calculated and compared to the number of cells in the control untreated culture to determine the % of the cells surviving.

The results of experiments in which *E*. *coli* cells were treated with 1-butanol at temperatures of 0, 30, and 37 °C are shown Table 11.

**Table 11. Percentage of E. coli cells surviving in 1-butanol at 0, 30 and 37 °C**

| 1-butanol % v/v | % Survival at 0 °C | % Survival at 30 °C | % Survival at 37 °C |
|---|---|---|---|
| 0 | 100 | 100 | 100 |
| 1 | nt | 100 | 72 |
| 1.5 | nt | 100 | 20 |
| 2 | nt | 100 | 0 |
| 2.5 | 100 | 23 | 0 |
| 3 | 100 | 0 | 0 |
| 3.5 | 100 | 0 | nt |
| 4 | 100 | nt | nt |
| 4.5 | 100 | nt | nt |

The concentration at which 1-butanol kills *E. coli* cells was affected by the treatment temperature. At 0 °C, concentrations of 1-butanol as high as 4.5% v/v had no toxic effect on *E. coli* cells during a one hour treatment. At 30 °C, *E. coli* cells were killed when treated with 3% v/v 1-butanol for one hour. At 37 °C, *E. coli* cells were killed when treated with 2% v/v 1-butanol for one hour.

The results of experiments in which *L. plantarum* PN0512 cells were treated with 1-butanol at temperatures of 0, 23, and 37 °C for one hour are shown Table 12.

**Table 12. Percentage of L. plantarum PN0512 cells surviving in 1-butanol at 0, 23 and 37 °C**

| 1-butanol % v/v | % Survival at 0 °C | % Survival at 23 °C | % Survival at 37 °C |
|---|---|---|---|
| 0 | 100 | 100 | 100 |
| 1 | nt | nt | 80 |
| 1.5 | nt | nt | 58 |
| 2 | nt | 100 | 29 |
| 2.5 | nt | 100 | 8 |
| 3 | 100 | 82 | 0 |
| 3.5 | 100 | 0 | 0 |
| 4 | 100 | 0 | nt |
| 4.5 | 100 | 0 | nt |
| 5 | 0 | nt | nt |
| 5.5 | 0 | nt | nt |

The concentration at which 1-butanol kills *L. plantarum* PN0512 cells was affected by the treatment temperature. At 0 °C, concentrations of 1-butanol as high as 4.5% v/v had no toxic effect on *L. plantarum* PN0512 cells during a one hour treatment. At 23 °C, *L. plantarum* PN0512 cells were killed when treated with 3.5% v/v 1-butanol for one hour. At 37 °C, *L. plantarum* PN0512 cells were killed when treated with 2.5 % v/v 1-butanol for one hour.

### EXAMPLE 5

### Cloning and Expression of Acetolactate Synthase

The purpose of this Example was to clone the *budB* gene from *Klebsiella pneumoniae* and express it in *E. coli* BL21-Al. The *budB* gene was amplified from *Klebsiella pneumoniae* strain ATCC 25955 genomic DNA using PCR, resulting in a 1.8 kbp product.

Genomic DNA was prepared using the Gentra Puregene kit (Gentra Systems, Inc., Minneapolis, MN; catalog number D-5000A). The *budB* gene was amplified from *Klebsiella pneumoniae* genomic DNA by PCR using primers N80 and N81 (see Table 2), given as SEQ ID NOs:11 and 12, respectively. Other PCR amplification reagents were supplied in manufacturers' kits, for example, Finnzymes Phusion™ High-Fidelity PCR Master Mix (New England Biolabs Inc., Beverly, MA; catalog no. F-531) and used according to the manufacturer's protocol. Amplification was carried out in a DNA Thermocycler GeneAmp 9700 (PE Applied Biosystems, Foster city, CA).

For expression studies the Gateway cloning technology (Invitrogen Corp., Carlsbad, CA) was used. The entry vector pENTRSDD-TOPO allowed directional cloning and provided a Shine-Dalgarno sequence for the gene of interest. The destination vector pDEST14 used a T7 promoter for expression of the gene with no tag. The forward primer incorporated four bases (CACC) immediately adjacent to the translational start codon to allow directional cloning into pENTRSDD-TOPO (Invitrogen) to generate the plasmid pENTRSDD-TOPObudB. The pENTR construct was transformed into *E. coli* Top10 (Invitrogen) cells and plated according to manufacturer's recommendations. Transformants were grown overnight and plasmid DNA was prepared using the QIAprep Spin Miniprep kit (Qiagen, Valencia, CA; catalog no. 27106) according to manufacturer's recommendations. Clones were sequenced to confirm that the genes inserted in the correct orientation and to confirm the sequence. The nucleotide sequence of the open reading frame (ORF) for this gene and the predicted amino acid sequence of the enzyme are given as SEQ ID NO:1 and SEQ ID NO:2, respectively.

To create an expression clone, the *budB* gene was transferred to the pDEST 14 vector by recombination to generate pDEST14budB. The pDEST14budB vector was transformed into *E*. *coli* BL21-Al cells (Invitrogen). Transformants were inoculated into Luria Bertani (LB) medium supplemented with 50 µg/mL of ampicillin and grown overnight. An aliquot of the overnight culture was used to inoculate 50 mL of LB supplemented with 50 µg/mL of ampicillin. The culture was incubated at 37 °C with shaking until the OD₆₀₀ reached 0.6-0.8. The culture was split into two 25-mL cultures and arabinose was added to one of the flasks to a final concentration of 0.2% w/v. The negative control flask was not induced with arabinose. The flasks were incubated for 4 h at 37 °C with shaking. Cells were harvested by centrifugation and the cell pellets were resuspended in 50 mM MOPS, pH 7.0 buffer. The cells were disrupted either by sonication or by passage through a French Pressure Cell. The whole cell lysate was centrifuged yielding the supernatant or cell free extract and the pellet or the insoluble fraction. An aliquot of each fraction (whole cell lysate, cell free extract and insoluble fraction) was resuspended in SDS (MES) loading buffer (Invitrogen), heated to 85 °C for 10 min and subjected to SDS-PAGE analysis (NuPAGE 4-12% Bis-Tris Gel, catalog no. NP0322Box, Invitrogen). A protein of the expected molecular weight of about 60 kDa, as deduced from the nucleic acid sequence, was present in the induced culture but not in the uninduced control.

Acetolactate synthase activity in the cell free extracts is measured using the method described by Bauerle et al. (Biochim. Biophys. Acta 92(1):142-149 (1964)).

### Example 6 (Prophetic)

### Cloning and Expression of Acetohydroxy Acid Reductoisomerase

The purpose of this prophetic Example is to describe how to clone the *ilvc* gene from *E. coli* K12 and express it in *E. coli* BL21-Al. The *ilvC* gene is amplified from *E. coli* genomic DNA using PCR.

The *ilvC* gene is cloned and expressed in the same manner as the *budB* gene described in Example 5. Genomic DNA from *E. coli* is prepared using the Gentra Puregene kit (Gentra Systems, Inc., Minneapolis, MN; catalog number D-5000A). The *ilvC* gene is amplified by PCR using primers N100 and N101 (see Table 2), given as SEQ ID NOs:13 and 14, respectively, creating a 1.5 kbp product. The forward primer incorporates four bases (CCAC) immediately adjacent to the translational start codon to allow directional cloning into pENTR/SD/D-TOPO (Invitrogen) to generate the plasmid pENTRSDD-TOPOilvC. Clones are sequenced to confirm that the genes are inserted in the correct orientation and to confirm the sequence. The nucleotide sequence of the open reading frame (ORF) for this gene and the predicted amino acid sequence of the enzyme are given as SEQ ID NO:3 and SEQ ID NO:4, respectively.

To create an expression clone, the *ilvC* gene is transferred to the pDEST 14 (Invitrogen) vector by recombination to generate pDEST1 4ilvC. The pDEST14ilvC vector is transformed into *E. coli* BL21-Al cells and expression from the T7 promoter is induced by addition of arabinose. A protein of the expected molecular weight of about 54 kDa, as deduced from the nucleic acid sequence, is present in the induced culture, but not in the uninduced control.

Acetohydroxy acid reductoisomerase activity in the cell free extracts is measured using the method described by Arfin and Umbarger (J. Biol. Chem. 244(5):1118-1127 (1969)).

### Example 7 (Prophetic)

### Cloning and Expression of Acetohydroxy Acid Dehydratase

The purpose of this prophetic Example is to describe how to clone the *ilvD* gene from *E. coli* K12 and express it in *E. coli* BL21-Al. The *ilvD* gene is amplified from *E. coli* genomic DNA using PCR.

The *ilvD* gene is cloned and expressed in the same manner as the *budB* gene described in Example 5. Genomic DNA from *E. coli* is prepared using the Gentra Puregene kit (Gentra Systems, Inc., Minneapolis, MN; catalog number D-5000A). The *ilvD* gene is amplified by PCR using primers N102 and N103 (see Table 2), given as SEQ ID NOs:15 and 16, respectively, creating a 1.9 kbp product. The forward primer incorporates four bases (CCAC) immediately adjacent to the translational start codon to allow directional cloning into pENTR/SD/D-TOPO (Invitrogen) to generate the plasmid pENTRSDD-TOPOilvD. Clones are submitted for sequencing to confirm that the genes are inserted in the correct orientation and to confirm the sequence. The nucleotide sequence of the open reading frame (ORF) for this gene and the predicted amino acid sequence of the enzyme are given as SEQ ID NO:5 and SEQ ID NO:6, respectively.

To create an expression clone, the *ilvD* gene is transferred to the pDEST 14 (Invitrogen) vector by recombination to generate pDEST14ilvD. The pDEST14ilvD vector is transformed into *E. coli* BL21-Al cells and expression from the T7 promoter is induced by addition of arabinose. A protein of the expected molecular weight of about 66 kDa, as deduced from the nucleic acid sequence, is present in the induced culture, but not in the uninduced control.

Acetohydroxy acid dehydratase activity in the cell free extracts is measured using the method described by Flint et al. (J. Biol. Chem. 268(20):14732-14742 (1993)).

### Example 8 (Prophetic)

### Cloning and Expression of Branched-Chain Keto Acid Decarboxylase

The purpose of this prophetic example is to describe how to clone the *kivD* gene from *Lactococcus lactis* and express it in *E. coli* BL21-Al.

A DNA sequence encoding the branched-chain keto acid decarboxylase *(kivD)* from *L. lactis* is obtained from GenScript (Piscataway, NJ). The sequence obtained is codon-optimized for expression in both *E. coli* and *B. subtilis* and is cloned into pUC57, to form pUC57-kivD. The codon-optimized nucleotide sequence of the open reading frame (ORF) for this gene and the predicted amino acid sequence of the enzyme are given as SEQ ID NO:7 and SEQ ID NO:8, respectively.

To create an expression clone *Ndel* and *BamHI* restriction sites are utilized to clone the 1.7 kbp kivD fragment from pUC57-kivD into vector pET-3a (Novagen, Madison, WI). This creates the expression clone pET-3a-kivD. The pET-3a-kivD vector is transformed into *E. coli* BL21-Al cells and expression from the T7 promoter is induced by addition of arabinose. A protein of the expected molecular weight of about 61 kDa, as deduced from the nucleic acid sequence, is present in the induced culture, but not in the uninduced control.

Branched-chain keto acid decarboxylase activity in the cell free extracts is measured using the method described by Smit et al. (Appl. Microbiol. Biotechnol. 64:396-402 (2003)).

### Example 9 (Prophetic)

### Cloning and Expression of Branched-Chain Alcohol Dehydrogenase

The purpose of this prophetic Example is to describe how to clone the *yqhD* gene from *E. coli* K12 and express it in *E. coli* BL21-Al. The *yqhD* gene is amplified from *E. coli* genomic DNA using PCR.

The *yqhD* gene is cloned and expressed in the same manner as the *budB* gene described in Example 5. Genomic DNA from *E. coli* is prepared using the Gentra Puregene kit (Gentra Systems, Inc., Minneapolis, MN; catalog number D-5000A). The *yqhD* gene is amplified by PCR using primers N104 and N105 (see Table 2), given as SEQ ID NOs:17 and 18, respectively, creating a 1.2 kbp product. The forward primer incorporates four bases (CCAC) immediately adjacent to the translational start codon to allow directional cloning into pENTR/SD/D-TOPO (Invitrogen) to generate the plasmid pENTRSDD-TOPOyqhD. Clones are submitted for sequencing to confirm that the genes are inserted in the correct orientation and to confirm the sequence. The nucleotide sequence of the open reading frame (ORF) for this gene and the predicted amino acid sequence of the enzyme are given as SEQ ID NO 9 and SEQ ID NO:10, respectively.

To create an expression clone, the *yqhD* gene is transferred to the pDEST 14 (Invitrogen) vector by recombination to generate pDEST14yqhD. The pDEST14ilvD vector is transformed into E. *coli* BL21-Al cells and expression from the T7 promoter is induced by addition of arabinose. A protein of the expected molecular weight of about 42 kDa, as deduced from the nucleic acid sequence, is present in the induced culture, but not in the uninduced control.

Branched-chain alcohol dehydrogenase activity in the cell free extracts is measured using the method described by Sulzenbacher et al. (J. Mol. Biol. 342(2):489-502 (2004)).

### Example 10 (Prophetic)

### Construction of a Transformation Vector for the Genes in an Isobutanol Biosynthetic Pathway

The purpose of this prophetic Example is to describe how to construct a transformation vector comprising the genes encoding the five steps in an isobutanol biosynthetic pathway. All genes are placed in a single operon under the control of a single promoter. The individual genes are amplified by PCR with primers that incorporate restriction sites for later cloning and the forward primers contain an optimized *E. coli* ribosome binding site (AAAGGAGG). PCR products are TOPO cloned into the pCR 4Blunt-TOPO vector and transformed into *E*. *coli Top10* cells (Invitrogen). Plasmid DNA is prepared from the TOPO clones and the sequence of the genes is verified. Restriction enzymes and T4 DNA ligase (New England Biolabs, Beverly, MA) are used according to manufacturer's recommendations. For cloning experiments, restriction fragments are gel-purified using QIAquick Gel Extraction kit (Qiagen). After confirmation of the sequence, the genes are subcloned into a modified pUC19 vector as a cloning platform. The pUC19 vector is modified by HindIII/Sapl digestion, creating pUC19dHS. The digest removes the lac promoter adjacent to the MCS (multiple cloning site), preventing transcription of the operons in the vector.

The *budB* gene is amplified from *K. pneumoniae* ATCC 25955 genomic DNA by PCR using primer pair N110 and N111 (see Table 2), given as SEQ ID NOs:19 and 20, respectively, creating a 1.8 kbp product. The forward primer incorporates SphI and AfIII restriction sites and a ribosome binding site (RBS). The reverse primer incorporates PacI and NsiI restriction sites. The PCR product is cloned into pCR4 Blunt-TOPO creating pCR4 Blunt-TOPO-budB. Plasmid DNA is prepared from the TOPO clones and the sequence of the gene is verified.

The *ilvC* gene is amplified from *E*. *coli* K12 genomic DNA by PCR using primer pair N112 and N113 (see Table 2) given as SEQ ID NOs:21 and 22, respectively, creating a 1.5 kbp product. The forward primer incorporates SalI and NheI restriction sites and a RBS. The reverse primer incorporates a XbaI restriction site. The PCR product is cloned into pCR4 Blunt-TOPO creating pCR4 Blunt-TOPO-ilvC. Plasmid DNA is prepared from the TOPO clones and the sequence of the gene is verified.

The *ilvD* gene is amplified from *E*. *coli* K12 genomic DNA by PCR using primer pair N114 and N115 (see Table 2) given as SEQ ID NOs:23 and 24, respectively, creating a 1.9 kbp product. The forward primer incorporates a XbaI restriction site and a RBS. The reverse primer incorporates a BamHI restriction site. The PCR product is cloned into pCR4 Blunt-TOPO creating pCR4 Blunt-TOPO-ilvD. Plasmid DNA is prepared from the TOPO clones and the sequence of the gene is verified.

The *kivD* gene is amplified from pUC57-kivD (described in Example 8) by PCR using primer pair N116 and N117 (see Table 2), given as SEQ ID NOs:25 and 26, respectively, creating a 1.7 bp product. The forward primer incorporates a BamHI restriction site and a RBS. The reverse primer incorporates a SacI restriction site. The PCR product is cloned into pCR4 Blunt-TOPO creating pCR4 Blunt-TOPO-kivD. Plasmid DNA is prepared from the TOPO clones and the sequence of the gene is verified.

The *yqhD* gene is amplified from *E*. *coli* K12 genomic DNA by PCR using primer pair N118 and N119 (see Table 2) given as SEQ ID NOs:27 and 28, respectively, creating a 1.2 kbp product. The forward primer incorporates a Sacl restriction site. The reverse primer incorporates Spel and EcoRI restriction sites. The PCR product is cloned into pCR4 Blunt-TOPO creating pCR4 Blunt-TOPO-yqhD. Plasmid DNA is prepared from the TOPO clones and the sequence of the gene is verified.

To construct the isobutanol pathway operon, the yqhD gene is excised from pCR4 Blunt-TOPO-*yqhD* with SacI and EcoRI, releasing a 1.2 kbp fragment. This is ligated with pUC19dHS, which has previously been digested with SacI and EcoRI. The resulting clone, pUC19dHS-yqhD, is confirmed by restriction digest. Next, the *ilvC* gene is excised from pCR4 Blunt-TOPO-ilvC with SalI and Xbal, releasing a 1.5 kbp fragment. This is ligated with pUC19dHS-yqhD, which has previously been digested with SalI and Xbal. The resulting clone, pUC19dHS-ilvC-yqhD, is confirmed by restriction digest. The *budB* gene is then excised from pCR4 Blunt-TOPO-*budB* with Sphl and Nsil, releasing a 1.8 kbp fragment. pUC19dHS-ilvC-yqhD is digested with Sphl and PstI and ligated with the SphI/NsiI *budB* fragment (Nsil and PstI generate compatible ends), forming pUC19dHS-budB-ilvC-yqhD. A 1.9 kbp fragment containing the *ilvD* gene is excised from pCR4 Blunt-TOPO-ilvD with Xbal and BamHI and ligated with pUC19dHS-budB-ilvC-yqhD, which is digested with these same enzymes, forming pUC19dHS-budB-ilvC-ilvD-yqhD. Finally, *kivD* is excised from pCR4 Blunt-TOPO-kivD with BamHI and SacI, releasing a 1.7 kbp fragment. This fragment is ligated with pUC19dHS-budB-ilvC-ilvD-yqhD, which has previously been digested with BamHI and Sacl, forming pUC19dHS-budB-ilvC-ilvD-kivD-yqhD.

The pUC19dHS-budB-ilvC-ilvD-kivD-yqhD vector is digested with AflII and Spel to release a 8.2 kbp operon fragment that is cloned into pBenAS, an *E.coli-B. subtilis* shuttle vector. Plasmid pBenAS is created by modification of the pBE93 vector, which is described by Nagarajan, (WO 93/24631, Example 4). To make pBenAS the *Bacillus amyloliquefaciens* neutral protease promoter (NPR), signal sequence, and the *phoA* gene are removed with a Ncol/Hindlll digest of pBE93. The NPR promoter is PCR amplified from pBE93 by primers BenNF and BenASR, given as SEQ ID NOS:29 and 30, respectively. Primer BenASR incorporates AflII, Spel, and HindIII sites downstream of the promoter. The PCR product is digested with Ncol and HindIII and the fragment is cloned into the corresponding sites in the vector creating pBenAS. The operon fragment is subcloned into the AfIII and Spel sites in pBenAS creating pBen-budB-ilvC-ilvD-kivD-yqhD.

### EXAMPLE 11 (Prophetic)

### Expression of the Isobutanol Biosynthetic Pathway in E. coli

The purpose of this prophetic Example is to describe how to express an isobutanol biosynthetic pathway in *E. coli.*

The plasmid pBen-budB-ilvC-ilvD-kivD-yqhD, constructed as described in Example 10, is transformed into *E. coli* NM522 (ATCC No. 47000) to give *E. coli* strain NM522/pBen-budB-ilvC-ilvD-kivD-yqhD and expression of the genes in the operon is monitored by SDS-PAGE analysis, enzyme assay and Western blot analysis. For Western blots, antibodies are raised to synthetic peptides by Sigma-Genosys (The Woodlands, TX).

*E. coli* strain NM522/pBen-budB-ilvC-ilvD-kivD-yqhD is inoculated into a 250 mL shake flask containing 50 mL of medium and shaken at 250 rpm and 35 °C. The medium is composed of: glucose (5 g/L), MOPS (0.05 M), ammonium sulfate (0.01 M), potassium phosphate, monobasic (0.005 M), S10 metal mix (1% (v/v)) yeast extract (0.1% (w/v)), casamino acids (0.1% (w/v)), thiamine (0.1 mg/L), proline (0.05 mg/L), and biotin (0.002 mg/L), and is titrated to pH 7.0 with KOH. S10 metal mix contains: MgCl₂ (200 mM), CaCl₂ (70 mM), MnCl₂ (5 mM), FeCl₃ (0.1 mM), ZnCl₂ (0.1 mM), thiamine hydrochloride (0.2 mM), CuSO₄ (172 µM), CoCl₂ (253 µM), and Na₂MoO₄ (242 µM). After 18 h, isobutanol is detected by HPLC or GC analysis, using methods that are well known in the art, for example, as described in the General Methods section above.

### EXAMPLE 12 (Prophetic)

### Expression of the Isobutanol Biosynthetic Pathway in Bacillus subtilis

The purpose of this prophetic Example is to describe how to express an isobutanol biosynthetic pathway in *Bacillus subtilis.* The same approach as described in Example 11 is used.

The plasmid pBen-budB-ilvC-ilvD-kivD-yqhD, constructed as described in Example 10, is used. This plasmid is transformed into *Bacillus subtilis* BE1010 (J. Bacteriol. 173:2278-2282 (1991)) to give B. *subtilis* strain BE1010/pBen-budB-ilvC-ilvD-kivD-yqhD and expression of the genes in each operon is monitored as described in Example 11.

*B. subtilis* strain BE1010/pBen-budB-ilvC-ilvD-kivD-yqhD is inoculated into a 250 mL shake flask containing 50 mL of medium and shaken at 250 rpm and 35 °C for 18 h. The medium is composed of: dextrose (5 g/L), MOPS (0.05 M), glutamic acid (0.02 M), ammonium sulfate (0.01 M), potassium phosphate, monobasic buffer (0.005 M), S10 metal mix (as described in Example 11, 1 % (v/v)), yeast extract (0.1 % (w/v)), casamino acids (0.1% (w/v)), tryptophan (50 mg/L), methionine (50 mg/L), and lysine (50 mg/L), and is titrated to pH 7.0 with KOH. After 18 h, isobutanol is detected by HPLC or GC analysis using methods that are well known in the art, for example, as described in the General Methods section above.

### EXAMPLE 13

### Cloning and Expression of Acetolactate Synthase

To create another acetolactate synthase expression clone, the *budB* gene was cloned into the vector pTrc99A. The *budB* gene was first amplified from pENTRSDD-TOPObudB (described in Example 5) using primers (N110.2 and N111.2, given as SEQ ID NOs:31 and 32, respectively) that introduced *Sac*I, *Spel* and *Mfe*l sites at the 5' end and *BbvC*I, *Afl*II*,* and *BamH*I sites at the 3' end. The resulting 1.75 kbp PCR product was cloned into pCR4-Blunt TOPO (Invitrogen) and the DNA sequence was confirmed (using N130Seq sequencing primers F1-F4 and R1-R4, given as SEQ ID NOs:40-47, respectively). The *budB* gene was then excised from this vector using *Sac*I and *BamH*I and cloned into pTrc99A (Amann et al. Gene 69(2):301-315 (1988)), generating pTrc99A::budB. The pTrc99A::budB vector was transformed into *E. coli* TOP10 cells and the transformants were inoculated into LB medium supplemented with 50 µg/mL of ampicillin and grown overnight at 37 °C. An aliquot of the overnight culture was used to inoculate 50 mL of LB medium supplemented with 50 µg/mL of ampicillin. The culture was incubated at 37 °C with shaking until the OD₆₀₀ reached 0.6 to 0.8. Expression of *budB* from the Trc promoter was then induced by the addition of 0.4 mM IPTG. Negative control flasks were also prepared that were not induced with IPTG. The flasks were incubated for 4 h at 37 °C with shaking. Cell-free extracts were prepared as described in Example 5.

Acetolactate synthase activity in the cell free extracts was measured as described in Example 5. Three hours after induction with IPTG, an acetolactate synthase activity of 8 units/mg was detected. The control strain carrying only the pTrc99A plasmid exhibited 0.03 units/mg of acetolactate synthase activity.

### EXAMPLE 14

### Cloning and Expression of Acetohydroxy Acid Reductoisomerase

The purpose of this Example was to clone the *ilvC* gene from *E. coli* K12 and express it in *E. coli* TOP10. The *ilvC* gene was amplified from *E. coli* K12 strain FM5 (ATCC 53911) genomic DNA using PCR.

The ilvC gene was cloned and expressed in a similar manner as described for the cloning and expression of *ilvC* in Example 6 above. PCR was used to amplify *ilvC* from the *E. coli* FM5 genome using primers N112.2 and N113.2 (SEQ ID NOs:33 and 34, respectively). The primers created *Sac*I and *Afll*II sites and an optimal RBS at the 5' end and *Not*l, *Nhe*I and *BamH*I sites at the 3' end of *ilvC.* The 1.5 kbp PCR product was cloned into pCR4Blunt TOPO according to the manufacturer's protocol (Invitrogen) generating pCR4Blunt TOPO::ilvC. The sequence of the PCR product was confirmed using sequencing primers (N131SeqF1-F3, and N131SeqR1-R3, given as SEQ ID NOs:48-53, respectively). To create an expression clone, the *ilvC* gene was excised from pCR4Blunt TOPO::ilvC using *Sac*I and *BamHI* and cloned into pTrc99A. The pTrc99A::ilvC vector was transformed into *E. coli* TOP*10* cells and expression from the Trc promoter was induced by addition of IPTG, as described in Example 13. Cell-free extracts were prepared as described in Example 5.

Acetohydroxy acid reductoisomerase activity in the cell free extracts was measured as described in Example 6. Three hours after induction with IPTG, an acetohydroxy acid reductoisomerase activity of 0.026 units/mg was detected. The control strain carrying only the pTrc99A plasmid exhibited less than 0.001 units/mg of acetohydroxy acid reductoisomerase activity.

### EXAMPLE 15

### Cloning and Expression of Acetohydroxy Acid Dehydratase

The purpose of this Example was to clone the *ilvD* gene from *E. coli* K12 and express it in *E. coli* Top10. The *ilvD* gene was amplified from *E. coli* K12 strain FM5 (ATCC 53911) genomic DNA using PCR.

The *ilvD* gene was cloned and expressed in a similar manner as the *ilvC* gene described in Example 14. PCR was used to amplify *ilvD* from the *E. coli* FM5 genome using primers N114.2 and N115.2 (SEQ ID NOs:35 and 36, respectively). The primers created *Sac*I and *Nhe*I sites and an optimal RBS at the 5' end and *Bsu36*I*,* PacI and *BamH*I sites at the 3' end of *ilvD.* The 1.9 kbp PCR product was cloned into pCR4Blunt TOPO according to the manufacturer's protocol (Invitrogen) generating pCR4Blunt TOPO::ilvD. The sequence of the PCR product was confirmed (sequencing primers N132SeqF1-F4 and N132SeqR1-R4, given as SEQ ID NOs:54-61, respectively). To create an expression clone, the *ilvD* gene was excised from plasmid pCR4Blunt TOPO::ilvD using *Sac*I and *BamH*I, and cloned into pTrc99A. The pTrc99A::ilvD vector was transformed into *E. coli* TOP10 cells and expression from the Trc promoter was induced by addition of IPTG, as described in Example 13. Cell-free extracts were prepared as described in Example 5.

Acetohydroxy acid dehydratase activity in the cell free extracts was measured as described in Example 7. Three hours after induction with IPTG, an acetohydroxy acid dehydratase activity of 46 units/mg was measured. The control strain carrying only the pTrc99A plasmid exhibited no detectable acetohydroxy acid dehydratase activity.

### EXAMPLE 16

### Cloning and Expression of Branched-Chain Keto Acid Decarboxylase

The purpose of this Example was to clone the *kivD* gene from *Lactococcus lactis* and express it in *E. coli TOP10.*

The *kivD* gene was cloned and expressed in a similar manner as that described for *ilvC* in Example 14 above. PCR was used to amplify *kivD* from the plasmid pUC57-kivD (see Example 8, above) using primers N116.2 and N117.2 (SEQ ID NOs:37 and 38, respectively). The primers created *Sac*I and *Pac*I sites and an optimal RBS at the 5' end and *Pci*l*, Avr*II, *Bgl*II and *BamH*I sites at the 3' end of *kivD.* The 1.7 kbp PCR product was cloned into pCR4Blunt TOPO according to the manufacturer's protocol (Invitrogen) generating pCR4Blunt TOPO::kivD. The sequence of the PCR product was confirmed using primers N133SeqF1-F4 and N133SeqR1-R4 (given as SEQ ID NOs:62-69, respectively). To create an expression clone, the *kivD* gene was excised from plasmid pCR4Blunt TOPO::kivD using *Sac*I and *BamH*I, and cloned into pTrc99A. The pTrc99A::kivD vector was transformed into *E. coli TOP10* cells and expression from the Trc promoter was induced by addition of IPTG, as described in Example 13. Cell-free extracts were prepared as described in Example 5.

Branched-chain keto acid decarboxylase activity in the cell free extracts was measured as described in Example 8, except that Purpald® reagent (Aldrich, Catalog No. 162892) was used to detect and quantify the aldehyde reaction products. Three hours after induction with IPTG, a branched-chain keto acid decarboxylase activity of greater than 3.7 units/mg was detected. The control strain carrying only the pTrc99A plasmid exhibited no detectable branched-chain keto acid decarboxylase activity.

### EXAMPLE 17

### Expression of Branched-Chain Alcohol Dehydrogenase

*E. coli* contains a native gene *(yqhD)* that was identified as a 1,3-propanediol dehydrogenase (U.S. Patent No. 6,514,733). The YqhD protein has 40% identity to AdhB (encoded by *adhB)* from *Clostridium,* a putative NADH-dependent butanol dehydrogenase. The *yqhD* gene was placed under the constitutive expression of a variant of the glucose isomerase promoter 1.6GI (SEQ ID NO. 70) in *E. coli* strain MG1655 1.6yqhD::Cm (WO 2004/033646) using λ Red technology (Datsenko and Wanner, Proc. Natl. Acad. Sci. U.S.A. 97:6640 (2000)). MG1655 1.6yqhD::Cm contains a FRT-CmR-FRT cassette so that the antibiotic marker can be removed. Similarly, the native promoter was replaced by the 1.5GI promoter (WO 2003/089621) (SEQ ID NO. 71), creating strain MG1655 1.5GI-yqhD::Cm, thus, replacing the 1.6GI promoter of MG1655 1.6yqhD::Cm with the 1.5GI promoter.

Strain MG1655 1.5GI-yqhD::Cm was grown in LB medium to mid-log phase and cell free extracts were prepared as described in Example 5. This strain was found to have NADPH-dependent isobutyraldehyde reductase activity when the cell extracts were assayed by following the decrease in absorbance at 340 nm at pH 7.5 and 35 °C.

To generate a second expression strain containing 1.5GI yqhD::Cm, a P1 lysate was prepared from MG1655 1.5GI yqhD::Cm and the cassette was transferred to BL21 (DE3) (Invitrogen) by transduction, creating BL21 (DE3) 1.5GI-yqhD::Cm.

### EXAMPLE 18

### Construction of a Transformation Vector for the First Four Genes in an Isobutanol Biosynthetic Pathway

The purpose of this Example was to construct a transformation vector comprising the first four genes (i.e., *budB, ilvC, ilvD* and *kivD*) in an isobutanol biosynthetic pathway.

To construct the transformation vector, first, the *ilvC* gene was obtained from pTrc99A::ilvC (described in Example 14) by digestion with *Afl*II and *BamH*I and cloned into pTrc99A::budB (described in Example 13), which was digested with *Afl*II and *BamH*I to produce plasmid pTrc99A::budB-ilvC. Next, the *ilvD* and *kivD* genes were obtained from pTrc99A::ilvD (described in Example 15) and pTrc99A::kivD (described in Example 16), respectively, by digestion with *Nhe*l and *Pac*l *(ilvD)* and *Pac*I and *BamH*I *(kivD).* These genes were introduced into pTrc99A::budB-ilvC,which was first digested with *Nhe*l and *BamH*I*,* by three-way ligation. The presence of all four genes in the final plasmid, pTrc99A::budB-ilvC-ilvD-kivD, was confirmed by PCR screening and restriction digestion.

### EXAMPLE 19

### Expression of an Isobutanol Biosynthetic Pathway in E. coli Grown on Glucose

To create E. coli isobutanol production strains, pTrc99A::budB-ilvC-ilvD-kivD (described in Example 18) was transformed into E. *coli MG1655* 1.5GI yqhD::Cm and E. *coli* BL21 (DE3) 1.5GI yqhD::Cm (described in Example 17). Transformants were initially grown in LB medium containing 50 µg/mL kanamycin and 100 µg/mL carbenicillin. The cells from these cultures were used to inoculate shake flasks (approximately 175 mL total volume) containing 50 or 170 mL of TM3a/glucose medium (with appropriate antibiotics) to represent high and low oxygen conditions, respectively. TM3a/glucose medium contained (per liter): glucose (10 g), KH₂PO₄ (13.6 g), citric acid monohydrate (2.0 g), (NH₄)₂SO₄ (3.0 g), MgSO₄·7H₂O (2.0 g), CaCl₂·2H₂O (0.2 g), ferric ammonium citrate (0.33 g), thiamine HCl (1.0 mg), yeast extract (0.50 g), and 10 mL of trace elements solution. The pH was adjusted to 6.8 with NH₄OH. The trace elements solution contained: citric acid H₂O (4.0 g/L), MnSO₄·H₂O (3.0 g/L), NaCl (1.0 g/L), FeSO₄·7H₂O (0.10 g/L), CoCl₂·6H₂O (0.10 g/L), ZnSO₄·7H₂O (0.10 g/L), CuSO₄·5H₂O (0.010 g/L), H₃BO₃ (0.010 g/L), and Na₂MoO₄. 2H₂O (0.010 g/L).

The flasks were inoculated at a starting OD₆₀₀ of ≤ 0.01 units and incubated at 34 °C with shaking at 300 rpm. The flasks containing 50 mL of medium were closed with 0.2 µm filter caps; the flasks containing 150 mL of medium were closed with sealed caps. IPTG was added to a final concentration of 0.04 mM when the cells reached an OD₆₀₀ of ≥ 0.4 units. Approximately 18 h after induction, an aliquot of the broth was analyzed by HPLC (Shodex Sugar SH1011 column (Showa Denko America, Inc. NY) with refractive index (RI) detection) and GC (Varian CP-WAX 58(FFAP) CB, 0.25 mm X 0.2 µm X 25 m (Varian, Inc., Palo Alto, CA) with flame ionization detection (FID)) for isobutanol content, as described in the General Methods section. No isobutanol was detected in control strains carrying only the pTrc99A vector (results not shown). Molar selectivities and titers of isobutanol produced by strains carrying pTrc99A::budB-ilvC-ilvD-kivD are shown in Table 13. Significantly higher titers of isobutanol were obtained in the cultures grown under low oxygen conditions.

**Table 13**

| Production of Isobutanol by *E. coli* Strains Grown on Glucose | | | |
|---|---|---|---|
| Strain | O₂ Conditions | Isobutanol mM* | Molar Selectivity (%) |
| MG1655 1.5GI yqhD/ pTrc99A:: bud B-ilvC-ilvD-kivD | High | 0.4 | 4.2 |
| MG1655 1.5GI yqhD/ pTrc99A::budB-ilvC-ilvD-kivD | Low | 9.9 | 39 |
| BL21 (DE3) 1.5GI yqhD/ pTrc99A::budB-ilvC-ilvD-kivD | High | 0.3 | 3.9 |
| BL21 (DE3) 1.5GI yqhD/ pTrc99A::budB-ilvC-ilvD-kivD | Low | 1.2 | 12 |

| | | | |
|---|---|---|---|
| *Determined by HPLC. | | | |

### EXAMPLE 20

### Expression of an Isobutanol Biosynthetic Pathway in E. coli Grown on Sucrose

Since the strains described in Example 19 were not capable of growth on sucrose, an additional plasmid was constructed to allow utilization of sucrose for isobutanol production. A sucrose utilization gene cluster *cscBKA*, given as SEQ ID NO:39, was isolated from genomic DNA of a sucrose-utilizing *E*. *coli* strain derived from ATCC strain 13281. The sucrose utilization genes (*cscA, cscK, and cscB*) encode a sucrose hydrolase (CscA), given as SEQ ID NO:139, D-fructokinase (CscK), given as SEQ ID NO:140, and sucrose permease (CscB), given as SEQ ID NO:141. The sucrose-specific repressor gene cscR was not included so that the three genes cscBKA were expressed constitutively from their native promoters in *E*. *coli.*

Genomic DNA from the sucrose-utilizing *E*. *coli* strain was digested to completion with *BamH*I and *EcoR*I*.* Fragments having an average size of about 4 kbp were isolated from an agarose gel and were ligated to plasmid pLitmus28 (New England Biolabs), digested with *BamH*I and *EcoR*I and transformed into ultracompetent *E. coli* TOP1 0F' cells (Invitrogen). The transformants were streaked onto MacConkey agar plates containing 1% sucrose and ampicillin (100 µg/mL) and screened for the appearance of purple colonies. Plasmid DNA was isolated from the purple transformants, and sequenced with M13 Forward and Reverse primers (Invitrogen), and Scr1-4 (given as SEQ ID NOs:72-75, respectively). The plasmid containing *cscB, cscK,* and *cscA* (*cscBKA*) genes was designated pScr1.

To create a sucrose utilization plasmid that was compatible with the isobutanol pathway plasmid (Example 18), the operon from pScr1 was subcloned into pBHR1 (MoBiTec, Goettingen, Germany). The *cscBKA* genes were isolated by digestion of pScr1 with *Xho*l (followed by incubation with Klenow enzyme to generate blunt ends) and then by digestion with *Age*l. The resulting 4.2 kbp fragment was ligated into pBHR1 that had been digested with *Nae*l and *Age*l, resulting in the 9.3 kbp plasmid pBHR1::cscBKA.

The sucrose plasmid pBHR1::cscBKA was transformed into *E. coli* BL21 (DE3) 1.5 yqhD /pTrc99A::budB-ilvC-ilvD-kivD and *E. coli* MG1655 1.5yqhD /pTrc99A::budB-ilvC-ilvD-kivD (described in Example 19) by electroporation. Transformants were first selected on LB medium containing 100 µg/mL ampicillin and 50 µg/mL kanamycin and then screened on MacConkey sucrose (1%) plates to confirm functional expression of the sucrose operon. For production of isobutanol, strains were grown in TM3a minimal defined medium (described in Example 19) containing 1% sucrose instead of glucose, and the culture medium was analyzed for the amount of isobutanol produced, as described in Example 19, except that samples were taken 14 h after induction. Again, no isobutanol was detected in control strains carrying only the pTrc99A vector (results not shown). Molar selectivities and titers of isobutanol produced by MG1655 1.5yqhD carrying pTrc99A::budB-ilvC-ilvD-kivD are shown in Table 14. Similar results were obtained with the analogous BL21 (DE3) strain.

**Table 14**

| Production of Isobutanol by *E. coli* strain MG1655 1.5yqhD /pTrc99A:: budB-ilvC-ilvD-kivD/pBHR1::cscBKA Grown on Sucrose | | | |
|---|---|---|---|
| O₂ Conditions | IPTG, mM | Isobutanol, mM* | Molar Selectivity, % |
| High | 0.04 | 0.17 | 2 |
| High | 0.4 | 1.59 | 21 |
| Low | 0.04 | 4.03 | 26 |
| Low | 0.4 | 3.95 | 29 |

| | | | |
|---|---|---|---|
| *Determined by HPLC. | | | |

### EXAMPLE 21

### Expression of Isobutanol Pathway Genes in Saccharomyces Cerevisiae

To express isobutanol pathway genes in *Saccharomyces cerevisiae,* a number of *E. coli*-yeast shuttle vectors were constructed. A PCR approach (Yu, et al. Fungal Genet. Biol. 41:973-981 (2004)) was used to fuse genes with yeast promoters and terminators. Specifically, the GPD promoter (SEQ ID NO:76) and CYC1 terminator (SEQ ID NO:77) were fused to the *alsS* gene from *Bacillus subtilis* (SEQ ID NO:78), the FBA promoter (SEQ ID NO:79) and CYC1 terminator were fused to the ILV5 gene from *S. cerevisiae* (SEQ ID NO:80), the ADH1 promoter (SEQ ID NO:81) and ADH1 terminator (SEQ ID NO:82) were fused to the ILV3 gene from *S. cerevisiae* (SEQ ID NO:83), and the GPM promoter (SEQ ID NO:84) and ADH1 terminator were fused to the *kivD* gene from *Lactococcus lactis* (SEQ ID NO:7). The primers, given in Table 15, were designed to include restriction sites for cloning promoter/gene/terminator products into *E. coli*-yeast shuttle vectors from the pRS400 series (Christianson et al. Gene 110:119-122 (1992)) and for exchanging promoters between constructs. Primers for the 5' ends of ILV5 and ILV3 (N138 and N155, respectively, given as SEQ ID NOs: 95 and 107, respectively) generated new start codons to eliminate mitochondrial targeting of these enzymes.

All fused PCR products were first cloned into pCR4-Blunt by TOPO cloning reaction (Invitrogen) and the sequences were confirmed (using M13 forward and reverse primers (Invitrogen) and the sequencing primers provided in Table 15. Two additional promoters (CUP1 and GAL1) were cloned by TOPO reaction into pCR4-Blunt and confirmed by sequencing; primer sequences are indicated in Table 15. The plasmids that were constructed are described in Table 16. The plasmids were transformed into either *Saccharomyces cerevisiae* BY4743 (ATCC 201390) or YJR148w (ATCC 4036939) to assess enzyme specific activities using the enzyme assays described in Examples 5-8 and Examples 13-16. For the determination of enzyme activities, cultures were grown to an OD₆₀₀ of 1.0 in synthetic complete medium *(*Methods in Yeast Genetics, 2005, Cold Spring Harbor Laboratory Press, Cold Spring Harbor, NY, pp. 201-202) lacking any metabolite(s) necessary for selection of the expression plasmid(s), harvested by centrifugation (2600 x g for 8 min at 4 °C), washed with buffer, centrifuged again, and frozen at -80 °C. The cells were thawed, resuspended in 20 mM Tris-HCl, pH 8.0 to a final volume of 2 mL, and then disrupted using a bead beater with 1.2 g of glass beads (0.5 mm size). Each sample was processed on high speed for 3 minutes total (with incubation on ice after each minute of beating). Extracts were cleared of cell debris by centrifugation (20,000 x g for 10 min at 4 °C).

**Table 15**

| Primer Sequences for Cloning and Sequencing of *S. cerevisiae* Expression Vectors | | | |
|---|---|---|---|
| Name | Sequence | Description | SEQ ID NO: |
| N98SeqF1 | | *B. subtilis alsS* sequencing primer | 85 |
| N98SeqF2 | | B. subtilis alsS sequencing primer | 86 |
| N98SeqF3 | | B. subtilis alsS sequencing primer | 87 |
| N98SeqF4 | | B. subtilis alsS sequencing primer | 88 |
| N99SeqR1 | | B. subtilis alsS sequencing primer | 89 |
| N99SeqR2 | | B. subtilis alsS sequencing primer | 90 |
| N99SeqR3 | | B. subtilis alsS sequencing primer | 91 |
| N99SeqR4 | | B. subtilis alsS sequencing primer | 92 |
| N136 | | FBA promoter forward primer with SacII/BglII sites | 93 |
| N137 | | FBA promoter reverse primer with BbvCl site and ILV5-annealing region | 94 |
| N138 | | ILV5 forward primer (creates alternate start cod on) | 95 |
| N139 | | ILV5 reverse primer | 96 |
| N140 | | CYC terminator forward primer with PacI site and ILV5-annealing region | 97 |
| N141 | | CYC terminator reverse primer with Notl site | 98 |
| N142 | | GPM promoter forward primer with BamHI site | 99 |
| N143 | | GPM promoter reverse primer with BbvCI site and kivD-annealing region | 100 |
| N144 | | kivD forward primer | 101 |
| N145 | | kivD reverse primer | 102 |
| N146 | | ADH terminator forward primer with PacI site and kivD-annealing region | 103 |
| N147 | | ADH terminator reverse primer with Spel site | 104 |
| N151 | | alsS reverse primer | 105 |
| N152 | | CYC terminator forward primer with Pacl site and alsS-annealing region | 106 |
| N155 | | ILV3 forward primer (alternate start codon) | 107 |
| N156 | | ILV3 reverse primer | 108 |
| N157 | | ADH terminator | 109 |
| | | forward primer with PacI site and ILV3-annealing region | |
| N158 | | ADH promoter forward primer with BamHI site | 110 |
| N159 | | ADH promoter reverse primer with BbvCI site and ILV3-annealing region | 111 |
| N160SeqF1 | GAAAACGTGGCATCCTCTC | FBA::ILV5::CYC sequencing primer | 112 |
| N160SeqF2 | | FBA::ILV5::CYC sequencing primer | 113 |
| N160SeqF3 | | FBA::ILV5::CYC sequencing primer | 114 |
| N160SeqF4 | | FBA::ILV5::CYC sequencing primer | 115 |
| N160SeqF5 | | FBA::ILV5::CYC sequencing primer | 116 |
| N160SeqR1 | | FBA::ILV5::CYC sequencing primer | 117 |
| N160SeqR2 | | FBA::ILV5::CYC sequencing primer | 118 |
| N160SeqR3 | | FBA::ILV5::CYC sequencing primer | 119 |
| N160SeqR4 | | FBA::ILV5::CYC sequencing primer | 120 |
| N160SeqR5 | | FBA::ILV5::CYC sequencing primer | 121 |
| N161SeqF1 | ACGGTATACGGCCTTCCTT | ADH::ILV3::ADH sequencing primer | 122 |
| N161SeqF2 | | ADH::ILV3::ADH sequencing primer | 123 |
| N161SeqF3 | | ADH::ILV3::ADH sequencing primer | 124 |
| N161SeqF4 | | ADH::ILV3::ADH sequencing primer | 125 |
| N161SeqF5 | | ADH::ILV3::ADH sequencing primer | 126 |
| N161SeqF6 | | ADH::ILV3::ADH sequencing primer | 127 |
| N161SeqR1 | | ADH::ILV3::ADH sequencing primer | 128 |
| N161SeqR2 | CCAAACCGTAACCCATCAG | ADH::ILV3::ADH sequencing primer | 129 |
| N161SeqR3 | CACAGATTGGGTACCACCA | ADH::ILV3::ADH sequencing primer | 130 |
| N161SeqR4 | | ADH::ILV3::ADH sequencing primer | 131 |
| N161SeqR5 | CATAGCTTTCAAACCCGCT | ADH::ILV3::ADH sequencing primer | 132 |
| N161SeqR6 | | ADH::ILV3::ADH sequencing primer | 133 |
| N162 | | *alsS* forward primer | 134 |
| N189 | | GPD forward primer with *Sac*lI/*Bgl*II sites | 135 |
| N190.1 | | GPD promoter reverse primer with *BbvC*l site and *alsS*-annealing region | 136 |
| N176 | | GAL1 promoter forward primer with *Sac*lI/*Bgl*II sites | 137 |
| N177 | | GAL1 promoter reverse with *BbvC*l site | 138 |
| N191 | | CUP1 promoter forward primer with SacII/BglII sites | 175 |
| N192 | | CUP1 promoter reverse with BbvCI site | 176 |

**Table 16**

| *E. coli-*Yeast Shuttle Vectors Carrying Isobutanol Pathway Genes | |
|---|---|
| Plasmid Name | Construction |
| pRS426 [ATCC No. 77107], URA3 selection | - |
| pRS426::GPD::alsS::CYC | GPD::alsS::CYC PCR product digested with *Sac*II/*Not*I cloned into pRS426 digested with same |
| pRS426::FBA::ILV5::CYC | FBA::ILV5::CYC PCR product digested with *Sac*II/*Not*I cloned into pRS426 digested with same |
| pRS425 [ATCC No. 77106], LEU2 selection | - |
| pRS425::ADH::ILV3::ADH | ADH::ILV3::ADH PCR product digested with *BamH*I/*Spe*I cloned into pRS425 digested with same |
| pRS425::GPM::kivD::ADH | GPM::kivD::ADH PCR product digested with *BamH*I/*Spe*I cloned into pRS425 digested with same |
| pRS426::CUP1::alsS | 7.7 kbp *Sac*II/*Bbv*CI fragment from pRS426::GPD::alsS::CYC ligated with SacII/BbvCI CUP1 fragment |
| pRS426::GAL1::ILV5 | 7 kbp SacII/BbvCI fragment from pRS426::FBA::ILV5::CYC ligated with SacII/BbvCI GAL1 fragment |
| pRS425::FBA::ILV3 | 8.9 kbp *BamH*I/*Bbv*Cl fragment from pRS425::ADH::ILV3::ADH ligated with 0.65 kbp |
| | *Bgl*II/*Bbv*Cl FBA fragment from pRS426::FBA::ILV5::CYC |
| pRS425::CUP1-alsS+FBA-ILV3 | 2.4 kbp *Sac*II/*Not*I fragment from pRS426::CUP1::alsS cloned into pRS425::FBA::ILV3 cut with *Sac*Il/*Not*I |
| pRS426::FBA-ILV5+GPM-kivD | 2.7 kbp *Bam*HI/Spel fragment from pRS425::GPM::kivD::ADH cloned into pRS426::FBA::ILV5::CYC cut with *BamH*I/*Spe*I |
| pRS426::GAL1-FBA+GPM-kivD | 8.5 kbp *Sac*Il/*Not*I fragment from pRS426:: FBA-ILV5+GPM-kivD ligated with 1.8 kbp *Sac*Il/*Not*I fragment from pRS426::GAL1::ILV5 |
| pRS423 [ATCC No. 77104], HIS3 selection | - |
| pRS423::CUP1-alsS+FBA-ILV3 | 5.2 kbp *Sac*I/*Sal*I fragment from pRS425::CUP1-alsS+FBA-ILV3 ligated into pRS423 cut with *Sac*I/*Sal*I |
| pHR81 [ATCC No. 87541], URA3 and leu2-d selection | - |
| pHR81::FBA-ILV5+GPM-kivD | 4.7 kbp *Sac*I/*Bam*HI fragment from pRS426::FBA-ILV5+GPM-kivD ligated into pHR81 cut with *Sac*I/*Bam*HI |

### EXAMPLE 22

### Production of Isobutanol by Recombinant Saccharomyces Cerevisiae

Plasmids pRS423::CUP1-alsS+FBA-ILV3 and pHR81::FBA-ILV5+GPM-kivD (described in Example 21) were transformed into *Saccharomyces cerevisiae* YJR148w to produce strain YJR148w/pRS423::CUP1-alsS+FBA-ILV3/ pHR81::FBA-ILV5+ GPM-kivD. A control strain was prepared by transforming vectors pRS423 and pHR81 (described in Example 21) into *Saccharomyces cerevisiae* YJR148w (strain YJR148w/pRS423/pHR81). Strains were maintained on standard *S. cerevisiae* synthetic complete medium (Methods in Yeast Genetics, 2005, Cold Spring Harbor Laboratory Press, Cold Spring Harbor, NY, pp. 201-202) containing either 2% glucose or sucrose but lacking uracil and histidine to ensure maintenance of plasmids.

For isobutanol production, cells were transferred to synthetic complete medium lacking uracil, histidine and leucine. Removal of leucine from the medium was intended to trigger an increase in copy number of the pHR81-based plasmid due to poor transcription of the leu2-d allele (Erhart and Hollenberg, J. Bacteriol. 156:625-635 (1983)). Aerobic cultures were grown in 175 mL capacity flasks containing 50 mL of medium in an Innova4000 incubator (New Brunswick Scientific, Edison, NJ) at 30 °C and 200 rpm. Low oxygen cultures were prepared by adding 45 mL of medium to 60 mL serum vials that were sealed with crimped caps after inoculation and kept at 30 °C. Sterile syringes were used for sampling and addition of inducer, as needed. Approximately 24 h after inoculation, the inducer CuSO₄ was added to a final concentration of 0.03 mM. Control cultures for each strain without CuSO₄ addition were also prepared. Culture supernatants were analyzed 18 or 19 h and 35 h after CuSO₄ addition by both GC and HPLC for isobutanol content, as described above in Example 19. The results for *S*. *cerevisiae* YJR148w/pRS423::CUP1-alsS+FBA-ILV3/pHR81::FBA-ILV5+GPM-kivD grown on glucose are presented in Table 17. For the results given in Table 17, the samples from the aerobic cultures were taken at 35 h and the samples from the low oxygen cultures were taken at 19 h and measured by HPLC.

The results for *S*. *cerevisiae* YJR148w/pRS423::CUP1-alsS+FBA-ILV3/pHR81::FBA-ILV5+GPM-kivD grown on sucrose are presented in Table 18. The results in this table were obtained with samples taken at 18 h and measured by HPLC.

**Table 17**

| Production of Isobutanol by *S*. *cerevisiae* YJR148w/pRS423::CUP1-alsS+FBA-ILV3/pHR81::FBA-ILV5+GPM-kivD Grown on Glucose | | | |
|---|---|---|---|
| Strain | O₂ level | Isobutanol, mM | Molar Selectivity % |
| YJR148w/pRS423/pHR81 (control) | Aerobic | 0.12 | 0.04 |
| YJR148w/pRS423/pHR81 (control) | Aerobic | 0.11 | 0.04 |
| YJR148w/pRS423::CUP1-alsS+FBA- | Aerobic | 0.97 | 0.34 |
| ILV3/ pHR81::FBA-ILV5+ GPM-kivD a | | | |
| YJR148w/pRS423::CUP1-alsS+FBA-ILV3/ pHR81::FBA-ILV5+ GPM-kivD b | Aerobic | 0.93 | 0.33 |
| YJR148w/pRS423::CU P1-alsS+FBA-ILV3/ pHR81::FBA-ILV5+ GPM-kivD c | Aerobic | 0.85 | 0.30 |
| YJR148w/pRS423/pHR81 (control) | Low | 0.11 | 0.1 |
| YJR148w/pRS423/pHR81 (control) | Low | 0.08 | 0.1 |
| YJR148w/pRS423::CUP1-alsS+FBA-ILV3/ pHR81::FBA-ILV5+ GPM-kivD a | Low | 0.28 | 0.5 |
| YJR148w/pRS423::CUP1-alsS+FBA-ILV3/ pHR81::FBA-ILV5+ GPM-kivD b | Low | 0.20 | 0.3 |
| YJR148w/pRS423::CUP1-alsS+FBA-ILV3/ pHR81::FBA-ILV5+ GPM-kivD c | Low | 0.33 | 0.6 |

**Table 18**

| Production of Isobutanol by *S*. *cerevisiae* YJR148w/pRS423::CUP1-alsS+FBA-ILV3/pHR81::FBA-ILV5+GPM-kivD Grown on Sucrose | | | |
|---|---|---|---|
| Strain | O₂ Level | Isobutanol mM | Molar Selectivity, % |
| YJR148w/pRS423/pHR81 (control) | Aerobic | 0.32 | 0.6 |
| YJR148w/pRS423/pHR81 (control) | Aerobic | 0.17 | 0.3 |
| YJR148w/pRS423::CUP1-alsS+FBA-ILV3/ pHR81::FBA-ILV5+ GPM-kivD a | Aerobic | 0.68 | 1.7 |
| YJR148w/pRS423::CUP1-alsS+FBA-ILV3/ pHR81::FBA-ILV5+ GPM-kivD b | Aerobic | 0.54 | 1.2 |
| YJR148w/pRS423::CUP1-alsS+FBA-ILV3/ pHR81::FBA-ILV5+ GPM-kivD c | Aerobic | 0.92 | 2.0 |
| YJR148w/pRS423/pHR81 (control) | Low | 0.18 | 0.3 |
| YJR148w/pRS423/pHR81 (control) | Low | 0.15 | 0.3 |
| YJR148w/pRS423::CUP1-alsS+FBA-ILV3/ pHR81::FBA-ILV5+ GPM-kivD a | Low | 0.27 | 1.2 |
| YJR148w/pRS423::CUP1-alsS+FBA-ILV3/ pHR81::FBA-ILV5+ GPM-kivD b | Low | 0.30 | 1.1 |
| YJR148w/pRS423::CUP1-alsS+FBA-ILV3/ pHR81::FBA-ILV5+ GPM-kivD c | Low | 0.21 | 0.8 |

| | | | |
|---|---|---|---|
| Strain suffixes "a", "b", and "c" indicate separate isolates. | | | |

The results indicate that, when grown on glucose or sucrose under both aerobic and low oxygen conditions, strain YJR148w/pRS423::CUP1-alsS+FBA-ILV3/ pHR81::FBA-ILV5+ GPM-kivD produced consistently higher levels of isobutanol than the control strain.

### EXAMPLE 23

### Production of Isobutanol by Recombinant Saccharomyces Cerevisiae

Plasmids pRS425::CUP1-alsS+FBA-ILV3 and pRS426::GAL1-ILV5+GPM-kivD (described in Example 21) were transformed into *Saccharomyces cerevisiae* YJR148w to produce strain YJR148w/pRS425::CUP1-alsS+ FBA-ILV3/pRS426::GAL1-ILV5+ GPM-kivD. A control strain was prepared by transforming vectors pRS425 and pRS426 (described in Example 21) into *Saccharomyces cerevisiae* YJR148w (strain YJR148w/pRS425/pRS426). Strains were maintained on synthetic complete medium, as described in Example 22.

For isobutanol production, cells were transferred to synthetic complete medium containing 2% galactose and 1% raffinose, and lacking uracil and leucine. Aerobic and low oxygen cultures were prepared as described in Example 22. Approximately 12 h after inoculation, the inducer CuSO₄ was added up to a final concentration of 0.5 mM. Control cultures for each strain without CuSO₄ addition were also prepared. Culture supernatants were sampled 23 h after CuSO₄ addition for determination of isobutanol by HPLC, as described in Example 22. The results are presented in Table 19. Due to the widely different final optical densities observed and associated with quantifying the residual carbon source, the concentration of isobutanol per OD₆₀₀ unit (instead of molar selectivities) is provided in the table to allow comparison of strains containing the isobutanol biosynthetic pathway genes with the controls.

**Table 19**

| Production of Isobutanol by *S*. *cerevisiae* YJR148w/pRS425::CUP1-alsS+FBA-ILV3/pRS426::GAL1-ILV5+GPM-kivD Grown on Galactose and Raffinose | | | | |
|---|---|---|---|---|
| Strain | O₂ level | CuSO₄, mM | Isobutanol mM | mM Isobutanol per OD unit |
| YJR148w/pRS425/pRS426 (control) | Aerobic | 0.1 | 0.12 | 0.01 |
| YJR148w/pRS425/pRS426 (control) | Aerobic | 0.5 | 0.13 | 0.01 |
| YJR148w/pRS425::CUP1-alsS+ FBA-ILV3/pRS426::GALl-ILV5+ GPM-kivD a | Aerobic | 0 | 0.20 | 0.03 |
| YJR148w/pRS425::CUP1-alsS+ FBA-ILV3/pRS426::GALl-ILV5+ GPM-kivD b | Aerobic | 0.03 | 0.82 | 0.09 |
| YJR148w/pRS425::CUP1-alsS+ FBA-ILV3/pRS426::GAL1-ILV5+ GPM-kivD c | Aerobic | 0.1 | 0.81 | 0.09 |
| YJR148w/pRS425::CUP1-alsS+ FBA-ILV3/pRS426::GAL1-ILV5+ GPM-kivD d | Aerobic | 0.5 | 0.16 | 0.04 |
| YJR148w/pRS425::CUP1-alsS+ FBA-ILV3/pRS426::GAL1-ILV5+ GPM-kivD e | Aerobic | 0.5 | 0.18 | 0.01 |
| YJR148w/pRS425/pRS426 (control) | Low | 0.1 | 0.042 | 0.007 |
| YJR148w/pRS425/pRS426 (control) | Low | 0.5 | 0.023 | 0.006 |
| YJR148w/pRS425::CUP1-alsS+ FBA-ILV3/pRS426::GAL1-ILV5+ GPM-kivD a | Low | 0 | 0.1 | 0.04 |
| YJR148w/pRS425::CUP1-alsS+ | Low | 0.03 | 0.024 | 0.02 |
| FBA-ILV3/pRS426::GAL1-ILV5+ GPM-kivD b | | | | |
| YJR148w/pRS425::CUP1-alsS+ FBA-ILV3/pRS426::GAL1-ILV5+ GPM-kivD c | Low | 0.1 | 0.030 | 0.04 |
| YJR148w/pRS425::CUP1-alsS+ FBA-ILV3/pRS426::GAL1-ILV5+ GPM-kivD d | Low | 0.5 | 0.008 | 0.02 |
| YJR148w/pRS425::CUP1-alsS+ FBA-ILV3/pRS426::GALl-ILV5+ GPM-kivD e | Low | 0.5 | 0.008 | 0.004 |

| | | | | |
|---|---|---|---|---|
| Strain suffixes "a", "b", "c", "d" and "e" indicate separate isolates. | | | | |

The results indicate that in general, higher levels of isobutanol per optical density unit were produced by the YJR148w/pRS425::CUP1-alsS+ FBA-ILV3/pRS426::GAL1-ILV5+ GPM-kivD strain compared to the control strain under both aerobic and low oxygen conditions.

### EXAMPLE 24

### Expression of an Isobutanol Biosynthetic Pathway in Bacillus subtilis

The purpose of this Example was to express an isobutanol biosynthetic pathway in *Bacillus subtilis.* The five genes of the isobutanol pathway (pathway steps (a) through (e) in Figure 1) were split into two operons for expression. The three genes *budB, ilvD,* and *kivD,* encoding acetolactate synthase, acetohydroxy acid dehydratase, and branched-chain keto acid decarboxylase, respectively, were integrated into the chromosome of *B*. *subtilis* BE1010 (Payne and Jackson, J. Bacteriol. 173:2278-2282 (1991)). The two genes *ilvC* and *bdhB,* encoding acetohydroxy acid isomeroreductase and butanol dehydrogenase, respectively, were cloned into an expression vector and transformed into the *Bacillus* strain carrying the integrated isobutanol genes.

Integration of the three genes, *budB, ilvD* and *kivD* into the chromosome of *B*. *subtilis* BE1010. *Bacillus* integration vectors pFP988DssPspac and pFP988DssPgroE were used for the chromosomal integration of the three genes, *budB* (SEQ ID NO:1), *ilvD* (SEQ ID NO:5), and *kivD* (SEQ ID NO:7). Both plasmids contain an *E*. *coli* replicon from pBR322, an ampicillin antibiotic marker for selection in *E*. *coli* and two sections of homology to the *sacB* gene in the *Bacillus* chromosome that direct integration of the vector and intervening sequence by homologous recombination. Between the *sacB* homology regions is a spac promoter (PgroE) on pFP988DssPspac or a groEL promoter (PgroE) on pFP988DssPgroE, and a selectable marker for *Bacillus,* erythromycin. The promoter region also contains the *lacO* sequence for regulation of expression by a *lacI* repressor protein. The sequences of pFP988DssPspac (6,341 bp) and pFP988DssPgroE (6,221 bp) are given as SEQ ID NO:142 and SEQ ID NO:143 respectively.

The cassette with three genes *budB-ilvD-kivD* was constructed by deleting the *ilvC* gene from plasmid pTrc99a budB-ilvC-ilvD-kivD. The construction of the plasmid pTrc99A::budB-ilvC-ilvD-kivD is described in Example 18. Plasmid pTrc99A::budB-ilvC-ilvD-kivD was digested with AflII and Nhel, treated with the Klenow fragment of DNA polymerase to make blunt ends, and the resulting 9.4 kbp fragment containing pTrc99a vector, *budB, ilvD,* and *kivD* was gel-purified. The 9.4 kbp vector fragment was self-ligated to create pTrc99A::budB-ilvD-kivD, and transformed into DH5α competent cells (Invitrogen). A clone of pTrc99a budB-ilvD-kivD was confirmed for the *ilvC* gene deletion by restriction mapping. The resulting plasmid pTrc99A::budB-ilvD-kivD was digested with Sacl and treated with the Klenow fragment of DNA polymerase to make blunt ends. The plasmid was then digested with BamHI and the resulting 5,297 bp *budB-ilvD-kivD* fragment was gel-purified. The 5,297 bp *budB-ilvD-kivD* fragment was ligated into the Smal and BamHI sites of the integration vector pFP988DssPspac. The ligation mixture was transformed into DH5α competent cells. Transformants were screened by PCR amplification of the 5.3 kbp *budB-ilvD-kivD* fragment with primers T-budB(BamHI) (SEQ ID NO:144) and B-kivD(BamHI) (SEQ ID NO:145). The correct clone was named pFP988DssPspac-budB-ilvD-kivD.

Plasmid pFP988DssPspac-budB-ilvD-kivD was prepared from the *E. coli* transformant, and transformed into *B. subtilis* BE1010 competent cells, which had been prepared as described by Doyle et al. (J. Bacteriol. 144:957 (1980)). Competent cells were harvested by centrifugation and the cell pellets were resuspended in a small volume of the supernatant. To one volume of competent cells, two volumes of SPII-EGTA medium (Methods for General and Molecular Bacteriology, P. Gerhardt et al., Ed., American Society for Microbiology, Washington, DC (1994)) was added. Aliquots (0.3 mL) of cells were dispensed into test tubes and then 2 to 3 µg of plasmid pFP988DssPspac-budB-ilvD-kivD was added to the tubes. The tubes were incubated for 30 min at 37 °C with shaking, after which 0.1 mL of 10% yeast extract was added to each tube and they were further incubated for 60 min. Transformants were grown for selection on LB plates containing erythromycin (1.0 µg/mL) using the double agar overlay method (*Methods for General and Molecular Bacteriology, supra*). Transformants were screened by PCR amplification with primers N130SeqF1 (SEQ ID NO:40) and N130SeqR1 (SEQ ID NO:44) for *budB,* and N133SeqF1 (SEQ ID NO:62) and N133SeqR1 (SEQ ID NO:66) for *kivD.* Positive integrants showed the expected 1.7 kbp *budB* and 1.7 kbp *kivD* PCR products. Two positive integrants were identified and named *B*. *subtilis* BE1010 ΔsacB::Pspac-budB-ilvD-kivD #2-3-2 and *B. subtilis* BE1010 ΔsacB::Pspac-budB-ilvD-kivD #6-12-7.

Assay of the enzyme activities in integrants *B. subtilis* BE1010 ΔsacB::Pspac-budB-ilvD-kivD #2-3-2 and *B. subtilis* BE1010 ΔsacB::Pspac-budB-ilvD-kivD #6-12-7 indicated that the activities of BudB, IIvD and KivD were low under the control of the spac promoter (Pspac). To improve expression of functional enzymes, the Pspac promoter was replaced by a PgroE promoter from plasmid pHT01 (MoBitec, Goettingen, Germany).

A 6,039 bp pFP988Dss vector fragment, given as SEQ ID NO:146, was excised from an unrelated plasmid by restriction digestion with Xhol and BamHI, and was gel-purified. The PgroE promoter was PCR-amplified from plasmid pHT01 with primers T-groE(Xhol) (SEQ ID NO:147) and B-groEL(Spel,BamH1) (SEQ ID NO:148). The PCR product was digested with Xhol and BamHI, ligated with the 6,039 bp pFP988Dss vector fragment, and transformed into DH5α competent cells. Transformants were screened by PCR amplification with primers T-groE(Xhol) and B-groEL(Spel,BamH1). Positive clones showed the expected 174 bp PgroE PCR product and were named pFP988DssPgroE. The plasmid pFP988DssPgroE was also confirmed by DNA sequence.

Plasmid pFP988DssPspac-budB-ilvD-kivD was digested with Spel and Pmel and the resulting 5,313 bp budB-ilvD-kivD fragment was gel-purified. The budB-ilvD-kivD fragment was ligated into Spel and Pmel sites of pFP988DssPgroE and transformed into DH5α competent cells. Positive clones were screened for a 1,690 bp PCR product by PCR amplification with primers T-groEL (SEQ ID NO:149) and N111 (SEQ ID NO:20). The positive clone was named pFP988DssPgroE-budB-ilvD-kivD.

Plasmid pFP988DssPgroE-budB-ilvD-kivD was prepared from the *E*. *coli* transformant, and transformed into *Bacillus subtilis* BE1010 competent cells as described above. Transformants were screened by PCR amplification with primers N130SeqF1 (SEQ ID NO:40) and N130SeqR1 (SEQ ID NO:44) for *budB,* and N133SeqF1 (SEQ ID NO:62) and N133SeqR1 (SEQ ID NO:66) for *kivD.* Positive integrants showed the expected 1.7 kbp *budB* and 1.7 kbp *kivD* PCR products. Two positive integrants were isolated and named *B. subtilis* BE1010 ΔsacB::PgroE-budB-ilvD-kivD #1-7 and *B. subtilis* BE1010 ΔsacB::PgroE-budB-ilvD-kivD #8-16.

Plasmid Expression of *ilvC* and *bdhB* genes. Two remaining isobutanol genes, *ilvC* and *bdhB,* were expressed from a plasmid. Plasmid pHT01 (MoBitec), a *Bacillus-E. coli* shuttle vector, was used to fuse an *ilvC* gene from *B. subtilis* to a PgroE promoter so that the *ilvC* gene was expressed from the PgroE promoter containing a *lacO* sequence. The *ilvc* gene, given as SEQ ID NO:186, was PCR-amplified from *B. subtilis* BR151 (ATCC 33677) genomic DNA with primers T-ilvCB.s.(BamHI) (SEQ ID NO:150) and B-ilvCB.s.(Spel BamHI) (SEQ ID NO:151). The 1,067 bp *ilvC* PCR product was digested with BamHI and ligated into the BamHI site of pHT01. The ligation mixture was transformed into DH5α competent cells. Positive clones were screened for a 1,188 bp PCR product by PCR amplification with primers T-groEL and B-ilvB.s.(Spel BamHI). The positive clone was named pHT01-ilvC(B.s). Plasmid pHT01-ilvC(B.s) was used as a template for PCR amplification of the PgroE-*ilvC* fused fragment.

Plasmid pBD64 (Minton et al., Nucleic Acids Res. 18:1651(1990)) is a fairly stable vector for expression of foreign genes in *B*. *subtilis* and contains a *repB* gene and chloramphenicol and kanamycin resistance genes for selection in *B. subtilis.* This plasmid was used for expression of *ilvC* and *bdhB* under the control of a PgroE promoter. To clone PgroE-*ilvC*, *bdhB* and a *lacI* repressor gene into plasmid pBD64, a one-step assembly method was used (Tsuge et al., Nucleic Acids Res. 31:e133 (2003)). A 3,588 bp pBD64 fragment containing a *repB* gene, which included the replication function, and the kanamycin antibiotic marker was PCR-amplified from pBD64 with primers T-BD64(DraIII) (SEQ ID NO:152), which introduced a DraIII sequence (CACCGAGTG), and B-BD64(DraIII) (SEQ ID NO:153), which introduced a DraIII sequence (CACCTGGTG). A 1,327 bp *lacI* repressor gene was PCR-amplified from pMUTIN4 (Vagner et al., Microbiol. 144:3097-3104 (1998)) with T-laclq(DraIII) (SEQ ID NO:154), which introduced a DraIII sequence (CACCAGGTG) and B-laclq(Dralll) (SEQ ID NO:155), which introduced a Dralll sequence (CACGGGGTG). A 1,224 bp PgroE-*ilvC* fused cassette was PCR-amplified from pHT01-ilvC(B.s) with T-groE(DraIII) (SEQ ID NO:156), which introduced a DraIII sequence (CACCCCGTG), and B-B.s.ilvC(DraIII) (SEQ ID NO:157), which introduced a DraIII sequence (CACCGTGTG). A 1.2 kbp *bdhB* gene (SEQ ID NO:158) was PCR-amplified from *Clostridium acetobutylicum* (ATCC 824) genomic DNA with primers T-bdhB(DraIII) (SEQ ID NO:159), which introduced a DraIII sequence (CACACGGTG), and B-bdhB(rrnBT1DraIII) (SEQ ID NO:160), which introduced a DraIII sequence (CACTCGGTG). The three underlined letters in the variable region of the DraIII recognition sequences were designed for specific base-pairing to assemble the four fragments with an order of pBD64-*lacI*-PgroE*ilvC*-*bdhB*. Each PCR product with DraIII sites at both ends was digested separately with DraIII, and the resulting DraIII fragments, 3,588 bp pBD64, *lacI*, PgroE*ilvC*, and *bdhB* were gel-purified using a QIAGEN gel extraction kit (QIAGEN). A mixture containing an equimolar concentration of each fragment with a total DNA concentration of 30 to 50 µg /100 µL was prepared for ligation. The ligation solution was then incubated at 16 °C overnight. The ligation generated high molecular weight tandem repeat DNA. The ligated long, linear DNA mixture was directly transformed into competent *B*. *subtilis* BE1010, prepared as described above. *B. subtilis* preferentially takes up long repeated linear DNA forms, rather than circular DNA to establish a plasmid. After transformation the culture was spread onto an LB plate containing 10 µg/ml of kanamycin for selection. Positive recombinant plasmids were screened by DraIII digestion, giving four fragments with an expected size of 3,588 bp (pBD64), 1,327 bp (*lacI*), 1,224 bp (PgorE-*ilvC*), and 1,194 bp (*bdhB*). The positive plasmid was named pBDPgroE-ilvC(B.s.)-bdhB.

Demonstration of isobutanol production from glucose or sucrose by *B. subtilis* BE1010 ΔsacB::PgroE-budB-ilvD-kivD/pBDPgroE-ilvC(B.s.)-bdhB. To construct the recombinant *B*. *subtilis* expressing the five genes of the isobutanol biosynthetic pathway, competent cells of the two integrants *B. subtilis* BE1010 ΔsacB-PgroE-budB-ilvD-kivD #1-7 and *B*. *subtilis* BE1010 ΔsacB::PgroE-budB-ilvD-kivD #8-16 were prepared as described above, and transformed with plasmid pBDPgroE-ilvC(B.s.)-bdhB, yielding *B. subtilis* BE1010 ΔsacB::PgroE-budB-ilvD-kivD #1-7/pBDPgroE-ilvC(B.s.)-bdhB and *B*. *subtilis* BE1010 ΔsacB::PgroE-budB-ilvD-kivD #8-16/pBDPgroE-ilvC(B.s.)-bdhB.

The two recombinant strains were inoculated in either 25 mL or 100 mL of glucose medium containing kanamycin (10 µg /mL) in 125 mL flasks to simulate high and low oxygen conditions, respectively, and aerobically grown at 37 °C with shaking at 200 rpm. The medium consisted of 10 mM (NH₄)₂SO₄, 5 mM potassium phosphate buffer (pH 7.0), 100 mM MOPS/KOH buffer (pH 7.0), 20 mM glutamic acid/KOH (pH 7.0), 2% S10 metal mix, 1% glucose, 0.01 % yeast extract, 0.01 % casamino acids, and 50 µg/mL each of L-tryptophan, L-methionine, and L-lysine. The S10 metal mix consisted of 200 mM MgCl₂, 70 mM CaCl₂, 5 mM MnCl₂, 0.1 mM FeCl₃, 0.1 mM ZnCl₂, 0.2 mM thiamine hydrochloride, 0.172 mM CuSO₄, 0.253 mM CoCl₂, and 0.242 mM Na₂MoO₄. The cells were induced with 1.0 mM isopropyl-β-D-thiogalactopyranoiside (IPTG) at early-log phase (OD₆₀₀ of approximately 0.2). At 24 h after inoculation, an aliquot of the broth was analyzed by HPLC (Shodex Sugar SH1011 column) with refractive index (RI) detection for isobutanol content, as described in the General Methods section. The HPLC results are shown in Table 20.

**Table 20**

| Production of Isobutanol from Glucose by *B*. *subtilis* BE1010 ΔsacB::PgroE-budB-ilvD-kivD/pBDPgroE-ilvC(B.s.)-bdhB Strains | | | |
|---|---|---|---|
| Strain | O₂ Level | isobutanol, mM | molar selectivity, % |
| *B. subtilis* a (induced) | high | 1.00 | 1.8 |
| *B. subtilis* b (induced) | high | 0.87 | 1.6 |
| *B. subtilis* a (induced) | low | 0.06 | 0.1 |
| *B. subtilis* b (induced) | low | 0.14 | 0.3 |

| | | | |
|---|---|---|---|
| *B. subtilis* a is *B*. *subtilis* BE1010 ΔsacB::PgroE-budB-ilvD-kivD #1-7/pBDPgroE-ilvC(B.s.)-bdhB *B. subtilis* b is *B. subtilis* BE1010 ΔsacB::PgroE-budB-ilvD-kivD #8-16/pBDPgroE-ilvC(B.s.)-bdhB | | | |

The isolate of *B. subtilis* BE1010 ΔsacB::PgroE-budB-ilvD-kivD #1-7/pBDPgroE-ilvC(B.s.)-bdhB was also examined for isobutanol production from sucrose, essentially as described above. The recombinant strain was inoculated in 25 mL or 75 mL of sucrose medium containing kanamycin (10 µg /mL) in 125 mL flasks to simulate high and medium oxygen levels, and grown at 37 °C with shaking at 200 rpm. The sucrose medium was identical to the glucose medium except that glucose (10 g/L) was replaced with 10 g/L of sucrose. The cells were uninduced, or induced with 1.0 mM isopropyl-p-D-thiogalactopyranoiside (IPTG) at early-log phase (OD₆₀₀ of approximately 0.2). At 24 h after inoculation, an aliquot of the broth was analyzed by HPLC (Shodex Sugar SH1011 column) with refractive index (RI) detection for isobutanol content, as described in the General Methods section. The HPLC results are given in Table 21.

**Table 21**

| Production of Isobutanol from Sucrose by *B. subtilis* Strain BE1010 ΔsacB::PgroE-budB-ilvD-kivD/pBDPgroE-ilvC(B.s.)-bdhB | | | |
|---|---|---|---|
| Strain | O₂ Level | isobutanol, mM | molar selectivity, % |
| *B. subtilis* a (uninduced) | high | Not detected | Not detected |
| *B. subtilis* a (induced) | high | 0.44 | 4.9 |
| *B. subtilis* a (induced) | medium | 0.83 | 8.6 |

| | | | |
|---|---|---|---|
| *B. subtilis* a is *B*. *subtilis* BE1010 ΔsacB::PgroE-budB-ilvD-kivD #1-7/pBDPgroE-ilvC(B.s.)-bdhB | | | |

### EXAMPLE 25 (Prophetic)

### Expression of an Isobutanol Biosynthetic Pathway in Lactobacillus plantarum

The purpose of this prophetic Example is to describe how to express an isobutanol biosynthetic pathway in *Lactobacillus plantarum.* The five genes of the isobutanol pathway, encoding five enzyme activities, are divided into two operons for expression. The *budB, ilvD* and *kivD* genes, encoding the enzymes acetolactate synthase, acetohydroxy acid dehydratase, and branched-chain α-keto acid decarboxylase, respectively, are integrated into the chromosome of *Lactobacillus plantarum* by homologous recombination using the method described by Hols et al. (*Appl. Environ. Microbiol.* 60:1401-1413 (1994)). The remaining two genes (*ilvC* and *bdhB,* encoding the enzymes acetohydroxy acid reductoisomerase and butanol dehydrogenase, respectively) are cloned into an expression plasmid and transformed into the *Lactobacillus* strain carrying the integrated isobutanol genes. *Lactobacillus plantarum* is grown in MRS medium (Difco Laboratories, Detroit, MI) at 37 °C, and chromosomal DNA is isolated as described by Moreira et al. (*BMC Microbiol.* 5:15 (2005)).

Integration. The *budB-ilvD-kivD* cassette under the control of the synthetic P11 promoter (Rud et al., *Microbiology* 152:1011-1019 (2006)) is integrated into the chromosome of *Lactobacillus plantarum* ATCC BAA-793 (NCIMB 8826) at the *IdhL1* locus by homologous recombination. To build the *IdhL* integration targeting vector, a DNA fragment from *Lactobacillus plantarum* (Genbank NC_004567) with homology to *IdhL* is PCR amplified with primers LDH EcoRV F (SEQ ID NO:161) and LDH AatIIR (SEQ ID NO:162). The 1986 bp PCR fragment is cloned into pCR4Blunt-TOPO and sequenced. The pCR4Blunt-TOPO-IdhL1 clone is digested with EcoRV and Aatll releasing a 1982 bp IdhL1 fragment that is gel-purified. The integration vector pFP988, given as SEQ ID NO:177, is digested with HindIII and treated with Klenow DNA polymerase to blunt the ends. The linearized plasmid is then digested with Aatll and the 2931 bp vector fragment is gel purified. The EcoRV/AatII IdhL1 fragment is ligated with the pFP988 vector fragment and transformed into *E*. *coli* Top10 cells. Transformants are selected on LB agar plates containing ampicillin (100 µg/mL) and are screened by colony PCR to confirm construction of pFP988-IdhL.

To add a selectable marker to the integrating DNA, the Cm gene with its promoter is PCR amplified from pC194 (GenBank NC_002013, SEQ ID NO:267) with primers Cm F (SEQ ID NO:163) and Cm R (SEQ ID NO:164), amplifying a 836 bp PCR product. This PCR product is cloned into pCR4Blunt-TOPO and transformed into *E*. *coli* Top10 cells, creating pCR4Blunt-TOPO-Cm. After sequencing to confirm that no errors are introduced by PCR, the Cm cassette is digested from pCR4Blunt-TOPO-Cm as an 828 bp Mlul/Swal fragment and is gel purified. The IdhL-homology containing integration vector pFP988-IdhL is digested with Mlul and Swal and the 4740 bp vector fragment is gel purified. The Cm cassette fragment is ligated with the pFP988-IdhL vector creating pFP988-DldhL::Cm.

Finally the *budB-ilvD-kivD* cassette from pFP988DssPspac-budB-ilvD-kivD, described in Example 24, is modified to replace the amylase promoter with the synthetic P11 promoter. Then, the whole operon is moved into pFP988-DIdhL::Cm. The P11 promoter is built by oligonucleotide annealing with primer P11 F-Stul (SEQ ID NO:165) and P11 R-Spel (SEQ ID NO:166). The annealed oligonucleotide is gel-purified on a 6% Ultra PAGE gel (Embi Tec, San Diego, CA). The plasmid pFP988DssPspac-budB-ilvD-kivD, containing the amylase promoter, is digested with Stul and Spel and the resulting 10.9 kbp vector fragment is gel-purified. The isolated P11 fragment is ligated with the digested pFP988DssPspac-budB-ilvD-kivD to create pFP988-P11-budB-ilvD-kivD. Plasmid pFP988-P11-budB-ilvD-kivD is then digested with Stul and BamHI and the resulting 5.4 kbp P11-budB-ilvD-kivD fragment is gel-purified. pFP988-DIdhL::Cm is digested with Hpal and BamHI and the 5.5 kbp vector fragment isolated. The budB-ilvD-kivD operon is ligated with the integration vector pFP988-DldhL::Cm to create pFP988-DldhL-P11-budB-ilvD-kivD::Cm.

Integration of pFP988-DldhL-P11-budB-ilvD-kivD::Cm into *L*. *plantarum BAA-793* to form *L. plantarum* ΔldhL1::budB-ilvD-kivD::Cm comprising exogenous *budB, ilvD,* and *kivD* genes. Electrocompetent cells of *L. plantarum* are prepared as described by Aukrust, T.W., et al. (In: Electroporation Protocols for Microorganisms; Nickoloff, J.A., Ed.; Methods in Molecular Biology, Vol. 47; Humana Press, Inc., Totowa, NJ, 1995, pp 201-208). After electroporation, cells are outgrown in MRSSM medium (MRS medium supplemented with 0.5 M sucrose and 0.1 M MgCl₂) as described by Aukrust et al. *supra* for 2 h at 37 °C without shaking. Electroporated cells are plated for selection on MRS plates containing chloramphenicol (10 µg/mL) and incubated at 37 °C. Transformants are initially screened by colony PCR amplification to confirm integration, and initial positive clones are then more rigorously screened by PCR amplification with a battery of primers.

Plasmid Expression of *ilvC* and *bdhB* genes. The remaining two isobutanol genes are expressed from plasmid pTRKH3 (O'Sullivan DJ and Klaenhammer TR, Gene 137:227-231 (1993)) under the control of the *L*. *plantarum* IdhL promoter (Ferain et al., J. Bacteriol. 176:596-601 (1994)). The IdhL promoter is PCR amplified from the genome of *L. plantarum* ATCC BAA-793 using primers PldhL F-HindIII (SEQ ID NO:167) and PldhL R-BamHI (SEQ ID NO:168). The 411 bp PCR product is cloned into pCR4Blunt-TOPO and sequenced. The resulting plasmid, pCR4Blunt-TOPO-PldhL is digested with Hindlll and BamHI releasing the PldhL fragment.

Plasmid pTRKH3 is digested with HindIII and Sphl and the gel-purified vector fragment is ligated with the PldhL fragment and the gel-purified 2.4 kbp BamHI/SphI fragment containing ilvC(B.s.)-bdhB from the *Bacillus* expression plasmid pBDPgroE-ilvC(B.s.)-bdhB (Example 24) in a three-way ligation. The ligation mixture is transformed into *E*. *coli* Top 10 cells and transformants are grown on Brain Heart Infusion (BHI, Difco Laboratories, Detroit, MI) plates containing erythromycin (150 mg/L). Transformants are screened by PCR to confirm construction. The resulting expression plasmid, pTRKH3-ilvC(B.s.)-bdhB is transformed into *L. plantarum* ΔldhL1::budB-ilvD-kivD::Cm by electroporation, as described above.

*L. plantarum* ΔldhL1::budB-ilvD-kivD::Cm containing pTRKH3-ilvC(B.s.)-bdhB is inoculated into a 250 mL shake flask containing 50 mL of MRS medium plus erythromycin (10 µg/mL) and grown at 37 °C for 18 to 24 h without shaking, after which isobutanol is detected by HPLC or GC analysis, as described in the General Methods section.

### EXAMPLE 26 (prophetic)

### Expression of an Isobutanol Biosynthetic Pathway in Enterococcus faecalis

The purpose of this prophetic Example is to describe how to express an isobutanol biosynthetic pathway in *Enterococcus faecalis.* The complete genome sequence of *Enterococcus faecalis* strain V583, which is used as the host strain for the expression of the isobutanol biosynthetic pathway in this Example, has been published (Paulsen et al., Science 299:2071-2074 (2003)). An *E. coli*/Gram-positive shuttle vector, Plasmid pTRKH3 (O'Sullivan DJ and Klaenhammer TR, Gene 137:227-231 (1993)), is used for expression of the five genes (*budB*, *ilvC*, *ilvD*, *kivD*, *bdhB*) of the isobutanol pathway in one operon. pTRKH3 contains an *E*. *coli* plasmid p15A replication origin, the pAMβ1 replicon, and two antibiotic resistance selection markers for tetracycline and erythromycin. Tetracycline resistance is only expressed in *E*. *coli,* and erythromycin resistance is expressed in both *E. coli* and Gram-positive bacteria. Plasmid pAMβ1 derivatives can replicate in *E*. *faecalis* (Poyart et al., FEMS Microbiol. Lett. 156:193-198 (1997)). The inducible nisA promoter (PnisA), which has been used for efficient control of gene expression by nisin in a variety of Gram-positive bacteria including *Enterococcus faecalis* (Eichenbaum et al., Appl. Environ. Microbiol. 64:2763-2769 (1998)), is used to control expression of the five desired genes encoding the enzymes of the isobutanol biosynthetic pathway.

The plasmid pTrc99A::budB-ilvC-ilvD-kivD (described in Example 18), which contains the isobutanol pathway operon, is modified to replace the *E*. *coli ilvC* gene (SEQ ID NO:3) with the *B. subtilis ilvC* gene (SEQ ID NO:184). Additionally, the *bdhB* gene(SEQ ID NO:158) from *Clostridium acetobutylicum* is added to the end of the operon. First, the *bdhB* gene from pBDPgroE-ilvC(B.s.)-bdhB (described in Example 24) is amplified using primers F-bdhB-Avrll (SEQ ID NO:169) and R-bdhB-BamHI (SEQ ID NO:170), and then TOPO cloned and sequenced. The 1194 bp *bdhB* fragment is isolated by digestion with AvrII and BamHI, followed by gel purification. This *bdhB* fragment is ligated with pTrc99A::budB-ilvC-ilvD-kivD that has previously been digested with AvrII and BamHI and the resulting fragment is gel purified. The ligation mixture is transformed into *E*. *coli Top10* cells by electroporation and transformants are selected following overnight growth at 37 °C on LB agar plates containing ampicillin (100 µg/mL). The transformants are then screened by colony PCR to confirm the correct clone containing pTrc99A::budB-ilvC-ilvD-kivD-bdhB.

Next, *ilvC*(B.s.) is amplified from pBDPgroE-ilvC(B.s.)-bdhB (described in Example 24) using primers F-ilvC(B.s.)-AfIII (SEQ ID NO:171) and R-ilvC(B.s.)-NotI (SEQ ID NO:172). The PCR product is TOPO cloned and sequenced. The 1051 bp *ilvC*(*B.s*.) fragment is isolated by digestion with AfIII and NotI followed by gel purification. This fragment is ligated with pTrc99A::budB-ilvC-ilvD-kivD-bdhB that has been cut with AfIII and Notl to release the *E*. *coli ilvC* (the 10.7 kbp vector band is gel purified prior to ligation with *ilvC*(*B.s*.)). The ligation mixture is transformed into *E*. *coli* Top10 cells by electroporation and transformants are selected following overnight growth at 37 °C on LB agar plates containing ampicillin (100 µg/mL). The transformants are then screened by colony PCR to confirm the correct clone containing pTrc99A::budB-ilvC(B.s.)-ilvD-kivD-bdhB.

To provide a promoter for the *E*. *colil* Gram-positive shuttle vector pTRKH3, the *nisA* promoter (Chandrapati et al., Mol. Microbiol. 46(2):467-477 (2002)) is PCR-amplified from *Lactococcus lactis* genomic DNA with primers F-PnisA(HindIII) (SEQ ID NO:173) and R-PnisA(Spel BamHI) (SEQ ID NO:174) and then TOPO cloned. After sequencing, the 213 bp *nisA* promoter fragment is isolated by digestion with HindIII and BamHI followed by gel purification. Plasmid pTRKH3 is digested with HindIII and BamHI and the vector fragment is gel-purified. The linearized pTRKH3 is ligated with the PnisA fragment and transformed into *E. coli* Top10 cells by electroporation. Transformants are selected following overnight growth at 37 °C on LB agar plates containing erythromycin (25 µg/mL). The transformants are then screened by colony PCR to confirm the correct clone of pTRKH3-PnisA.

Plasmid pTRKH3-PnisA is digested with Spel and BamHI, and the vector is gel-purified. Plasmid pTrc99A::budB-ilvC(B.s)-ilvD-kivD-bdhB, described above, is digested with Spel and BamHI, and the 7.5 kbp fragment is gel-purified. The 7.5 kbp *budB-ilvC*(B.s)*-ilvD-kivD-bdhB* fragment is ligated into the pTRKH3-PnisA vector at the Spel and BamHI sites. The ligation mixture is transformed into *E*. *coli* Top10 cells by electroporation and transformants are selected following overnight growth on LB agar plates containing erythromycin (25 µg/mL) at 37 °C. The transformants are then screened by colony PCR. The resulting plasmid is named pTRKH3-PnisA-budB-ilvC(B.s)-ilvD-kivD-bdhB. This plasmid is prepared from the *E*. *coli* transformants and transformed into electrocompetent *E*. *faecalis* V583 cells by electroporation using methods known in the art (Aukrust, T.W., et al. In: Electroporation Protocols for Microorganisms; Nickoloff, J.A., Ed.; Methods in Molecular Biology, Vol. 47; Humana Press, Inc., Totowa, NJ., 1995, pp 217-226), resulting in E. *faecalis* V583/ pTRKH3-PnisA-budB-ilvC(B.s)-ilvD-kivD-bdhB.

The second plasmid containing *nisA* regulatory genes, *nisR* and *nisK,* the *add9* spectinomycin resistance gene, and the pSH71 origin of replication is transformed into *E*. *faecalis* V583/ pTRKH3-PnisA-budB-ilvC(B.s)-ilvD-kivD-bdhB by electroporation. The plasmid containing pSH71 origin of replication is compatible with pAMβ1 derivatives in *E*. *faecalis* (Eichenbaum et al., *supra*). Double drug resistant transformants are selected on LB agar plates containing erythromycin (25 µg/mL) and spectinomycin (100 µg/mL), grown at 37 °C.

The resulting *E*. *faecalis* strain V583B harboring two plasmids, i.e., an expression plasmid (pTRKH3-PnisA-budB-ilvC(B.s)-ilvD-kivD-bdhB) and a regulatory plasmid (pSH71-nisRK), is inoculated into a 250 mL shake flask containing 50 mL of Todd-Hewitt broth supplemented with yeast extract (0.2%) (Fischetti et al., J. Exp. Med. 161:1384-1401 (1985)), nisin (20 µg/mL) (Eichenbaum et al., *supra*), erythromycin (25 µg/mL), and spectinomycin (100 µg/mL). The flask is incubated without shaking at 37 °C for 18-24 h, after which time, isobutanol production is measured by HPLC or GC analysis, as described in the General Methods section.

### EXAMPLE 27

### Increased tolerance of Saccharomyces cerevisiae to isobutanol at decreased growth temperatures

Tolerance levels were determined for yeast strain *Saccharomyces cerevisiae* BY4741 (ATCC 201388) at 25 °C and 30 °C as follows. The strain was cultured in YPD medium. Overnight cultures in the absence of any test compound were started in 25 mL of YPD medium in 150 mL flasks with incubation at 30 °C or at 25 °C in shaking water baths. The next morning, each overnight culture was diluted into a 500 mL flask containing 300 mL of fresh medium to an initial OD600 of about 0.1. The flasks were incubated in shaking water baths at 30 °C or 25 °, using the same temperature as used for each overnight culture. The large cultures were incubated for 3 hours and then were split into flasks in the absence (control) and in the presence of 2% or 3% of isobutanol. Growth was followed by measuring OD₆₀₀ for six hours after addition of the isobutanol. The ΔOD₆₀₀ was calculated by subtracting the initial OD₆₀₀ from the final OD₆₀₀ at 6 hours. The percent growth inhibition relative to the control culture was calculated as follows: % Growth Inhibition = 100 - [100(Sample ΔOD₆₀₀/Control ΔOD₆₀₀)]. The results are summarized in Table 22 below and indicate that growth of strain BY4741 is less inhibited by 2% isobutanol at 25 °C than by 2% isobutanol at 30 °C.

**Table 22. Growth of Saccharomyces cerevisiae Strain BY4741 at 25 °C and 30 °C with Isobutanol.**

| % Isobutanol | Temperature | % Growth Inhibition |
|---|---|---|
| 2 | 30 | 99 |
| 2 | 25 | 84 |
| 3 | 30 | No growth |
| 3 | 25 | No growth |

## Claims

1. A method for the production of isobutanol comprising:
a) providing a recombinant microbial production host which produces isobutanol, wherein said recombinant microbial host comprises DNA molecules encoding polypeptides that catalyse each of the following substrate to product conversions:
i) pyruvate to acetolactate;
ii) acetolactate to 2,3-dihydroxyisovalerate;
iii) 2,3-dihydroxyisovalerate to α-ketoisovalerate;
iv) α-ketoisovalerate to isobutyraldehyde; and
v) isobutyraldehyde to isobutanol;
wherein at least one of said DNA molecules is heterologous to said recombinant microbial host;
b) seeding the production host of (a) into a fermentation medium comprising a fermentable carbon substrate to create a fermentation culture;
c) growing the production host in the fermentation culture at a first temperature for a first period of time, wherein the first temperature is from about 25°C to about 40 °C;
d) lowering the temperature of the fermentation culture to a second temperature; and
e) incubating the production host at the second temperature of step (d) for a second period of time;
whereby isobutanol is produced.

2. The method according to Claim 1 wherein the fermentable carbon substrate is derived from a grain or sugar source selected from the group consisting of wheat, corn, barley, oats, rye, sugar cane, sugar beets, cassava, sweet sorghum, and mixtures thereof.

3. The method according to Claim 1 wherein the fermentable carbon substrate is derived from cellulosic or lignocellulosic biomass selected from the group consisting of corn cobs, crop residues, corn husks, corn stover, grasses, wheat straw, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers, animal manure, and mixtures thereof.

4. The method according to Claim 1 wherein the fermentable carbon substrate is selected from the group consisting of monosaccharides, oligosaccharides, and polysaccharides.

5. The method according to any one of Claims 1-4 wherein the fermentation culture is maintained under conditions selected from the group consisting of anaerobic conditions and microaerobic conditions.

6. The method according to any one of Claims 1-5 wherein while growing the production host in (c) at a first temperature over a first period of time, a metabolic parameter of the fermentation culture is monitored.

7. The method according to Claim 6 wherein the metabolic parameter that is monitored is selected from the group consisting of optical density, pH, respiratory quotient, fermentable carbon substrate utilization, CO₂ production, and isobutanol production.

8. The method according to any one of Claims 1-7 wherein lowering the temperature of the fermentation culture of step (d) occurs at a predetermined time.

9. The method according to any one of Claims 1-7 wherein the lowering of the temperature of the fermentation culture of step (d) coincides with a change in a metabolic parameter.

10. The method according to Claim 9 wherein the change in metabolic parameter is a decrease in the rate of isobutanol production.

11. The method according to any one of Claims 1-10 wherein the second temperature is from about 3 °C to about 25 °C lower than the first temperature.

12. The method according to any one of Claims 1-11 wherein steps (d) and (e) are repeated one or more times.

13. The method according to any one of Claims 1-12 wherein the recombinant microbial production host is selected from the group consisting of *Clostridium, Zymomonas, Escherichia, Salmonela, Rhodococcus, Pseudomonas, Bacillus, Lactobacillus, Enterococcus, Alcaligenes, Klebsiela, Paenibacillus, Arthrobacter, Corynebacterium, Brevibacterium, Saccharomyces,* and *Pichia.*

14. The method according to any one of Claims 1-13 wherein the polypeptide that catalyzes a substrate to product conversion of pyruvate to acetolactate is acetolactate synthase and the acetolactate synthase has an amino acid sequence having at least 95% identity to an amino acid sequence selected from the group consisting of SEQ ID NO:2, SEQ ID NO:178, and SEQ ID NO:180, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of acetolactate to 2,3-dihydroxyisovalerate is acetohydroxy acid isomeroreductase
and the acetohydroxy acid isomeroreductase has an amino acid sequence having at least 95% identity to an amino acid sequence selected from the group consisting of SEQ ID NO:4, SEQ ID NO:181, SEQ ID NO:183, and SEQ ID NO:185, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of 2, 3-dihydroxyisovalerate to α-ketoisovalerate is acetohydroxy acid dehydratase and the acetohydroxy acid dehydratase has an amino acid sequence having at least 95% identity to an amino acid sequence selected from the group consisting of SEQ ID NO:6, SEQ ID NO:186, SEQ ID NO:188, and SEQ ID NO:190, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of α-ketoisovalerate to isobutyraldehyde is branched-chain α-keto acid decarboxylase and the branched-chain α-keto acid decarboxylase has an amino acid sequence having at least 95% identity to an amino acid sequence selected from the group consisting of SEQ ID NO:8, SEQ ID NO:193, SEQ ID NO:195, and SEQ ID NO:197, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of isobutyraldehyde to isobutanol is branched-chain alcohol dehydrogenase and the branched-chain alcohol dehydrogenase has an amino acid sequence having at least 95% identity to an amino acid sequence selected from the group consisting of SEQ ID NO:10, SEQ ID NO:199, SEQ ID NO:201, SEQ ID NO:203, and SEQ ID NO:204, based on the Clustal W method of alignment using the default parameters of GAP PENALTY=10, GAP LENGTH PENALTY=0.1, and Gonnet 250 series of protein weight matrix.

## Patentansprüche

1. Verfahren zur Verstellung von Isobutanol, wobei das Verfahren aufweist:
a) Bereitstellen eines rekombinanten mikrobiellen Produktionswirts, der Isobutanol erzeugt, wobei der rekombinante mikrobielle Wirt DNA-Molelküle aufweist, die für Polypeptide codieren, die jede der folgenden Substrat-Produkt-Umwandlungen katalysieren:
i) Pyruvat in Acetolacetat;
ii) Acetolacetat in 2,3-Dihydroxyisovalerat;
iii) 2,3-Dihydroxyisovalerat in α-Ketoisovalerat;
iv) α-Ketoisovalerat in Isobutyraldehyd; und
v) Isobutyraldehyd in Isobutanol;
wobei mindestens eines der DNA-Moleküle zu dem rekombinanter mikrobiellen Wirt heterolog ist;
b) Einimpfen des Produktionswirts gemäß (a) in ein Fermentationsmedium, das ein fermentierbares Kohlenstoffsubstrat aufweist, um eine Fermentationskultur zu erzeugen;
c) Vermehren des Produktionswirts in der Permentationskultur bei einer ersten Temperatur während einer ersten Zeitspanne, wobei die Temperatur etwa 25°C bis etwa 40°C beträgt;
d) Absenken der Temperatur der Fermentationskultur auf eine zweite Temperatur; und
e) Inkubieren des Produktionswirts bei der zweiten Temperatur gemäß Schritt (d) während einer zweiten Zeitspanne;
wodurch Isobutanol produziert wird.

2. Verfahren nach Anspruch 1, wobei das fermentierbare Kohlenstoffsubstrat aus einer Getreide- oder Zuckerquelle gewonnen wird, die aus der Gruppe ausgewählt ist, die aus Weizen, Mais, Gerste, Hafer, Roggen, Zuckerrohr, Zuckerrüben, Maniok, süßem Sorghum und Gemischen davon besteht.

3. Verfahren nach Anspruch 1, wobei das fermentierbare Kohlenstoffsubstrat aus cellulose- oder lignocellulosehaltiger Biomasse gewonnen wird, die aus der Gruppe ausgewählt ist, die aus Maiskolben, Ernterückständen, Maishülsen, Maisstroh, Gräsern, Weizenstroh, Gerstestroh, Heu, Reisstroh, Hirse, Papierabfall, Zuckerrohrbagasse, Sorghum, Sojabohnen, aus dem Mahlen von Getreiden gewonnenen Bestandteilen, Bäumen, Ästen bzw. Zweigen, Wurzeln, Blättern, Holzschnitzeln, Sägemehl, Sträuchem und Büschen, Gemüse, Obst, Blumen, tierischem Dünger und Gemischen davon besteht.

4. Verfahren nach Anspruch 1, wobei das fermentierbare Kohlenstoffsubstrat aus der Gruppe ausgewählt ist, die aus Monosacchariden, Oligosacchariden und Polysacchariden besteht.

5. Verfahren nach einem der Ansprüche 1-4, wobei die Fermentationskultur unter Bedingungen gehalten wird, die aus der Gruppe ausgewählt sind, die aus anaeroben Bedingungen und mikroaeroben Bedingungen besteht.

6. Verfahren nach einem der Ansprüche 1-5, wobei während der Vermehrung des Produktionswirts in (c) bei einer ersten Temperatur während einer ersten Zeitspanne ein Stoffwechselparameter der Fermentationskultur überwacht wird.

7. Verfahren nach Anspruch 6, wobei der überwachte Stoffwechselparameter aus der Gruppe ausgewählt ist, die aus optischer Dichte, pH-Wert, respiratorischem Quotient, Verwertung des fermentierbaren Kohlenstoffsubstrats, CO₂-Produktion und Isobutanol-Produktion besteht.

8. Verfahren nach einem der Ansprüche 1-7, wobei die Temperaturabsenkung der Fermentationskultur gemäß Schritt (d) zu einem vorgegebenen Zeitpunkt erfolgt.

9. Verfahren nach einem der Ansprüche 1-7, wobei die Temperaturabsenkung der Fermentationskultur gemäß Schritt (d) mit einer Veränderung eines Stoffwechselparameters zusammenfällt.

10. Verfahren nach Anspruch 9, wobei die Veränderung des Stoffwechselparameters eine Abnahme der Produktionsgeschwindigkeit von Isobutanol ist.

11. Verfahren nach einem der Ansprüche 1-10, wobei die zweite Temperatur um etwa 3°C bis etwa 25°C niedriger ist als die erste Temperatur.

12. Verfahren nach einem der Ansprüche 1-11, wobei die Schritte (d) und (e) einmal oder mehrmals wiederholt werden.

13. Verfahren nach einem der Ansprüche 1-12, wobei der rekombinante mikrobielle Produktionswirt
aus der Gruppe ausgewählt ist, die aus *Clostridium, Zymomonas. Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Saccharomyces* und *Pichia* besteht.

14. Verfahren nach einem der Ansprüche 1-13, wobei das Polypeptid, das eine Substrat-Produkt-Umwandlung von Pyruvat in Acetolacetat katalysiert, Acetolacetatsynthase ist und die Acetolacetatsynthase eine Aminosäuresequenz mit einer Identität von mindestens 95% zu einer Aminosäuresequenz aufweist, die aus der Gruppe ausgewählt ist, die aus SEQ ID Nr.: 2, SEQ ID Nr.: 178 und SEQ ID Nr.: 180 besteht, basierend auf der Clustal W-Alignment-Methode unter Verwendung der Standardparameter GAP PENALTY=10 und GAP LENGTH PENALTY=0,1 und der Proteingewichtsmatrix-Serie Gonnet 250; oder
wobei das Polypeptid, das eine Substrat-Produkt-Umwandlung von Acetolacetat in 2,3-Dihydroxyisovalerat katalysiert, Hydroxyessigsäure-Isomeroreduktase ist und die Hydroxyessigsäure-Isomervreduktase eine Aminosäuresequenz mit einer Identität von mindestens 95% zu einer Aminosäuresequenz aufweist, die aus der Gruppe ausgewählt ist, die aus SEQ ID Nr.: 4, SEQ ID Nr.: 181, SEQ ID Nr.: 183 und SEQ ID Nr.: 185 besteht, basierend auf der Clustal W-Alignment-Methode unter Verwendung der Standardpararneter GAP PENALTY=10 und GAP LENGTH PENALTY=0,1 und der Proteingewichtsmatrix-Serie Gonnet 250; oder
wobei das Polypeptid, das eine Substrat-Produkt-Umwandlung von 2,3-Dihydroxyisovalerat in α-Ketoisovalerat katalysiert, Hydroxyessigsäuredehydratase ist und die Hydroxyessigsäuredehydratase eine Aminosäuresequenz mit einer Identität von mindestens 95% zu einer Aminosäuresequenz aufweist, die aus der Gruppe ausgewählt ist, die aus SEQ ID Nr.: 6, SEQ ID Nr.: 186, SEQ ID Nr.: 188 und SEQ ID Nr.: 190 besteht, basierend auf der Clustal W-Alignment-Methode unter Verwendung der Standardparameter GAP PENALTY=10 und GAP LENGTH PENALTY=0,1 und der Proteingewichtsmatrix-Serie Gonnet 250; oder
wobei das Polypeptid, das eine Substrat-Produkt-Umwandlung von α-Ketoisovalerat in Isobutyraldehyd katalysiert, verzweigtkettige α-Ketosäuredecarboxylase ist und die verzweigtkettige α -Ketosäuredecarboxylase eine Aminosäuresequenz mit einer Identität von mindestens 95% zu einer Aminosäuresequenz aufweist, die aus der Gruppe ausgewählt ist, die aus SEQ ID Nr.: 8, SEQ ID Nr.: 193, SEQ ID Nr.: 195 und SEQ ID Nr.: 197 besteht, basierend auf der Clustal W-Alignment-Methode unter Verwendung der Standardparameter GAP PENALTY=10 und GAP LENGTH PENALTY=0,1 und der Proteingewichtsmatrix-Serie Gonnet 250; oder
wobei das Polypeptid, das eine Substrat-Produkt-Umwandlung von Isobutyraldehyd in Isobutanol katalysiert, verzweigtkettige Alkoholdehydrogenase ist und die verzweigtkettige Alkoholdehydrogenase eine Aminosäuresequenz mit einer Identität von mindestens 95% zu einer Aminosäuresequenz aufweist, die aus der Gruppe ausgewählt ist, die aus SEQ ID Nr.: 10, SEQ ID Nr.: 199, SEQ ID Nr.: 201, SEQ ID Nr.: 203 und SEQ ID Nr.: 204 besteht, basierend auf der Clustal W-Alignment-Methode unter Verwendung der Standardparameter GAP PENALTY=10 und GAP LENGTH PENALTY=0,1 und der Proteingewichtsmatrix-Serie Gonnet 250.

## Revendications

1. Procédé pour la production d'isobutanol comprenant:
a) la fourniture d'un hôte de production microbien recombinant qui produit de l'isobutanol, dans lequel ledit hôte microbien recombinant comprend des molécules d'ADN codant pour des polypeptides qui catalysent chacune des conversions de substrat en produit suivantes:
i) pyruvate en acétolactate;
ii) acétolactate en 2,3-dihydroxyisovalérate;
iii) 2,3-dihydroxyisovalérate en α-cétoisovalérate;
iv) α-cétoisovalérate en isobutyraldéhyde; et
v) isobutyraldéhyde en isobutanol;
dans lequel au moins une desdites molécules d'ADN est hétérologue pour ledit hôte microbien recombinant;
b) l'ensemencement de l'hôte de production de (a) dans un milieu de fermentation comprenant un substrat de carbone fermentable pour créer une culture de fermentation;
c) la croissance de l'hôte de production dans la culture de fermentation à une première température pendant une première période de temps, dans lequel la première température est d'environ 25°C à environ 40°C;
d) l'abaissement de la température de la culture de fermentation à une deuxième température; et
e) l'incubation de l'hôte de production à la deuxième température de l'étape (d) pendant une deuxième période de temps;
en conséquence de quoi de l'isobutanol est produit.

2. Procédé selon la revendication 1, dans lequel le substrat de carbone fermentable est dérivé d'une graine ou d'une source de sucre choisi dans le groupe constitué de blé, de maïs, d'orge, d'avoines, de seigle, de canne à sucre, de betteraves à sucre, de manioc, de sorgho sucré et de mélanges de ceux-ci.

3. Procédé selon la revendication 1, dans lequel le substrat de carbone fermentable est dérivé d'une biomasse cellulosique ou lignocellulosique choisie dans le groupe constitué d'épis de maïs, de résidus de récolte, d'enveloppes de maïs, de tige de maïs, d'herbes, de paille de blé, de paille d'orge, de foin, de paille de riz, de panic raide, de déchet de papier, de bagasse de canne à sucre, de sorgho, de soja, de composants obtenus à partir de broyage de graines, d'arbres, de branches, de racines, de feuilles, de copeaux de bois, de sciure, d'arbustes et de buissons, de légumes, de fruits, de fleurs, de fumier animal et de mélanges de ceux-ci.

4. Procédé selon la revendication 1, dans lequel le substrat de carbone fermentable est choisi dans le groupe constitué de monosaccharides, d'oligosaccharides et de polysaccharides,

5. Procédé selon l'une quelconque des revendications 1-4, dans lequel la culture de fermentation est maintenue dans des conditions choisies dans le groupe constitué de conditions anaérobiques et de conditions microaérobiques.

6. Procédé selon l'une quelconque des revendications 1-5, dans lequel, pendant la croissance de l'hôte de production dans (c) à une première température sur une première période de temps, un paramètre métabolique de la culture de fermentation est surveillé.

7. Procédé selon la revendication 6, dans lequel le paramètre métabolique qui est surveillé est choisi dans le groupe constitué de la densité optique, du pH, du quotient respiratoire, de l'utilisation du substrat de carbone fermentable, de la production de CO₂ et de la production d'isobutanol.

8. Procédé selon l'une quelconque des revendications 1-7, dans lequel l'abaissement de la température de la culture de fermentation de l'étape (d) se produit à un temps prédéterminé.

9. Procédé selon l'une quelconque des revendications 1-7, dans lequel l'abaissement de la température de la culture de fermentation de l'étape (d) coïncide avec un changement dans un paramètre métabolique.

10. Procédé selon la revendication 9, dans lequel le changement dans un paramètre métabolique est une diminution dans le taux de production d'isobutanol.

11. Procédé selon l'une quelconque des revendications 1-10, dans lequel la deuxième température est d'environ 3°C à environ 25°C inférieure à la première température.

12. Procédé selon l'une quelconque des revendications 1-11, dans lequel les étapes (d) et (e) sont répétées une ou plusieurs fois.

13. Procédé selon l'une quelconque des revendications 1-12, dans lequel l'hôte de production microbien recombinant est choisi dans le groupe constitué de *Clostridium, Zymamonas, Escherichia, Salmonela, Rhodococcus, Pseudomonas, Bacillus, Lactobacillus, Enterococcus, Alcaligenes, Klebsiela, Paenibacillus, Arthrobacter, Corynebacterium, Brevibacterium, Saccharomyces et Pichia.*

14. Procédé selon l'une quelconque des revendications 1-13, dans lequel le polypeptide qui catalyse une conversion de substrat en produit de pyruvate en acétolactate est l'acétolactate-synthase et l'acétolactate-synthase possède une séquence d'acides aminés présentant au moins 95% d'identité avec une séquence d'acides aminés choisie dans le groupe constitué de la SEQ ID NO:2, la SEQ ID NO:178 et la SEQ ID NO:180, sur la base de la méthode d'alignement Clustal W en utilisant les paramètres par défaut de GAP PENALTY = 10, GAP LENGTH PENALTY = 0,1 et série Gonnet 250 de matrice de poids de protéine; ou
dans lequel le polypeptide qui catalyse une conversion de substrat en produit d'acétolactate en 2,3-dihydroxyisovalérate est l'acétohydroxyacide-isoméroréductase et l'acétohydroxyacide-isoméroréductase possède une séquence d'acides aminés présentant au moins 95% d'identité avec une séquence d'acides aminés choisie dans le groupe constitué de la SEQ ID NO:4, la SEQ ID NO:181, la SEQ ID NO:183 et la SEQ ID NO:185, sur la base de la méthode d'alignement Clustal W en utilisant les paramètres par défaut de GAP PENALTY = 10, GAP LENGTH PENALTY = 0,1 et série Gonnet 250 de matrice de poids de protéine; ou
dans lequel le polypeptide qui catalyse une conversion de substrat en produit de 2,3-dihydroxyisovalérate en α-cétoisovalérate est l'acétohydroxyacide-déshydratase et l'acétohydroxyacide-déshydratase possède une séquence d'acides aminés présentant au moins 95% d'identité avec une séquence d'acides aminés choisie dans le groupe constitué de la SEQ ID NO:6, la SEQ ID NO:186, la SEQ ID NO:188 et la SEQ ID NO:190, sur la base de la méthode d'alignement Clustal W en utilisant les paramètres par défaut de GAP PENALTY = 10, GAP LENGTH PENALTY = 0,1 et série Gonnet 250 de matrice de poids de protéine; ou
dans lequel le polypeptide qui catalyse une conversion de substrat en produit d'α-cétoisovalérate en isobutyraldéhyde est l'α-cétoacide à chaîne ramifiée-décarboxylase et l'α-cétoacide à chaîne ramifiée-décarboxylase possède une séquence d'acides aminés présentant au moins 95% d'identité avec une séquence d'acides aminés choisie dans le groupe constitué de la SEQ ID NO:8, la SEQ ID NO:193, la SEQ ID NO:195 et la SEQ ID NO:197, sur la base de la méthode d'alignement Clustal W en utilisant les paramètres par défaut de GAP PENALTY = 10, GAP LENGTH PENALTY = 0,1 et série Gonnet 250 de matrice de poids de protéine; ou
dans lequel le polypeptide qui catalyse une conversion de substrat en produit d'isobutyraldéhyde en isobutanol est l'alcool à chaîne ramifiée-déshydrogénase et l'alcool à chaîne ramifiée-déshydrogénase possède une séquence d'acides aminés présentant au moins 95% d'identité avec une séquence d'acides aminés choisie dans le groupe constitué de la SEQ ID NO:10, la SEQ ID NO:199, la SEQ ID NO:201, la SEQ ID NO:203 et la SEQ ID NO:204, sur la base de la méthode d'alignement Clustal W en utilisant les paramètres par défaut de GAP PENALTY = 10, GAP LENGTH PENALTY = 0,1 et série Gonnet 250 de matrice de poids de protéine.
